# EUROPÄISCHE PATENTSCHRIFT

(11) **EP 0 623 176 B1**
(45) Veröffentlichungstag und Bekanntmachung des Hinweises auf die Patenterteilung: **19.01.2000**
(21) Anmeldenummer: 93911715.6
(22) Anmeldetag: 10.05.1993
(51) Int. Cl.: C12Q 1/48, C12Q 1/68

(54) **VERFAHREN ZUM NACHWEIS REVERSER TRANSCRIPTASE**
PROCESS FOR DETECTING REVERSE TRANSCRIPTASE
PROCEDE DE MISE EN EVIDENCE DE LA TRANSCRIPTASE REVERSE

(30) Priorität: 11.05.1992 CH 150292
(43) Veröffentlichungstag der Anmeldung: 09.11.1994
(73) Patentinhaber: SCHÜPBACH, Jörg, CH-5408 Ennetbaden (CH); BÖNI, Jürg, 5430 Wettingen (CH)
(72) Erfinder: SCHÜPBACH, Jörg, CH-5408 Ennetbaden (CH); BÖNI, Jürg, 5430 Wettingen (CH)
(86) Internationale Anmeldenummer: CH9300116
(87) Internationale Veröffentlichungsnummer: WO9323560

(56) Entgegenhaltungen:
- EP-A- 0 272 098
- WO-A-92/04467
- US-A- 5 021 335
- JOURNAL OF CLINICAL MICROBIOLOGY Bd. 30, Nr. 7, Juli 1992, WASHINGTON, D.C.,USA Seiten 1752 - 1757 S. MENZO ET AL.

## Beschreibung

Die Erfindung befasst sich mit Verfahren zum ultrasensitiven Nachweis von Enzymen des Typs der Reversen Transcriptase (RNA-abhängige DNA-Polymerase oder Deoxynucleosidtriphospat:DNA deoxynucleotidyl transferase) in einem Untersuchungsmaterial. Die Erfindung umfasst auch Screening Kits zum Nachweis von Enzymen des Typs der Reversen Transcriptase mittels dieser Verfahren sowie Typisierungskits, welche die Reverse Transcriptase Aktivität einem bestimmten Agens oder einer Gruppe von Agentien mit bestimmten gemeinsamen Merkmalen zuordnen.

Reverse Transcriptase (im folgenden als RT bezeichnet) ist ein obligatorischer Bestandteil reifer Virionen replikationskompetenter Retroviren (Baltimore D. Nature 1970;226:1209-1211; Temin HM und Mizutani S. Nature 1970;226:1211-3). Enzyme mit zumindest teilweise ähnlichen Aktivitäten können aber auch in verschiedenen anderen Retrolementen, die nicht der Familie der Retroviren angehören, vorkommen. Zu diesen Retroelementen gehören einerseits die retrovirusähnlichen Elemente (retrovirus-like elements) mit den Untereinheiten Retrotransposons, Pararetroviren (Caulimoviren und Hepadnaviren) und LINE-Elemente, andererseits die nicht-retrovirus-ähnlichen Elemente (non-retrovirus-like elements) wie mitochondriale Plasmide oder msDNA (für eine kürzliche Uebersicht über Retroelemente S. Ricchetti M. Bull Inst Pasteur 1991;89:147-58). Die Erfindung beschreibt daher auch Verfahren zum ultrasensitiven Nachweis aller Retroviren sowie aller anderen Retroelemente, die aktive RT enthalten oder exprimieren. RT sind Enzyme, welche aufgrund einer vorgegebenen Template-RNA (Substrat) die dazu komplementäre DNA (cDNA) herstellen (RNA-abhängige DNA-Polymerasen). Als Produkt dieser Reaktion, welche als reverse Transkriptionsreaktion (RT-Reaktion) bezeichnet wird, entsteht eine RNA/cDNA-Heteroduplex. Retrovirale RT besitzen als weitere Aktivität eine RNase H-Funktion. Schliesslich besitzen sie auch DNA-abhängige DNA-Polymerase Aktivität. Eine Uebersicht über die Eigenschaften der retroviralen Reversen Transcriptasen findet sich bei H. Varmus und R. Swanstrom: Replication of Retroviruses. In RNA Tumor Viruses, 2. Auflage. R. Weiss, N. Teich, H. Varmus, J. Coffin, Hrsg. Cold Spring Harbor Laboratory 1984 und 1985, Kapitel 5 und S5.

Die existierenden Tests zum Nachweis von RT beruhen generell auf der RNA-abhängigen DNA-Synthese. Als natürliche Vorlage (Template) für das Enzym fungiert die in den Virionen vorhandene 705 genomische RNA, doch können synthetische homopolymere RNA 10 bis 1000fach effizienter verwendet werden. Voraussetzung für das Zustandekommen der RT-Reaktion, sei die Template-Nukleinsäure homo- oder heteropelymer, ist die Anwesenheit eines geeigneten RT-Primers. Natürlicherweise ist dies eine für das jeweilige Virus charakteristische tRNA, doch werden in diagnostischen RT Assays üblicherweise Oligodeoxyribonucleotide als Primer eingesetzt. Als Template-Primer-Kombination werden heute vorwiegend synthetische Homopolymere des Typs poly(rA)-oligo(dT) verwendet. Da diese auch von verschiedenen eukaryotischen DNA-Polymerasen verwendet werden kann, wird auch häufig das von diesen nicht verwendbare poly(rC)-oligo(dG) eingesetzt. Dieses kann aber immer noch von gewissen prokaryotischen DNA-Polymerasen verwendet werden. Diese Enzyme können sogar hetero-polymere RNA-Templates effizient verarbeiten (Gerard GF and Grandgenett DP. Retrovirus Reverse Transcriptase. In Molecular Biology of RNA Tumor Viruses, JR Stephenson Hrsg, Academic Press, New York, 1980, Kapitel 9).

Ein heute bekanntes Standardverfahren zum Nachweis von RT kann so beschrieben werden: Als Substrat wird ein poly-rA (bzw. poly-rC) Homopolymer verwendet, an welches ein synthetisches Deoxyribonukleotid-Oligomer dT (bzw. dG) von etwa 12-24 Einheiten Länge als Primer hybridisiert wurde. Das die RT enthaltende Untersuchungsmaterial wird nach geeigneter Aufbereitung in ein Reaktionsgemisch gegeben, in welchem die Template-Primer-Kombination, ³H-markiertes Nukleosidtriphosphat dTTP (bzw. dGTP), ein geeignetes divalentes Kation (Mg⁺⁺ oder Mn⁺⁺), sowie möglicherweise weitere die Reaktion unterstützende Substanzen in einem geeigneten Puffersystem vorgegeben sind. Nach Inkubation über eine geeignete Zeit und Temperatur wird die Reaktion gestoppt und die gebildete Heteroduplex durch Zugabe von Trichloressigsäure präzipitiert und auf einen Filter transferiert. Der getrocknete Filter wird in Szintillationsflüssigkeit gegeben und die inkorporierte Radioaktivität mittels Flüssigszintillationstechniken gemessen (s. PS-US 3,755,086; ähnliche Techniken wurden aber auch beschrieben durch Poiesz B et al., Proc. Natl Acad Sci USA 1980;77:1415; Hoffmann AD et al., Virology 1985; 147:326 sowie verschiedenste weitere Autoren). Modifikationen des Assays, welche zu leicht erhöhter Sensitivität und/oder leichterer Durchführbarkeit führen und/oder gewisse andere Vorteile haben mögen, umfassen die Verwendung anderer Isotopen [z.B. ³⁵S, ³²P, oder Gamma-Strahler (s. z.B. PCT Application WO 90/06373)], nichtradioaktiv markierter Nukleosidtriphosphate, die Verwendung eines auf einem Träger immobilisierten Substrats (z.B. EP Application 392459 vom 10.4.90), oder die Verwendung von ELISA-Formaten (z.B. Eberle J et al., Reverse transcriptase activity measured by ELISA, Abstract M.A. 1084, VII International Conference on AIDS, Florence 16-21, June 1991).

Selbst bei Verwendung von synthetischen homopolymeren Templates sind die heutigen RT Assays von geringer analytischer Sensitivität, wenn sie mit anderen Methoden zum Nachweis von Retroviren verglichen werden. Dies wird dadurch illustriert, dass für den Nachweis von RT-Aktivität in mit Human Immundeficiency Virus (HIV) infizierten Zellkulturen eine etwa 100x höhere Konzentration von HIV erforderlich ist, als wenn der Virusnachweis über den virusspezifischen (p24) Antigennachweis erfolgt (Lee YS. J Virol Meth 1988;20:89-94) Gegenüber dem Antigennachweis besitzen Methoden zum Virusnachweis in Zellkulturen (Ho DD et al. N Engl J Med 1989;321:1621-5) oder verschiedene molekulardiagnostische Verfahren zum Nachweis spezifischer Nukleinsäuresequenzen des nachzuweisenden Erregers nochmals eine um etliche Grössenordnungen erhöhte analytische Sensitivität. Diese wird dadurch erreicht, dass auserwählte Nukleinsäuresequenzen mittels verschiedener Verfahren zur Nukleinsäurevermehrung zu hohen Mengen amplifiziert werden. Zu diesen Amplifikationsverfahren gehören die folgenden:
1. Polymerase Chain Reaction (PCR)
   - US 4,683,202: Process for Amplifying Nucleic Acid Sequences
   - US 4,683,195: Process for Amplifying, Detecting and/or Cloning Nucleic Acid Sequences
2. Ligase Detection Reaction (LDR)/Ligase Chain Reaction (LCR)
   - WO 89/12696: Method and Reagents tor Detecting Nucleic Acid Sequences
   - WO 89/09835: Ligase-Based Amplification Method
3. Amplifikation/Detektion basierend auf Transkriptionsverfahren
   - EP 0408295: Nucleic Acid Sequence Amplification Methods
   - WO 89/01050: Selective Amplification of Target Polynucleotide Sequence
   - WO 88/10315: Transcription-Based Nucleic Acid Amplification Detection System
4. Auf replikativer RNA basierende Verfahren
   - WO 90/03445: Target Nucleic Acid Amplification/Detection System
   - WO 90/02820: Replicative RNA-Based Amplification/Detection
   - US 4,957,858: Replicative RNA Reporter Systems
   - US 4,786,600: Autocatalytic Replication of Recombinant RNA
5. Auf replikativer DNA basierende Verfahren
   - DE 3929030: Verfahren zur Vermehrung von Nukleinsäuren
   - WO 90/10064: Improved Methods tor in vitro DNA Amplification and Genomic Cloning and Mapping
6. Varia
   - US 4,994,368: Amplification Method tor Polynucleotide Assays
   - WO 90/01069: Process tor Amplifying and Detecting Nucleic Acid

Gemeinsam ist allen diesen Verfahren das Prinzip, dass Nukleinsäureabschnitte selektiv zu hohen Quantitäten vermehrt werden, bevor sie durch herkömmliche molekulare Identifikationsverfahren analysiert und identifiziert werden. Eine Variante davon besteht darin, dass nicht die nachzuweisende Sequenz selbst vermehrt wird, sondern dass an diese eine andere Nukleinsäure hybridisiert wird, die ausser der komplementären Sequenz noch eine spezifische Indikator- oder Reportersequenz enthält, die dann amplifiziert wird (sog. Probe Amplification).

Ein schwerwiegender Nachteil aller molekulardiagnostischen Verfahren zum Nachweis von Retroviren, besonders im Zusammenhang mit einem Screening für verschiedene Retroviren, ist jedoch, dass sie sequenz- und damit erregerspezifisch sind. Dies bedeutet, dass sie nur Erreger mit zumindest teilweise bekannter Genomsequenz identifizieren können und dass ein Test in der Regel nur einen einzigen Erreger erfasst. Werden Sequenzen gewählt, die mehreren Retroviren gemeinsam sind, dann besteht die Gefahr, dass auch irrelevante Sequenzen, z.B. endogene Retroviren oder andere obligate Bestandteile des zellulären Genoms der untersuchten Spezies, erfasst werden und der Test unspezifisch wird, oder dass die Sensitivität des Nachweises darunter leidet. Auch die aufwendigen kulturellen Nachweismethoden sind prinzipiell erregerspezifisch und nur dann anwendbar, wenn die Wirtszelle bekannt ist und sich in vitro propagieren lässt.

Da alle replikationskompetenten Retroviren eine aktive RT besitzen, können mit einer Nachweismethode für dieses Enzym sämtliche existierenden Retroviren nachgewiesen werden. Darüber hinaus können auch andere mit aktiver RT assoziierte Retroelemente nachgewiesen werden. Für einen effizienten diagnostischen Einsatz des Reverse Transcriptase Assays in diesem Sinne ist freilich eine beträchtliche Erhöhung der analytischen Sensitivität Voraussetzung.

Es ist Aufgabe der Erfindung ein Verfahren vorzuschlagen, einen sog. PERT (Product Enhanced Reverse Transcriptase) Assay, mit welchem eine Sensitivitätssteigerung in der Grössenordnung von mehreren Millionen erzielt werden kann, und die entsprechenden Kits zur Durchführung dieses Verfahrens anzugeben.

Hierzu wird erfindungsgemäss die im Untersuchungsmaterial vorhandene RT-Aktivität so zur Bereitstellung einer zu vermehrenden Nukleinsäure verwendet, dass eine zu vermehrende Nukleinsäure ausschliesslich dann bereitgestellt wird, wenn im Untersuchungsmaterial RT-Aktivität vorhanden ist. Die zu vermehrende Nukleinsäure wird anschliessend durch ein geeignetes Amplifikationsverfahren vermehrt und mit einem geeigneten Analyseverfahren nachgewiesen.

Damit können auch geringste Mengen von RT-Aktivität nachgewiesen werden.

Verfahren und Kits, welche eine Sensitivitätssteigerung des RT Assay in der Grössenordnung von mehreren Millionen bewirken, sind in den Patentansprüchen 1 - 34 beschrieben.

Die Erfindung wird im Folgenden anhand von Zeichnungen und Beispielen erläutert. Es zeigen:
Fig. 1 den schematischen Aufbau des PERT Assay
Fig. 2 den Aufbau des PERT Assay in einer illustrativen Variante, in welcher die zu vermehrende Nukleinsäure eine Sequenz der cDNA ist, die mittels der Polymerase Chain Reaction amplifiziert wird.
Fig. 3 den Aufbau des PERT Assay in einer illustrativen Variante, in welcher die zu vermehrende Nukleinsäure eine Sequenz einer Reportersonde ist, die mittels der Polymerase Chain Reaction amplifiziert wird.
Fig. 4 den Aufbau des PERT Assay in einer illustrativen Variante, in welcher die zu vermehrende Nukleinsäure eine Sequenz der cDNA ist, die mittels der Ligase Chain Reaction amplifiziert wird.
Fig. 5 den Aufbau des PERT Assay in einer illustrativen Variante, in welcher die zu vermehrende Nukleinsäure eine Sequenz einer Reportersonde ist, die mittels der Ligase Chain Reaction amplifiziert wird.
Fig. 6 den Aufbau des PERT Assay in einer illustrativen Variante, in welcher die zu vermehrende Nukleinsäure eine Sequenz der cDNA ist, die nach einem Verfahren amplifiziert wird, das sowohl Elemente der PCR als auch der LCR vereint.
Fig. 7 den Aufbau des PERT Assay in einer illustrativen Variante, in welcher die zu vermehrende Nukleinsäure eine Sequenz einer Reportersonde ist, die nach einem Verfahren amplifiziert wird, das sowohl Elemente der PCR als auch der LCR vereint.
Fig. 8 den Aufbau des PERT Assay in 3 illustrativen Versionen, in welcher die cDNA die zu vermehrende Nukleinsäure enthält, die mittels eines isothermen multienzymatischen Amplifikationsverfahrens amplifiziert wird.
Fig. 9 den Aufbau des PERT Assay in 3 illustrativen Versionen, in welcher die zu vermehrende Nukleinsäure eine von der cDNA abgeleitete RNA ist, die mittels eines isothermen multienzymatischen Amplifikationsverfahrens amplifiziert wird.
Fig. 10 den Aufbau des PERT Assay in einer illustrativen Variante, in welcher die zu vermehrende Nukleinsäure eine Sequenz einer Reportersonde ist, die mittels eines isothermen multienzymatischen Amplifikationsverfahrens amplifiziert wird.
Fig. 11 - 14 den Aufbau des PERT Assay in 4 illustrativen Varianten, in welchen die zu vermehrende Nukleinsäure eine von der Sequenz der cDNA mindestens teilweise abgeleitete primäre replikative RNA ist, die mittels eines geeigneten RNA-Replikationssystems amplifiziert wird.
Fig. 15 den Aufbau des PERT Assay in einer illustrativen Variante, in welcher die zu vermehrende Nukleinsäure eine Reportersonde ist, die aus einer replikativen RNA besteht.
Fig. 16 den Aufbau des PERT Assay in einer illustrativen Variante, in welcher die zu vermehrende Nukleinsäure eine von einer DNA-Reportersonde abgeleitete primäre replikative RNA ist, die mittels eines geeigneten RNA-Replikationssystems amplifiziert wird.
Fig. 17 den Aufbau des PERT Assay in einer illustrativen Variante, bei welcher die zu vermehrende Nukleinsäure eine von der Sequenz einer Reportersonde abgeleitete primäre replikative RNA ist, wobei die Reportersonde mittels einer "smart probe" mit der cDNA verbunden wird.
Fig. 18 - 19 den Aufbau des PERT Assay in 2 illustrativen Varianten, in welchen die zu vermehrende Nukleinsäure eine von der Sequenz der cDNA mindestens teilweise abgeleitete primäre replikative DNA ist, die mittels eines geeigneten DNA-Replikationssystems amplifiziert wird.
Fig. 20 den Aufbau des PERT Assay in einer illustrativen Variante, in welcher die zu vermehrende Nukleinsäure eine von der Sequenz der cDNA abgeleitete primäre replikative DNA ist und worin als RT-Primer bzw. zum Einführen von ori-Sequenzen anstelle von einzelsträngigen Oligonukleotiden Adaptoren verwendet werden.
Fig. 21 den Aufbau des PERT Assay in einer illustrativen Variante, in welcher die zu vermehrende Nukleinsäure eine primäre replikative DNA ist, welche gleichzeitig eine Reportersonde ist, die mittels eines geeigneten DNA-Replikationssystems amplifiziert wird.
Fig. 22 den Aufbau des PERT Assay in einer illustrativen Variante, in welcher die zu vermehrende Nukleinsäure eine von einer Reportersonde abgeleitete primäre replikative DNA ist, die mittels eines geeigneten DNA-Replikationssystems amplifiziert wird.
Fig. 23 den Aufbau des PERT Assay, in welchem die zu vermehrende Nukleinsäure eine Sequenz einer Reportersonde ist, die mittels eines zyklischen Verfahrens, welches die Synthese einer dsDNA und deren Verdauung mittels eines Restriktionsenzyms beinhaltet, amplifiziert wird.
Fig. 24 ein erstes Ausführungsbeispiel: Vergleich der analytischen Sensitivität eines Reverse Transcriptase Assay (RT) und eines nach Fig. 2 durchgeführten PERT Assay unter Verwendung von MuLV Reverser Transcriptase.
Fig. 25 ein weiteres Anwendungsbeispiel zu dem in Fig. 2 geschilderten Verfahren, nämlich den direkten Nachweis von RT-Aktivität im Blutplasma von 5 HIV-infizierten Patienten bzw. deren Abwesenheit im Blutplasma von 5 gesunden Blutspendern.

### Definitionen der verwendeten Begriffe

### Nukleinsäuren, Nukleinsäurestrukturen und Bestandteile

Der Begriff "Nukleotid" bezeichnet jedes Ribonukleotidmonophosphat und Deoxyribonukleotidmonophosphat mit einer beliebigen natürlich vorkommenden oder modifizierten Base in derjenigen Struktur, wie es [in polymerisierter Form] als Baustein in einer Nukleinsäure vorkommt.

Die Begriffe "rNTP" und "dNTP" bezeichnen ein Ribonukleotid-Triphosphat, respektive ein Deoxyribonukleotid-Triphosphat mit einer beliebigen natürlich vorkommenden oder modifizierten Base. Die Begriffe "rNTPs" und "dNTPs" bezeichnen eine Mischung von Ribonukleotid-Triphosphaten, respektive Deoxyribonukleotid-Triphosphaten, welche aus mindestens zwei verschiedenen Ribonukleotid-Triphosphaten oder Deoxyribonukleotid-Triphosphaten bestehen.

Der Begriff "Nukleinsäure" bezeichnet ein aus Nukleotiden bestehendes, einzelsträngiges, teilweise oder vollständig doppelsträngiges Oligomer oder Polymer. Falls die Nukleinsäure ausschliesslich aus Ribonukleotidmonophosphaten besteht ist sie eine "RNA", falls sie ausschliesslich aus Deoxyribonukleotidmonophosphaten besteht, eine "DNA". Eine Nukleinsäure kann aber auch Ribonukleotidmonophosphate und Deoxyribonukleotidmonophosphate enthalten. Die Zusatzbezeichnungen "ss", respektive "ds", kennzeichnen eine Nukleinsäure als einzelsträngig, respektive doppelsträngig.

Der Begriff "Oligonukleotid" bezeichnet eine einzelsträngige Nukleinsäure, die aus mindestens zwei, aber nicht mehr als hundert, natürlichen oder modifizierten Nukleotiden oder einer Mischung davon besteht. Das Oligonukleotid kann eine natürliche Nukleinsäure darstellen oder daraus hergestellt werden, oder durch chemische oder enzymatische Synthese hergestellt werden.

Der Begriff "Polynukleotid" bezeichnet eine einzelsträngige oder doppelsträngig Nukleinsäure, die aus mehr als hundert natürlichen oder modifizierten Nukleotiden oder einer Mischung davon besteht.

Der Begriff "homopolymer" charakterisiert Nukleinsäuren oder Nukleinsäureabschnitte, deren Nukleotide alle eine identische Base aufweisen. Als identisch gelten im Zusammenhang mit dem Begriff "homopolymer" auch solche Basen, die nur in ihrer Bindungsfunktion zu einer komplementären Base identisch, bezüglich ihrer chemischen Struktur aber abweichend sind.

Der Begriff "heteropolymer" charakterisiert Nukleinsäuren oder Nukleinsäureabschnitte, deren Nukleotide mindestens zwei in ihrer Bindungsfunktion zu einer komplementären Base verschiedene Basen aufweisen.

Die Begriffe "upstream" und "downstream" stellen relative Lage- resp. Richtungsbezeichnungen innerhalb eines Nukleinsäurestranges dar. "upstream" bezeichnet, von einem Bezugselement aus gesehen, eine Lage oder Richtung gegen das 5'-Ende, "downstream" eine solche gegen das 3'-Ende eines Nukleinsäurestranges zu.

Der Begriff "Adaptor" bezeichnet eine teilweise doppelsträngige Nukleinsäure, welche vorzugsweise aus einem Oligonukleotid und einem zweiten, kürzeren Oligonukleotid besteht. Das kürzere der beiden Oligonukleotide ist zum längeren komplementär und an dessen 5'- oder 3'-Ende hybridisiert, wodurch das doppelsträngige Stück Nukleinsäure entsteht. Der Rest des längeren Oligonukleotids ist einzelsträngig und weist an seinem anderen Ende eine Hybridisierungssequenz auf [eine zu einer bestimmten Nukleinsäure komplementäre Sequenz auf], durch welche es an eine definierte Nukleinsäure hybridisiert werden kann (siehe unter "Hybridisierungssequenz").

### Enzyme und enzymatische Aktivitäten

Der Begriff "Reverse Transcriptase" (Abkürzung: "RT") bezeichnet ein Enzym, welches natürlicherweise in seiner Funktion in einer primer-abhängigen Reaktion unter Verwendung von dNTPs eine zu einer Template-RNA komplementäre DNA synthetisiert.

Der Begriff "Reverse Transcriptase-Aktivität" (Abkürzung: "RT-Aktivität") bezeichnet eine Aktivität eines beliebigen Enzyms, durch welche in einer primer-abhängigen Reaktion unter Verwendung von dNTPs eine zu einer Template-RNA komplementäre DNA synthetisiert wird.

Der Begriff "RNA-Polymerase", auch "DNA-abhängige-RNA-Polymerase", bezeichnet ein Enzym, das in einer sequenzspezifischen Art und Weise an einen Transcriptionspromotor bindet, die Transcription einer RNA ausgehend von einer definierten Position downstream dieses Transcriptionspromotors initiiert und von einer einzelsträngigen oder doppelsträngigen DNA unter Verwendung von rNTPs eine zum transcribierten DNA-Strang komplementäre RNA synthetisiert.

Der Begriff "DNA-Polymerase", auch "DNA-abhängige-DNA-Polymerase", bezeichnet ein Enzym, welches in einer primer-abhängigen Reaktion und unter Verwendung von dNTPs einen zu einem DNA-Strang komplementären DNA-Strang synthetisiert.

Der Begriff "RNA-Replicase" bezeichnet ein Enzym oder Enzymkomplex mit RNA-abhängiger-RNA-Polymerase-Aktivität, welches oder welcher unter Verwendung von rNTPs eine auf die RNA-Replicase abgestimmte replikative RNA replizieren kann, wobei als Produkt des Replikationsvorgangs ein neues Molekül replikativer RNA entsteht, welches zur replizierten replikativen RNA komplementär ist.

Der Begriff "DNA-Replicase" bezeichnet ein Enzym oder einen Enzymkomplex, welches oder welcher in einem Protein-geprimten Prozess die Replikation einer auf die DNA-Replicase abgestimmten replikativen DNA unter Verwendung von dNTPs an einem DNA-ori initiiert und infolge seiner DNA-abhängigen-DNA-Polymerase-Aktivität die Replikation der replikativen DNA bewirkt, wobei die DNA-Replicase während des Replikationsvorgangs den zum replizierten DNA-Strang schon vorhandenen komplementären DNA-Strang von diesem verdrängt.

### Funktionelle Sequenzen und Funktionen

Der Begriff "funktionelle Sequenz" bezeichnet einen Abschnitt auf einem Strang einer Nukleinsäure, dem innerhalb des Verfahrens eine bestimmte Funktion zugeordnet ist, wobei diese Funktion durch jene Nukleinsäure, durch welche die funktionelle Sequenz eingeführt wird, und/oder durch eine davon abgeleitete nachfolgende einzelsträngige Nukleinsäure mit sinngemäss gleicher oder komplementärer Basensequenz, oder durch eine doppelsträngige Nukleinsäure wahrgenommen wird. Die in einer funktionellen Sequenz enthaltene Basensequenz ist durch ihre Funktion entweder genau definiert oder aber frei wählbar.

Der Begriff "Hybridisierungssequenz" bezeichnet diejenige funktionelle Sequenz, welche zum Zweck der Hybridisierung mit einer anderen Nukleinsäure eine zu der Hybridisierungssequenz der anderen Nukleinsäure komplementäre Basensequenz aufweist.

Der Begriff "flankierende Sequenz" bezeichnet diejenige funktionelle Sequenz, welche upstream oder downstream einer beliebigen anderen funktionellen Sequenz das 5'- oder 3'-Ende einer Nukleinsäure bildet.

Der Begriff "Spacer-Sequenz" bezeichnet diejenige funktionelle Sequenz, welche die Funktion ausübt, zwei andere funktionelle Sequenzen auf einer Nukleinsäure voneinander zu trennen.

Der Begriff "Transcriptionspromotor-Sequenz" bezeichnet diejenige funktionelle Sequenz mit einer definierten Basensequenz, welche mit der Funktion eines Transcriptionspromotors assoziiert ist. Die "P(+)-Sequenz" eines Transcriptionspromotors bezeichnet diejenige Basensequenz, wie sie auf dem zur synthetisierten RNA komplementären DNA-Strang vorliegt. Als "P(-)-Sequenz" eines Transcriptionspromotors wird diejenige Basensequenz bezeichnet, wie sie auf dem DNA-Strang mit der gleichen Polarïtät wie die synthetisierte RNA vorliegt.

Der Begriff "Transcriptionspromotor" bezeichnet den aus den komplementären P(-)- und P(+)-Transcriptionspromotor-Sequenzen bestehenden doppelsträngigen DNA-Abschnitt, welcher durch Bindung einer sequenzspezifischen RNA-Polymerase [an diese] in einer durch die Orientierung der definierten Basensequenz bestimmten Richtung die Synthese einer RNA initiiert. In denjenigen Fällen, wo die zu transcribierende DNA einzelsträngig ist, umfasst der Transcriptionspromotor zusätzlich mindestens die ersten drei Nukleotide der zu transcribierenden DNA in Form einer doppelsträngigen DNA.

Der Begriff "Replicasen-Bindungsdomäne" bezeichnet diejenige funktionelle Sequenz, welche innerhalb einer einzelsträngigen RNA durch Ausbildung von Sekundärstrukturen die Bindung einer auf die Replicasen-Bindungsdomäne abgestimmten RNA-Replicase vermittelt.

Der Begriff "RNA-ori-Sequenz" bezeichnet eine funktionelle Sequenz mit einer definierten Basensequenz auf einem Nukleinsäurestrang, welche, wenn sie am 3'-Ende einer einzelsträngigen RNA vorliegt, die Inititiation der RNA-Replikation durch eine der RNA-ori-Sequenz entsprechende, an die RNA gebundene RNA-Replicase ermöglicht.

Der Begriff "5'-RNA-ori-Sequenz" bezeichnet diejenige RNA-ori-Sequenz, welche am 5'-Ende der replikativen RNA liegt oder zu liegen kommt.

Der Begriff "3'-RNA-ori-Sequenz" bezeichnet diejenige RNA-ori-Sequenz , welche am 3'-Ende der replikativen RNA liegt oder zu liegen kommt.

Der Begriff "RNA-ori(+)-Sequenz" bezeichnet die Basensequenz in derjenigen Polarität, in der sie, am 3'-Ende einer RNA gelegen, die RNA-Replikation ermöglicht.

Der Begriff "RNA-ori(-)-Sequenz" bezeichnet die zur RNA-ori (+)-Sequenz komplementäre Basensequenz.

Der Begriff "DNA-ori-Sequenz" bezeichnet diejenige funktionelle Sequenz mit einer definierten Basensequenz auf einem Nukleinsäurestrang, welche mit der Initiation der DNA-Replikation in einem DNA-Replikationssystem assoziiert ist. Als "DNA-ori(+)-Sequenz" wird diejenige Basensequenz bezeichnet, welche bei der Replikation der DNA zuerst komplementiert wird. Die "DNA-ori(-)-Sequenz" bezeichnet die zur "DNA-ori (+)-Sequenz" komplementäre Basensequenz.

Der Begriff "DNA-ori" bezeichnet den aus den komplementären DNA-ori(+)- und DNA-ori (-)-Sequenzen bestehenden doppelsträngigen DNA-Abschnitt, der, am Ende einer doppelsträngigen DNA gelegen, in einem dem "DNA-ori" entsprechenden in vitro DNA-Replikationssystem durch Bindung von bestimmten Kofaktoren [Proteinen] die Replikation durch eine DNA-Replicase in einem in vitro-Replikationssystem initiiert. Ein "DNA-ori" kann auch durch die miteinander hybridisierten DNA-ori(+)- und DNA-ori(-)-Sequenzen eines einzigen Strangs einer replikativen DNA gebildet werden.

### Nukleinsäuren mit definierter Funktion

Der Begriff "Primer" bezeichnet eine Nukleinsäure, welche infolge ihrer zu einer andern Nukleinsäure komplementären Sequenz, in hybridisiertem Zustand über ihre freie OH-Gruppe am 3'-Ende als Startpunkt der Synthese eines Nukleinsäurestranges durch eine RT-Aktivität oder eine DNA-abhängige-DNA-Polymerase dient.

Der Begriff "RT-Primer" bezeichnet denjenigen Primer, welcher die Funktion des Primers für die RT-Aktivität ausübt.

Der Begriff "Template-Nukleinsäure" bezeichnet diejenige gesamte einzelsträngige Nukleinsäure, zu welcher mindestens in Teilen durch reverse Transcriptase-Aktivität ein komplementärer DNA-Strang synthetisiert wird.

Der Begriff "Template-RNA" bezeichnet diejenigen Anteile der Template-Nukleinsäure, welche aus RNA bestehen und zu welchen mindestens in Teilen eine cDNA synthetisiert wird.

Der Begriff "Template-DNA" bezeichnet diejenigen Anteile der Template-Nukleinsäure, welche aus DNA bestehen.

Der Begriff "Template-Primer-Kombination" bezeichnet die als Substrat für die [RT oder] RT-Aktivität vorgelegte Kombination einer Template-Nukleinsäure mit allen an diese hybridisierten Nukleinsäuren inklusive RT-Primern.

Der Begriff "cDNA" bezeichnet diejenige Nukleinsäure, welche die durch RT-Aktivität synthetisierte, zur Template-Nukleinsäure komplementäre DNA sowie alle Bestandteile des RT-Primers umfasst.

Der Begriff "cDNA/Template-Duplex" bezeichnet diejenige zumindest teilweise doppelsträngige Nukleinsäure, welche aus der Template-Nukleinsäure und der mit ihr hybridisierten cDNA besteht.

Der Begriff "cDNA/RNA-Heteroduplex" bezeichnet diejenigen Abschnitte der cDNA-Template-Duplex, welche aus Template-RNA und der mit ihr hybridisierten cDNA besteht.

Der Begriff "replikative RNA" bezeichnet eine einzelsträngige RNA, welche alle zur Replikation mit Hilfe eines auf diese RNA abgestimmten in vitro RNA-Replikationssystems notwendigen funktionellen Sequenzen enthält und welche in diesem System repliziert wird.

Der Begriff "primäre replikative RNA" bezeichnet diejenige replikative RNA, die als zu vermehrende Nukleinsäure in Schritt 1 entsteht.

Der Begriff "replikative DNA" bezeichnet eine einzelsträngige oder zumindest partiell doppelsträngige DNA, welche in einem der replikativen DNA entsprechenden in vitro DNA-Replikationssystem repliziert wird. Sie ist dadurch gekennzeichnet, dass an beiden Enden des replizierten DNA-Strangs eine DNA-ori-Sequenz vorliegt und dass entweder diese in einer einzelsträngigen DNA einen DNA-ori bilden, oder dass zumindest an einem Ende der partiell doppelsträngigen DNA ein aktiver DNA-ori vorliegt.

Der Begriff "primäre replikative DNA" bezeichnet diejenige replikative DNA, die als zu vermehrende Nukleinsäure in Schritt 1 entsteht.

Der Begriff "Reportersonde" bezeichnet eine von der cDNA verschiedene, aber mit dieser direkt oder indirekt verbundene Nukleinsäure, welche zum Zweck des Nachweises der erfolgten Synthese einer cDNA eingesetzt wird. Die Reportersonde enthält entweder die zu vermehrende Nukleinsäure oder ist das Edukt einer Reaktion oder Reaktionsfolge, welche zur zu vermehrenden Nukleinsäure führt.

### Varia

Der Begriff "Retroelement" bezeichnet diejenigen genetischen Elemente und deren Expressionsprodukte, welche durch Reverse Transcriptase-Aktivität entstanden sind und/oder sich mit Hilfe von reverser Transcriptase replizieren.

Der Begriff "Carrier" bezeichnet eine feste Trägerphase, welche z.B. Beads, die Oberfläche einer Vertiefung einer Microtiterplatte, eine Filter-Membran etc. sein kann.

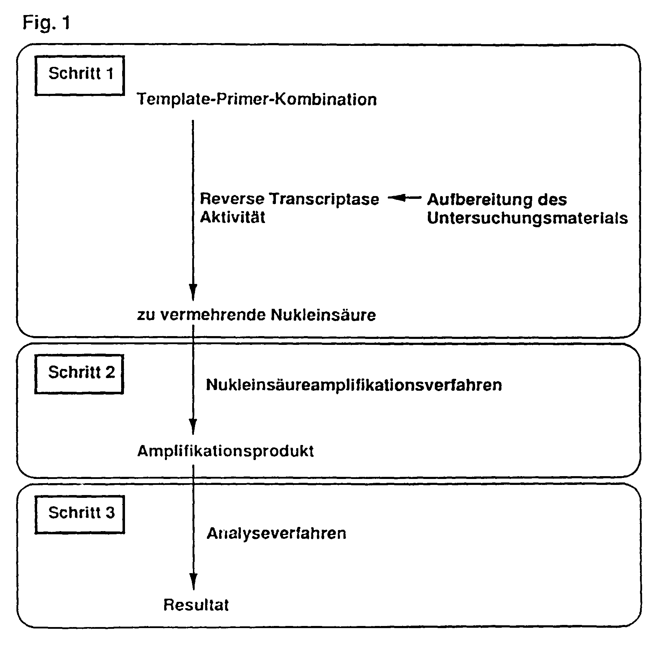

**Fig. 1** zeigt den PERT Assay, welcher aus drei miteinander verknüpften Schritten besteht: Im ersten Schritt wird unter Einbezug der im Untersuchungsmaterial vorhandenen RT-Aktivität eine zu vermehrende Nukleinsäure bereitgestellt. Im zweiten Schritt wird diese in einem geeigneten Nukleinsäureamplifikationsverfahren vermehrt, und im dritten Schritt wird die vermehrte Nukleinsäure mit einem geeigneten Analyseverfahren identifiziert.

Im **ersten Schritt** wird das Untersuchungsmaterial aufgearbeitet und in eine Form gebracht, die für den Nachweis der RT-Aktivität geeignet ist. Eine Template-Nukleinsäure wird zusammen mit mindestens einem geeigneten RT-Primer als Substrat für die im aufgearbeiteten Untersuchungsmaterial möglicherweise enthaltene RT-Aktivität angeboten, zusammen mit den übrigen für die cDNA-Synthese erforderlichen Reagentien, wie den verschiedenen Deoxyribonukleosidtriphosphaten (dNTPs), divalenten Kationen (Mg⁺⁺ oder Mn⁺⁺), einem geeigneten Puffersystem und anderen die spezifische Synthese der cDNA optimal fördernden Substanzen. Infolge der Wirkung der RT-Aktivität wird als erstes Reaktionsprodukt eine zur gewählten Template-Nukleinsäure komplementäre cDNA synthetisiert. Diese cDNA ist in Form einer cDNA-Template-Duplex, die mindestens teilweise eine cDNA-RNA-Heteroduplex ist, mit der Template-Nukleinsäure verbunden und liegt in einem Nukleinsäuregemisch, bestehend aus unreagierten Template-Primer-Kombinationen, ungebundenen Primermolekülen und nicht geprimten Template-Molekülen, vor.

Für die Bereitstellung der zu vermehrenden Nukleinsäure existiert eine Vielfalt verschiedener Möglichkeiten. In gewissen Varianten von PERT Assays ist die zu vermehrende Nukleinsäure identisch mit der cDNA oder stellt einen Teil derselben dar. In anderen Fällen stellt die im Nukleinsäuregemisch vorliegende cDNA, bzw. Teile derselben, das Edukt einer Reaktion oder einer Reaktionsfolge dar, welche, unter Umständen unter Einbezug weiterer Nukleinsäuren, zur zu vermehrenden Nukleinsäure führen. Ferner existiert auch die Möglichkeit, dass mittels Anteilen der cDNA die Selektion einer Nukleinsäure bewirkt wird, die entweder eine Reportersonde ist oder die Verbindung zu einer Reportersonde herstellt, wobei die Reportersonde die zu vermehrende Nukleinsäure darstellt oder enthält, bzw. das Edukt einer Reaktion oder Reaktionsfolge bildet, welche, unter Umständen unter Einbezug weiterer Nukleinsäuren, zur zu vermehrenden Nukleinsäure führt. Schliesslich gibt es noch die Möglichkeit, dass für die Bereitstellung der zu vermehrenden Nukleinsäure nicht allein die cDNA, sondern die cDNA-Template-Duplex verwendet wird. In diesem Fall ist bzw. enthält die cDNA-Template-Duplex entweder die zu vermehrende Nukleinsäure, oder sie bzw. ein Teil von ihr bildet das Edukt einer Reaktion oder Reaktionsfolge, welche zur zu vermehrenden Nukleinsäure führt. Zum ersten Schritt gehören ferner auch Massnahmen, welche dazu dienen, vorgelegte vermehrbare, aber nicht zu vermehrende Nukleinsäuren zu eliminieren.

Im **zweiten Schritt** wird das Produkt des ersten Schritts, die zu vermehrende Nukleinsäure, in einem auf diese abgestimmten Nukleinsäure-Amplifikationsverfahren zu hohen Mengen vermehrt. Das Produkt besteht, abhängig vom eingesetzten Amplifikationsverfahren, entweder aus einer definierten DNA und/oder definierten RNA.

Im **dritten Schritt** des PERT Assay wird die amplifizierte Nukleinsäure identifiziert und gegebenenfalls quantifiziert. Die Identifikation kann durch eine beliebige Technik erfolgen, die geeignet ist, eine bestimmte Nukleinsäuresequenz zu identifizieren und allenfalls zu quantifizieren oder deren Synthese nachzuweisen. Eine Anzahl solcher Analyseverfahren ist jedem ausgebildeten und versierten Molekularbiologen geläufig. Durch die Kombination der ersten beiden Schritte wird in Schritt 3 die Identifikation auch geringster Mengen der RT-Aktivität, im Grenzfall der Schwellendosis, die gerade hinreichend ist, um ein einziges Molekül cDNA zu synthetisieren, ermöglicht.

Im Folgenden sollen allgemeine Aspekte der verschiedenen Schritte des PERT Assay mehr im Detail beschrieben werden:

### Untersuchungsmaterialien und Probenaufbereitung

Die Art der Aufarbeitung des Untersuchungsmaterials ist davon abhängig, woraus es besteht und welche Arten von Retroelement-assoziierter RT-Aktivität im PERT Assay erfasst, bzw. nicht erfasst werden sollen. Sie entscheidet damit bis zu einem gewissen Grad über die Spezifität und die Sensitivität des PERT Assay. Sie entscheidet aber in der Regel nicht darüber, welche Variante des PERT Assay zu wählen ist.

Im weitesten Sinn besteht das Untersuchungsmaterial zumindest in Teilen aus biologischem Material, wobei dieses menschlicher, tierischer oder pflanzlicher Herkunft sein kann und auch pro- sowie eukaryotische Einzeller mit einschliesst. Als illustrative Beispiele für Untersuchungsmaterialien seien genannt:

Körpergewebe- oder Organe menschlichen oder tierischen Ursprungs, einschliesslich
(a) flüssige Gewebe wie Blut, oder Fraktionen davon, z.B. Serum, Plasma, bestimmte Blutzellpopulationen oder -fraktionen, Plasma- oder Serumfraktionen, Blutprodukte wie z.B. Bluteiweisse, Blutgerinnungsfaktoren, Hyperimmunseren, Antikörperkonzentrate, Hormone u.a.,
(b) feste Gewebe und Organe, z.B. Transplantate, Biopsie- oder Autopsiematerial, Abstriche von Schleimhäuten und anderen Oberflächen, einzelne Zellen oder Präparate oder Extrakte, wie Hormonpräparate aus Drüsengeweben, Plazenta, etc., Fleisch und Fleischprodukte von Nutztieren,
(c) Körperflüssigkeiten oder -ausscheidungen physiologischer oder pathologischer Art, z.B. Liquor cerebrospinalis, Urin, Speichel, Galle, Schweiss, Milch, andere Drüsensekrete, Bläscheninhalte, Fruchtwasser, Synovialflüssigkeit, Kammuerwasser, Ascites oder andere Körperhöhlenergüsse, Lymphe, Stuhl, etc., oder Produkte, die solche Materialien als Bestandteile enthalten,
(d) Proben aus in vitro propagierten pro- und eukaryotischen Zellkulturen jeglicher Art, sowie daraus gewonnene biologische Erzeugnisse wie Impfstoffe, Antikörper, Wachstums- und Differenzierungsfaktoren, Hormone, rekombinante Proteine etc.,
(e) Verschiedenes, z.B. Proben von Nahrungsmitteln, Wasser und anderen Getränken, Umweltproben, Hygieneproben, Proben von Nutzpflanzen (Gemüse, Frucht-, Holz-, Faserpflanzen).

Generell gilt für die Probenaufbereitung, dass die im Untersuchungsmaterial enthaltene RT-Aktivität in eine Form gebracht werden muss, die es ihr ermöglicht, die RT-Reaktion zu katalysieren. Falls die RT-Aktivität im Untersuchungsmaterial in Zellen, Zellorganellen oder Viruspartikeln eingeschlossen ist, müssen diese erst durch geeignete Verfahren aufgeschlossen und das Enzym extrahiert werden.

Für grösstmögliche Spezifität. beim Nachweis von z.B. retroviraler RT ist Kontamination durch zelluläre DNA-Polymerasen zu vermeiden. Zu diesem Zweck kann Untersuchungsmaterial durch verschiedene Massnahmen vorbehandelt werden. Diese umfassen einerseits physikalische Massnahmen, z.B. Zentrifugation unter Bedingungen, welche pro- und eukaryotische Zellen, nicht aber Viren oder gelöstes Enzym, pelletieren, oder Ultrafiltration, welche Zellen zurückhält, nicht aber Viruspartikel oder gelöstes Enzym. Ein anderes Beispiel ist die Anreicherung von Viruspartikeln in der einen Phase eines geeigneten Zweiphasensystems. Andererseits können auch immunologische Methoden eingesetzt werden, um die Spezifität oder Sensitivität des PERT in eine gewünschte Richtung zu lenken. Beispielsweise können durch Immunadsorption oder -präzipitation mittels geeigneter Antikörper intakte Viruspartikel einer bestimmten Art oder Teile davon oder freies Enzym im Probenmaterial angereichert werden. Neben Immunreagentien können für solche Zwecke z.B. auch Lectine, die Affinität für bestimmte Glykoproteine haben, sowie andere Agentien mit Affinität zu Retroelementen oder deren Bestandteilen oder anderen Agentien eingesetzt werden. Ein weiteres Beispiel ist PolyU, welches Affinität für RT besitzt. Andererseits ist es auch möglich, mittels solcher Verfahren unerwünschte Aktivitäten aus dem Probenmaterial zu entfernen.

Ein häufiges Untersuchungsmaterial werden menschliche oder tierische Blutproben sein, und aus diesen besonders die flüssigen Bestandteile, Plasma oder Serum. In diesem Fall kann eine Probenaufbereitung einen oder mehrere der folgenden Schritte enthalten:
(a) Hochtourige Zentrifugation, z.B. 15'000x g, zur Entfernung von Zellen, Zelltrümmern und anderem unerwünschtem partikulärem Material,
(b) Ultrafiltration mit Filtern geeigneter Porengrösse zur weiteren Entfernung unerwünschten partikulären Materials,
(c) Ultrazentrifugation zur Pelletierung von Viruspartikeln, oder Adsorption von Viruspartikeln an eine mit Antikörpern oder anderen geeigneten Molekülen beschichtete feste Phase oder einen anderen geeigneten Träger, oder Präzipitation mittels Immunreagentien oder chemischen Substanzen (z.B. Polyaethylenglykol),
(d) Extraktion der RT-Aktivität mit einem ein geignetes Detergens enthaltenden Puffer oder andere das Enzym schonende chemische oder physikalische Verfahren.

Weitere Untersuchungsmaterialien, die nach diesem Prozedere aufgearbeitet werden können, umfassen alle flüssigen Materialien oder solche, die in einer Flüssigkeit gelöst oder suspendiert werden können, z.B. Pulver jeglicher Art (z.B. lyophilisierte Produkte).

### Eingesetzte Nukleinsäuren

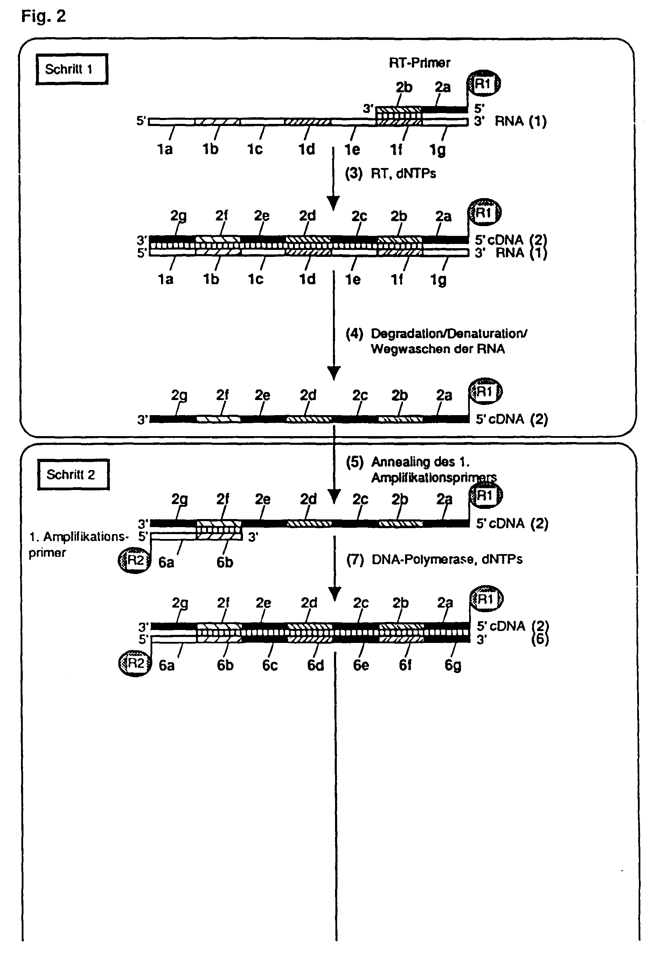

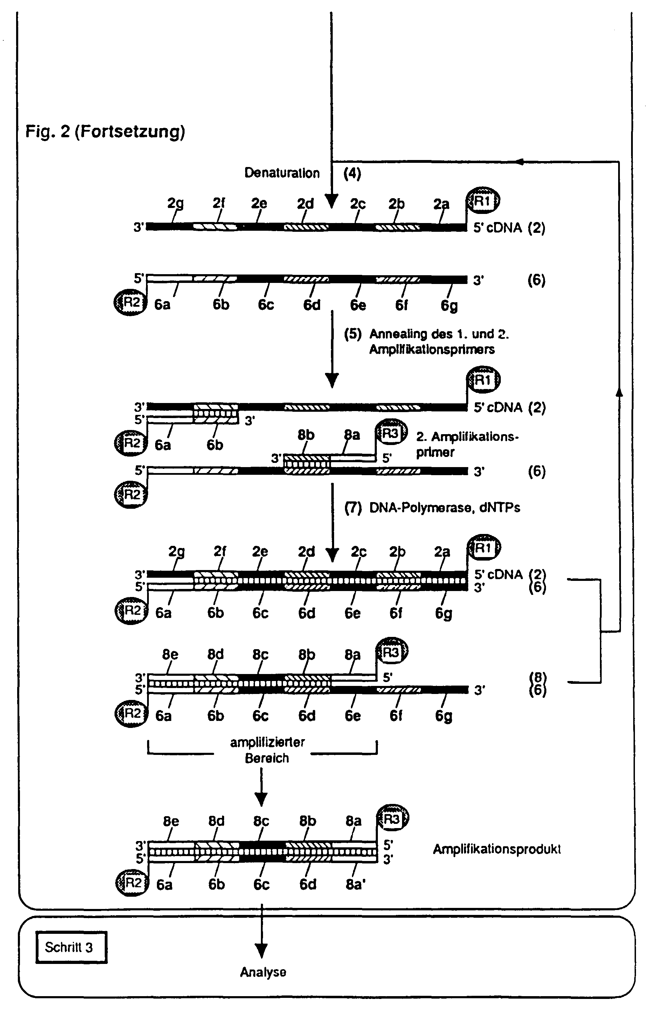

Funktionelle Sequenzen: Alle im Verfahren eingesetzten Nukleinsäuren sind aus funktionellen Sequenzen aufgebaut. In den Fig. 2 - 23 sind diese durch Balken mit verschiedener Zeichnung dargestellt. Der Hybridisierungsbereich zweier Nukleinsäuren ist jeweils durch senkrechte Schraffierung zwischen den beiden angegeben. Die Tatsache, dass die funktionellen Sequenzen in der Regel mit derselben Länge aufgeführt sind, bedeutet nicht, dass sie dieselbe Länge aufweisen müssen. Eine gegenüber der Norm grössere oder geringere Länge der Balken hat keine Bedeutung bezüglich der Länge der symbolisierten Sequenzen; solche Abweichungen dienen lediglich der besseren Uebersichtlichkeit der Figuren. Wenn nicht anders vermerkt, hat jede funktionelle Sequenz eine bestimmte, von den anderen funktionellen Sequenzen verschiedene Basensequenz. Jeder funktionellen Sequenz ist, falls nicht anders vermerkt, ausschliesslich eine einzige Funktion zugeordnet. Mögliche Abweichungen davon sind jeweils in der Figurenbeschreibung oder unter "Modifikationen" beschrieben. Eine gegebene Funktion kann zudem für das Funktionieren der nachstehend beschriebenen Varianten des PERT Assay notwendig oder aber nicht notwendig sein. Alle eingesetzten Nukleinsäuren bestehen aus zumindest einer Hybridisierungssequenz. Funktionelle Gruppen: Häufig tragen die im Verfahren eingesetzten Nukleinsäuren funktionelle Gruppen, die in den Figuren mit (R1), (R2), etc. bezeichnet sind. Diese befinden sich bevorzugterweise am 5'-Ende; der Einfachkeit halber ist in den Figuren jeweils nur die Lokalisation am 5'-Ende angegeben. Diese funktionellen Gruppen verleihen im Rahmen des technisch Möglichen und unter der Voraussetzung, dass sie nicht mit Funktion und Ablauf des PERT Assay interferieren, beliebige zusätzliche Eigenschaften. (R) ist beispielsweise ein Ligand, etwa ein Biotinmolekül, welches die mit (R) versehene Nukleinsäure an einen mit Avidin beschichteten Carrier zu koppeln vermag. Andere Beispiele für Liganden sind Moleküle mit antigenen Eigenschaften, welche an ihnen entsprechende Carrier-immobilisierte spezifische Antikörper binden können. (R) kann aber auch selbst ein Carrier sein; in diesem Fall ist die mit (R) versehene Nukleinsäure kovalent an einen Carrier gebunden. Bevorzugterweise ist jeweils nicht mehr als ein (R) ein Carrier. Alternativ können Nukleinsäuren auch dadurch mit (R) markiert werden, dass für die Synthese dNTPs oder rNTPs verwendet werden, die mit funktionellen Gruppen versehen sind.

Template-Primer-Kombination: Diese besteht aus der Template-Nukleinsäure und allen an diese hybridisierten Nukleinsäuren. Alle für den PERT Assay verwendeten Template-Primer-Kombinationen enthalten eine heteropolymere Template-Nukleinsäure. Ein Abschnitt derselben, der bevorzugterweise unmittelbar an das 5'-Ende einer Hybridisierungssequenz für einen RT-Primer angrenzt, besteht aus RNA und wird als "Template-RNA" bezeichnet. Die Template-RNA ist das eigentliche Substrat einer RT oder RT-Aktivität; die Synthese eines zur Template-RNA komplementären DNA-Strangs definiert die Anwesenheit einer RT oder RT-Aktivität in der Reaktionsmischung. In den meisten Varianten von PERT Assays besteht die ganze Template-Nukleinsäure aus RNA natürlicher, rekombinanter oder synthetischer Provenienz. In einigen Varianten werden jedoch gewisse funktionelle Sequenzen der Template-Nukleinsäure, die upstream der Template-RNA gelegen sind, in Form von DNA vorgelegt. Diese DNA-Anteile der Template-Nukleinsäure bilden die "Template-DNA". Mit der Template-Nukleinsäure ist jeweils mindestens ein RT-Primer verbunden, wobei mindestens dessen 3'-Ende an die Template-Nukleinsäure hybridisiert ist. Zusätzlich zum RT-Primer kann an eine gegen das 5'-Ende hin gelegene Hybridisierungssequenz der Template-Nukleinsäure eine weitere einzel- oder teilweise doppelsträngige Nukleinsäure, bevorzugterweise eine DNA, hybridisiert sein, die für die Einführung einer für das Verfahren essentiellen funktionellen Sequenz benützt wird. Nach Ablauf der RT-Reaktion wird diese DNA mittels einer DNA-Ligase (z.B. T4-DNA-Ligase) mit der in der RT-Reaktion synthetisierten cDNA verbunden. Teile der Template-Nukleinsäure, die downstream der am weitesten downstream gelegenen Hybridisierungssequenz, d.h. der minimal erforderlichen Hybridisierungssequenz für den RT-Primer, liegen, können aus RNA und/oder aus DNA bestehen.

Die Wahl der Template-Primer-Kombination erfolgt so, dass der PERT Assay höchstmögliche Spezifität und Sensitivität für die Erfassung der Retroelemente, die nachgewiesen werden sollen, garantiert. Unter allen Umständen muss garantiert werden, dass ein Nukleinsäureamplifikationsprozess ausschliesslich dann initiiert wird, wenn in Schritt 1 ein hinreichend langes Stück Template-RNA revers transcribiert wurde. Es muss ferner ausgeschlossen werden, dass von einer im Untersuchungsmaterial vorhandenen Nukleinsäure (RNA und/oder DNA) in Schritt 2 ein Amplifikationsprozess initiiert wird, der unabhängig von der in Schritt 1 synthetisierten cDNA ist. Für die Spezifität und Sensitivität ist deshalb wesentlich, dass die Template-Nukleinsäure mit keiner im Untersuchungsmaterial vorhandenen Nukleinsäure identisch oder teilweise identisch ist, sei diese Teil eines normalen Genoms einer Spezies oder eine Nukleinsäure eines infektiösen (übertragbaren) Agens, oder eine andere kontaminierende Nukleinsäure, deren Anwesenheit im Untersuchungsmaterial nicht ausgeschlossen werden kann. Zwecks grösstmöglicher Spezifität des Tests für retrovirale RT wird bevorzugterweise eine heteropolymere Template-RNA verwendet. Zusätzlich kann insbesondere über die Länge und Beschaffenheit (Sekundärstruktur, der Template-RNA die Effizienz der cDNA-Synthese durch verschiedene Typen von Enzymen mit RT-Aktivität beeinflusst und damit die Sensitivität und Spezifität des PERT Assay reguliert werden.

Die meisten Template-Nukleinsäuren haben zusätzlich zu der minimal erforderlichen einen Hybridisierungssequenz mindestens eine weitere Hybridisierungssequenz. Diese dienen dem Annealing der Nukleinsäuren, die für das Priming der RT-Reaktion und/oder für die Einführung von essentiellen funktionellen Sequenzen benötigt werden. Bei der Verwendung von Reportersonden kann die Template-Nukleinsäure immer dann, wenn die Hybridisierungssequenz ausschliesslich zur Selektion einer Reportersonde eingesetzt wird, d.h. keine zusätzliche Funktion ausübt, mehrere hintereinandergeschaltete, bevorzugterweise jeweils durch eine kurze Spacer-Sequenz voneinander getrennte Hybridisierungssequenzen enthalten. An ein Molekül der cDNA können dadurch mehrere Reportersondenmoleküle hybridisiert werden, wodurch die Sensitivität erhöht wird. Bei Verwendung von mehr als einer Hybridisierungssequenz für Reportersonden können diese auch unterschiedliche Basensequenzen aufweisen; dadurch wird die Verwendung von verschiedenen Reportersonden ermöglicht. Auch für den RT-Primer kann auf der Template-Nukleinsäure mehr als eine Hybridisierungssequenz angelegt werden. Je nach Variante des PERT Assay kann oder muss die Template-Nukleinsäure ausser den Hybridisierungssequenzen weitere funktionelle Sequenzen enthalten.

Wie erwähnt, kann es vorteilhaft sein, eine Template-Nukleinsäure zu verwenden, die sich sowohl aus RNA- als auch aus DNA-Sequenzen zusammensetzt. Diese Möglichkeit ist dann attraktiv, wenn die zu vermehrende Nukleinsäure eine zumindest teilweise doppelsträngige DNA erfordert oder wenn für die Synthese der zu vermehrenden Nukleinsäure bzw. für die Initiation des Vermehrungsverfahrens eine zumindest teilweise doppelsträngige DNA notwendig ist. Dies ist dann der Fall, wenn in Schritt 2 ein auf der Verwendung replikativer Nukleinsäuren oder ein auf Transcription beruhendes Nukleinsäureamplifikationsverfahren benutzt und ein doppelsträngiger DNA-ori bzw. ein Transcriptionspromotor benötigt wird. Bei der Konstruktion der Template-Nukleinsäuren für solche Verfahren muss darauf geachtet werden, dass zwischen der Template-DNA und der für den RT-Primer bestimmten Hybridisierungssequenz eine ausreichend lange und entsprechend gestaltete Template-RNA liegt, so dass die cDNA-Synthese ausschliesslich durch Einwirkung einer RT-Aktivität initiiert werden kann. Anschliessend an die reverse Transcription der Template-RNA wird die Template-DNA dupliziert. Dabei kann es von Vorteil sein, die schwache DNA-abhängige DNA-Polymeraseaktivität der im Untersuchungsmaterial vorhandenen RT durch Zugabe einer DNA-abhängigen DNA-Polymerase, die aber keine RT-Aktivität besitzt, zu ergänzen.

Bei der Verwendung replikativer Nukleinsäuren in Schritt 2 benötigt eine aus DNA/RNA bestehende Template-Nukleinsäure nur eine einzige Hybridisierungssequenz, nämlich die für den RT-Primer. Beispiele von Template-Nukleinsäuren, die RNA und DNA enthalten, werden in den Figurenbeschreibungen gegeben.

RT-Primer: Alle im Verfahren verwendeten RT-Primer sind Nukleinsäuren, die entweder aus RNA, DNA, oder einer Kombination aus beiden bestehen und entweder einzelsträngig oder teilweise doppelsträngig sind. Sie bestehen mindestens aus einer Hybridisierungssequenz, mit welcher sie an die Template-Nukleinsäure hybridisieren. In bestimmten Fällen können upstream der am 3'-Ende gelegenen Hybridisierungssequenz eine oder mehrere, zur Template-Nukleinsäure nicht-komplementäre Sequenzen anschliessen. Die im Replikationszyklus von Retroviren als RT-Primer verwendete natürliche tRNA ist ein Beispiel für einen solchen zweiteiligen RT-Primer. Falls der RT-Primer eine DNA ist, können die nichtkomplementären Sequenzen für die Einführung neuer, in der Template-Nukleinsäure noch nicht vorhandener funktioneller Sequenzen benützt werden. An das nicht-komplementäre 5'-Ende des RT-Primers kann auch eine weitere Nukleinsäure anhybridisiert sein. Der RT-Primer ist dann in diesem Bereich doppelsträngig. Bezüglich der Länge und Beschaffenheit der nichtkomplementären Primersequenzen ist man frei, unter dem Vorbehalt, dass sie nicht in unzulässiger Art mit dem optimalen Ablauf des PERT Assay interferieren. Der RT-Primer kann zudem eine funktionelle Gruppe (R1) tragen.

Nukleinsäuren zur Herstellung einer dsDNA: In vielen Varianten des PERT Assay ist es notwendig, nach erfolgter cDNA-Synthese einen zweiten, zur cDNA zumindest teilweise komplementären zweiten DNA-Strang zu schaffen. Dafür muss mindestens eine weitere Nukleinsäure, bevorzugterweise ein DNA-Oligonukleotid, an die cDNA hybridisiert werden. Eine solche weitere DNA besteht zumindest aus einer Hybridisierungssequenz. Zu dieser kommt in gewissen Fällen zumindest eine weitere, zur cDNA nichtkomplementäre funktionelle Sequenz hinzu, die im 2. DNA-Strang in Bezug auf die Hybridisierungssequenz terminal zu liegen kommt und einer flankierenden Sequenz entspricht. Falls diese nichtkomplementären Sequenzen aus DNA bestehen, können sie für die Einführung weiterer benötigter, in der cDNA noch nicht vorhandener funktioneller Sequenzen oder weiterer gewünschter Funktionen benützt werden. Bezüglich der Länge und Beschaffenheit dieser Sequenzen ist man frei, unter dem Vorbehalt, dass sie nicht in unzulässiger Art mit dem optimalen Ablauf des PERT Assay interferieren. Diese Nukleinsäuren können zudem eine funktionelle Gruppe (R) tragen.

Reportersonden: Diese Nukleinsäuren werden zum Nachweis der erfolgten Synthese einer cDNA eingesetzt, indem sie direkt oder indirekt durch Hybridisierung mit der cDNA verbunden werden. Diese Verbindung kann entweder so erfolgen, dass eine Hybridisierungssequenz der Reportersonde direkt mit einer Hybridisierungssequenz der cDNA hybridisiert wird. In diesem Fall ist die Reportersonde von der Template-Nukleinsäure verschieden, mit Ausnahme derjenigen Sequenz, durch welche die Hybridisierung mit der cDNA erfolgt. Es ist aber auch möglich, die Reportersonde indirekt mit der cDNA zu verbinden, indem mindestens eine weitere Nukleinsäure zwischen die Reportersonde und die cDNA zwischengeschaltet und mit beiden durch Hybridisierung verbunden wird. In diesem Fall ist die Sequenz der Reportersonde bevorzugterweise völlig verschieden von der Sequenz der Template-Nukleinsäure. Die Reportersonde stellt entweder die zu vermehrende Nukleinsäure dar oder enthält sie, oder sie oder ein Teil von ihr ist das Edukt einer weiteren Reaktion oder Reaktionsfolge, die zu der zu vermehrenden Nukleinsäure führt.

### Allgemeine Verfahrensaspekte zu Schritt 1

Bereitstellung der zu vermehrenden Nukleinsäure: Ausgehend von einem mittels der Template-Primer-Kombination vorgelegten Satz sinnvoll angeordneter funktioneller Sequenzen wird in Schritt 1 durch die RT-Aktivität eine diese funktionellen Sequenzen ebenfalls enthaltende cDNA geschaffen, die mit der Template-Nukleinsäure zusammen die cDNA-Template-Duplex bildet. Falls diese noch nicht alle für die zu vermehrende Nukleinsäure und deren Synthese erforderlichen funktionellen Sequenzen in der notwendigen Konstellation (RNA oder DNA, einzel- oder doppelsträngig) aufweist, müssen die noch fehlenden über weitere Nukleinsäuren bzw. Reaktionen eingebracht und mit den bereits vorhandenen funktionellen Sequenzen zu der zu vermehrenden Nukleinsäure zusammengefügt werden.

Das Einbringen der funktionellen Sequenzen, welche für die Herstellung und Amplifikation der zu vermehrenden Nukleinsäure benötigt werden, kann entweder so erfolgen, dass bereits die Template-Nukleinsäure alle funktionellen Sequenzen enthält, wobei der RT-Primer und die Reaktionsbedingungen so gewählt werden, dass auch die cDNA bzw. die cDNA-Template-Duplex diese funktionellen Sequenzen enthält. cDNA bzw. cDNA-Template-Duplex können dann die zu vermehrende Nukleinsäure darstellen oder enthalten. Andererseits ist es möglich, eine Template-Nukleinsäure vorzulegen, welche nicht alle der für die Herstellung und Amplifikation der zu vermehrenden Nukleinsäure benötigten funktionellen Sequenzen enthält, wobei die fehlenden vermittelst weiterer Nukleinsäuren eingebracht werden müssen. Möglichkeiten dafür bieten der RT-Primer, weitere an die Template-Nukleinsäure anhybridisierte DNA-Moleküle, die nach erfolgter RT-Reaktion durch Ligation mit der synthetisierten cDNA verbunden werden, ein an die cDNA hybridisierter Primer für die Synthese einer dsDNA oder andere Nukleinsäuren, von denen mindestens eine an die in der cDNA am weitesten downstream gelegene Hybridisierungssequenz hybridisiert wird. Zu den letzteren Nukleinsäuren gehören die Reportersonden oder die Nukleinsäuren, welche die spezifische Verknüpfung zwischen der cDNA und einer Reportersonde herstellen.

Reaktionsbedingungen für die RT-Reaktion: Für die Sensitivität des PERT Assay ist wesentlich, dass der nachzuweisenden RT-Aktivität optimale Reaktionsbedingungen hinsichtlich pH, Salzkonzentration, Ionenstärke, Art und Konzentration des eingesetzten divalenten Kations (Mg, Mn), Konzentration der verwendeten dNTPs, sowie der Reaktionstemperatur angeboten werden. Von Bedeutung ist ferner, die funktionelle Integrität der Template-Primer-Kombination über die Dauer der RT-Reaktion zu gewährleisten.

Synthese und Zusammensetzung der cDNA: Diese besteht an ihrem 5'-Ende aus dem RT-Primer. Das 3'-Ende wird gebildet durch den in der RT-Reaktion synthetisierten Abschnitt. Falls die Template-Nukleinsäure nebst der Template-RNA auch eine Template-DNA umfasst, besteht die cDNA am 5'-Ende aus dem RT-Primer, in der Mitte aus dem zur Template-RNA komplementären Teil sowie am 3'-Ende aus einem Teil, der mindestens zum 3'-Ende der Template-DNA komplementär ist. Nicht Bestandteil der cDNA sind hingegen jene an die Template-Nukleinsäure anhybridisierten vorgelegten Nukleinsäuren; diese werden erst in einer weiteren Reaktion durch Ligation mit der cDNA verbunden.

Elimination der Template-Nukleinsäure: In gewissen Varianten des PERT Assay ist es vorteilhaft oder notwendig, nach Ablauf der RT-Reaktion die Template-Nukleinsäure als Teilnehmer an den nachfolgenden Reaktionen zu eliminieren. Die Massnahmen, die dazu eingesetzt werden können, bestehen z.B. darin, dass die im Reaktionsgemisch vorhandene RNA durch enzymatische Behandlung, z.B. durch RNase, oder chemische Behandlung (z.B. basische Hydrolyse) vollständig degradiert wird, ohne dass dabei die cDNA-Moleküle eliminiert werden. Durch die Degradation der RNA wird auch die Funktion der Template-Nukleinsäure zerstört, da sie obligatorischerweise eine Template-RNA enthält. Bei allen PERT-Varianten besteht zudem die Möglichkeit, den RT-Primer über dessen 5'-Ende, z.B. mittels einer funktionellen Gruppe (R1), an einen Carrier zu binden. Die durch die RT-Aktivität synthetisierte cDNA wird dadurch ebenfalls an den Carrier gebunden. Es entsteht dadurch generell die Möglichkeit, die Template-Nukleinsäure durch Denaturation und anschliessendes Wegwaschen zu entfernen, so dass sie in den folgenden Reaktionen nicht mehr interferiert. Ist Waschen nicht ausreichend, können die RNA-Anteile der Template-Nukleinsäure auch durch basische Hydrolyse zerstört und anschliessend abgesaugt werden; nach Waschen mit einem neutralen Puffer werden die in den folgenden Reaktionen benötigten Reagentien in einem geeigneten Puffer zugegeben.

Es ist für jeden geübten Molekularbiologen offensichtlich, dass sich anhand der hier und im folgenden dargestellten Prinzipien und unter Einbezug sinnvoller Kombinationen eine grosse Vielfalt von Möglichkeiten ergibt, einen PERT Assay durchzuführen, welcher die erfindungsgemässen Vorgaben erfüllt. Essentiell ist in jedem Fall, dass sämtliche im Verfahren eingesetzten Nukleinsäuren, sowie deren funktionelle Sequenzen und funktionellen Gruppen, die weiteren Reagentien und die gewählten Reaktionsbedingungen derart untereinander und gegenüber dem Untersuchungsmaterial abgestimmt sind, dass die vorgegebene Spezifität und Sensitivität des Verfahrens gewährleistet sind.

### Aufbau des PERT Assay bei Verwendung der PCR in Schritt 2

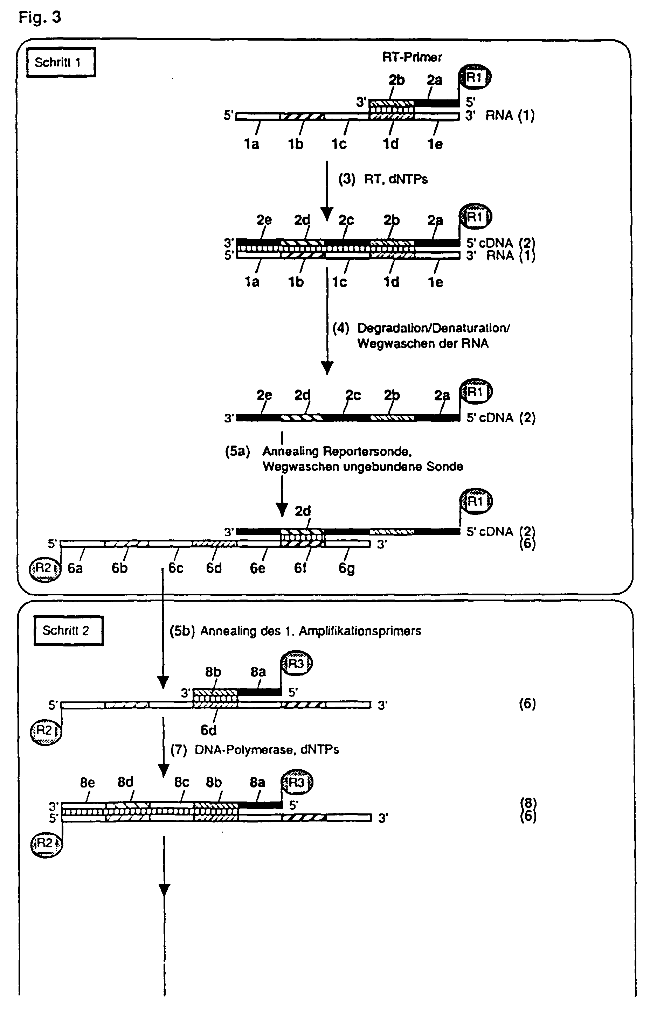

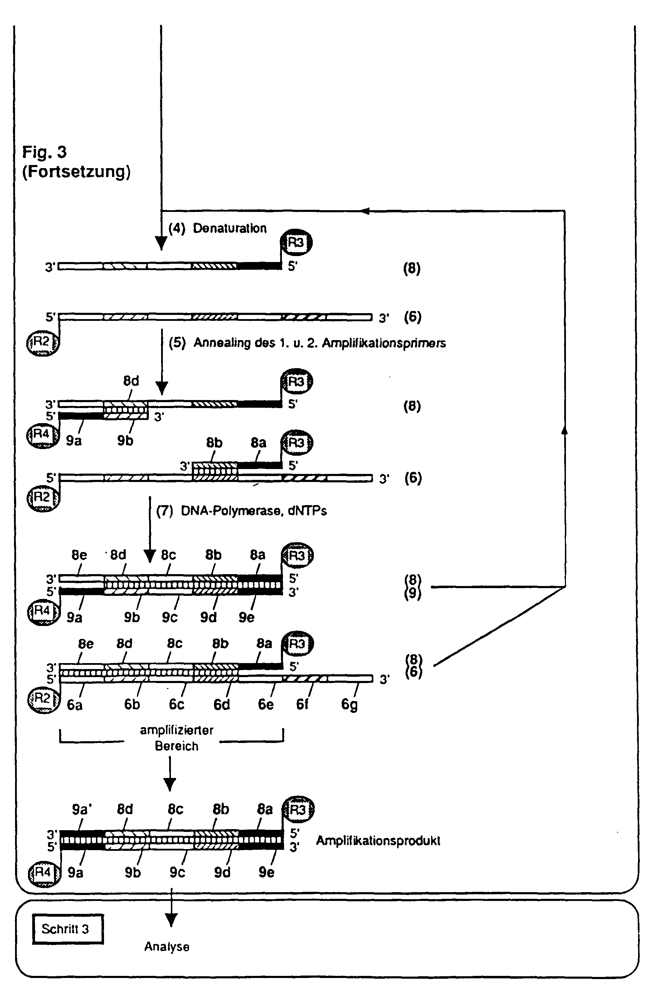

Fig. 2 und 3 zeigen Varianten von PERT Assays, die auf der Verwendung der Polymerase Chain Reaction in Schritt 2 beruhen. Bevorzugterweise wird dabei als Template-Nukleinsäure eine reine RNA verwendet. Es existieren zwei prinzipiell verschiedene Möglichkeiten, die durch Einwirkung der RT-Aktivität synthetisierte cDNA sensitiv, d.h. über einen Amplifikationsprozess, nachzuweisen: Einerseits kann die cDNA, bzw. zumindest eine Teilsequenz davon, direkt amplifiziert werden. Dieses Prinzip ist in Fig. 2 illustriert. Andererseits kann anstelle der cDNA eine Teilsequenz einer DNA-Reportersonde, die mit der cDNA spezifisch, d.h. durch Hybridisierung, verbunden wird, amplifiziert werden (Fig. 3). In beiden Fällen gelangen lediglich drei Arten von funktionellen Sequenzen zum Einsatz, nämlich Hybridisierungs-, Spacer- und flankierende Sequenzen.

**Fig. 2** zeigt eine Variante, bei der die zu vermehrende Nukleinsäure eine Sequenz der cDNA ist.

Schritt 1: Vorgelegt wird eine Template-Nukleinsäure, welche die funktionellen Sequenzen (1a)(1b)(1c)(1d)(1e)(1f)(1g) enthält. Von diesen sind (1b), (1d) und (1f) Hybridisierungssequenzen und notwendig. (1c) und (1e) sind nicht notwendige Spacer-Sequenzen; (1c) ist aber bevorzugterweise vorhanden. Nicht notwendig sind die flankierenden Sequenzen (1a) und (1g). Von den drei Hybridisierungssequenzen dient (1f) dem Annealing des RT-Primers. (1b) und (1d) dienen in Schritt 2 dem Annealing der Primer für die Amplifikation des Produkts der RT-Reaktion (Amplifikationsprimer). Die in (1b) angelegte Sequenz dient dabei dem Annealing des 1. Amplifikationsprimers, die in (1d) angelegte Sequenz dem Annealing des 2. Amplifikationsprimers. Der RT-Primer (R1)(2a)(2b) ist bevorzugterweise ein Oligonukleotid. Das am 3'-Ende gelegene und notwendige (2b) vermittelt das Annealing an (1f) der Template-Nukleinsäure. Das am 5'-Ende gelegene nichtkomplementäre (2a) hingegen ist nicht notwendig und dient dazu, allenfalls erwünschte weitere Funktionen in die cDNA einzubringen. Bei Anwesenheit von RT-Aktivität im aufgearbeiteten Untersuchungsmaterial wird in der Reaktion (3) unter Verwendung der benötigten dNTPs eine cDNA (2) synthetisiert, welche maximal die Elemente (R1)(2a)(2b)(2c)(2d)(2e)(2f)(2g) enthält. Für das Funktionieren des PERT Assay müssen in der RT-Reaktion zumindest die funktionellen Sequenzen bis und mit (2f) synthetisiert werden. Diese cDNA enthält die mittels der PCR zu vermehrende Nukleinsäure.

Es muss nun durch entsprechende Massnahmen sichergestellt werden, dass die Amplifikation in Schritt 2 ausschliesslich von der in der RT-Reaktion synthetisierten cDNA (2), nicht aber von der aus RNA bestehenden Template-Nukleinsäure (1) ausgeht. Letzteres kann z.B. dann eintreten, wenn die in Schritt 2 eingesetzte DNA-abhängige DNA-Polymerase auch eine gewisse RNA-abhängige DNA-Polymeraseaktivität besitzt oder die eingesetzte Enzympräparation durch ein Enzym mit solcher Aktivität kontaminiert ist. Falls das in Schritt 2 verwendete DNA-Amplifikationsverfahren Gewähr bietet, dass dabei kein einziges RNA-Molekül in cDNA umgesetzt wird, oder dass, falls tatsächlich RNA-Moleküle in cDNA umgesetzt werden, die Sensitivität des PERT Assay unter den gewählten Bedingungen zur Erfassung dieser unspezifischen Aktivität nicht ausreicht, kann auf solche Massnahmen verzichtet werden. Generell muss aber auch sichergestellt werden, dass die in grossem Ueberschuss vorhandene Template-Nukleinsäure (1) in den nachfolgenden Reaktionen nicht durch Absättigung von Amplifikationsprimern eine effiziente Vermehrung der DNA-Sequenzen verunmöglicht, so dass es zu falsch-negativen Resultaten kommt. Bevorzugterweise wird daher in Reaktion (4) die vorhandene Template-Nukleinsäure (1) mittels einer der bereits erwähnten Massnahmen als Teilnehmer an nachfolgenden Reaktionen eliminiert. Die einzelsträngige cDNA (2) ist das Produkt von Schritt 1.

Schritt 2: An die cDNA (2) wird sodann in Reaktion (5) der erste Amplifikationsprimer (R2)(6a)(6b) anhybridisiert. Dieser ist bevorzugterweise ein DNA-Oligonukleotid. (R2) ist bevorzugterweise verschieden von (R1). Das am 3'-Ende des Primers gelegene, notwendige (6b) dient der Hybridisierung an (2f) der cDNA. Das am 5'-Ende gelegene, nicht notwendige (6a) besitzt keine Komplementarität zur cDNA. Durch Zugabe einer DNA-abhängigen DNA-Polymerase wird in Reaktion (7) unter Einbezug von dNTPs eine dsDNA (2)//(6) synthetisiert.

In Reaktion (4) wird diese dsDNA denaturiert, worauf in Reaktion (5) ein erneutes Annealing des ersten Amplifikationsprimers (R2)(6a)(6a) sowie ein erstmaliges Annealing des zweiten Amplifikationsprimers (R3)(8a)(8b) an den entsprechenden DNA-Einzelstrang erfolgt. Der zweite Amplifikationsprimer ist ebenfalls bevorzugterweise ein DNA-Oligonukleotid, das keine Komplementarität mit dem 1. Amplifikationsprimer aufweist. (R3) ist wiederum bevorzugterweise verschieden von (R1). Das am 3'-Ende des Primers gelegene obligatorische (8b) dient der Hybridisierung an (6d) des zweiten DNA-Strangs. Das am 5'-Ende gelegene fakultative (8a) besitzt keine Homologie zur cDNA. Durch die DNA-abhängige DNA-Polymerase werden in Reaktion (7) unter Einbezug von dNTPs die dsDNA (2)//(6) bzw. (6)//(8) synthetisiert. Diese werden in den nächsten Amplifikationszyklus eingeschleust. Als Hauptprodukt des Amplifikationprozesses entsteht eine dsDNA bestehend aus den Elementen (R2)(6a)(6b)(6c)(6d)(8a') auf dem einen Strang und den Elementen (R3)(8a)(8b)(8c)(8d)(8e) auf dem anderen Strang.

Schritt 3: Diese dsDNA kann für die Analyse gemäss den später folgenden generellen Anmerkungen zu Schritt 3 herangezogen werden. Alternativ können die funktionellen Gruppen (R) als Markierungen eingesetzt werden und ermöglichen so einfache Detektionssysteme zum Nachweis von dsDNA-Molekülen, die zwei dieser Markierungen, bevorzugt (R2) und (R3), tragen. Solche Detektionssysteme können beispielsweise auf ELISA-Basis, aber auch auf der einfachen und kostengünstigen Partikelagglutination beruhen.

### Modifikationen:

Die als fakultativ benannten funktionellen Sequenzen und funktionellen Gruppen in Template-Nukleinsäure und den Primern können, einzeln oder in den verschiedenen möglichen Kombinationen, entfallen. Falls in den Amplifikationsprimern (6a) und (8a) weggelassen werden, entsteht als Amplifikationsprodukt eine doppelsträngige DNA bestehend aus den Sequenzen (6b)(6c)(6d)//(8b)-(8c)(8d).

Bei der Template-Nukleinsäure (1) können die flankierenden Sequenzen (1a) und (1g) beliebige Funktionen haben, mit der einzigen Restriktion, dass sie nicht mit dem optimalen Ablauf des PERT in unzulässiger Art interferieren. Unter diesem Vorbehalt ist es möglich, natürlich vorkommende RNA-Moleküle als Template-Nukleinsäure (1) einzusetzen.

Abweichend von der Norm kann eine funktionelle Sequenz in gewissen Situationen mehr als einer Funktion dienen. Beispielsweise können die Hybridisierungsfunktionen von (1d) und (1f) zusammengelegt werden. In diesem Fall wird in Schritt 1 die Sequenz (1d) das Annealing eines RT-Primers (R3)(8a)(8b) an die Template-Nukleinsäure (1) vermitteln und in Schritt 2 die von (1d) abgeleitete Sequenz (6d) das Annealing des 2. Amplifikationsprimers (R3)(8a)(8b). Es entfallen dann die funktionellen Sequenzen (1e) und (1f) der Template-Nukleinsäure (1), die von diesen abgeleiteten funktionellen Sequenzen sowie der separate RT-Primer (R1)(2a)(2b).

Als weitere Modifikation können diese Hybridisierungsfunktionen so angelegt werden, dass die sie ausübenden Sequenzen (1d) und (1f) sich teilweise überlappen. Dann entfällt die Spacer-Sequenz (1e) und die von dieser abgeleiteten Sequenzen.

Die Verwendung unterschiedlicher Sequenzen für das Annealing des RT-Primers und des 2. Amplifikationsprimers ermöglicht andererseits die Verwendung von RNA im RT-Primer; dadurch können Sensitivität und/oder Spezifität des PERT Assay beeinflusst werden.

Abweichend von der Norm existiert auch die Möglichkeit, für verschiedene funktionelle Sequenzen identische Basensequenzen zu verwenden, diese können auch homopolymer sein. Dies betrifft die flankierenden Sequenzen (1a) und (1g) sowie die Spacer-Sequenzen (1c) und (1e) der Template-Nukleinsäure (1), die homopolymer und/oder untereinander identisch sein können.

Schliesslich existiert die Möglichkeit, eine Template-Nukleinsäure mit mehreren Hybridisierungssequenzen für RT-Primer auszustatten.

**Fig. 3** zeigt die Verwendung einer Reportersonde.

Schritt 1: Wenn anstelle der cDNA eine Reportersonde zur Amplifikation verwendet wird, braucht die Template-Nukleinsäure nicht alle der in Fig. 2 aufgeführten funktionellen Sequenzen zu enthalten. Vorgelegt wird nun eine Template-Nukleinsäure, welche die funktionellen Sequenzen (1a)(1b)(1c)(1d)(1e) enthält. (1d) dient der Bindung des RT-Primers und ist notwendig; (1c) hat Spacerfunktion und ist nicht erforderlich. Die Hybridisierungssequenz (1b) ist notwendig; die davon abgeleitete Sequenz (2d) der cDNA dient der Bindung der Reportersonde. (1a) und (1e) sind nicht notwendige, flankierende Sequenzen.

Die Template-Nukleinsäure ist über die Hybridisierungssequenz (1d) mit dem RT-Primer (R1)(2a)(2b) verbunden. Für diesen gelten die in Fig. 2/Schritt 1 zum RT-Primer gemachten Angaben. Bei Anwesenheit von RT-Aktivität im aufgearbeiteten Untersuchungsmaterial wird in der Reaktion (3) unter Einbau der benötigten dNTPs die cDNA (2) synthetisiert, welche maximal die Elemente (R1)(2a)(2b) (2c)(2d)(2e) enthält. Für das Funktionieren des PERT Assay müssen minimal die Elemente (R1)(2a)(2b)(2c)(2d) vorhanden sein. Diese cDNA (2) ist mit der Template-Nukleinsäure (1) in Form einer Heteroduplex verbunden und ist in der Lage, vermittelst (R1) an einen Carrier gebunden zu werden, bzw. ist dann, wenn (R1) Carrier ist, bereits Carrier-gebunden.

Wiederum muss sichergestellt werden, dass die Template-Nukleinsäure (1) nicht als spezifischer Kompetitor für die Reportersonde (6) auftritt und dadurch zu falsch-negativen Resultaten führt. In Reaktion (4) wird daher mit einer der genannten Methoden die vorhandene Template-Nukleinsäure, die RNA (1), als relevanter Teilnehmer an der Annealingreaktion (5a) eliminiert. An die cDNA (2) wird sodann in Reaktion (5a) eine DNA-Reportersonde (6) anhybridisiert, welche die zu vermehrende Nukleinsäure darstellt bzw. enthält und aus den funktionellen Sequenzen (6a)(6b)(6c)(6d) (6e)(6e)(6f)(6g) besteht. Von diesen ist (6f) notwendig und diejenige Sequenz, die spezifisch mit dem in der RT-Reaktion (3) synthetisierten (2d) der cDNA (2) hybridisiert. Alle anderen funktionellen Sequenzen der Reportersonde (6) hybridisieren nicht mit der cDNA (2) und weisen keine Sequenzidentität mit funktionellen Sequenzen von ihr auf. (6b) und (6d) sind notwendige Hybridisierungssequenzen für die Amplifikationsprimer. (6d) dient dem Annealing des ersten Amplifikationsprimers, die zu (6b) komplementäre Sequenz dient dem Annealing des zweiten Amplifikationsprimers. (6c) und (6e) sind nicht notwendige Spacer-Sequenzen; (6c) ist jedoch bevorzugterweise vorhanden. (6a) und (6g) sind fakultative flankierende Sequenzen. Die Reportersonde (6) kann an ihrem 5'-Ende oder anderswo ebenfalls eine funktionelle Gruppe (R2) tragen; diese muss von (R1) verschieden sein.

Spätestens nach der Hybridisierung (5a) der Reportersonde (6) an die cDNA (2) wird die cDNA mittels der funktionellen Gruppe (R1) an einen Carrier gebunden und nichtgebundene Reportersonde unter nichtdenaturierenden Bedingungen vollständig aus dem Reaktionsgemisch entfernt. Damit rigoros gewaschen werden kann, ist eine starke Bindung zwischen der carriergebundenen cDNA (2) und der Reportersonde (6) notwendig. Der Hybridisierungsbereich dieser beiden Nukleinsäuren, (2d) bzw. (6f), muss daher von genügender Länge und geeigneter Beschaffenheit sein, und das Annealing der Reportersonde (6) muss unter optimalen Reaktionsbedingungen erfolgen. Ebenso muss darauf geachtet werden, dass die Bindung der cDNA (2) an den Carrier bestehen bleibt.

Schritt 2: Nach vollständigem Wegwaschen ungebundener Reportersonde (6) wird in einer weiteren Reaktion (5b) in einem geeigneten Puffer der 1. Amplifikationsprimer (R3)(8a)(8b) zugegeben. Dieser ist bevorzugterweise ein DNA-Oligonukleotid; bevorzugterweise ist (R3) verschieden von (R1). Das am 3'-Ende gelegene (8b) dient dem Annealing an (6d) der Reportersonde. Das am 5'-Ende gelegene (8a) ist fakultativ, hat keine Komplementarität zur Reportersonde und weder Identität noch Komplementarität zur cDNA (2). Durch Zugabe einer DNA-abhängigen DNA-Polymerase wird unter Einbezug von dNTPs in Reaktion (7) die dsDNA (6)//(8) synthetisiert. In Reaktion (4) wird diese dsDNA denaturiert, worauf in einer erneuten Reaktion (5) das Annealing nun sowohl des 1. als auch des 2. Amplifikationsprimers an den entsprechenden DNA-Einzelstrang erfolgt. Der 2. Amplifikationsprimer (R4)(9a)(9b) ist bevorzugterweise ein DNA-Oligonukleotid, das weder Identität noch Komplementarität mit der cDNA aufweist; bevorzugterweise ist (R4) von (R1) verschieden. Das am 5'-Ende gelegene nichtkomplementäre (9a) ist fakultativ. Das am 3'-Ende gelegene obligatorische (9b) vermittelt das Annealing mit (8d). Durch die DNA-abhängige DNA-Polymerase werden unter Einbezug von dNTPs in Reaktion (7) die dsDNA (6)//(8) bzw. (8)//(9) synthetisiert. Diese werden in den nächsten Amplifikationszyklus eingeschleust. Als Hauptprodukt der Amplifikation entsteht eine dsDNA (R3)(8a)(8b)(8c)(8d)(9a')//(R4)(9a)(9b)(9c)(9d)(9e).

Schritt 3: Ist analog Schritt 3 von Fig. 2.

### Modifikationen:

In der Template-Nukleinsäure (1) können mehrere Hybridisierungssequenzen (1b) für Reportersonden, bevorzugterweise jeweils durch eine kurze Spacer-Sequenz voneinander getrennt, plaziert werden. Die Hybridisierungssequenzen brauchen nicht identisch zu sein, so dass verschiedene Reportersonden eingesetzt werden können. Ebenso existiert die Möglichkeit, die Template-Nukleinsäure mit mehreren Hybridisierungssequenzen für RT-Primer auszustatten.

Die als fakultativ benannten funktionellen Sequenzen und funktionellen Gruppen in Template-Nukleinsäure, Primern und Reportersonde können, einzeln oder in den verschiedenen möglichen Kombinationen, entfallen. Falls bei den Amplifikationsprimern auf (8a) und (9a) verzichtet wird, entsteht als Amplifikationsprodukt eine doppelsträngige DNA mit den funktionellen Sequenzen (8b)(8c) (8d)//(9b)(9c)(9d). Auf (R1) als Bestandteil des RT-Primers kann dann verzichtet werden, wenn die Bedingungen für die RT-Reaktion so gewählt werden, dass dabei mit Liganden markierte Deoxyribonukleotidmonophosphate in die neusynthetisierte cDNA eingebaut werden; diese funktionellen Gruppen können dann im Sinne von (R1) die cDNA an einen Carrier binden.

Bei der Reportersonde (6) können (6a) und (6g) beliebige Funktionen aufweisen. Dies ermöglicht, natürlich vorkommende lineare oder ringförmige Nukleinsäuren als Reportersonde einzusetzen.

Es ist ferner möglich, die zu amplifizierende Region der Reportersonde (6) nicht upstream, sondern downstream der cDNA-bindenden Region (6f) zu plazieren, so dass sie in den Bereich von (6g) zu liegen kommt. Die zu amplifizierende Region kann (6f) auch einschliessen oder mit diesem überlappen; dies letztere ist allerdings keine bevorzugte Konstellation.

Abweichend von der Norm kann eine funktionelle Sequenz in gewissen Situationen mehr als eine Funktion aufweisen. Beispielsweise können die Hybridisierungsfunktionen von (6d) und (6f) zusammengelegt werden. In diesem Fall wird (6d) vorerst das Annealing der Reportersonde an die cDNA vermitteln (dies bedingt natürlich auch eine in (1b) entsprechend modifizierte Template-Nukleinsäure!) und dann, nach dem Wegwaschen der ungebundenen Reportersonde (6) und einem in diesem Fall notwendig werdenden Denaturierungsschritt, auch den Primer (R)(8a)(8b) binden. Dadurch entfallen (6e) und (6f) der Reportersonde sowie die von diesen abgeleiteten funktionellen Sequenzen. Als weitere Modifikation können die Hybridisierungsfunktionen von (6d) und (6f) so angelegt werden, dass die sie ausübenden Sequenzen sich teilweise überlappen. Dann entfällt die Spacer-Sequenz (6e) und die von dieser abgeleiteten Sequenzen.

Die Möglichkeit der teilweisen, allerdings nur geringfügigen, Ueberlappung existiert schliesslich auch für (1b) und (1d) der Template-Nukleinsäure (1), solange die Reportersonde dadurch nicht an (2b) hybridisiert.

Abweichend von der Norm können für verschiedene funktionelle Sequenzen identische Basensequenzen verwendet werden, diese können auch homopolymer sein. Dies betrifft die flankierenden Sequenzen (1a) und (1e) sowie die Spacer-Sequenz (1c) der Template-Nukleinsäure, die homopolymer und/oder untereinander identisch sein können. Homopolymer und/oder untereinander identisch können auch die flankierenden Sequenzen (6a) und (6g) sowie die Spacer-Sequenzen (6c) und (6e) der Reportersonde (6) sein.

### Aufbau des PERT Assay bei Verwendung eines auf der Ligase Chain Reaction (LCR) basierenden Amplifikationsverfahrens in Schritt 2

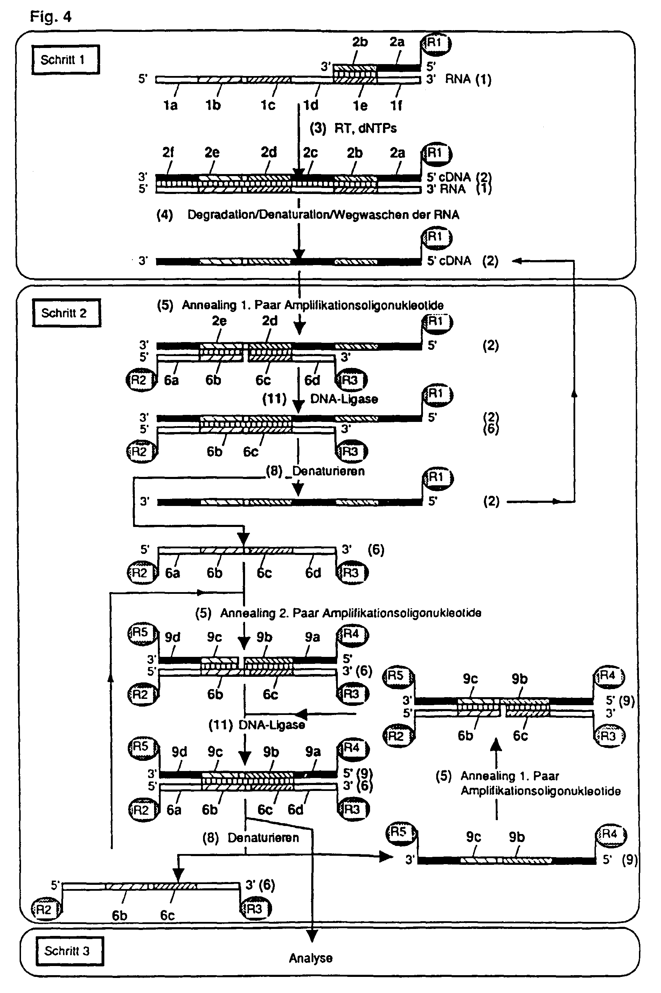

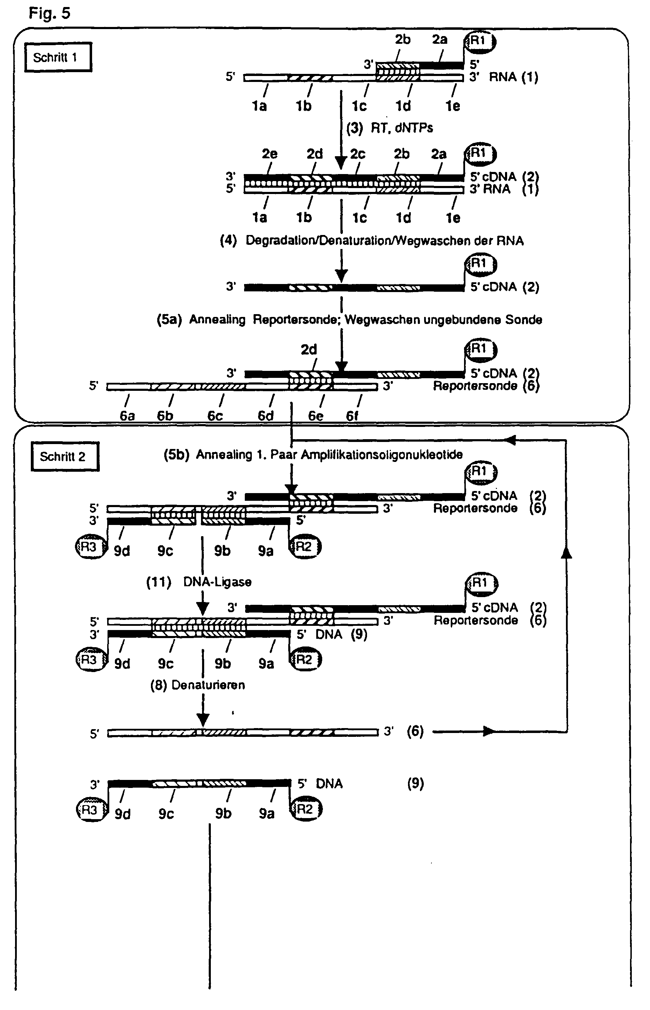

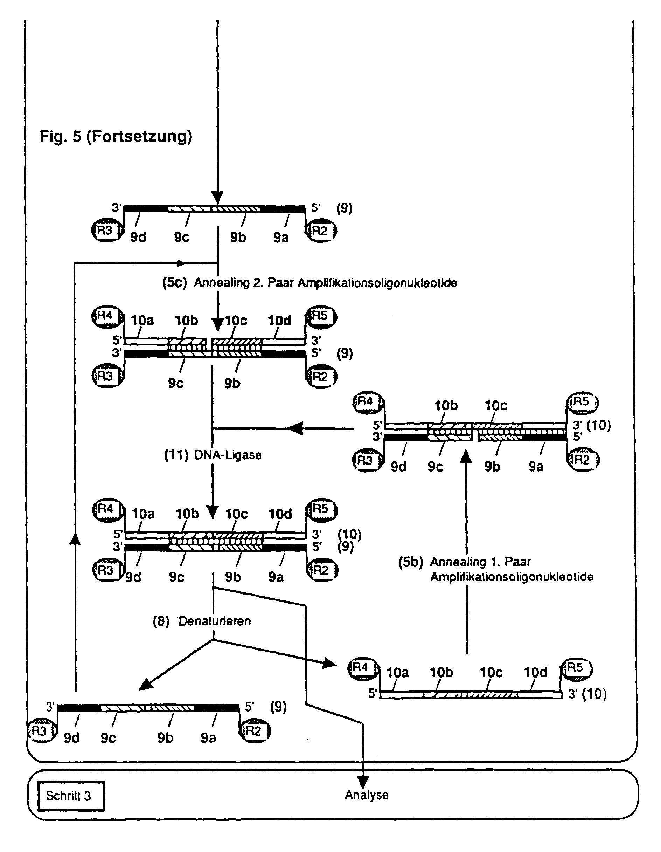

Gemäss den PS WO 89/12696 und WO 89/09835 ist die LCR ein weiteres Verfahren zur Amplifikation von Nukleinsäuresequenzen. Ihre Verwendung im Rahmen des PERT Assay ist in den Figuren 4 und 5 beschrieben. Bevorzugterweise wird dabei als Template-Nukleinsäure eine reine RNA verwendet; keinesfalls darf die in Schritt 2 zu vermehrende Sequenz der Template-Nukleinsäure aus DNA bestehen. Die zum Einsatz gelangenden funktionellen Sequenzen sind Hybridisierungs-, Spacer- und flankierende Sequenzen.

**Fig. 4** zeigt den prinzipiellen Verfahrensablauf, wenn eine Teilsequenz der cDNA selbst amplifiziert wird.

Schritt 1: Vorgelegt wird eine Template-Nukleinsäure (1), welche die funktionellen Sequenzen (1a) bis (1f) enthält. Von diesen sind (1b), (1c) und (1e) Hybridisierungssequenzen und für das Funktionieren des PERT Assay notwendig. (1d) ist eine nicht notwendige Spacer-Sequenz, und (1a) und (1f) sind nicht notwendige flankierende Sequenzen. Von den drei Hybridisierungssequenzen dient (1e) dem Annealing des RT-Primers. (1b) und (1c), welche unmittelbar aneinander anschliessen, sowie die zu ihnen komplementären Sequenzen dienen dem Annealing der beiden DNA-Oligonukleotidpaare, mit deren Hilfe die LCR-Amplifikation einer Teilsequenz des Produkts der RT-Reaktion erfolgt. Der RT-Primer (R1)(2a)(2b) besitzt an seinem 3'-Ende die notwendige Hybridisierungssequenz (2b). Das am 5'-Ende gelegene (2a) ist fakultativ und besitzt keine Komplementarität zur Template-Nukleinsäure. (R1) ist ebenfalls fakultativ. Falls der RT-Primer eine DNA ist, ermöglicht (2a), zusätzliche gewünschte Funktionen in die cDNA einzubringen. Bei Anwesenheit von RT-Aktivität im aufgearbeiteten Untersuchungsmaterial wird in der Reaktion (3) unter Verwendung von dNTPs eine cDNA (2) synthetisiert, welche maximal die Elemente (R1)(2a)(2b)(2c) (2d)(2e)(2f) enthält. Für das Funktionieren des PERT Assay müssen in der RT-Reaktion zumindest die funktionellen Sequenzen bis und mit (2e) synthetisiert werden. Diese cDNA enthält die mittels der LCR zu vermehrende Nukleinsäure.

Für einen optimalen Ablauf des PERT Assay wird zunächst die Template-RNA der Template-Nukleinsäure (1) in Reaktion (4) degradiert und als Reaktionspartner in den kommenden Reaktionen eliminiert. Dies ist wünschenswert, weil damit das Annealing des zweiten Oligonukleotidpaares an die Template-Nukleinsäure verhindert werden kann. Ein solches Annealing kann, je nach den gewählten Reaktionsbedingungen und Reagentien, zu falsch-positiven oder falsch-negativen Resultaten des PERT Assay führen: Falsch-positive Resultate entstehen dann, wenn das an die Template-Nukleinsäure gebundene zweite Oligonukleotidpaar, bestehend aus (R4)(9a)(9b) und (9c)(9d)(R5) [s. unten], durch eine Ligase wie diejenige aus dem Bacteriophagen T4 zu einem kohärenten DNA-Strang ligiert wird.

Falsch-negative Resultate entstehen dann, wenn das an die Template-Nukleinsäure (1) gebundene zweite Oligonukleotidpaar durch die eingesetzte Ligase zwar nicht zu einem kohärenten DNA-Strang ligiert, aber infolge seiner Bindung an die in grosser Menge vorliegende Template-Nukleinsäure der Reaktion entzogen wird.

Schritt 2: In Reaktion (5) werden zwei DNA-Oligonukleotide, (R2)(6a)(6b) und (6c)(6d)(R3), an die cDNA angelagert. Diese zwei Oligonukleotide sind in ihren Hybridisierungssequenzen (6b) bzw. (6c) vollständig komplementär zu (2e) bzw. (2d) der cDNA und nach dem Annealing an dieselben lediglich durch ein Nick voneinander getrennt. (6a) bzw. (6d) sind fakultativ, weisen keine Identität oder Komplementarität zur cDNA auf und werden zur Einführung allfällig gewünschter zusätzlicher DNA-Sequenzen in das Amplifikationsprodukt verwendet. Oligonukleotid (6c)(6d)(R3) ist am 5'-Ende phosphoryliert. Bezüglich Lokalisation der ebenfalls fakultativen funktionellen Gruppen gilt, dass (R2) bevorzugterweise möglichst weit gegen das 5'-Ende von (6a)(6b) und (R3) möglichst weit gegen das 3'-Ende von (6c)(6d) liegt. In Reaktion (11) wird eine DNA-Ligase (z.B. des Bacteriophagen T4; mit Vorteil wird ein hitzestabiles Enzym verwendet) zur Ligation der beiden Oligonukleotide eingesetzt, wodurch der DNA-Strang (6) geschaffen wird. In Reaktion (8) wird denaturiert, worauf Strang (2) wiederum für das Annealing der beiden Oligonukleotide (R2)(6a)(6b) und (6c)(6d)(R3) zur Verfügung steht. Strang (6) wird für die weitere Amplifikation verwendet.

Zunächst erfolgt in Reaktion (5) das Annealing eines weiteren, analog zum ersten Paar konstruierten DNA-Oligonuklotidpaares. Dieses umfasst die Oligonukleotide (R4)(9a)(9b) und (9c)(9d)(R5), deren Hybridisierungssequenzen (9b) bzw. (9c) vollkommen komplementär zu (6c) bzw. (6b) sind und nach dem Annealing an diese unmittelbar nebeneinander zu liegen kommen, und zwar optimalerweise so, dass das Nick zwischen dem 3'-Ende des einen und dem 5'-Ende des anderen gegenüber dem Nick, das in Strang (6) vorhanden war, um ein Nukleotid verschoben ist. Das 5'-Ende von (9c)(9d)(R5) muss ausserdem eine Phosphatgruppe tragen. Bezüglich Lokalisation der fakultativen funktionellen Gruppen gilt, dass (R4) bevorzugterweise möglichst weit gegen das 5'-Ende von (9a)(9b) und (R5) möglichst weit gegen das 3'-Ende von (9c)(9d) liegt. Wiederum sind (9a) und (9d) fakultativ. (9a) ist entweder komplementär zu (6d) oder nicht; desgleichen ist (9d) komplemenär zu (6a) oder nicht. Nicht-komplementäre Sequenzen (9a) bzw. (9d) dienen zur Einführung weiterer funktioneller Sequenzen in das Amplifikat. Komplementäre Sequenzen (9a) bzw. (9d) bewirken dagegen eine Verlängerung der Hybridisierungssequenzen. Durch erneute Aktion der DNA-Ligase (11) werden die beiden Oligonukleotide (R4)(9a)(9b) und (9c)(9d)(R5) zu Strang (9) ligiert, der mit Strang (6) partiell eine Duplex bildet. Nach erneuter Denaturierung (8) erfolgt wiederum das Annealing (5) der entsprechenden komplementären Oligonukleotidpaare an die separierten Stränge (6) und (9); als Resultat dieser zyklischen Amplifikation entstehen grosse Mengen des doppelsträngigen DNA-Oligonukleotiddimers (6)//(9).

Schritt 3: Dieses Produkt kann für die Analyse gemäss den später folgenden generellen Anmerkungen zu Schritt 3 herangezogen werden. Alternativ können, falls mittels (R) Markierungen eingeführt wurden, einfache Detektionssysteme zum Nachweis von DNA-Molekülen, welche bestimmte neugebildete Kombinationen von funktionellen Gruppen (R) tragen, eingesetzt werden. Diese Kombinationen sind: (R2) und (R3), (R2) und (R4), (R5) und (R4), (R5) und (R3). Solche Detektionssysteme können beispielsweise auf ELISA-Basis, aber auch auf Partikelagglutination beruhen. Die mittels der Oligonukleotide eingeführten nichtkomplementären funktionellen Sequenzen (6a), (6d), (9a), und (9d) können, da die DNA in diesen Bereichen einzelsträngig ist, beim Nachweis mittels Hybridisierung Verwendung finden.

### Modifikationen:

Die als fakultativ benannten funktionellen Sequenzen und funktionellen Gruppen der eingesetzten Nukleinsäuren können, einzeln oder in einer der verschiedenen möglichen Kombinationen, entfallen. Beim kombinierten Verzicht auf (6a), (6d), (9a) und (9d) ist das Amplifikationsprodukt (6)//(9) ein dsDNA-Molekül, welches aus den Fragmenten (6b)(6c)//(9b)(9c) besteht. Die flankierenden Sequenzen (9a) bzw. (9d) können so gestaltet werden, dass sie komplementär zu (6d) bzw. (6a) sind.

Bei der Template-Nukleinsäure (1) können die flankierenden Sequenzen (1a) und (1f) beliebige Funktionen aufweisen, so dass natürlich vorkommende RNA-Moleküle als Template-Nukleinsäure (1) eingesetzt werden können.

Die Template-Nukleinsäure (1) kann mehr als eine Hybridisierungssequenz für den RT-Primer enthalten.

Abweichend von der Norm kann eine funktionelle Sequenz in gewissen Situationen mehr als einer Funktion dienen. Beispielsweise können die Hybridisierungsfunktionen von (1c) und (1e) zusammengelegt werden. In diesem Fall vermittelt (1c) vorerst das Annealing des nun auch als RT-Primer dienenden (R4)(9a)(9b) an die Template-Nukleinsäure (1). In Schritt 2 ist (R4)(9a)(9b) bekanntermassen eines der verwendeten Amplifikationsoligonukleotide. Dadurch entfallen die funktionellen Sequenzen (1d) und (1e) der Template-Nukleinsäure (1) sowie alle von diesen abgeleiteten funktionellen Sequenzen. Ein separater RT-Primer (R1)(2a)(2b) erübrigt sich ebenfalls.

Als weitere Modifikation können diese Hybridisierungsfunktionen so angelegt werden, dass die sie ausübenden Sequenzen (1c) und (1e) sich teilweise überlappen. Dann entfällt die Spacer-Sequenz (1d) und die von dieser abgeleiteten Sequenzen.

Die Verwendung unterschiedlicher Sequenzen für das Annealing des RT-Primers und des Amplifikationsoligonukleotids (R4)(9a)(9b) ermöglicht andererseits die Verwendung eines RNA-Primers; dadurch können Sensitivität und/oder Spezifität des PERT Assay beeinflusst werden.

Abweichend von der Norm existiert die Möglichkeit, für verschiedene funktionelle Sequenzen identische Basensequenzen zu verwenden, diese können auch homopolymer sein. Dies betrifft die flankierenden Sequenzen (1a) und (1f) sowie die Spacer-Sequenz (1d) der Template-Nukleinsäure (1), die homopolymer und/oder untereinander identisch sein können.

Eine nicht bevorzugte, aber mögliche Modifikation besteht darin, für die Hybridisierungssequenzen (1b) und (1c) der Template-Nukleinsäure (1) ein und dieselbe, von den Sequenzen des Spacerelements (1d), der Hybridisierungssequenz (1e) für den RT-Primer und der flankierenden Sequenzen (1a) und (1f) verschiedene Sequenz zu verwenden. Die Amplifikationsoligonukleotide dürfen dann nur aus einer Hybridisierungssequenz bestehen und müssen am 5'-Ende phosphoryliert sein.

**Fig. 5** zeigt den Verfahrensablauf, wenn anstelle der cDNA zumindest eine Teilsequenz einer Reportersonde amplifiziert wird.

Schritt 1: Template-Primer-Kombination, RT-Reaktion (3) sowie deren Produkt sind in allen Aspekten identisch denjenigen aus Figur 3. Das aus Reaktion (3) hervorgehende Gemisch von Nukleinsäuren wird in Reaktion (4) derart modifiziert, dass sichergestellt wird, dass die Template-Nukleinsäure (1) in den folgenden Reaktionen nicht als spezifischer Kompetitor für die Reportersonde (6) auftritt und dadurch zu falsch-negativen Resultaten führt. In Reaktion (4) wird daher mit einer der genannten Methoden die vorhandene Template-Nukleinsäure (1) als relevanter Teilnehmer an der Annealingreaktionen (5a) eliminiert.

An die cDNA (2) wird sodann in Reaktion (5a) eine DNA-Reportersonde (6) anhybridisiert, welche die zu vermehrende Nukleinsäure darstellt oder enthält und aus (6a)(6b)(6c)(6d)(6e)(6f) besteht. Von diesen ist (6e) die Hybridisierungssequenz, die spezifisch mit dem in der RT-Reaktion (3) synthetisierten (2d) der cDNA hybridisiert. Alle anderen funktionellen Sequenzen der Reportersonde hybridisieren nicht mit der cDNA. (6b) und (6c) schliessen unmittelbar aneinander an und sind notwendige Hybridisierungssequenzen für die Amplifikationsoligonukleotide. Sie besitzen mit Vorteil weder Identität noch Komplementarität zueinander und dürfen weder Identität noch Komplementarität zur cDNA besitzen. (6d) ist eine nicht notwendige Spacer-Sequenz. (6a) und (6f) sind nicht notwendige flankierende Sequenzen. Spätestens nach der Hybridisierung (5a) der Reportersonde (6) an die cDNA (2) wird die cDNA mittels der funktionellen Gruppe (R1) an einen Carrier gebunden und nichtgebundene Reportersonde unter nichtdenaturierenden Bedingungen vollständig aus dem Reaktionsgemisch entfernt, wie für Fig. 3 beschrieben.

Schritt 2: Danach wird in einer weiteren Annealingreaktion (5b) in einem geeigneten Puffer das erste Paar von Amplifikationsoligonukleotiden, (R2)(9a)(9b) und (9c)(9d)(R3), zugegeben. (9b) bzw. (9c) dieser beiden Oligonukleotide sind vollständig komplementär zu (6c) bzw. (6b) und nach dem Annealing an dieselben lediglich durch ein Nick voneinander getrennt. (9c)(9d)(R3) ist ausserdem am 5'-Ende phosphoryliert. (9a) bzw. (9d) sind fakultativ, weisen keine Identität oder Komplementarität zur Reportersonde auf und werden zur Einführung allfällig gewünschter zusätzlicher DNA-Sequenzen in das Amplifikationsprodukt verwendet. Bezüglich Lokalisation der fakultativen funktionellen Gruppen gilt, dass (R2) bevorzugterweise möglichst weit gegen das 5'-Ende von (9a)(9b) und (R3) möglichst weit gegen das 3'-Ende von (9c)(9d) liegt. (R2) und (R3) sollen zudem von (R1) verschieden und nicht Carrier sein. In Reaktion (11) werden vermittelst der DNA-Ligase die beiden Oligonukleotide (R2)(9a)(9b) und (9c)(9d)(R3) zu einer kohärenten Sequenz (9) ligiert. In Reaktion (8) wird denaturiert, worauf die Reportersonde (6) wiederum für das Annealing der beiden Oligonukleotide (R2)(9a) (9b) und (9c)(9d)(R3) zur Verfügung steht. Strang (9) wird für die weitere Amplifikation verwendet.

Zunächst erfolgt in Reaktion (5) das Annealing eines weiteren analog zum ersten Paar konstruierten DNA-Oligonuklotidpaars. Dieses umfasst die Oligonukleotide (R4)(10a)(10b) und (10c)(10d)(R5), deren funktionelle Sequenzen (10b) bzw. (10c) vollkommen komplementär sind zu (9c) bzw. (9b) und nach dem Annealing an diese unmittelbar nebeneinander zu liegen kommen, und zwar optimalerweise so, dass das Nick zwischen dem 3'-Ende des einen und dem 5'-Ende des anderen gegenüber dem Nick, das in Strang (9) vorhanden war, um ein Nukleotid verschoben ist. Das 5'-Ende des Oligonukleotids (10c)(10d)(R5) muss ausserdem eine Phosphatgruppe tragen.

Bezüglich Lokalisation der fakultativen funktionellen Gruppen gilt, dass (R4) bevorzugterweise möglichst weit gegen das 5'-Ende von (10a)(10b) und (R5) möglichst weit gegen das 3'-Ende von (10c)(10d) liegt. (R4) und (R5) sind zudem bevorzugterweise von (R1) verschieden und nicht Carrier. (10a) bzw. (10d) sind fakultative nicht-komplementäre flankierende Sequenzen und dienen zur Einführung weiterer Funktionen in das Amplifikat.

Durch erneute Aktion der DNA-Ligase (11) werden die beiden Oligonukleotide (R4)(10a)(10b) und (10c)(10d)(R5) zu Strang (10) ligiert, der mit Strang (9) partiell eine Duplex bildet. Nach erneuter Denaturierung (8) erfolgt wiederum das Annealing (5) der entsprechenden komplementären Oligonukleotidpaare an die separierten Stränge (9) und (10); als Resultat dieser zyklischen Amplifikation entstehen grosse Mengen des teilweise doppelsträngigen DNA-Oligonukleotiddimers (9)//(10).

Schritt 3: Das teilweise doppelsträngige DNA-Oligonukleotiddimer (9)//(10) wird mit den in Fig. 4 erwähnten Methoden nachgewiesen.

### Modifikationen:

In der Template-Nukleinsäure (1) können mehrere Hybridisierungssequenzen (1b) für Reportersonden, bevorzugterweise durch jeweils eine kurze Spacer-Sequenz voneinander getrennt, plaziert werden. Die Hybridisierungssequenzen brauchen nicht identisch zu sein, so dass verschiedene Reportersonden eingesetzt werden können. Desgleichen können mehrere Hybridisierungssequenzen für den RT-Primer vorhanden sein.

Die als fakultativ benannten Elemente in Template-Nukleinsäure, RT-Primer, Reportersonde und Amplifikationsoligonukleotiden können, einzeln oder in einer der verschiedenen möglichen Kombinationen, entfallen. Die flankierenden Sequenzen (10a) bzw. (10d) können so gestaltet werden, dass sie komplementär zu (9d) bzw. (9a) sind. Auf (R1) als Bestandteil des RT-Primers kann dann verzichtet werden, wenn die Bedingungen für die RT-Reaktion so gewählt werden, dass dabei mit Liganden markierte Deoxyribonukleotidmonophosphate in die neusynthetisierte cDNA eingebaut werden; diese funktionellen Gruppen können dann im Sinne von (R1) die cDNA an einen Carrier binden.

Bei der Reportersonde (6) können die flankierenden Sequenzen (6a) und (6f) beliebiger Basensequenz sein. So ist es möglich, natürlich vorkommende lineare oder ringförmige DNA-Moleküle als Reportersonde einzusetzen.

Es ist ferner möglich, die zu amplifizierende Region der Reportersonde (6) nicht upstream, sondern downstream der cDNA-bindenden Hybridisierungssequenz (6e) zu plazieren, so dass sie in den Bereich von (6f) zu liegen kommt. Die zu amplifizierende Region kann auch mit (6e) überlappen; dies ist allerdings keine bevorzugte Konstellation.

Abweichend von der Norm kann eine gegebene funktionelle Sequenz in gewissen Situationen mehr als eine Funktion erfüllen. Beispielsweise können die Hybridisierungsfunktionen von (6c) und (6e) zusammengelegt werden. In diesem Fall wird (6c) vorerst das Annealing der Reportersonde an die cDNA vermitteln (dies bedingt natürlich auch eine entsprechend modifizierte Template-Nukleinsäure!) und dann, nach dem Wegwaschen der ungebundenen Reportersonde und einem in diesem Fall notwendig werdenden Denaturierungsschritt, auch das Amplifikationsoligonukleotid (R2)(9a)(9b) binden. Infolge der Zusammenlegung dieser beiden Funktionen auf (6c) entfallen die funktionellen Sequenzen (6d) und (6e) der Reportersonde sowie die von diesen abgeleiteten funktionellen Sequenzen.

Als weitere Modifikation können diese Hybridisierungsfunktionen so angelegt werden, dass die sie ausübenden Sequenzen sich teilweise überlappen. Dann entfällt die Spacerregion (6d) und die von dieser abgeleiteten Sequenzen.

Die Möglichkeit der teilweisen, nur wenige Nukleotide umfassenden Ueberlappung existiert schliesslich auch für die funktionellen Sequenzen (1b) und (1d) der Template-Nukleinsäure (1), solange die Reportersonde dadurch nicht an (2b) hybridisiert. Abweichend von der Norm existiert die Möglichkeit, für verschiedene funktionelle Sequenzen identische Basensequenzen zu verwenden, diese können auch homopolymer sein. Dies betrifft die flankierenden Sequenzen (1a) und (1e) sowie die Spacer-Sequenz (1c) der Template-Nukleinsäure, die homopolymer und/oder untereinander identisch sein können. Homopolymer und/oder untereinander identisch können auch die flankierenden Sequenzen (6a) und (6f) sowie das Spacerelement (6d) der Reportersonde (6) sein.

Eine nicht bevorzugte, aber mögliche Modifikation besteht darin, für die Oligonukleotidhybridisierungsregionen (6b) und (6c) der Reportersonde (6) ein und dieselbe, von den Sequenzen des Spacerelements (6d), der Hybridisierungssequenz (6e) und der flankierenden Sequenzen (6a) und (6f) verschiedene, Basensequenz zu verwenden. Die Amplifikationsoligonukleotide dürfen dann lediglich aus einer Hybriddisationssequenz bestehen und müssen am 5'-Ende phosphoryliert sein.

### Aufbau des PERT Assay bei Verwendung des "Process for Amplifying and Detecting Nucleic Acid Sequences" (WO 90/01069) in Schritt 2

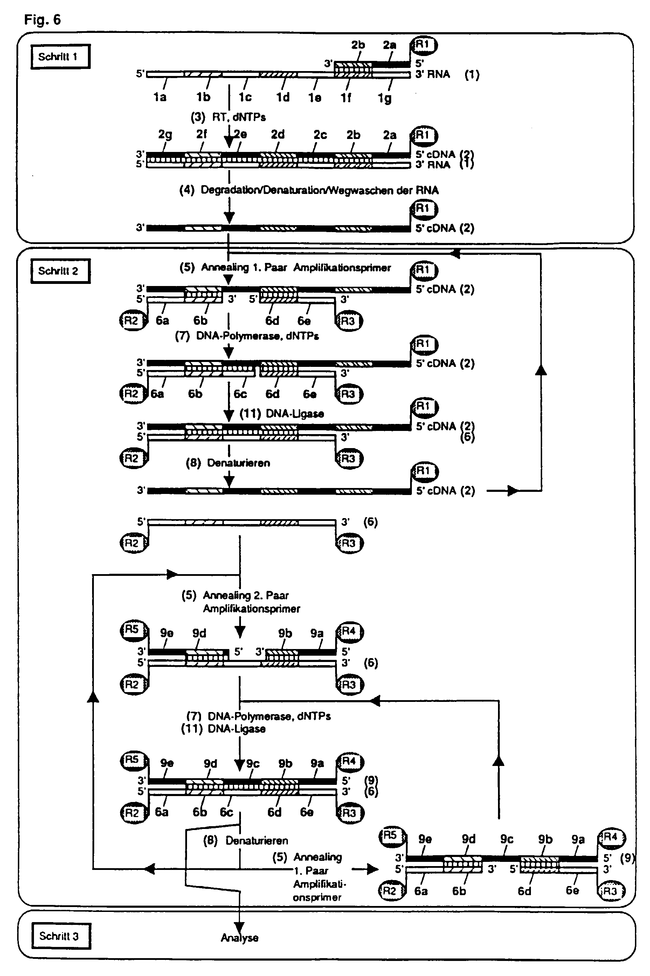

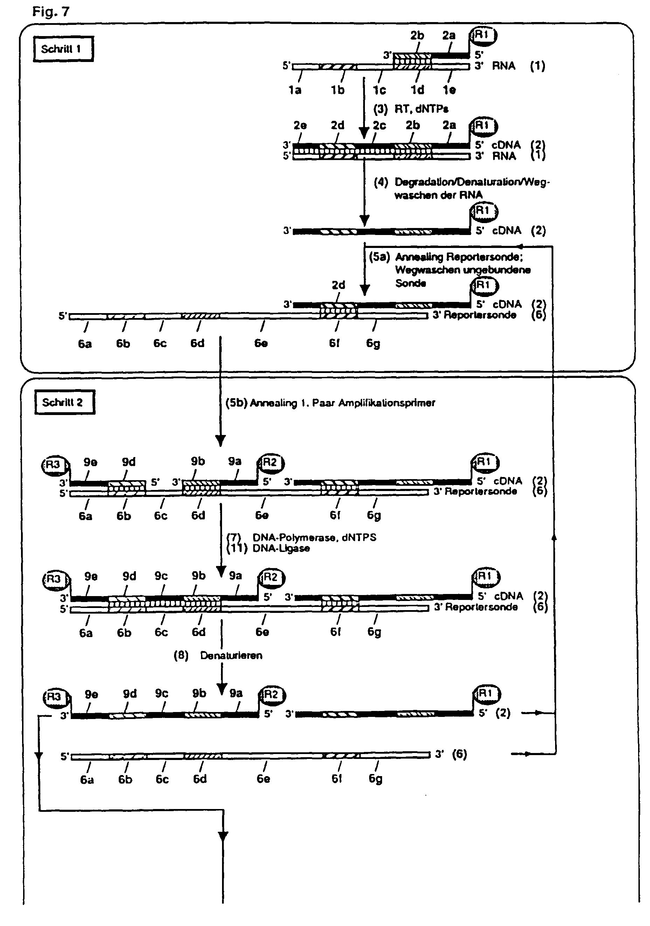

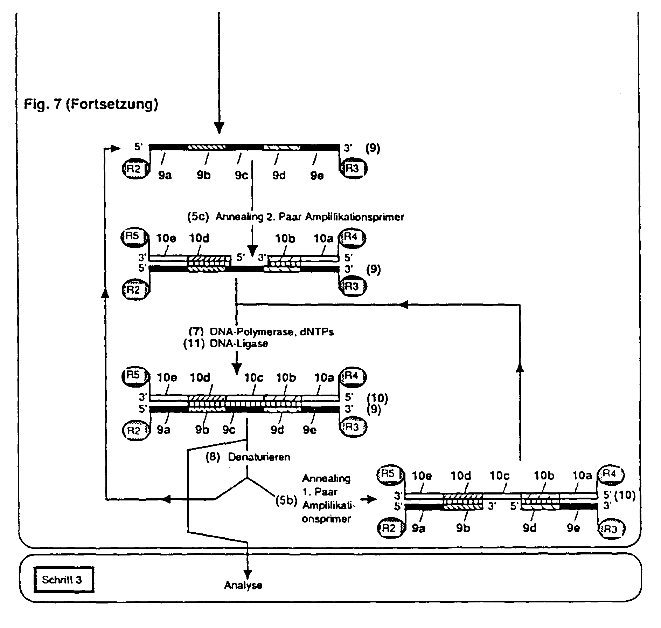

Fig. 6 und 7 zeigen den Reaktionsablauf des PERT Assays unter Einbezug dieses Amplifikationsverfahrens, in welchem sowohl Elemente der Ligase Chain Reaction als auch der Polymerase Chain Reaction verwendet werden. Bevorzugterweise wird dabei als Template-Nukleinsäure eine reine RNA verwendet; keinesfalls darf die in Schritt 2 zu vermehrende Sequenz der Template-Nukleinsäure aus DNA bestehen. An funktionellen Sequenzen werden Hybridisierungs-, Spacer- und flankierende Sequenzen verwendet.

**Fig. 6** zeigt eine Variante, bei der die zu vermehrende Nukleinsäure eine Teilsequenz der cDNA ist.

Schritt 1: Vorgelegt wird eine Template-Nukleinsäure, welche die funktionellen Sequenzen (1a) bis (1g) enthält. Die Template-Nukleinnsäure (1), der RT-Primer (R1)(2a)(2b) und die cDNA (2) sind bezüglich Struktur, Funktionszuteilung und Bezeichnung identisch mit denjenigen aus Fig. 2. Abweichend zu Fig. 2 ist die Spacer-Sequenz (1c) hier immer notwendig. Identisch sind auch die Reagentien und Reaktionsbedingungen für die RT-Reaktion und deren Produkt. Für das Funktionieren des PERT Assay müssen in dieser zumindest die funktionellen Sequenzen bis und mit (2f) synthetisiert werden.

Für einen optimalen Ablauf des PERT Assay wird zunächst die Template-Nukleinsäure in Reaktion (4) durch eine der erwähnten Methoden degradiert und als Reaktionspartner in den kommenden Reaktionen eliminiert. Dies ist wünschenswert, weil damit das Annealing des zweiten Oligonukleotidpaares an die Template-Nukleinsäure verhindert werden kann. Ein solches Annealing kann, je nach den gewählten Reaktionsbedingungen und Reagentien, zu falsch-positiven oder aber falsch-negativen Resultaten des PERT Assay führen: Falsch-positive Resultate entstehen dann, wenn die in den folgenden Reaktionen eingesetzte DNA-abhängige DNA-Polymerase auch eine RNA-abhängige DNA-Polymeraseaktivität besitzt und wenn gleichzeitig die verwendete Ligase die DNA-Fragmente auch dann ligieren kann, wenn sie in Form einer RNA//DNA Heteroduplex vorliegen. Eine Ligase mit solcher Funktion ist beispielsweise diejenige aus dem Bacteriophagen T4. Falsch-negative Resultate entstehen dann, wenn das an die Template-Nukleinsäure (1) gebundene zweite Oligonukleotidpaar durch die eingesetzte Ligase zwar nicht zu einem kohärenten DNA-Strang ligiert, aber doch infolge seiner Bindung an die Template-Nukleinsäure der Bindung an das ligierte erste Oligonukleotidpaar entzogen wird, so dass es zu einem suboptimalen Ablauf der PCR kommt.

Schritt 2: An die cDNA (2) wird nun in Reaktion (5) ein erstes Paar von DNA-Oligonulcleotidprimern anhybridisiert. Das am 3'-Ende des Primers (R2)(6a)(6b) gelegene obligatorische (6b) dient der Hybridisierung an (2f) der cDNA. Das am 5'-Ende gelegene (6a) ist fakultativ, besitzt keine Komplementarität zur cDNA und dient der Einführung einer neuen, in der cDNA noch nicht vorhandenen Sequenz. Auch (R2) ist fakultativ und bevorzugterweise verschieden von (R1). Der zweite der beiden Primer, (6d)(6e)(R3), hat dieselbe Polarität wie der erste und besteht aus einer obligatorischen, am 5'-Ende gelegenen Hybridisierungssequenz (6d), die komplementär zu (2d) der cDNA und am 5'-Ende phosphoryliert ist, sowie dem fakultativen (6e), das am 3'-Ende des Primers gelegen ist und der Einführung einer allfälligen weiteren Sequenz dient. Das fakultative (R3) ist bevorzugterweise verschieden von (R1).

Im Gegensatz zur LCR, wo die beiden Amplifikationsoligonukieotide eines Paars unmittelbar aneinander zu liegen kommen, sind sie hier durch die Spacer-Sequenz (2e) der cDNA voneinander getrennt. Die nach PS-WO 90/01069 bevorzugte Länge von (2e) beträgt nur wenige Nukleotide, doch funktioniert der PERT Assay auch bei längerem (2e). Durch eine DNA-abhängige DNA-Polymerase - es dürfen dafür nur DNA-Polymerasen ohne syntheseabhängige 5'->3' Exonucleaseaktivität verwendet werden - wird in Reaktion (7) unter Einbezug von dNTPs in einer "gap filling" Reaktion das zu (2e) komplementäre (6c) synthetisiert, das von Primer (6d)(6e)(R3) durch ein Nick getrennt bleibt. In Reaktion (11) wird eine DNA-Ligase (z.B. aus dem Bacteriophagen T4; mit Vorteil wird ein hitzestabiles Enzym verwendet) zur Ligation der beiden Fragmente eingesetzt, wodurch der kohärente DNA-Strang (6) geschaffen wird. In Reaktion (8) wird denaturiert, worauf Strang (2) wiederum für das Annealing des ersten Primerpaars zur Verfügung steht. Strang (6) wird für die weitere Amplifikation verwendet.

Zunächst erfolgt in Reaktion (5) das Annealing des zweiten Paars von DNA-Oligonukleotid-Primern. Die dafür verwendeten Hybridisierungssequenzen (9b) bzw. (9d) sind komplementär zu (6d) bzw. (6b) des ersten Primerpaars. Der erste Primer des 2. Primerpaars besteht aus den Elementen (R4)(9a)(9b), wobei wie erwähnt das am 3'-Ende dieses Primers gelegene obligatorische (9b) der Hybridisierung an (6d) des zweiten DNA-Strangs dient. Das am 5'-Ende gelegene (9a) ist fakultativ, nicht-komplementär zu (6e) und dient dazu, allenfalls erwünschte weitere Funktionen in das Amplifikationsprodukt einzubringen. (R4) ist fakultativ und bevorzugterweise verschieden von (R1). Der zweite Primer dieses Paars ist von derselben Polarität und besteht aus den funktionellen Sequenzen (9d)(9e)(R5). An seinem 5'-Ende ist er phosphoryliert. Das am 5'-Ende gelegene obligatorische (9d) ist komplementär zu (6b). Das am 3'-Ende gelegene (9e) ist fakultativ, nicht-komplementär zu (6a) und dient dazu, allenfalls erwünschte weitere Funktionen in das Amplifikationsprodukt einzubringen. (R5) ist fakultativ und bevorzugterweise verschieden von (R1). Dieses Primerpaar wird also an den DNA-Strang (6) anhybridisiert. Durch erneute Aktion der DNA-Polymerase und unter Verwendung von dNTPs wird in einer weiteren "gap filling" Reaktion die Lücke zwischen den beiden Primern geschlossen und die beiden Fragmente vermittelst der DNA-Ligase in Reaktion (11) zu Strang (9) verbunden. Dieser ist über die funktionellen Sequenzen (9b)(9c)(9d) mit Strang (6) zu einer partiellen Duplex verbunden. (9a) und (9e) haben dagegen keine Komplementarität zu (6e) bzw. (6a) von Strang (6). Nach erneuter Denaturierung (8) erfolgt wiederum das Annealing (5) der entsprechenden komplementären Primerpaare an die separierten Stränge (6) und (9); als Resultat dieser zyklischen Amplifikation entstehen grosse Mengen der partiellen Duplex (6)//(9).

Schritt 3: Dieses Produkt kann für die Analyse gemäss den später folgenden generellen Anmerkungen zu Schritt 3 herangezogen werden. Alternativ können, falls mittels (R) Markierungen eingesetzt wurden, einfache Detektionssysteme zum Nachweis von DNA-Molekülen, welche bestimmte neugebildete Kombinationen von funktionellen Gruppen (R) tragen, eingesetzt werden. Diese Kombinationen sind: (R2) und (R3), (R2) und (R4), (R5) und (R4), (R5) und (R3). Solche Detektionssysteme können auch hier beispielsweise auf ELISA-Basis oder auf der einfachen und kostengünstigen Partikelagglutination beruhen. Die mittels der Oligonukleotide eingeführten nichtkomplementären funktionellen Sequenzen (6a), (6e), (9a), und (9e) können, da die DNA in diesen Bereichen einzelsträngig ist, beim Nachweis mittels Hybridisierung Verwendung finden.

### Modifikationen:

Die als fakultativ benannten funktionellen Sequenzen und funktionellen Gruppen der eingesetzten Nukleinsäuren können, einzeln oder in den verschiedenen möglichen, Kombinationen, entfallen. Bei Wegfall aller vier (6a), (6e), (9a) und (9e) entsteht als Amplifikationsprodukt eine doppelsträngige DNA bestehend aus den Sequenzen (6b)(6c)(6d)//(8b)(8c)(8d). Die flankierenden Sequenzen (9a) bzw. (9e) können auch so gestaltet werden, dass sie komplementär zu (6e) bzw. (6a) sind.

Bei der Template-Nukleinsäure (1) können (1a) und (1g) beliebiger Sequenz sein, mit der einzigen Restriktion, dass sie nicht mit dem optimalen Ablauf des PERT in unzulässiger Art interferieren. Unter diesem Vorbehalt ist es auch möglich, natürlich vorkommende RNA-Moleküle als Template-Nukleinsäure (1) einzusetzen. Die Template-Nukleinsäure kann mehrere Hybridisierungssequenzen für RT-Primer aufweisen.

Abweichend von der Norm können die Hybridisierungssequenzen (1d) und (1f) zusammengelegt werden. In diesem Fall vermittelt (1d) vorerst das Annealing des nun auch als RT-Primer dienenden (R4)(9a)(9b) an die Template-Nukleinsäure (1). In Schritt 2 ist (R4)(9a)(9b) bekanntermassen einer der Amplifikationsprimer. Dadurch entfallen (1e) und (1f) der Template-Nukleinsäure (1). Ein separater RT-Primer (R1)(2a)(2b) erübrigt sich ebenfalls.

Als weitere Modifikation können dieHybridisierungssequenzen (1d) und (1f) so angelegt werden, dass sie sich teilweise überlappen. Dann entfällt die Spacer-Sequenz (1e) und die von dieser abgeleiteten Sequenzen.

Die Verwendung unterschiedlicher Sequenzen für das Annealing des RT-Primers und des 2. Amplifikationsprimers ermöglicht andererseits die Verwendung eines RNA-Primers; dadurch können Sensitivität und/oder Spezifität des PERT Assay beeinflusst werden. Abweichend von der Norm existiert die Möglichkeit, für verschiedene funktionelle Sequenzen identische Basensequenzen zu verwenden, diese können auch homopolymer sein. Dies betrifft die flankierenden Sequenzen (1a) und (1g) sowie die Spacer-Sequenzen (1c) und (1e) der Template-Nukleinsäure (1), die homopolymer und/oder untereinander identisch sein können.

Im weiteren ist es möglich, für zumindest zwei der Hybridisierungssequenzen (1b), (1d) und (1f) der Template-Nukleinsäure (1) ein und dieselbe, von den Spacer- und flankierenden Sequenzen verschiedene, Basensequenz zu verwenden. Als Beispiel sei die Situation, in welcher alle drei Hybridisierungssequenzen identisch sind, skizziert: Für den einen noch notwendigen Primer, bzw. auch für den zu ihm komplementären Primer, gilt dann, dass er ausschliesslich aus einer Hybridisierungssequenz bestehe und an seinem 5'-Ende phosphoryliert sei. Unter diesen Bedingungen binden maximal drei dieser Primermoleküle an die Template-Nukleinsäure (1); cDNA-Synthese erfolgt komplementär zu (1e), (1c) und (1a), und als Produkt der RT-Reaktion entstehen drei jeweils durch ein Nick getrennte, an die Template-Nukleinsäure gebundene Fragmente, (2b)(2c), (2d)(2e) und (2f)(2g). Diese Fragmente werden mittels der erwähnten DNA-Ligase zu (2b)(2c)(2d)(2e)(2f)(2g) ligiert. Diese Version lässt sich weiter dadurch vereinfachen, dass aus der Template-Nukleinsäure die funktionellen Sequenzen (1e) und (1f), wie oben beschrieben, eliminiert würden. In diesem Fall werden lediglich die cDNA-Fragmente (2d)(2e) und (2f)(2g) gebildet und mittels DNA-Ligase ligiert. Die Stränge (2b)(2d)(2e)(2f)(2g) wie auch (2d)(2e)(2f)(2g) würden dann unter Zuhilfenahme auch des zweiten Primers nach demselben Prinzip in einem Amplifikationsprozess gemäss PS-WO 90/01069 vermehrt.

**Fig. 7** zeigt eine Variante in welcher die in der RT-Reaktion synthetisierte cDNA nur zur spezifischen Anhybridisierung einer teilweise komplementären Reportersonde, welche die zu vermehrende Nukleinsäure darstellt, benutzt wird.

Schritt 1: Template-Primer-Kombination, RT-Reaktion (3) und deren Produkt sind in allen Aspekten identisch mit denjenigen aus Fig. 3. Das aus der RT-Reaktion (3) hervorgehende Gemisch von Nukleinsäuren wird in Reaktion (4) derart modifiziert, dass sichergestellt werden kann, dass die Template-Nukleinsäure (1) in den folgenden Reaktionen nicht als spezifischer Kompetitor für die Reportersonde (6) auftreten und dadurch zu falsch-negativen Resultaten führen kann. In Reaktion (4) wird daher mit einer der genannten Methoden die vorhandene Template-Nukleinsäure (1) als relevanter Teilnehmer an der Annealingreaktion (5) eliminiert. An die cDNA (2) wird sodann in Reaktion (5a) eine DNA-Reportersonde (6) anhybridisiert, welche die zu vermehrende Nukleinsäure darstellt oder enthält und aus (6a)(6b)(6c)(6d)(6e)(6f)(6g) besteht. Von diesen ist (6f) diejenige notwendige Hybridisierungssequenz, die mit dem in Reaktion (3) synthetisierten (2d) der cDNA hybridisiert. Alle anderen funktionellen Sequenzen der Reportersonde (6) hybridisieren nicht mit der cDNA (2) und besitzen keine Sequenzidentität mit Teilen von ihr. (6b) und (6d) sind ebenfalls notwendige Hybridisierungssequenzen für die Amplifikationsprimer. (6d) dient dem Annealing des ersten Amplifikationsprimers, die zu (6b) komplementäre Sequenz dient dem Annealing des zweiten Amplifikationsprimers.(6c) und (6e) haben Spacerfunktion; von den beiden ist nur (6c) notwendig. (6a) und (6g) sind fakultative flankierende Sequenzen.

Spätestens nach der Hybridisierung (5a) der Reportersonde (6) an die cDNA (2) wird die cDNA mittels der funktionellen Gruppe (R1) an einen Carrier gebunden und nichtgebundene Reportersonde unter nichtdenaturierenden Bedingungen vollständig aus dem Reaktionsgemisch entfernt, wie in Fig. 3 beschrieben.

Schritt 2: In einer weiteren Annealingreaktion (5b) wird in einem geeigneten Puffer das erste Paar Amplifikationsoligonuklotidprimer, (R2)(9a)(9b) und (9d)(9e)(R3), zugegeben. Diese zwei Primer sind in (9b) bzw. (9d) vollständig komplementär zu (6d) bzw. (6b). (9d)(9e)(R3) ist ausserdem am 5'-Ende phosphoryliert. (R2) bzw. (R3) sind fakultativ und bevorzugterweise von (R1) verschieden. In Reaktion (7) wird dann vermittelst der DNA-abhängigen DNA-Polymerase unter Verwendung von dNTPs die Lücke zwischen den beiden Primern geschlossen und in Reaktion (11) die beiden Fragmente, (R2)(9a)(9b)(9c) und (9d)(9e)(R3), mittels der DNA-Ligase zu einem einzigen Strang (9) vereint. In Reaktion (8) wird denaturiert, worauf die Reportersonde (6) wiederum für das Annealing der beiden Oligonukleotide (R2)(9a)(9b) und (9d)(9e)(R3) zur Verfügung steht. Strang (9) wird für die weitere Amplifikation verwendet.

Zunächst erfolgt in Reaktion (5c) das Annealing des zweiten Paars von DNA-Oligonukleotid-Primern. Der erste Primer des 2. Primerpaars besteht aus (R4)(10a)(10b), wobei das am 3'-Ende gelegene obligatorische (10b) der Hybridisierung an (9d) dient. Das am 5'-Ende gelegene (10a) ist fakultativ und dient dazu, allenfalls erwünschte weitere Funktionen in das Amplifikationsprodukt einzubringen. Es gelten dafür dieselben Restriktionen wie für (2a) oder (9a). (R4) ist fakultativ und bevorzugterweise von (R1) verschieden und kein Carrier. Der zweite Primer (10d)(10e)(R5) dieses Paares ist von derselben Polarität und an seinem 5' -Ende phosphoryliert. Das am 5'-Ende gelegene (10d) ist komplementär zu (9b). Das am 3'-Ende gelegene fakultative (10e) dient wiederum der Einführung einer weiteren funktionellen Sequenz. (R5) ist eine weitere fakultative funktionelle Gruppe, bevorzugterweise von (R1) verschieden und nicht Carrier.

Dieses Primerpaar wird an den DNA-Strang (9) anhybridisiert. Durch erneute Aktion der DNA-Polymerase und unter Verwendung von dNTPs wird in einer weiteren "gap filling" Reaktion die Lücke zwischen den beiden Primern geschlossen und die beiden Fragmente in Reaktion (11) vermittelst der DNA-Ligase zu Strang (10) verbunden. Dieser ist über die funktionellen Sequenzen (10b)(10c) (10d) mit Strang (9) zu einer partiellen Duplex verbunden. (10a) und (10e) haben dagegen keine Komplementarität zu (9e) bzw. (9a) von Strang (9). Nach erneuter Denaturierung (8) erfolgt wiederum das Annealing (5) der entsprechenden komplementären Primerpaare an die separierten Stränge (9) und (10); als Resultat dieser zyklischen Amplifikation entstehen grosse Mengen der partiellen Duplex (9)//(10).

Schritt 3: Dieses Produkt kann für die Analyse gemäss den später folgenden generellen Anmerkungen zu Schritt 3 herangezogen werden. Alternativ können, falls mittels (R) Markierungen eingeführt wurden, einfache Detektionssysteme zum Nachweis von DNA-Molekülen, welche bestimmte Kombinationen von (R) tragen, eingesetzt werden. Diese Kombinationen sind: (R2) und (R3), (R2) und (R4), (R5) und (R4), (R5) und (R3).Solche Detektionssysteme können auch hier auf ELISA-Basis oder auf der einfachen und kostengünstigen Partikelagglutination beruhen. Die mittels der Oligonukleotide eingeführten nichtkomplementären funktionellen Sequenzen (9a), (9e), (10a), und (10e) können, da die DNA in diesen Bereichen einzelsträngig ist, beim Nachweis mittels Hybridisierung Verwendung finden.

### Modifikationen:

In der Template-Nukleinsäure (1) können mehrere Hybridisierungssequenzen (1b) für Reportersonden, bevorzugterweise durch jeweils eine kurze Spacer-Sequenz voneinander getrennt, plaziert werden. Die Hybridisierungssequenzen brauchen nicht identisch zu sein, so dass verschiedene Reportersonden eingesetzt werden können. Ebenso können mehrere Hybridisierungssequenzen für RT-Primer eingesetzt werden.

Die als fakultativ benannten funktionellen Sequenzen und funktionellen Gruppen in den eingesetzten Nukleinsäuren können einzeln oder in den verschiedenen möglichen Kombinationen entfallen. In der Template-Nukleinsäure (1) sind dies die flankierenden Sequenzen (1a) und (1e) sowie die Spacer-Sequenz (1c). Bei der Reportersonde (6) können die flankierenden Sequenzen (6a) und (6g), sowie die Spacer-Sequenz (6e) entfallen. Bei den verschiedenen Primern kann auf die nicht-komplementären Sequenzen (2a), (9a), (9e), (10a) und (10e) verzichtet werden. Wenn auf alle vier von (9a), (9e), (10a) und (10e) verzichtet wird, entsteht als Amplifikationsprodukt eine doppelsträngige DNA bestehend aus (9b)(9c) (9d)//(10b)(10c)(10d). Die flankierenden Sequenzen (10a) bzw. (10e) des 2. Paares von Amplifikationsprimern können so gestaltet werden, dass sie komplementär zu (9e) bzw. (9a) sind. Es kann auch auf eine, mehrere oder alle der funktionellen Gruppen (R2)(R3)(R4)(R5) verzichtet werden. Auf (R1) als Bestandteil des RT-Primers kann dann verzichtet werden, wenn die Bedingungen für die RT-Reaktion so gewählt werden, dass dabei mit Liganden markierte Deoxyribonukleotidmonophosphate in die neusynthetisierte cDNA eingebaut werden; diese funktionellen Gruppen können dann im Sinne von (R1) die cDNA an einen Carrier binden.

Bei der Reportersonde (6) können (6a) und (6g) beliebige Sequenz aufweisen. So ist es möglich, natürlich vorkommende lineare oder ringförmige DNA-Moleküle als Reportersonde einzusetzen.

Es ist ferner möglich, die zu amplifizierende Region der Reportersonde (6) nicht upstream, sondern downstream der cDNA-bindenden Hybridisierungssequenz (6f) zu plazieren, so dass sie in den Bereich von (6g) zu liegen kommt. Die zu amplifizierende Region kann (6f) auch einschliessen oder mit diesem überlappen; dies letztere ist allerdings keine bevorzugte Konstellation.

Abweichend von der Norm kann eine funktionelle Sequenz in gewissen Situationen mehr als eine Funktion aufweisen. Beispielsweise können die Hybridisierungsfunktionen von (6d) und (6f) zusammengelegt werden. In diesem Fall wird in Reaktion (5a) Sequenz (6d) vorerst das Annealing der Reportersonde an die cDNA vermitteln (dies bedingt natürlich auch eine entsprechend modifizierte Template-Nukleinsäure!) und dann, nach dem Wegwaschen der ungebundenen Reportersonde und einem in diesem Fall notwendig werdenden Denaturierungsschritt, auch den Primer (R2)(9a)(9b) binden. Beim Weglassen der Sequenz (1a) der Template-Nukleinsäure ist Denaturierung nicht notwendig. Infolge der Zusammenlegung dieser beiden Funktionen auf (6d) erübrigen sich die funktionellen Sequenzen (6e) und (6f) der Reportersonde. Als weitere Modifikation können diese Hybridisierungsfunktionen so angelegt werden, dass die sie ausübenden Sequenzen (6d) und (6f) sich teilweise überlappen. Dann entfällt die Spacer-Sequenz (6e) und die von dieser abgeleiteten Sequenzen.

Die Möglichkeit der teilweisen, nur wenige Nukleotide umfassende Ueberlappung existiert schliesslich auch für die funktionellen Sequenzen (1b) und (1d) der Template-Nukleinsäure (1), solange die Reportersonde dadurch nicht an (2b) hybridisiert.

Abweichend von der Norm können für verschiedene funktionelle Sequenzen identische Basensequenzen verwendet werden, diese können auch homopolymer sein. Dies betrifft die flankierenden Sequenzen (1a) und (1e) sowie die Spacer-Sequenz (1c) der Template-Nukleinsäure, die homopolymer und/oder untereinander identisch sein können. Homopolymer und/oder untereinander identisch können auch die flankierenden Sequenzen (6a) und (6g) sowie die Spacer-Sequenzen (6c) und (6e) der Reportersonde (6) sein.

Im weiteren ist es möglich, für zumindest zwei der Hybridisierungssequenzen (6b), (6d) und (6f) der Reportersonde (6) ein und dieselbe, von den Sequenzen der Spacer- und flankierenden Sequenzen verschiedene, Basensequenz zu verwenden. Als Beispiel sei die Situation, in welcher alle drei Hybridisierungssequenzen identisch sind, skizziert: Für den einen noch notwendigen Primer, bzw. auch den zu ihm komplementären Primer, gilt dann, dass er ausschliesslich aus einer Hybridisierungssequenz bestehe und an seinem 5'-Ende phosphoryliert sei. Unter diesen Bedingungen hybridisiert in Reaktion (5a) entweder (6b), (6d) oder (6f) an die Hybridisierungssequenz (2d) der cDNA. Nach Waschen und Denaturation binden in Reaktion (5b) maximal drei der genannten Primermoleküle an die Reportersonde (6); DNA-Synthese (7) erfolgt komplementär zu (6e), (6c) und (6a). Als Produkt entstehen drei jeweils durch ein Nick getrennte, an die Reportersonde gebundene Fragmente, (6b)(6c), (6d)(6e) und (6f)(6g), die durch Zugabe der Ligase (11) ligiert werden. Diese Version würde weiter dadurch vereinfacht, dass aus der Reportersonde die funktionellen Sequenzen (6e) und (6f), wie oben beschrieben, deletiert würden. In diesem Fall würden nur zwei statt der drei Fragmente entstehen, ligiert und weiter amplifiziert.

### Aufbau des PERT Assay bei Verwendung eines auf Transcription basierenden Amplifikationsverfahrens

Auch bei diesen PERT-Varianten wird durch die im Untersuchungsmaterial vorhandene RT-Aktivität eine cDNA aufgrund einer geprimten Template-Nukleinsäure synthetisiert. Falls nicht anders vermerkt, wird dabei bevorzugterweise eine reine RNA als Template-Nukleinsäure verwendet. Keinesfalls darf die in Schritt 2 zu vermehrende Sequenz der Template-Nukleinsäure aus DNA bestehen. Für das Verfahren ist wesentlich, dass nach der RT-Reaktion (3a) die Template-RNA eliminiert wird. Die zu vermehrende Nukleinsäure ist in der cDNA enthalten oder sie wird in weiteren Reaktionen, ausgehend von der cDNA, synthetisiert. Dazu wird eine zumindest partiell doppelsträngige DNA mit einem funktionellen Promotor für eine DNA-abhängige RNA-Polymerase (z.B. des Bacteriophagen T7, T3 oder SP6) geschaffen, von welcher anschliessend eine RNA synthetisiert wird.

Für den Aufbau der promotorhaltigen zumindest partiell doppelsträngigen DNA gibt es verschiedene Möglichkeiten. Der Transcriptionspromotor kann entweder in der Nähe des 5'-Endes der cDNA oder in der Nähe des 5'-Endes des 2. DNA-Strangs plaziert werden. In beiden Fällen kann die Sequenzinformation für den Promotor bereits in der Template-Nukleinsäure angelegt sein, oder sie kann über einen Primer eingeführt werden. Wird der RT-Primer dafür benützt, kommt der Promotor in die Nähe des 5'-Endes der cDNA zu liegen. Wird hingegen der Primer für den 2. DNA-Strang dazu benützt, kommt der Promotor in die Nähe des 5'-Endes des 2. DNA-Strangs zu liegen.

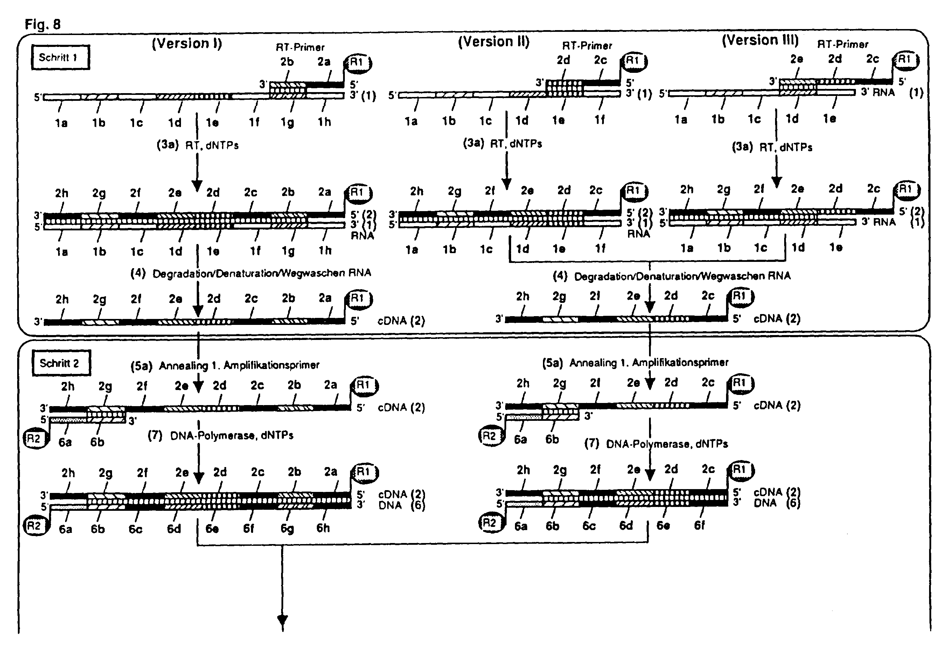

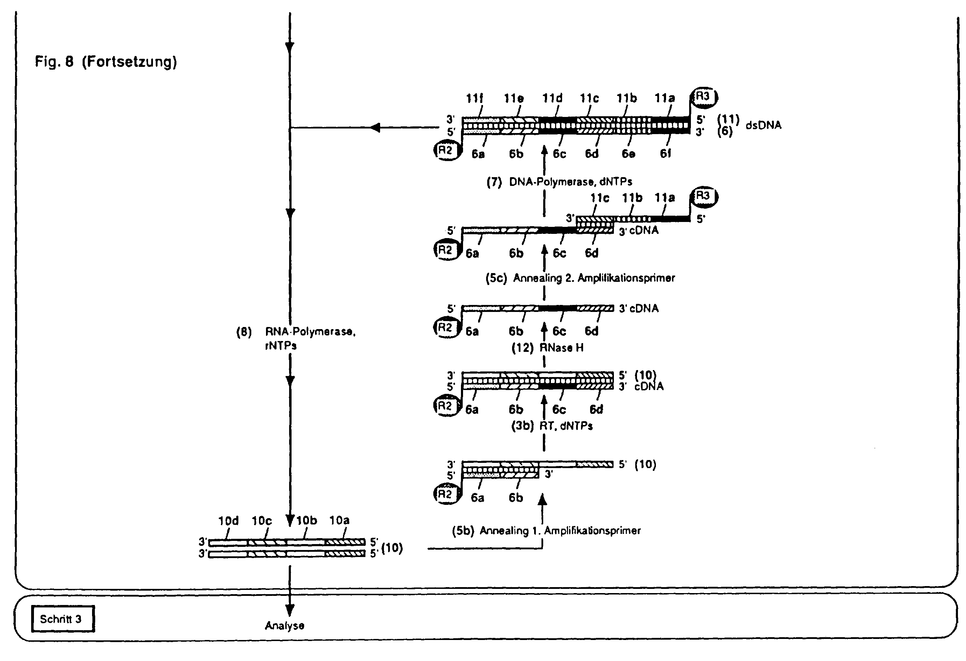

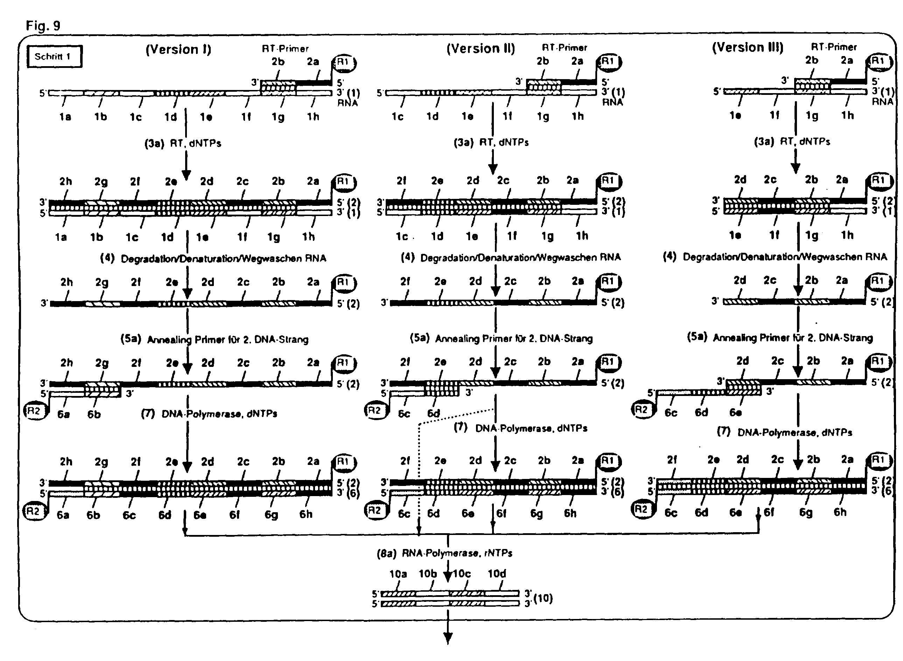

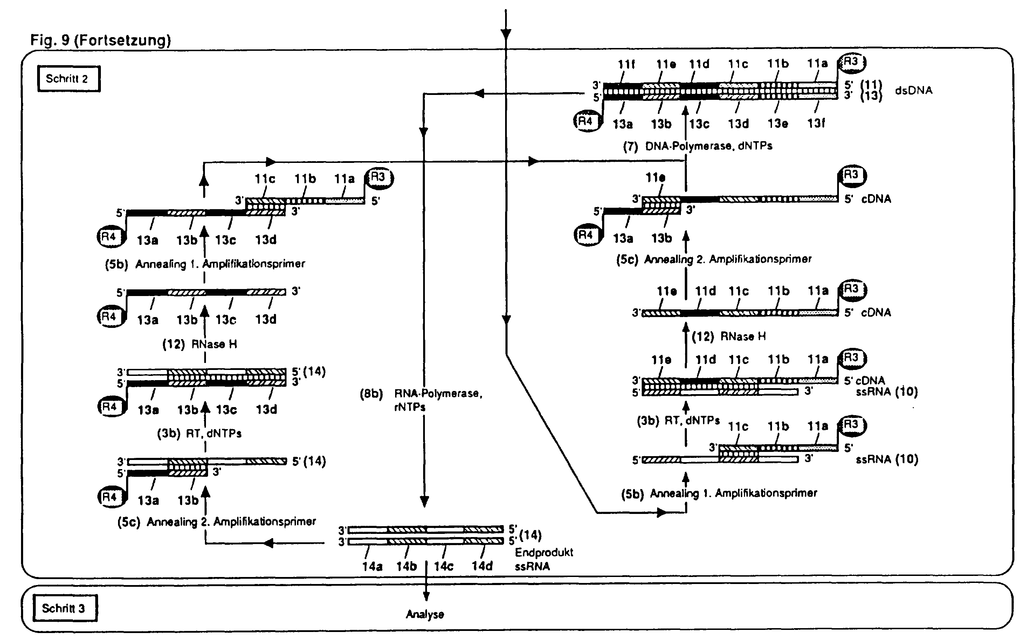

Prototypen dieser zwei Typen von PERT Assays sind in den Fig. 8 und 9 beschrieben.

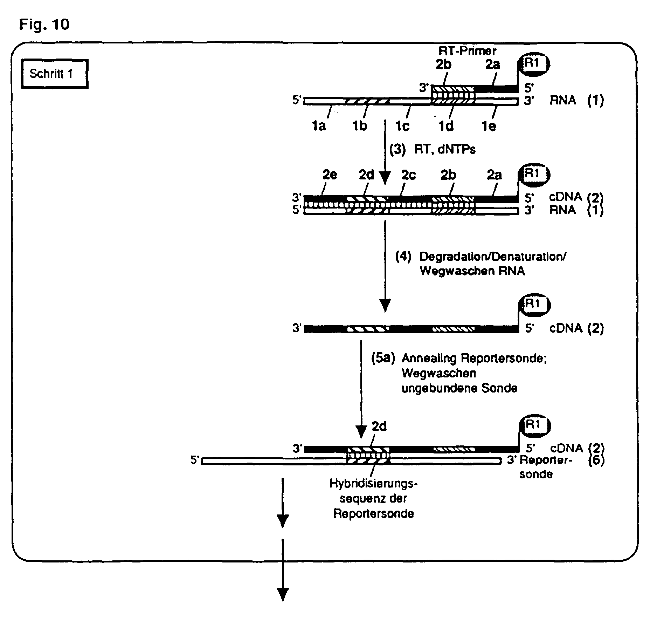

Wie bei den vorgängig beschriebenen Varianten des PERT Assay ist es auch hier möglich, anstelle der cDNA einen Teil einer zu dieser teilweise komplementären Reportersonde für die Amplifikation zu verwenden. Die Reportersonde kann entweder aus DNA oder RNA bestehen (Fig. 10). Ebenso kann eine von einer DNA-Reportersonde abgeleitete RNA die zu vermehrende Nukleinsäure enthalten.

In allen Fällen erfolgt die Amplifikation der zu vermehrenden Nukleinsäure mit einem multienzymatischen Verfahren gemäss PS-EP 0408295, WO 89/01050 und WO 88/10315, das bei PS-EP 0408295, nicht aber den anderen beiden, isotherm verläuft.

**Fig. 8** zeigt verschiedene Versionen des PERT Assay, in denen die in der RT-Reaktion synthetisierte cDNA die zu vermehrende Nukleinsäure enthält. In all diesen Versionen enthält die in Reaktion (3a) synthetisierte cDNA in der Nähe des 5'-Endes die P(-)-Sequenz eines Transcriptionspromotors. Die Transcriptionspromotorsequenz kann dabei entweder bereits in der Template-Nukleinsäure enthalten sein (Versionen I und II) oder/und mittels des RT-Primers zugeführt werden (Versionen II und III). In die Template-Nukleinsäure integriert, entspricht sie der (P+)-Sequenz des Promotors. Als Bestandteil des RT-Primers entspricht sie der (P-)-Sequenz.

Schritt 1: Version 1: Vorgelegt wird eine Template-Nukleinsäure, welche die funktionellen Sequenzen (1a)(1b)(1c)(1d)(1e)(1f)(1g)(1h) enthält. Von diesen sind (1b), (1d) und (1g) notwendige Hybridisierungssequenzen. Ebenfalls notwendig ist (1e), welches für den Promotor für die gewählte DNA-abhängige RNA-Polymerase kodiert. Fakultativ sind die Spacer-Sequenzen (1c) und (1f) und die flankierenden Sequenzen (1a) und (1h). Von den drei Hybridisierungssequenzen dient (1g) dem Annealing des RT-Primers. Die Hybridisierungssequenz (1b) dient in ihrer komplementären Form (2g) in Schritt 2 dem Annealing des ersten Amplifikationsprimers für die Synthese des 2. DNA-Strangs sowie als (10c) dem Annealing ersten Amplifikationsprimers zur Amplifikation. (1d) bzw. das ihm entsprechende (6d) dient in Schritt 2 dem Annealing des zweiten Amplifikationsprimers.

Der RT-Primer (R1)(2a)(2b) bindet mittels der notwendigen Hybridisierungssequenz (2b) an (1g) der Template-Nukleinsäure. Das am 5'-Ende gelegene (2a) ist fakultativ und dient, falls der RT-Primer ein DNA-Oligonukleotid ist, der Einführung einer neuen, in der Template-Nukleinsäure noch nicht vorhandenen Sequenz. (R1) ist fakultativ.

Bei Anwesenheit von RT-Aktivität im aufgearbeiteten Untersuchungsmaterial wird in der Reaktion (3a) unter Verwendung von dNTPs eine cDNA (2) synthetisiert, welche maximal die funktionellen Sequenzen (2a) bis (2h) enthält. Für das Funktionieren des PERT Assay müssen in der RT-Reaktion zumindest die Sequenzen bis und mit (2g) synthetisiert werden.

Version II: Vorgelegt wird eine Template-Nukleinsäure bestehend aus (1a)(1b)(1c)(1d)(1e)(1f); im Vergleich zu Version I sind (1g) und (1h) deletiert. (1b), (1d) und (1e) sind notwendige Hybridisierungssequenzen. (1e) enthält zudem die in RNA kodierte Sequenz des Transcriptionspromotors; die dem Primer-Annealing dienende Sequenz kann zu einer oder zu beiden Seiten über die den Promotor kodierende Sequenz hinausreichen. Fakultativ sind die Spacer-Sequenz (1c) sowie die flankierenden Sequenzen (1a) und (1f). Von den drei Hybridisierungssequenzen dient (1e) dem Annealing des RT-Primers. (1b) und (1d) haben dieselbe Funktion wie in Version I. Entsprechend der im Vergleich zu Version I verkürzten Template-Nukleinsäure ist der RT-Primer, ein DNA-Oligonukleotid (R1)(2c)(2d), verschieden von dem in Version I verwendeten. Das am 3'-Ende gelegene notwendige (2d) vermittelt das Annealing an (1e) der Template-Nukleinsäure (1) und enthält zudem die (P-)-Sequenz des Transcriptionspromotors. Die dem Annealing dienende Sequenz von (2d) kann, wie bei (1e), zu einer oder zu beiden Seiten über die Promotorsequenz hinausreichen. Das am 5'-Ende gelegene (2c) ist fakultativ und hat die Funktion von (2a) in Version I. (R1) ist fakultativ. Als Produkt der RT-Reaktion (3a) entsteht eine cDNA (2) bestehend aus maximal den funktionellen Sequenzen (2c) bis (2h). Für das Funktionieren des PERT Assay müssen in der RT-Reaktion zumindest die Sequenzen bis und mit (2g) synthetisiert werden.

Version III: Vorgelegt wird eine Template-Nukleinsäure, welche die funktionellen Sequenzen (1a)(1b)(1c)(1d)(1e) enthält; im Vergleich zu Version II ist also auch noch die für den Transcriptionspromotor kodierende Sequenz deletiert. (1b) und (1d) sind notwendige Hybridisierungssequenzen. Fakultativ sind die Spacer-Sequenz (1c) sowie die flankierenden Sequenzen (1a) und (1e). Von den zwei Hybridisierungssequenzen dient (1d) dem Annealing sowohl des RT-Primers als auch des 2. Amplifikationsprimers. (1b) hat dieselbe Funktion wie in Version I. Die in der Template-Nukleinsäure fehlende Transcriptionspromotor-Sequenz wird mittels des RT-Primers zugeführt. Dieser, ein DNA-Oligonukleotid (R1)(2c)(2d)(2e) hat eine am 3'-Ende gelegene, notwendige Hybridisierungssequenz (2e), welche das Annealing an (1d) der Template-Nukleinsäure (1) vermittelt. Unmittelbar upstream davon enthält (2d) die (P-)-Sequenz des Transcriptionspromotors. Das am 5'-Ende gelegene (2c) ist wie in Version II. (R1) ist fakultativ. Als Produkt der RT-Reaktion (3a) entsteht eine cDNA (2) bestehend aus maximal den funktionellen Sequenzen (2c) bis (2h). Für das Funktionieren des PERT Assay müssen in der RT-Reaktion zumindest die Sequenzen bis und mit (2g) synthetisiert werden.

Alle Versionen: Zunächst wird bei allen drei Versionen die aus RNA bestehende Tempiate-Nukleinsäure (1) in Reaktion (4) durch eine der erwähnten Methoden degradiert und damit als Reaktionspartner in den kommenden Reaktionen eliminiert. Die durch diese Massnahmen nicht betroffene einzelsträngige cDNA (2) enthält in den Sequenzen (2e) bis (2g) die zu vermehrende Nukleinsäure.

Schritt 2: Die cDNA (2) wird sodann in eine Reaktionsmischung gemäss den Angaben der eingangs aufgeführten Patentschriften überführt, welche mehrere Enzyme enthält, nämlich die dem gewählten Promotor entsprechende DNA-abhängige RNA-Polymerase, eine DNA-abhängige DNA-Polymerase sowie eine RNA-abhängige DNA-Polymerase (RT). Falls diese letztere ungenügende RNase H-Wirkung aufweist, kann zusätzlich noch RNase H zugegeben werden. Zudem muss vor oder mit dem Ueberführen in die Reaktionmischung die Stabilität von RNA durch eine der erwähnten Methoden wieder garantiert werden.

An die cDNA (2) wird sodann bei allen Versionen in Reaktion (5a) der erste Amplifikationsprimer, ein DNA-Oligonukleotid (R2) (6a)(6b), anhybridisiert. (R2) ist bevorzugterweise verschieden von (R1) und nicht ein Carrier. Die am 3'-Ende des Primers gelegene, obligatorische Hybridisierungssequenz (6b) hat in allen Versionen Komplementarität zu (2g) der cDNA (2). Das am 5'-Ende gelegene fakultative nichtkomplementäre (6a) dient der Einführung einer neuen, in der cDNA noch nicht vorhandenen Sequenz. Durch die in der Reaktionsmischung vorliegende DNA-abhängige DNA-Polymerase und unter Verwendung von dNTPs wird in Reaktion (7) der 2. DNA-Strang synthetisiert; als Resultat entsteht eine partiell doppelsträngige DNA (2)//(6). Für Version I besteht diese aus (R1)(2a) bis (2h)//(R2)(6a) bis (6h), für Versionen II und III dagegen aus (R1)(2c) bis (2h)//(R2)(6c) bis (6h).

Von der in die Reaktionsmischung eingegebenen dsDNA (2)//(6), die in (2d)//(6e) aller drei Versionen einen funktionellen Promotor für die gewählte RNA-Polymerase aufweist, erfolgt in Reaktion (8) vermittelst der DNA-abhängigen RNA-Polymerase und unter Verwendung von rNTPs die Synthese einer ssRNA-Spezies (10) enthaltend (10a)(10b)(10c)(10d). Diese ssRNA (10) hat in allen drei Versionen die Polarität der cDNA; (10a),(10b) und (10c) sind komplementär zu (1d), (1c) und (1b) der verschiedenen Template-RNAs (1). (10d) ist hingegen von (6a) abgeleitet.

In den anschliessenden Reaktionen erfolgt, ausgehend von der ssRNA (10) erneut der Aufbau einer transcriptionsfähigen dsDNA (11)//(6) durch eine RT-Reaktion. In Reaktion (5b) erfolgt zunächst das Annealing des 1. Amplifikationsprimers (R2)(6a)(6b) an die ssRNA (10). Dabei dient die gesamte DNA-Sequenz des Oligonukleotids (R2)(6a)(6b) dem Annealing.

Vermittelst der in der Reaktionsmischung vorhandenen RT wird in Reaktion (3b) unter Verwendung von dNTPs die cDNA (R2)(6a)(6b) (6c)(6d) synthetisiert. Durch die RNase H-Wirkung der RT oder durch Einwirkung nötigenfalls zusätzlich beigefügter RNase H wird in Reaktion (12) die RNA (10) von der cDNA-RNA-Heteroduplex soweit degradiert, dass in Reaktion (5c) das Annealing des zweiten Amplifikationsprimers (R3)(11a)(11b)(11c) an (6d) der cDNA erfolgen kann. Dieser Amplifikationsprimer besteht an seinem 3'-Ende aus einer obligatorischen, zu (6d) komplementären Hybridisierungssequenz (11c). Upstream davon befindet sich die ebenfalls obligatorische funktione le Sequenz (11b) für die (P-)-Sequenz des Promotors der RNA-Polymerase; am 5'-Ende ist eine fakultative flankierende Sequenz (11a). (R3) ist ebenfalls fakultativ.

Durch erneute Einwirkung der DNA-abhängigen DNA-Polymerase und unter der Verwendung von dNTPs entsteht in Reaktion (7) die dsDNA (11)//(6). Dabei enthält Strang (11) die Elemente (R3)(11a) bis (11f) und Strang (6) die Elemente (R2)(6a) bis (6f). Diese dsDNA besitzt in (11b)//(6e) wiederum einen funktionellen Promotor für die RNA-Polymerase, wodurch in Reaktion (8) erneut die ssRNA (10a)(10b)(10c)(10d) synthetisiert wird. Die ssRNA (10) wird wiederum in den Amplifikationszyklus eingeschleust und stellt zugleich das hauptsächliche Produkt von Schritt 2 dar.

Schritt 3: Die aus dem Amplifikationsprozess resultierende ssRNA (10a)(10b)(10c)(10d) wird für die Analyse gemäss den später folgenden generellen Anmerkungen zu Schritt 3 verwendet.

### Modifikationen:

Die als fakultativ benannten funktionellen Sequenzen und funktionellen Gruppen (R) der verschiedenen eingesetzten Nukleinsäuren können, einzeln oder in den verschiedenen möglichen Kombinationen, entfallen. Verzicht auf die am 5'-Ende des RT-Primers situierte funktionelle Sequenz [(2a) bei Version I; (2c) bei Versionen I und II] hat keine Auswirkung auf die Zusammensetzung der in Schritt 2 produzierten ssRNA. Verzicht auf Sequenz (6a) des ersten Amplifikationsprimers hat zur Folge, dass die am 3'-Ende der ssRNA (10) liegende Sequenz (10d) fehlt. Verzicht auf (1a) der Template-Nukleinsäure und/oder (11a) des 2. Amplifikationsprimers hat hingegen keinen Einfluss auf die Zusammensetzung der ssRNA (10). In Reaktion (4) kann das Vorhandensein von (R1) vorteilhaft sein.

Eine zusätzliche Spacer-Sequenz kann in der Template-Nukleinsäure zwischen den Elementen (1d) und (1e), im RT-Primer zwischen (2d) und (2e), und/oder im 2. Amplifikationsprimer zwischen (11b) und (11c) eingeschoben werden.

In Version III können bei der Template-Nukleinsäure (1) beide flankierenden Sequenzen beliebige Basensequenzen aufweisen; dies ermöglicht, natürlich vorkommende RNA-Moleküle als Template-Nukleinsäure (1) einzusetzen.

Abweichend von der Norm kann eine funktionelle Sequenz, oder eine Kombination von funktionellen Sequenzen, mehr als einer Funktion dienen. So kann in allen Versionen der 2. Amplifikationsprimer (R3)(11a)(11b)(11c) auch die Funktion des RT-Primers übernehmen. Das Annealing des RT-Primers, vermittelst der Hybridisierungssequenz (11c), erfolgt dann an die Hybridisierungssequenz (1d) der Template-Nukleinsäure (1). Eine Template-Nukleinsäure (1), welche gegen das 3'-Ende die Sequenzen bis und mit (1d) umfasst, ist unter diesen Umständen ausreichend für das Funktionieren des PERT Assay.

Die Verwendung verschiedener Moleküle für den RT-Primer und den 2. Amplifikationsprimer ermöglicht andererseits die Verwendung eines RNA-Primers für die RT-Reaktion, wodurch Sensitivität und/oder Spezifität des PERT Assay beeinflusst werden können.

Abweichend von der Norm existiert die Möglichkeit, für verschiedene funktionelle Sequenzen identische Basensequenzen zu verwenden, diese können auch homopolymer sein. Dies betrifft in allen Versionen die flankierenden oder Spacer-Sequenzen der Template-Nukleinsäure (1), die homopolymer und/oder untereinander identisch sein können.

Im Weiteren ist es möglich, diese Versionen derart zu modifizieren, dass der Transcriptionspromotor gegen das 5'-Ende des 2. DNA Strangs hin zu liegen kommt. Die in Schritt 2 synthetisierte ssRNA (10) weist dann die gleiche Polarität wie die Template-Nukleinsäure (1) auf. Die Transcriptionspromotorsequenz kann als P(-)-Sequenz über die Template-Nukleinsäure eingebracht werden, wobei diese upstream der Hybridisierungssequenz (1b) liegen muss. Immer jedoch muss der zur Synthese des 2. DNA Stranges eingesetzte Amplifikationsprimer upstream der am 3'-Ende gelegenen Hybridisierungssequenz die P(-)-Sequenz eines Transcriptionspromotors enthalten. Wird die Transcriptionspromotorsequenz ausschliesslich über den Primer zur Synthese des 2. DNA-Strangs eingebracht, kann als Template-Nukleinsäure eine natürliche RNA verwendet werden. Um die partiell doppelsträngige DNA mit einem funktionelle Promotor aufzubauen, ist es in diesem Fall jedoch notwendig, dass nach der Synthese des 2. DNA-Strangs die DNA denaturiert wird, um durch Annealing des 1. Amplifikationsprimers umd erneuter DNA-Synthese einen funktionellen Promotor aufzubauen. In diesem Fall stellt die durch die RT-Aktivität synthetisierte cDNA (2) nur das Edukt zur Synthese der zu vernehrenden Nukleinsäure dar. Die in Schritt 2 synthetisierte ssRNA (10) weist dann die gleiche Polarität wie die Template-Nukleinsäure (1) auf.

**Fig. 9** beschreibt verschiedene Versionen des PERT Assay, in welchen die zu vermehrende Nukleinsäure eine RNA ist. Der zur Synthese dieser RNA notwendige Transcriptionspromotor kommt in allen illustrierten Versionen in die Nähe des 5'-Endes des zweiten DNA-Stranges zu liegen. Die Transcriptionspromotorsequenz kann dabei bereits als (P-)-Sequenz in der Template-Nukleinsäure enthalten sein (Versionen I und II), oder sie kann, ebenfalls der (P-)-Sequenz entsprechend, über den Primer für den zweiten DNA-Strang zugeführt werden.

Schritt 1: Version I: Vorgelegt wird eine Template-Nukleinsäure, welche die funktionellen Sequenzen (1a) bis (1h) enthält. Davon sind (1b), (1e) und (1g) notwendige Hybridisierungssequenzen. Ebenfalls notwendig ist (1d), welches für den Transcriptionspromotor kodiert. Fakultativ sind die Spacer-Sequenzen (1c) und (1f) sowie die flankierenden Sequenzen (1a) und (1h). Von den drei Hybridisierungssequenzen dient (1g) dem Annealing des RT-Primers und in Schritt 2 die davon abgeleitete Sequenz (13d) dem Annealing des ersten Amplifikationsprimers. (1b) dient in seiner komplementären Form (2g) dem Annealing des Primers für den 2. DNA-Strang in Schritt 1. (1e) dient in seiner komplementären Form (14b) in Schritt 2 dem Annealing des zweiten Amplifikationsprimers. Der RT-Primer (R1)(2a)(2b) ist ein RNA- oder DNA-Oligonukleotid. Das am 3'-Ende gelegene, notwendige (2b) vermittelt das Annealing an (1g) der Template-Nukleinsäure. Die funktonelle Gruppe (R1) ist fakultativ. Bei Anwesenheit von RT-Aktivität im aufgearbeiteten Untersuchungsmaterial wird in Reaktion (3a) unter Verwendung der benötigten dNTPs eine cDNA (2) synthetisiert, welche maximal die funktionellen Sequenzen (2a) bis (2h) enthält. Für das Funktionieren des PERT Assay müssen in der RT-Reaktion (3a) zumindest die Sequenzen bis und mit (2g) synthetisiert werden.

Version II: Vorgelegt wird eine Template-Nukleinsäure, welche (1c)(1d)(1e)(1f)(1g)(1h) enthält; im Vergleich zu Version I sind (1a) und (1b) deletiert. Bezüglich der Funktion der verbleibenden Sequenzen gilt das unter Version I Gesagte, mit der Ausnahme, dass (1c) jetzt flankierende Sequenz ist. Auch der RT-Primer (R1)(2a) (2b) ist wie in Version I. Bei Anwesenheit von RT-Aktivität im aufgearbeiteten Untersuchungsmaterial wird in Reaktion (3a) unter Verwendung der benötigten dNTPs eine cDNA (2) synthetisiert, welche maximal die funktionellen Sequenzen (2a) bis (2f) enthält. Für das Funktionieren des PERT Assay müssen in der RT-Reaktion (3a) zumindest die Sequenzen bis und mit (2e) synthetisiert werden.

Version III: Vorgelegt wird eine Template-Nukleinsäure, welche (1e)(1f)(1g)(1h) enthält; im Vergleich zu Version II sind (1c) und (1d) deletiert. Bezüglich der Funktion der verbleibenden funktionellen Sequenzen gilt das unter Version I Gesagte. Auch der RT-Primer (R1)(2a)(2b) ist wie in Version I. Bei Anwesenheit von RT-Aktivität im aufgearbeiteten Untersuchungsmaterial wird in Reaktion (3a) unter Verwendung der benötigten dNTPs eine cDNA (2) synthetisiert, welche maximal die funktionellen Sequenzen (2a) bis (2d) enthält. Für das Funktionieren des PERT Assay müssen in der RT-Reaktion (3a) die Sequenzen (2c) und (2d) synthetisiert werden.

Alle Versionen: Die RNA wird in Reaktion (4) durch eine der unter Fig. 2 erwähnten Methoden als Reaktionspartner in den kommenden Reaktionen eliminiert.

In Version I wird nun an die cDNA (2) in Reaktion (5a) der Primer für den 2. DNA-Strang, ein DNA-Oligonukleotid (R2)(6a)(6b), anhybridisiert. Dessen am 3'-Ende gelegene obligatorische Hybridisierungssequenz (6b) hat Komplementarität zu (2g) der cDNA. Das am 5'-Ende gelegene (6a) ist fakultativ, besitzt keine Komplementarität zur cDNA und dient der Einführung einer neuen, in der cDNA noch nicht vorhandenen Sequenz. Durch Zugabe einer DNA-abhängigen DNA-Polymerase und unter Verwendung von dNTPs wird in Reaktion (7) der 2. DNA-Strang (6) synthetisiert; als Resultat entsteht eine dsDNA (2)//(6) bestehend aus (R1)(2a) bis (2h)//(R2) (6a) bis (6h).

In Version II wird in Reaktion (5a) anstelle des in Version I verwendeten Primers ein dem verkürzten 3'-Ende der cDNA entsprechender DNA-Oligonukleotidprimer (R2)(6c)(6d) an die aus Schritt I hervorgehende cDNA (2) anhybridisiert. Dessen am 3'-Ende gelegene, obligatorische Hybridisierungssequenz (6d) hat Komplementarität zu (2e) der cDNA und enthält gleichzeitig die (P-)-Sequenz des gewählten Transcriptionspromotors. Die Hybridisierungssequenz kann die Sequenz des Transcriptionspromotors zu beiden Seiten überlappen. Das am 5'-Ende gelegene (6c) ist fakultativ, besitzt keine Komplementarität zur cDNA und dient der Einführung einer neuen, in der cDNA noch nicht vorhandenen Sequenz. Durch Zugabe einer DNA-abhängigen DNA-Polymerase und unter Verwendung von dNTPs wird in Reaktion (7) der 2. DNA-Strang (6) synthetisiert; als Resultat entsteht eine dsDNA (2)//(6) bestehend aus (R1)(2a) bis (2f)//(R2)(6c) bis (6h). Falls (6d) an seinem 3'-Ende mindestens 3 Nukleotide über das 3'-Ende der Polymerasepromotor-Sequenz hinausreicht, entsteht schon durch das Annealing des Primers (R2)(6c)(6d) an die cDNA ein funktioneller Promotor für die RNA-Polymerase; für die RNA-Synthese (8a) wird dann keine Reaktion (7) mehr benötigt.

In Version III wird in Reaktion (5a) ein dem noch weiter verkürzten 3'-Ende der cDNA entsprechender DNA-Oligonukleotidprimer (R2)(6c)(6d)(6e) an die cDNA (2) anhybridisiert. Die am 3'-Ende des Primers (R2)(6c)(6d)(6e) gelegene obligatorische Hybridisierungssequenz (6e) hat Komplementarität zu (2d) der cDNA. (6c) und (6d) sind wie in Version II. Durch Zugabe einer DNA-abhängigen DNA-Polymerase und unter Verwendung von dNTPs werden in Reaktion (7) die beiden 3'-Enden der zwei DNA-Stränge verlängert; als Resultat entsteht eine dsDNA (2)//(6) bestehend aus (R1)(2a) bis (2f)//(R2)-(6c) bis (6h).

Bei allen Versionen müssen spätestens jetzt wieder Reaktionsbedingungen hergestellt werden, welche die Stabilität der RNA in den folgenden Reaktionen garantieren. Die dazu verwendeten Methoden sind wie in Fig. 8.

Von der in die Reaktionsmischung eingegebenen dsDNA (2)//(6), die in (2e)//(6d) aller drei Versionen einen funktionellen Promotor für die gewählte RNA-Polymerase aufweisen, erfolgt in Reaktion (8a) vermittelst der DNA-abhängigen RNA-Polymerase und unter Verwendung von rNTPs die Synthese einer ssRNA-Spezies (10) enthaltend (10a) (10b)(10c)(10d). Diese ssRNA (10) hat bei allen drei Versionen die Polarität des 2. DNA-Strangs (6) bzw. der Template-Nukleinsäure (1); (10a), (10b) und (10c) sind identisch mit (1e),(1f) und (1g) der verschiedenen Template-RNAs (1). (10d) ist hingegen von (2a) abgeleitet und komplementär zu diesem.

Schritt 2: Die ssRNA (10) wird in das Amplifikationsverfahren eingegeben. In einer Annealing-Reaktion (5b) wird der 1. Amplifikationsprimer (R3)(11a)(11b)(11c) durch die am 3'-Ende gelegene Hybridisierungssequenz (11c) an (10c) der ssRNA hybridisiert. (11b) ist eine P(-)-Sequenz eines Transcriptionspromotors und (11a) stellt eine flankierende Sequenz dar. (11b) und (11c) sind notwendig, (11a) und die funktionelle Gruppe (R3) sind fakultativ. Vermittelst der in der Reaktionsmischung vorhandenen RT wird in Reaktion (3b) unter Verwendung von dNTPs die cDNA (R3)(11a)(11b) (11c)(11d)(11e) synthetisiert. Durch die RNase H-Wirkung der RT oder durch Einwirkung nötigenfalls zusätzlich beigefügter RNase H wird in Reaktion (12) die RNA (10) von der cDNA-RNA-Heteroduplex soweit degradiert, dass in Reaktion (5c) das Annealing des zweiten Amplifikationsprimers (R4)(13a)(13b) an (11e) der cDNA erfolgen kann. Dieser Amplifikationsprimer besteht an seinem 3'-Ende aus einer obligatorischen, zu (11e) komplementären Hybridisierungssequenz (13b). Upstream davon befindet sich die fakultative flankierende Sequenz (13a). (R4) ist ebenfalls fakultativ. Durch erneute Einwirkung der DNA-abhängigen DNA-Polymerase und unter der Verwendung von dNTPs entsteht in Reaktion (7) eine dsDNA (11)//(13), von welcher in Reaktion (8b) durch eine RNA-Polymerase und unter Verwendung von rNTPS eine ssRNA (14) synthetisiert wird, die in den Sequenzen (14a), (14b) und (14c) komplementär zu (10d), (10c) und (10b) sind.

Ausgehend von RNA (14) erfolgt der erneute Aufbau einr dsDNA (11)11(13). In einer Annealing-Reaktion (5c) wird der 2. Amplifikationsprimer (R4)(13a)(13b) an (14c) der RNA hybridisiert. Durch die in der Reaktionsmischung vorhandenen RT wird in Reaktion (3b) unter Verwendung von dNTPs die cDNA (R4)(13a)(13b)(13c)(13d) synthetisiert. Infolge der RNase H-Wirkung der RT oder durch Einwirkung nötigenfalls zusätzlich beigefügter RNase H wird in Reaktion (12) die RNA (14) von der cDNA-RNA-Heteroduplex soweit degradiert, dass in Reaktion (5b) das Annealing des 1. Amplifikationsprimers (R3)(11a)(11b)(11c) an (13d) der cDNA erfolgen kann. Durch erneute Einwirkung der DNA-abhängigen DNA-Polymerase und unter der Verwendung von dNTPs entsteht in Reaktion (7) wieder eine dsDNA (11)//(13), von welcher in Reaktion (8b) durch eine RNA-Polymerase und unter Verwendung von rNTPS die ssRNA (14) synthetisiert wird.

Schritt 3: Die aus dein Amplifikationsprozess resultierende ssRNA (14a)(14b)(14c)(14d) wird für die Analyse gemäss den später folgenden generellen Anmerkungen zu Schritt 3 verwendet.

### Modifikationen:

Die als fakultativ benannten funktionellen Sequenzen und funktionellen Gruppen (R) aller eingesetzten Nukleinsäuren können, einzeln oder in den verschiedenen möglichen Kombinationen, entfallen. Verzicht auf die am 5'-Ende des RT-Primers situierte funktionelle Sequenz (2a) hat in allen Versionen zur Folge, dass (10d) der ssRNA (10) entfällt. Verzicht auf die am 5'-Ende gelegene funktionelle Sequenz des Primers für den 2. DNA-Strang [(6a) in Version I, (6c) in Versionen II und III] hat dagegen keine Auswirkung in Bezug auf die Zusammensetzung der in Schritt 2 synthetisierten ssRNA (10). Verzicht auf (11a) des 1. Amplifikationsprimers hat keinen Einfluss auf die Zusammensetzung der ssRNA (14), während bei Verzicht auf (13a) in der von der dsDNA (11)//(13) transcribierten ssRNA (14) die Sequenz (14d) entfällt. In Reaktion (4) kann das Vorhandensein von (R1) vorteilhaft sein.

Ein zusätzliches Spacer-Element kann in der Template-Nukleinsäure zwischen den Elementen (1d) und (1e), im Primer für den zweiten DNA-Strang zwischen (6d) und (6e), und /oder im 2. Amplifikationsprimer zwischen (11b) und (11c) eingeschoben werden.

In Version III kann bei der Template-Nukleinsäure (1) die am 3'-Ende gelegene flankierende Sequenz (1h) eine beliebige Basensequenz aufweisen. Dies ermöglicht, natürlich vorkommende RNA-Molekühe als Template-Nukleinsäure (1) einzusetzen.

Abweichend von der Norm kann eine funktionelle Sequenz, oder eine Kombination funktioneller Sequenzen, mehr als einer Funktion dienen. So kann in allen Versionen der 1. Amplifikationsprimer (R3)(11a)(11b)(11c) auch die Funktion des RT-Primers übernehmen. Das Annealing dieses RT-Primers, vermittelst (11c), erfolgt dann an (1g) der Template-Nukleinsäure (1).

Die Verwendung verschiedener Moleküle für den RT-Primer und den 1. Amplifikationsprimer ermöglicht andererseits die Verwendung eines RNA-Primers für die RT-Reaktion, wodurch Sensitivität und/oder Spezifität des PERT Assay beeinflusst werden können.

Abweichend von der Norm existiert die Möglichkeit, für verschiedene funktionelle Sequenzen identische Basensequenzen zu verwenden, diese können auch homopolymer sein. Dies betrifft in allen Versionen die flankierenden Sequenzen und Spacer-Sequenzen der Template-Nukleinsäure (1), die homopolymer und/oder untereinander identisch sein können.

Als eine weitere Modifikation lässt sich in Schritt 1 eine dsDNA (2)//(6) aufbauen, die an beiden Enden je einen funktionellen Transcriptionspromotor trägt. Es werden dann zwei verschiedene Spezies von ssRNAs produziert, die sich teilweise überlappen und entgegengesetzter Polarität sind.

Auf der Basis von Version II lässt sich durch Verwendung einer Template-Nukleinsäure, die zusätzlich zur Template-RNA eine Template-DNA enthält, ein stark vereinfachtes Verfahren erreichen: Vorgelegt wird eine Template-Nukleinsäure (1c)(1d)(1e)(1f)(1g)(1h), wobei (1d) und mindestens die ersten 3 Nukleotide von (1e) aus DNA, das restliche (1e) und (1f) jedoch aus RNA bestehen. Bei den übrigen funktionellen Sequenzen ist gleichgültig, ob sie aus DNA oder RNA bestehen. Unter Verwendung des RT-Primers (R1)(2a)(2b) wird in der RT-Reaktion eine doppelsträngige Nukleinsäure synthetisiert, die in der dsDNA (1d)//(2e) einen funktionellen Transcriptionspromotor besitzt, von welchem nach Eingabe in die in Schritt 2 verwendete Reaktionsmischung die zu vermehrende Nukleinsäure, die RNA (10a)(10b)(10c)(10d), direkt synthetisiert werden kann. Auch in dieser Version muss jedoch die Template-Nukleinsäure vor der Eingabe in die in Schritt 2 verwendete Reaktionsmischung eliminiert werden.

**Fig. 10** zeigt die Amplifikation mittels einer Reportersonde.

Schritt 1: Template-Primer-Kombination, RT-Reaktion (3) und deren Produkt sind in allen Aspekten identisch mit denjenigen aus Fig. 3. In Reaktion (4) wird die vorhandene Template-Nukleinsäure, die RNA (1), als relevanter Teilnehmer an der Annealingreaktion (5a) mit einer der genannten Methoden eliminiert. An die cDNA wird in Reaktion (5a) eine Reportersonde (6) anhybridisiert. Die Anforderungen und Möglichkeiten für diese entsprechen denjenigen in PS-EP 0408295; es können DNA- oder RNA-Reportersonden verwendet werden. Voraussetzung ist, dass eine Hybridisierungssequenz der Reportersonde (6) Komplementarität zur Hybridisierungssequenz (2d) der cDNA (2) aufweist und dadurch das Annealing an diese vermittelt. Spätestens nach der Hybridisierung der Reportersonde (6) an die cDNA (2) wird diese mittels der funktionellen Gruppe (R1) an einen Carrier gebunden und nichtgebundene Reportersonde unter nichtdenaturierenden Bedingungen vollständig aus dem Reaktionsgemisch entfernt, so wie in Fig. 3 beschrieben. Spätestens nach dem vollständigem Wegwaschen ungebundener Reportersonde müssen, wie in **Fig. 8** ausgeführt, wieder Reaktionsbedingungen hergestellt werden, welche die Amplifikation zumindest einer Teilsequenz der Reportersonde (6) gemäss PS-EP 0408295 zulassen. Als Hauptprodukt der Amplifikation entsteht eine ssRNA, die gemäss den Angaben zu Fig. 8 und 9 analysiert wird.

### Modifikationen:

Es stehen alle in Fig. 3 erwähnten Möglichkeiten bezüglich Wahl der Template-Nukleinsäure und des RT-Primers offen. Es ist jedoch darauf zu achten, dass weder Template-Nukleinsäure noch RT-Primer Sequenzhomologie oder -komplementarität zur Reportersonde aufweisen. Einzige Ausnahme ist die der Hybridisierung der Reportersonde dienende Sequenz.

Eine Modifikation, welche die Effizienz der initialen Synthese einer RNA erhöht, besteht darin, dass die Hybridisierungssequenz (1b) der Template-Nukleinsäure (1) gleichzeitig die P(+)-Sequenz eines Transcriptionspromotors enthält. Als Reportersonde wird eine Nukleinsäure verwendet, deren Hybridisierungssequenz so gestaltet ist, dass sie sowohl mit (2d), welches die P(-)-Sequenz des Transcriptionspromotors darstellt, als auch (2e) hybridisiert.

Upstream dieser doppelten Hybridisierungssequenz enthält die Reportersonde zunächst eine Spacersequenz und wiederum eine Hybridisierungssequenz. Alle drei funktionellen Sequenzen bestehen aus DNA, so dass durch die Hybridisierung der Reportersonde an die cDNA ein Transcriptionspromotor entsteht, von welchem ausgehend nach Zugabe der RNA-Polymerase die Synthese der zu vermehrenden ssRNA erfolgt.

### Aufbau des PERT Assay bei Verwendung eines auf replikativer RNA beruhenden Amplifikationsverfahrens

Diese PERT Varianten benützen ein in vitro Replikationssystem zur Amplifikation einer primären replikativen RNA. Die für die Replikation notwendigen funktionellen Sequenzen einer RNA sind hier am Beispiel des am besten dokumentierten Replikationssystem des Bakteriophagen Qβ(beta) illustriert. Um als Template-Nukleinsäure für die Qß-Replicase zu dienen, muss das 3'-Ende der primären RNA eine cytidinreiche Region aufweisen (definitionsgemäss eine 3'-RNA-ori(+)-Sequenz), die der Initiation der RNA-Replikation dient. Das 5'-Ende der primären replikativen RNA muss eine guanylreiche Sequenz enthalten (definitionsgemäss eine 5'-ori-(-)-Sequenz), die in ihrer komplementären Sequenz eine cytidinreiche Region darstellt, und die Initiation der RNA-Synthese des komplementären Strangs übernimmt. Die zwischen den beiden terminalen Replikations-Initiationssequenzen liegenden RNA-Abschnitte müssen zudem noch eine Replicasen-Bindungsdomäne, d.h. ein für die Bindung der RNA-Replicase notwendiges diskontinuierliches Sequenzelement, aufweisen. Das Vorhandensein eines solchen Typs RNA in Verbindung mit Qβ-Replicase und den benötigten rNTPs in einer Reaktionsmischung führt zu einer autokatalytischen Vermehrung des RNA-Moleküls mit einer Verdoppelungszeit von unter einer Minute. Neben dem Qβ-Replikationssystem können in analoger Weise auch Replikationssysteme anderer RNA-Phagen benutzt werden. Als Beispiele, und in keiner Weise abschliessend, können diejenigen der Phagen MS2, f2, und R17 genannt werden.

Die Adaptation des PERT Assay an dieses Replikationssystem kann einerseits durch die Verwendung von Template-Nukleinsäuren erfolgen, die von replikativen RNAs abgeleitet sind und/oder deren Basensequenz mindestens teilweise in die primäre replikative RNA einfliesst, welche dann in Schritt 2 durch eine RNA-Replicase vermehrt wird. Andererseits kann die in der RT-Reaktion synthetisierte cDNA dazu verwendet werden, um mit Hilfe einer Reportersonde eine primäre replikative RNA als die zu vermehrende Nukleinsäure bereitzustellen. Ausser wenn anders vermerkt, werden in den folgenden illustrierten Varianten bevorzugterweise reine RNAs als Template-Nukleinsäure eingesetzt.

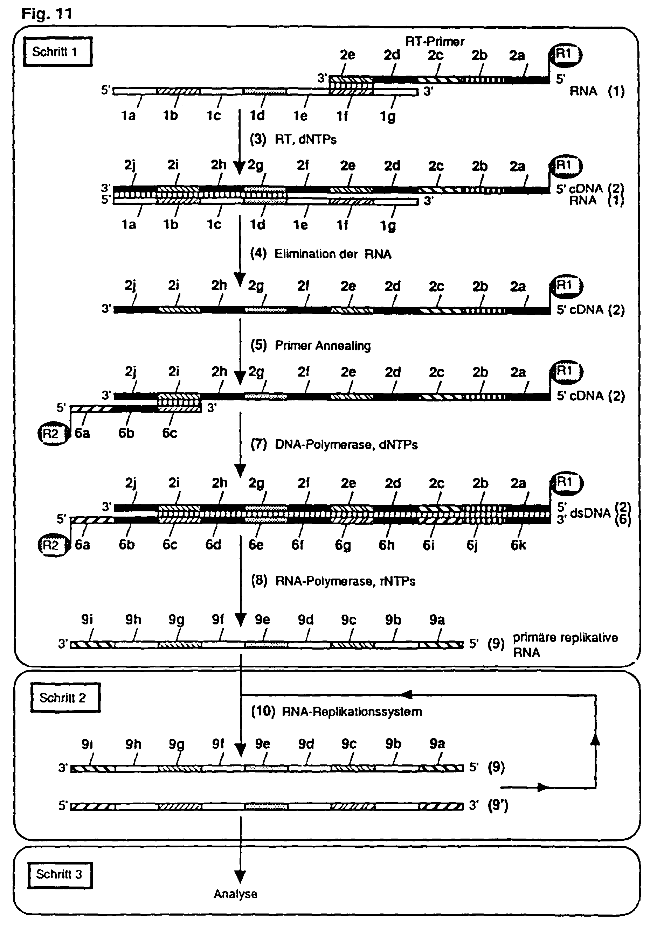

Bei der Verwendung von Template-Nukleinsäuren, die von replikativen RNAs abgeleitet sind, wird im ersten Schritt zuerst eine zumindest partiell doppelsträngige DNA synthetisiert, welche direkt an einen Transcriptionspromotor anschliessend alle notwendigen funktionellen Sequenzen einer replikativen RNA enthält. Ausgehend von dieser zumindest partiell doppelsträngigen DNA wird dann mit Hilfe einer DNA-abhängigen RNA-Polymerase eine replikative RNA synthetisiert, die in Schritt 2 mit dem dieser RNA entsprechenden Replikationssystem vermehrt wird. Es ist wesentlich, dass die Wahl der Template-Nukleinsäure-Sequenz und der Primer-Sequenz so erfolgt, dass die Abfolge der enzymatischen Reaktionen nur dann zu der Synthese einer vermehrbaren RNA führt, wenn im ersten Schritt eine im Untersuchungsmaterial vorliegende RT-Aktivität eine cDNA synthetisiert hat. Es müssen Vorkehrungen getroffen werden, welche die Replikation der Template-Nukleinsäure in Schritt 2, und somit falsch-positive oder falsch negative-Resultate (je nach Art der Analyse des Amplifikationsprodukts ist beides möglich) verunmöglichen. Die in der Folge genannten Varianten (Fig. 11 - 17) dienen dazu, die Vielfalt der Möglichkeiten zu illustrieren, nach denen ein PERT Assay nach diesem Reaktionsprinzip ablaufen kann.

**Fig. 11** zeigt einen ersten möglichen Reaktionsablauf eines solchen PERT Assay. Die notwendigen funktionellen Sequenzen zur späteren RNA-Synthese und zu deren Vermehrung, mit Ausnahme der Replicasen-Bindungsdomäne, werden durch ssDNAs in die DNA eingebracht. Die Transcriptionspromotor-Sequenz wird über den RT-Primer in der Nähe des 5'-Endes der cDNA eingeführt. Die am Ende von Schritt 1 synthetisierte primäre replikative RNA weist eine zur Template-Nukleinsäure gegensätzliche Polarität auf.

Schritt 1: Die vorgelegte Template-Nukleinsäure (1) besteht aus den folgenden funktionellen Sequenzen: Einer flankierenden Sequenz (1a) am 5'-Ende, der Hybridisierungssequenz (1b), zwei Spacer-Sequenzen (1c) bzw. (1e), der Replicasen-Bindungsdomäne (1d), einer zweiten Hybridisierungssequenz (1f) für das Annealing des RT-Primers, und der flankierenden Sequenz (1g) am 3'-Ende. Die flankierenden Sequenzen (1a) und (1g) sowie die Spacer-Sequenzen (1c) und (1e) sind fakultativ; alle anderen Sequenzen sind notwendig.

Als RT-Primer fungiert eine ssDNA (R1) (2a) (2b) (2c) (2d) (2e), vorzugsweise ein Oligonukleotid. Diese ssDNA enthält in (2b) die P(-)-Sequenz eines Transcriptionspromotors, daran anschliessend die 5'-ori (-)-Sequenz (2c) und an seinem 3'-Ende die Hybridisierungssequenz (2e) zur Hybridisierung an (1f) der Template-Nukleinsäure (1). (2a) und (2d) sind eine flankierende, respektive eine Spacer-Sequenz. (2b), (2c) und (2e) sind notwendig, (2a) und (2d) sind fakultativ. Auch (R1) ist fakultativ.

Die im aufgearbeiteten Untersuchungsmaterial enthaltene RT-Aktivität synthetisiert in Reaktion (3) eine cDNA (2) umfassend (R1) (2a) (2b) (2c) (2d) (2e) (2f) (2g) (2h) (2i) (2j). Für das Funktionieren des PERT Assay müssen in der RT-Reaktion zumindest die funktionellen Sequenzen bis (2g) synthetisiert werden.

Das aus Reaktion (3) hervorgehende Gemisch von Nukleinsäuren wird in Reaktion (4) derart modifiziert, dass sichergestellt werden kann, dass keine dieser Komponenten in unerwünscher Weise mit dem optimalen Ablauf der nachfolgenden Reaktionen interferiert. Insbesondere muss verhindert werden, dass die Template-Nukleinsäure (1) und/oder die Template-Primer-Kombinationen (1)//(R1) (2a) (2b) (2c) (2d) (2e) mit der Hybridisierung (5) des zweiten Primers (R2) (6a) (6b) (6c) an die cDNA (2) kompetitiert, und dass in Schritt 2 die Bindung der RNA-Replicase an die in grossem Ueberschuss vorhandene Template-Nukleinsäure (1) anstatt an die replikative RNA (9) erfolgt. Beides führt zu einer verminderten Sensitivität des Nachweises oder gar zu falsch negativen Resultaten. Aus diesem Grund wird in Reaktion (4) die aus RNA bestehende Template-Nukleinsäure (1) mit einer der erwähnten Methoden eliminiert.

In Reaktion (5) erfolgt das Annealing einer zweiten ssDNA (R2) (6a) (6b) (6c), vorzugsweise ein Oligonukleotid. Diese DNA enthält als notwendige Sequenzen am 5'-Ende eine 3'-ori (-)-Sequenz (6a) und am 3'-Ende eine Hybridisierungsequenz (6c) zur Hybridisierung an die funktionenlle Sequenz (2i) der cDNA (2). Fakultative Elemente sind eine Spacer-Sequenz (6b) sowie eine funktionelle Gruppe (R2) . In Reaktion (7) wird durch Wirkung einer DNA-Polymerase und unter Verwendung von dNTPs eine dsDNA bestehend aus den Strängen (2) und (6) synthetisiert, wobei das Segment (2b)//(6j) den aktiven Transcriptionspromotor für die RNA-Polymerase darstellt. Spätestens jetzt müssen, wie in Fig. 8 beschrieben, wieder Reaktionsbedingungen hergestellt werden, welche die Stabilität der RNA in den folgenden Reaktionen garantieren. Nach Zugabe der dem Transcriptionspromotor entsprechenden RNA-Polymerase wird unter Verwendung der notwendigen rNTPs in Reaktion (8) die zu vermehrende Nukleinsäure, die primäre replikative RNA (9) synthetisiert, welche alle für die Vermehrung im gewählten Replikationssystem erforderlichen Funktionen besitzt. Deren Anteile (9c), (9d), (9e), (9f) und (9g) sind dabei komplementär zu (1f), (1e), (1d), (1c), und (1b) der Template-Nukleinsäure.

Schritt 2: Diese RNA (9) wird nun in das RNA-Replikationssystem (10) eingegeben. Dieses enthält in einem geeigneten Puffer alle Enzyme und anderen Reagentien wie rNTPs usw., die für die RNA-Replikation notwendig sind. In diesem Replikationssystem wird die RNA vermehrt, wobei einzelsträngige RNA-Moleküle beider Polaritäten gebildet werden, die in Schritt 3 mit geeigneten Methoden nachgewiesen werden.

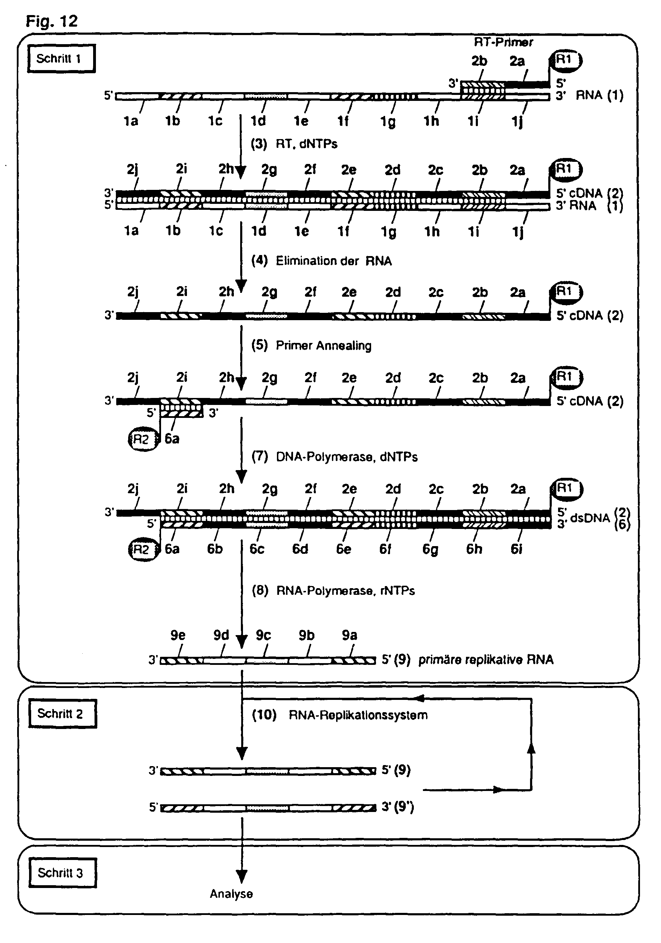

**Fig. 12** zeigt einen Reaktionsablauf, bei dem alle zur späteren RNA-Synthese und zu deren Vermehrung notwendigen Sequenzen schon in der Template-Nukleinsäure vorliegen. Die Transcriptionspromotor-Sequenz kommt wiederum in der Nähe des 5'-Endes der cDNA zu liegen. Die am Ende von Schritt 1 synthetisierte primäre replikative RNA weist eine zur Template-Nukleinsäure gegensätzliche Polarität auf.

Schritt 1: Die vorgelegte Template-Nukleinsäure (1) enthält die funktionellen Sequenzen (1a)...(1j). Abweichend von den allgemein geltenden Prinzipien sind in der hier beschriebenen Variante den funktionellen Sequenzen (1b), respektive (6a), zwei Funktionen zugeordnet.

Im Unterschied zu Fig. 11 kodiert die eingesetzte Template-Nukleinsäure (1) für alle Funktionen, die für die RNA-Transcription und für die RNA-Replikation benötigt werden. (1b) enthält eine 3'-RNA-ori(-)-Sequenz und ist gleichzeitig eine Hybridisierungssequenz, wobei die zur Hybridisierung verwendete Sequenz länger sein kann als die zur Replikation minimal benötigte RNA-ori-Sequenz. Voraussetzung ist, dass die RNA-ori-Sequenz das 5'-Ende von (1b) bildet. (1c), (1e) und (1h) sind Spacer-Sequenzen, (1d) die Replicasen-Bindungsdomäne und (1f) eine 5'-RNA-ori(+)-Sequenz. (1g) ist die P(+)-Sequenz eines Transcriptionspromotors und (1g) die Hybridisierungssequenz für den RT-Primer. (1a) und (1h) sind flankierende Sequenzen. Sowohl die flankierenden als auch die Spacer-Sequenzen sind fakultativ. Alle restlichen funktionellen Sequenzen sind für diese Variante notwendig.

Der RT-Primer (R1) (2a) (2b) ist eine Nukleinsäure, vorzugsweise ein DNA- oder RNA-Oligonukleotid, und besteht aus der fakultativen funktionellen Gruppe (R1), der ebenfalls entbehrlichen und nicht hybridisierenden flankierenden Sequenz (2a) sowie der zur Hybridisierung an (1g) verwendeten notwendigen Hybridisierungssequenz (2b).

Die im Untersuchungsmaterial enthaltene RT-Aktivität synthetisiert in Reaktion (3) eine cDNA mit maximal den funktionellen Sequenzen (2a) (2b) (2c) (2d) (2e) (2f) (2g) (2h) (2i) (2j). Für das Funktionieren des PERT Assay müssen in der RT-Reaktion zumindest die funktionellen Sequenzen bis (2i) synthetisiert werden.

Wie in Fig. 11 wird in Reaktion (4) die Template-Nukleinsäure (1) degradiert. In Reaktion (5) erfolgt sodann das Annealing des zweiten Primers (R2) (6a), eines DNA-Oligonukleotids. Er besteht aus der zu (2g) komplementären Hybridisierungssequenz (6a) und enthält am 5'-Ende eine 3'-RNA-ori (-)-Sequenz. Die funktionelle Gruppe (R2) ist fakultativ.

In Reaktion (7) findet die Synthese einer dsDNA, bestehend aus den Strängen (2) und (6), durch eine DNA-Polymerase statt. Spätestens zum jetzigen Zeitpunkt müssen ausserdem, wie in Fig. 8 beschrieben, wieder Reaktionsbedingungen hergestellt werden, welche die Stabilität der RNA in den folgenden Reaktionen garantieren. Anschliessend erfolgt in Reaktion (8) durch die dem Transcriptionspromotor entsprechende RNA-Polymerase die Synthese der primären replikativen RNA (9). Deren funktionelle Sequenzen (9a), (9b), (9c), (9d) und (9e) sind dabei komplementär zu (1f), (1e), (1d), (1c), und (1b) der Template-Nukleinsäure.

Schritt 2: Diese RNA (9) wird nun in das RNA-Replikationssystem (10) eingegeben und darin vermehrt, wobei einzelsträngige RNA-Moleküle beider Polaritäten gebildet werden, welche in Schritt 3 mit geeigneten Methoden nachgewiesen werden.

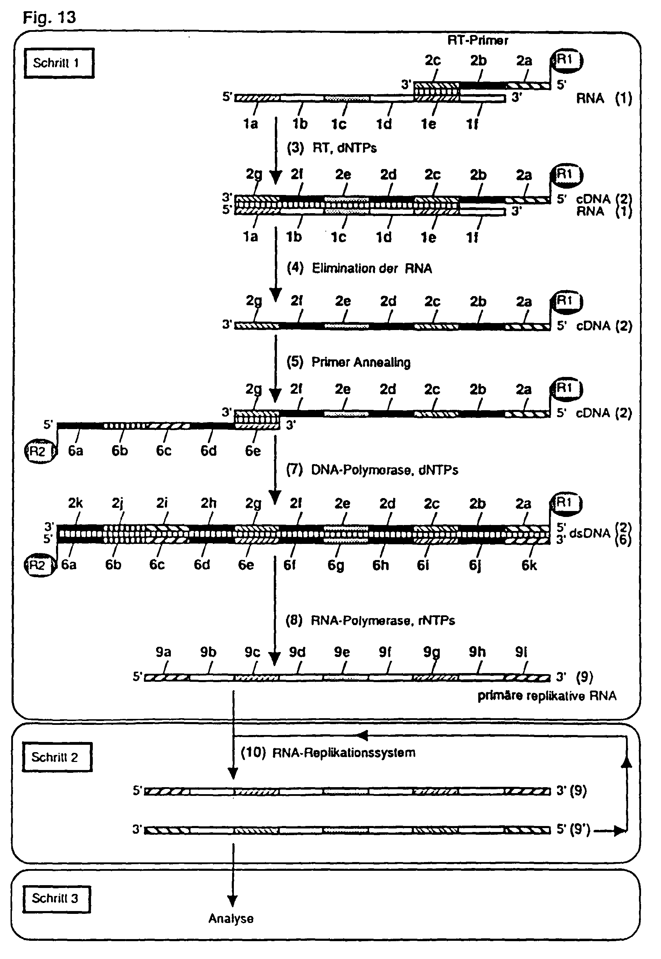

**Fig. 13** zeigt eine weitere Variante der Template-Primer-Konfiguration. Die zur RNA-Synthese und zur Vermehrung notwendigen funktionellen Sequenzen, mit Ausnahme der Replicasen-Bindungsdomäne, werden wiederum ausschliesslich durch Oligonukleotide in die DNA eingebracht. Die Transcriptionspromotor-Sequenz wird jedoch über den 2. Primer in die DNA eingeführt. Die am Ende von Schritt 1 synthetisierte primäre replikative RNA weist die gleiche Polarität auf wie die Template-Nukleinsäure.

Schritt 1: Die vorgelegte Template-Nukleinsäure (1) besteht aus den folgenden, für das Funktionieren dieser Variante notwendigen funktionellen Sequenzen: (1a), am 5'-Ende der Template-Nukleinsäure (1) gelegen, ist eine Hybridisierungssequenz, (1c) ist die Replicasen-Bindungsdomäne, und (1e) ist die für das Annealing des RT-Primers verantwortliche Hybridisierungssequenz. Falkultativ sind die Spacer-Sequenzen (1b) und (1d) und die flankierende Sequenz (1f).

Der Primer für die RT-Reaktion (3) ist eine ssDNA (R1) (2a) (2b) (2c), vorzugsweise ein Oligonukleotid. Er besteht aus (R1), der 3'-RNA-ori(-)-Sequenz (2a), der Spacer-Sequenz (2b) und einer zu (1c) komplementären Hybridisierungssequenz (2c) an seinem 3'-Ende. Die beiden Sequenzen (2a) und (2c) sind für das Verfahren notwendig, die anderen zwei können auch entfallen.

Die im Untersuchungsmaterial enthaltene RT-Aktivität synthetisiert in Reaktion (3) eine cDNA mit den Anteilen (R1) (2a) (2b) (2c) (2d) (2e) (2f) (2g) . Für das Funktionieren des PERT Assay müssen in der RT-Reaktion alle funktionellen Sequenzen bis (2g) synthetisiert werden.

Wie in Fig. 11 wird in Reaktion (4) die Template-Nukleinsäure, die RNA (1), degradiert. In Reaktion (5) erfolgt sodann das Annealing des zweiten Primers (R2)(6a) ... (6e), einer ssDNA, vorzugsweise ein Oligonukleotid. Er enthält in der Nähe des 5'-Endes die P(-)- Sequenz des Transcriptionspromotors (6b) und unmittelbar downstream derselben eine 5'-RNA-ori(-)-Sequenz (6c). Am 3' -Ende des Primers befindet sich die zu (2g) der cDNA komplementäre Hybridisierungssequenz (6e), (6b), (6c) und (6e) sind für das Funktionieren dieser Variante notwendig. Zusätzlich zu diesen können als fakultative Elemente eine flankierende (6a) und eine Spacer-Sequenz (6d) vorhanden sein. Zudem kann der Primer eine funktionelle Gruppe (R2) aufweisen.

In Reaktion (7) wird durch Wirkung einer DNA-Polymerase und unter Verwendung von dNTPs eine dsDNA bestehend aus den Strängen (2) und (6) synthetisiert, wobei nun das Segment (6b)//(2j) einen aktiven Transcriptionspromotor darstellt. Spätestens zum jetzigen Zeitpunkt müssen ausserdem, wie in Fig. 8 beschrieben, wieder Reaktionsbedingungen hergestellt werden, welche die Stabilität der RNA in den folgenden Reaktionen garantieren.

Nach Zugabe der RNA-Polymerase wird unter Verwendung der notwendigen rNTPs in Reaktion (8) die zu vermehrende Nukleinsäure, die primäre replikative RNA (9) synthetisiert, welche alle für die Vermehrung im gewählten Replikationssystem erforderlichen funktionellen Sequenzen besitzt. Deren funktionelle Sequenzen (9c), (9d), (9e), (9f), (9g) entsprechen dabei (1a), (1b), (1c), (1d) und (1e) der Template-Nukleinsäure (1).

Schritt 2: Die primäre replikative RNA (9), die die gleichen funktionellen Sequenzen aufweist wie die primäre replikative RNA in Figur 11, wird analog zu Schritt 2/Fig. 11 amplifiziert und anschliessend in Schritt 3 nachgewiesen.

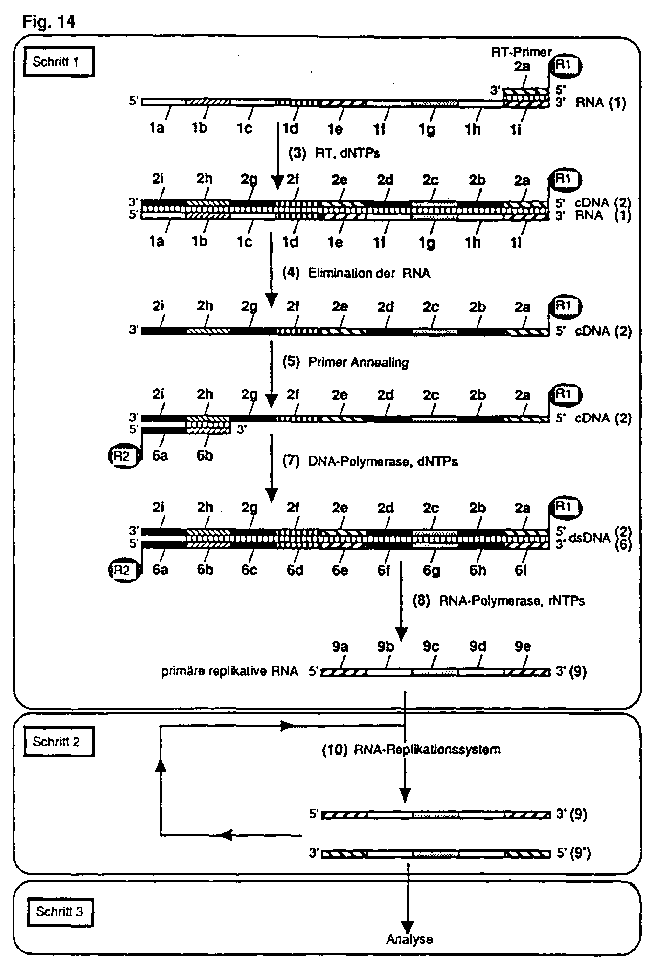

**Fig. 14** zeigt eine Variante, in welcher die Template-Nukleinsäure schon alle für die spätere RNA-Synthese und Amplifikation benötigten funktionellen Sequenzen enthält. Die Transcriptionspromotorsequenz kommt in der Nähe des 3'-Endes der cDNA zu liegen. Die am Ende von Schritt 1 synthetisierte RNA weist die gleiche Polarität auf wie die Template-Nukleinsäure.

Schritt 1: Die vorgelegte Template-Nukleinsäure (1) enthält am 5'-Ende eine flankierende Sequenz (1a), gefolgt von einer Hybridisierungssequenz (1b). Daran anschliessend befindet sich eine Spacer-Sequenz (1c). Es folgen eine P(-)-Sequenz eines Transcriptionspromotors (1d) und unmittelbar daran anschliessend eine 5'-RNA-ori(-)-Sequenz (1e). (1f) bezeichnet eine Spacer-Sequenz, (1g) ist die Replicasen-Bindungsdomäne und (1h) eine weitere Spacer-Sequenz. (1i) ist in dieser Variante mit einer Doppelfunktion belegt und enthält gleichzeitig eine 3'-RNA-ori(+)-Sequenz und eine Hybridisierungssequenz für den RT-Primer (2a), wobei die RNA-ori-Sequenz am 3'-Ende liegen muss, aber nur einen Teil der zur Hybridisierung verwendeten Sequenz darzustellen braucht. (1b), (1d), (1e), (1g) und (1i) sind für das Funktionieren dieser Variante notwendig, währenddem (1a), (1c), (1f) und (1h) entbehrlich sind.

Der RT-Primer (R1)(2a) ist ein DNA-Oligonukleotid. Das notwendige (2a) ist eine Hybridisierungssequenz, welche mit (1i) der Template-Nukleinsäure (1) hybridisiert, und enthält ausserdem eine 3'-ori (-)-Sequenz am 5'-Ende. Die funktionelle Gruppe (R1) ist fakultativ.

Die im Untersuchungsmaterial enthaltene RT-Aktivität synthetisiert in Reaktion (3) eine cDNA mit maximal den Anteilen (2a) (2b) (2c)(2d)(2e)(2f)(2g)(2h)(2i). Für das Funktionieren des PERT Assay müssen in der RT-Reaktion zumindest die Sequenzen bis (2f) synthetisiert werden.

Wie in Fig. 11 wird zunächst in Reaktion (4) die Template-Nukleinsäure, die RNA (1), degradiert. In dieser Variante kommt als weiterer Grund, die RNA zu degradieren, die Möglichkeit einer Interferenz durch den Umstand hinzu, dass die Template-Nukleinsäure (1) in ihrer freien Form (d.h. nicht mit Primer (2a) gepaart) durch das Vorliegen einer Replicasen-Bindungsdomäne (1g) und einer 3'-RNA-ori(+)-Sequenz (1i) an ihrem 3'-Ende ein Substrat für die RNA-Replicase darstellt. In Reaktion (5) erfolgt sodann das Annealing des zweiten Primers (R2)(6a)(6b), der vorzugsweise ein DNA-Oligonukleotid ist. Er besteht aus der zu (2f) komplementären notwendigen Hybridisierungssequenz (6b) und enthält am 5' -Ende eine fakultative flankierende Sequenz (6a). Ebenfalls fakultativ ist die funktionelle Gruppe (R2).

In Reaktion (7) erfolgt die Synthese einer dsDNA, bestehend aus den Strängen (2) und (6), durch eine DNA-Polymerase. Spätestens zum jetzigen Zeitpunkt müssen ausserdem, wie in Fig. 8 beschrieben, wieder Reaktionsbedingungen hergestellt werden, welche die Stabilität der RNA in den folgenden Reaktionen garantieren. In der Transcriptionsreaktion (8) erfolgt die Synthese der primären replikativen RNA, welche die zu vermehrende Nukleinsäure ist. Deren funktionelle Sequenzen (9a), (9b), (9c), (9d) und (9e) sind dabei identisch zu (1e), (1f), (1g), (1h) und (1i) der Template-Nukleinsäure.

Schritt 2: Die primäre replikative RNA (9), die die gleichen funktionellen Sequenzen aufweist wie die primäre replikative RNA in Fig. 12, wird analog zu Fig. 12 amplifiziert und anschliessend in Schritt 3 nachgewiesen.

### Modifikationen:

Die innerhalb einer Variante als fakultativ benannten funktionellen Sequenzen und funktionellen Gruppen können, einzeln oder in den verschiedenen möglichen Kombinationen, entfallen.

Die in den Fig. 11, 12, 13 und 14 beschriebenen Varianten stellen jeweils zwei mögliche Extreme dar, was die Art und Weise betrifft, wie die für die RNA-Synthese und RNA-Replikation notwendigen funktionellen Sequenzen, mit Ausnahme der Replicasen-Bindungsdomäne, in die dsDNA eingeführt werden. Sowohl die Transcriptionspromotor-Sequenz wie auch die RNA-ori-Sequenzen sind hier entweder ausschliesslich und vollständig auf den verwendeten Oligonukleotiden oder vollständig auf der Template-Nukleinsäure kodiert. Diese Unterscheidung ist nicht obligat, sondern nur als Beispiel zu verstehen. Es ist ohne weiteres möglich, Kombinationen vorzunehmen. Zudem ist es möglich, die Template-Nukleinsäure so zu gestalten, dass nur Teile einer bestimmten notwendigen funktionellen Sequenz in ihr vorliegen und die fehlenden Anteile über einen der Primer eingeführt wird. Alle diese Möglichkeiten gelten auch für die Replicasen-Bindungsdomäne.

Ebenso ist die in Fig. 11 - 14 dargestellte klare Trennung der Hybridisierungssequenzen von den zur RNA-Synthese und RNA-Replikation notwendigen funktionellen Sequenzen nicht zwingend. Eine Hybridisierungssequenz kann mit einer RNA-ori-Sequenz, der Replicasen-Bindungsdomäne, der Transcriptionspromotor-Sequenz oder auch mit zwei benachbarten dieser funktionellen Sequenzen teilweise überlappen, diese vollständig in sich enthalten oder mit einer dieser, wie in Fig. 12 und 14 für die 3'-RNA-ori-Sequenz beschrieben, identisch sein.

So kann z.B. die in Fig. 14 beschriebene Variante dahingehend einfach modifiziert werden, dass der Primer für den zweiten DNA-Strang als Hybridisierungssequenz (6b) eine P(-)-Sequenz eines Transcriptionspromotors, sowie mindestens drei weitere zur cDNA komplementäre Nukleotide enthält. Der Primer hybridisiert dann mit der funktionellen Sequenz (2f) der cDNA, wobei eine partiell dsDNA mit einem funktionellen Transcriptionspromotor(6b)//(2f) für die RNA-Polymerase entsteht. Die replikative RNA (9) kann deshalb direkt von dieser partiell doppelsträngigen DNA (2)//(6) synthetisiert (8) werden. Die Synthese der Doppelstrang-DNA mit DNA-Polymerase (7) kann dadurch entfallen. Ebenso sind die funktionellen Sequenzen (1a)...(1c) der Template-Nukleinsäure (1) nicht mehr notwendig und können entfallen.

Wie erwähnt, kann die Verwendung einer Template-Nukleinsäure, die nebst der Template-RNA auch eine Template-DNA enthält, in gewissen Konstellationen von Vorteil sein, indem die Synthese einer primären replikativen RNA stark vereinfacht wird. Wenn beispielsweise bei der in Fig. 12 gezeigten Template-Nukleinsäure (1) die flankierende Sequenz (1a) weggelassen wird, (1b) (1c) (1d) (1e) (1f) (1g) in DNA und von den restlichen funktionellen Sequenzen mindestens das in diesem Fall notwendige (1h) in RNA vorgelegt werden, entsteht in der RT-Reaktion (3) in (1g)//(2d) ein funktioneller Transcriptionspromotor, von welchem ausgehend nach Zugabe einer entsprechenden DNA-abhängigen RNA-Polymerase sowie der benötigten rNTPs die Synthese der primären replikativen RNA (9) der Zusammensetzung (9a) (9b) (9c) (9d) (9e) erfolgt. Wenn bei der in Fig. 14 gezeigten Template-Nukleinsäure (1) die (P-)-Sequenz des Transcriptionspromotors (1d) sowie mindestens die unmittelbar downstream anschliessenden drei ersten Nukleotide von (1e) in DNA vorgelegt werden, entsteht in der RT-Reaktion (3) ebenfalls ein funktioneller Transcriptionspromotor, welcher die direkte Synthese der primären replikativen RNA (9) ermöglicht, welche komplementär zu den funktionellen Sequenzen (2e) (2d) (2c) (2b) (2a) der cDNA (2) synthetisiert wird. Dabei können unterschiedlich grosse Anteile der cDNA (2) in Form des RT-Primers vorgelegt werden, wichtig ist lediglich, dass die Template-Primer-Kombination alle funktionellen Sequenzen einer replikativen RNA in der richtigen Anordnung enthält und dass sich zwischen der Template-DNA und der Hybridisierungssequenz für den RT-Primer ein ausreichend langes Stück Template-RNA befindet.

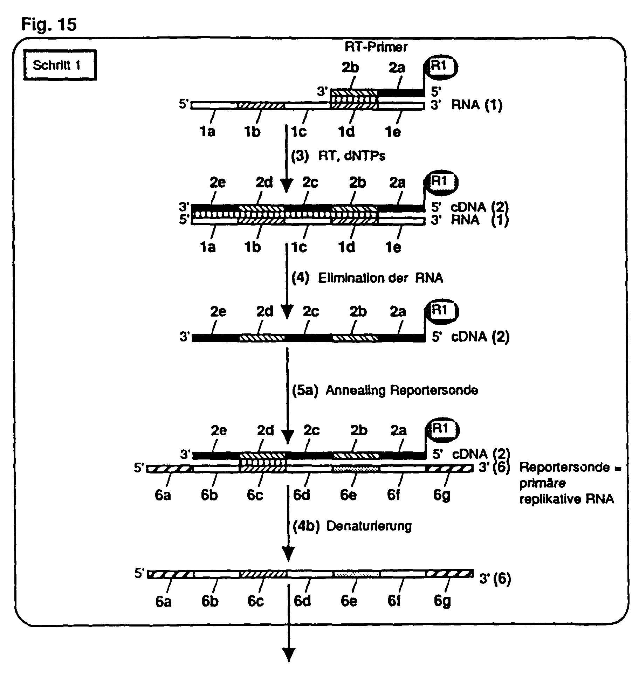

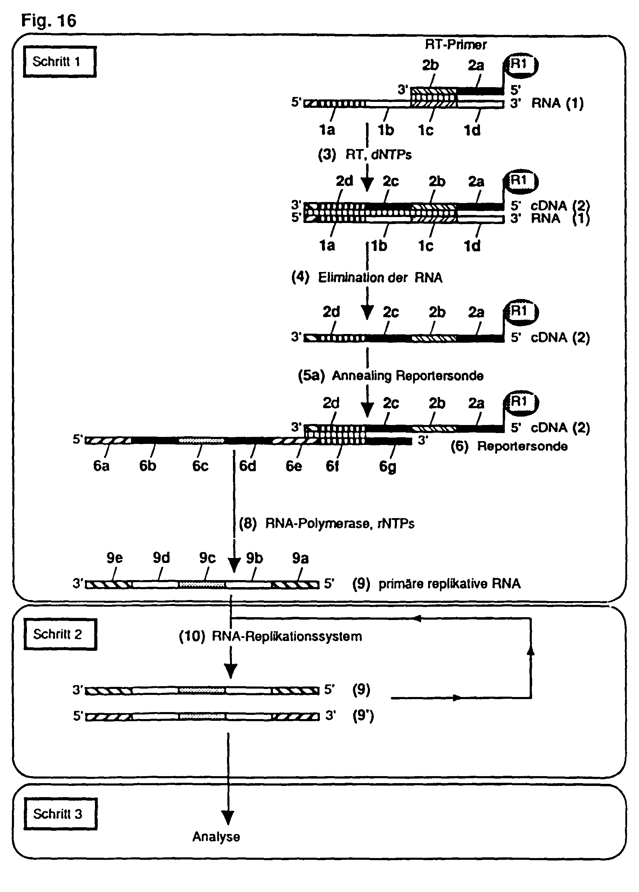

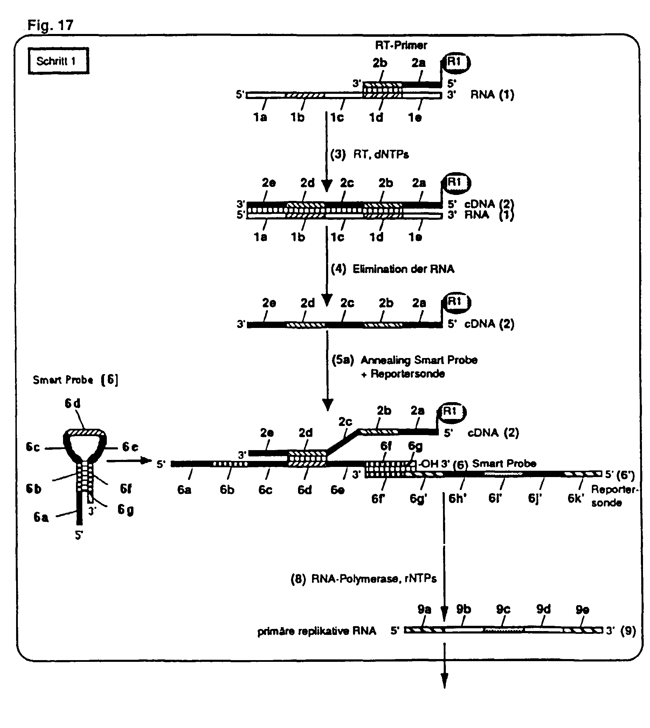

Die in der Folge beschriebenen Varianten (Fig. 15 -17) benützen die in der RT-Reaktion (3) synthetisierte cDNA nur zur Selektion einer Reportersonde, welche entweder selbst in Schritt 2 amplifiziert wird (Fig. 15) oder welche zuerst zur Synthese einer primären replikativen RNA benützt wird (Fig. 16, 17), bevor diese anschliessend in Schritt 2 vermehrt wird.

**Fig. 15** zeigt eine Variante, bei der als Reportersonde eine replikative RNA an die synthetisierte cDNA hybridisiert wird. Nachdem die ungebundene replikative RNA weggewaschen ist, wird die spezifisch gebundene RNA von der cDNA abgelöst und anschliessend amplifiziert.

Schritt 1: Sowohl die notwendigen und fakultativen funktionellen Sequenzen von Template-Nukleinsäure (1) und RT-Primer (R1) (2a) (2b) als auch die Möglichkeiten und Anforderungen für den Rest (R1) sind identisch zu denjenigen in Fig. 3. Im übrigen gilt die Anforderung, dass die Hybridisierungssequenz (2d) der cDNA als einzige und ausschliesslich mit der Hybridisierungssequenz der als Reportersonde eingesetzten replikativen RNA reagiert. Keine andere Sequenz der Template-Nukleinsäure (1), des RT-Primers (R1) (2a) (2b) oder der cDNA (2) darf mit der als Reportersonde eingesetzten replikativen RNA eine spezifische Interaktion eingehen können.

Das aus der RT-Reaktion (3) resultierende Gemisch von Nukleinsäuren wird in Reaktion (4) derart modifiziert, dass sichergestellt werden kann, dass keine ihrer Komponenten, insbesondere die Template-Nukleinsäure (1) und/oder die Template-Primer-Kombinationen (1)//(R1) (2a) (2b), in der folgenden Hybridisierungsreaktion (5a) der Reportersonde (6) mit der cDNA (2) als spezifischer Kompetitor auftreten kann und dadurch zu einer verminderten Sensitivität des Nachweises oder gar zu falsch negativen Resultaten führt. In Reaktion (4) wird die Template-Nukleinsäure (1) deshalb mit einer der genannten Methoden eliminiert.

Falls zur Entfernung der Template-Nukleinsäure (1) degradierende Bedingungen angewendet wurden, müssen jetzt, wie in Fig. 8 beschrieben, wieder Reaktionsbedingungen hergestellt werden, welche die Stabilität der RNA in den folgenden Reaktionen garantieren. An die cDNA (2) wird zunächst in Reaktion (5a) als Reportersonde (6) eine vollständige replikative RNA anhybridisiert, welche aus den funktionellen Sequenzen (6a) (6b) (6c) (6d) (6e) (6f) (6g) besteht. Von diesen ist (6a) eine 5'-RNA-ori(-)-Sequenz; (6b), (6d) und (6f) sind Spacer-Sequenzen, (6c) eine Hybridisierungssequenz für die Hybridisierung an (2d) der cDNA, (6e) die Replicasen-Bindungsdomäne, und (6g) ist eine am 3'-Ende gelegenen 3'-RNA-ori(+)-Sequenz. (6a), (6c), (6e) und (6g) sind für das Verfahren notwendig, die Spacer-Sequenzen sind fakultativ.

Spätestens nach dem Annealing dieser Reportersonde (6) an die cDNA (2) wird die cDNA mittels der funktionellen Gruppe (R1) an einen Carrier gebunden und nichtgebundene Reportersonde unter nichtdenaturierenden Bedingungen vollständig aus dem Reaktionsgemisch entfernt. Damit rigoros gewaschen werden kann, ist eine starke Bindung zwischen der carriergebundenen cDNA (2) und der Reportersonde (6) notwendig. Der Hybridisationsbereich dieser beiden Nukleinsäuren, (2d) bzw. (6c), muss daher von genügender Länge und geeigneter Beschaffenheit sein, und das Annealing der Reportersonde (6) muss unter optimalen Reaktionsbedingungen erfolgen. Ebenso muss darauf geachtet werden, dass die Bindung der cDNA (2) an den Carrier bestehen bleibt.

Nach vollständigem Wegwaschen ungebundener Reportersonde (6) wird die spezifisch gebundene replikative RNA (6) von der cDNA (2) mittels Denaturierung losgelöst (4b) und in Schritt 2 im entsprechenden RNA-Replikationssystem amplifiziert und anschliessend in Schritt 3 mit geeigneten Methoden nachgewiesen.

### Modifikationen:

Die Hybridisierungssequenz (6c) der Reportersonde kann auch mit der Replicasen-Bindungsdomäne, mit einer der RNA-ori-Sequenzen oder zwei benachbarten notwendigen funktionellen Sequenzen überlappend angelegt werden, mit einer dieser Sequenzen identisch sein oder eine dieser, vorzugsweise jedoch nicht eine RNA-ori-Sequenz, vollständig in sich enthalten.

**Fig. 16** zeigt eine Variante, bei der die zum Nachweis der in Schritt 1 synthetisierten cDNA eingesetzte Reportersonde zur Synthese einer replikativen RNA verwendet wird.

Schritt 1: Die vorgelegte Template-Nukleinsäure (1) enthält am 5'-Ende eine Hybridisierungssequenz (1a). Diese enthält gleichzeitig die P(-)-Sequenz eines Transcriptionspromotors sowie mindestens die ersten drei Nukleotide einer 3'-RNA-ori(+)-Sequenz. Daran anschliessend befindet sich die Spacer-Sequenz (1b). Es folgen die Hybridisierungssequenz für den RT-Primer (1c) und die flankierende Sequenz (1d), (1a) und (1c) sind für das Funktionieren dieser Variante notwendig, währenddem (1b) und (1d) entbehrlich sind. Der RT-Primer (R1)(2a)(2b) ist identisch zu demjenigen in Fig. 3, mit der Abweichung, dass die funktionelle Gruppe (R1) jetzt fakultativ ist. Im übrigen gilt die Anforderung, dass die Hybridisierungssequenz (2d) der cDNA als einzige und ausschliesslich mit der Hybridisierungssequenz der Reportersonde (6) reagiert. Keine andere Sequenz der Template-Nukleinsäure (1), des RT-Primers (R1)(2a)(2b) oder der cDNA (2) darf mit der als Reportersonde eingesetzten replikativen RNA eine spezifische Interaktion eingehen können. Da die Reportersonde (6) für sich allein kein Substrat für die RNA-Polymerase darstellt, erübrigt sich bei dieser Variante die Elimination der Template-RNA. An die cDNA (2) wird daher zunächst in Reaktion (5a) eine Reportersonde (6) anhybridisiert.

Diese ist eine ssDNA und enthält die folgenden funktionellen Sequenzen: (6a) ist eine 5'-RNA-ori(-)-Sequenz; (6b) und (6d) sind Spacer-Sequenzen; (6c) ist eine Replicasen-Bindungsdomäne; (6e) ist eine 3'-RNA-ori(+)-Sequenz und mit mindestens 3 Nukleotiden am 3'-Ende an der Hybridisierungssequenz beteiligt; (6f) ist die P(-)-Sequenz eines Transcriptionspromotors und gleichzeitg Teil der Hybridisierungssequenz und (6g) ist eine flankierende Sequenz. (6a), (6c), (6e) und (6f) sind für das Verfahren notwendig; (6g), (6b) und (6d) sind fakultativ. Durch die Hybridisierung der Reportersonde (6) an die cDNA (2) entsteht in Reaktion (5a) ein aktiver Transcriptionspromotor (2d)//(6e teilweise)(6f), von welchem in Reaktion (8) durch eine RNA-Polymerase unter Verwendung von rNTPs die primäre replikative RNA (9) synthetisiert wird.

In Schritt 2 wird die primäre replikative RNA im entsprechenden RNA-Replikationssystem amplifiziert und anschliessend in Schritt 3 mit geeigneten Methoden nachgewiesen.

### Modifikationen:

Es besteht die Möglichkeit, die oben dargestellte Varianten so zu modifizieren, dass die Hybridisierungssequenz auf der Reportersonde downstream von der P(-)-Sequenz des Transcriptionspromotors liegt. Die Hybridisierungssequenz (1a) der Template-Nukleinsäure enthält dann keine RNA-ori(+)-Sequenzanteile und keine P(-)-Sequenz eines Transcriptionspromotors. Die Herstellung eines aktiven Transcriptionspromotors wird dann durch Annealing eines zusätzlichen ssDNA-Oligonukleotids (6f') erreicht, welches die komplementären Sequenzen zu der P(-)-Sequenz des Transcriptionspromotors (6f) und mindestens die ersten 3 Nukleotide der RNA-ori(+)-Sequenz enthält. Als zusätzliche Modifikation kann dann die Template-Nukleinsäure (1) an ihrem 5'-Ende eine flankierende Sequenz enthalten. In all diesen Fällen muss jedoch spätestens nach dem Annealing der Reportersonde (6) an die cDNA (2) diese an einen Carrier gebunden und überschüssige Reportersonde weggewaschen werden, und anschliessend müssen Reaktionsbedinungen geschaffen werden, welche die Stabilität der RNA garantieren.

**Fig. 17** zeigt eine Variante des PERT Assay, in welcher der Nachweis einer durch RT-Aktivität gebildeten cDNA, mit Hilfe einer durch eine "smart probe" induzierten Synthese einer replikativen RNA, wie in PS-WO 90/03445 beschrieben, erfolgt.

Schritt 1: sowohl die notwendigen und entbehrlichen funktionellen Sequenzen von Template-Nukleinsäure (1) und RT-Primer (R1)(2a)(2b) als auch die Möglichkeiten und Anforderungen für den Rest (R1) sind identisch zu denjenigen in Fig. 3. Im übrigen gilt die Anforderung, dass die Hybridisierungssequenz (2d) der cDNA als einzige und ausschliesslich mit der zur spezifischen Hybridisierung bestimmten Sequenz der "smart probe", nicht jedoch mit anderen funktionellen Sequenzen der "smart probe" oder mit der die replikative RNA kodierenden ssDNA-Reportersonde reagiert. Keine andere Sequenz der Template-Nukleinsäure (1), des RT-Primers (R1)(2a)(2b) oder der cDNA (2) darf mit einer der beiden zur Synthese von replikativer RNA eingesetzten Nukleinsäuren eine spezifische Interaktion eingehen können.

Aus den gleichen Gründen und auf die gleiche Art und Weise wie in Fig. 15 beschrieben, wird die Template-Nukleinsäure (1) eliminiert (4). Die weiteren Reaktionen bis zur Synthese der primären replikativen RNA erfolgen nun gemäss dem in PS-WO 90/03445 offenbarten Verfahren durch den Nachweis der nur in der cDNA (2) vorhandenen Sequenz (2d). In Reaktion (5a) wird zunächst eine "smart probe" (6) an (2d) der cDNA (2) und an eine Reportersonde (6') hybridisiert. Die "smart probe", eine ssDNA, besteht aus den folgenden Anteilen: Beginnend vom 5'-Ende her, enthält sie zuerst eine flankierende Sequenz (6a), gefolgt von der P(+)-Sequenz eines Transcriptionspromotors (6b). Daran anschliessend enthält sie eine Zwischenssequenz (6c), die zu (2d) der cDNA komplementäre Sequenz (6d), und eine zweite Zwischensequenz (6e). Auf diese folgt die P(-)-Sequenz (6f) des gleichen Transcriptionspromotors, und am 3'-Ende eine noch mindestens drei Nukleotide umfassende Sequenz (6g). Die zwei Zwischensequenzen (6b) und (6e) sind zueinander nicht komplementär und trennen die zur Hybridisierungssequenz (2d) der cDNA komplementäre Hybridisierungssequenz (6d) von den Promotersequenzen (6b) und (6f). Die Reportersonde (6'), eine ssDNA, enthält in den funktionellen Sequenzen (6i')(6h')(6g') (6j')(6k') die Information für eine replikative RNA, wobei (6k') die 5'-RNA-ori(-)-Sequenz, (6j') eine fakultative Spacer-Sequenz, (6i') die Replicasen-Bindungsdomäne, (6h') eine weitere fakultative Spacer-Sequenz und (6g') die 3'-RNA-ori(+)-Sequenz bezeichnen und in (6f') die P(+)-Sequenz eines Transcriptionspromotors vorliegt. In Abwesenheit der Targetsequenz (2d) der cDNA bildet die "smart probe" durch intramolekulare Hybridisierung der beiden zueinander komplementären Transcriptionspromotor-Sequenzen (6b) und (6f) als Sekundärstruktur eine Stem-Loop-Struktur, wie links in Fig. 17 gezeigt. Falls aber (6d) der "smart probe" an (2d) der cDNA (2) hybridisiert, werden (6f) und (6g) der "smart probe" für die Hybridisierung an (6f') und Teilen von (6g') der einzelsträngigen Reportersonde (6') verfügbar. Durch diese Hybridisierung entsteht ein funktioneller Transcriptionspromotor (6f)(6g)//(6f')(erste 3 Nukleotide von 6g'), und nach Zugabe der entsprechenden RNA-Polymerase und benötigten rNTPs wird die primäre replikative RNA (9) synthetisiert (8).

Falls erfindungsgemäss zur Entfernung der Template-Nukleinsäure (1) in Reaktion (4) degradierende Bedingungen angewendet wurden, müssen spätestens unmittelbar vor der Zugabe der RNA-Polymerase zur Synthese der primären replikativen RNA, wie in Fig. 8 beschrieben, wieder Reaktionsbedingungen hergestellt werden, welche die Stabilität der RNA in den folgenden Reaktionen garantieren. Die in Reaktion (8) durch die RNA-Polymerase synthetisierte primäre replikative RNA (9) wird in Schritt 2 im entsprechenden RNA-Replikationssystem amplifiziert und anschliessend in Schritt 3 mit geeigneten Methoden nachgewiesen.

Für alle Varianten, die eine Reportersonde verwenden, gilt, dass die auf der Template-Nukleinsäure (1), dem RT-Primer (R1)(2a) (2b) und der Reportersonde (6) als fakultativ benannten funktionellen Sequenzen einzeln oder in den verschiedenen möglichen Kombinationen entfallen können.

### Aufbau des PERT Assay bei Verwendung eines auf replikativer DNA beruhenden Amplifikationsverfahrens

Bei diesen PERT-Varianten erfolgt der Nachweis der RT-Aktivität im Untersuchungsmaterial dadurch, dass von der cDNA abgeleitete mindestens teilweise doppelsträngige DNAs oder Teile der cDNA-Template-Duplex mit Hilfe eines in vitro DNA-Replikationssystems vermehrt und anschliessend nachgewiesen wird. Ein Beispiel für ein derartiges Replikationssystem ist das des Bacteriophagen Φ29, welches die Proteine p2 (eine DNA-Replicase) und p3 (das sog. terminale Protein), dNTPs und ein geeignetes Puffersystem umfasst. Die zu replizierende DNA weist an beiden Enden einen DNA-ori auf, eine im Minimum 12 Basenpaare lange, definierte und zumindest partiell doppelsträngige Sequenz. Das terminale Protein p2 bindet in Anwesenheit von dATP kovalent an den DNA-ori, wodurch die DNA-Replicase die Replikation in einer protein-geprimten Reaktion beginnt und diese unter Verdrängung des nicht replizierten Strangs ausführt.

Für die Amplifikation können auch andere DNA-Replikationssysteme, die nach analogen Prinzipien funktionieren, eingesetzt werden. So sollen als Beispiele, und in keiner Weise abschliessend, diejenigen der Bacteriophagen M29, Nf oder Cp1, sowie das der eukaryotischen Adenoviren genannt werden.

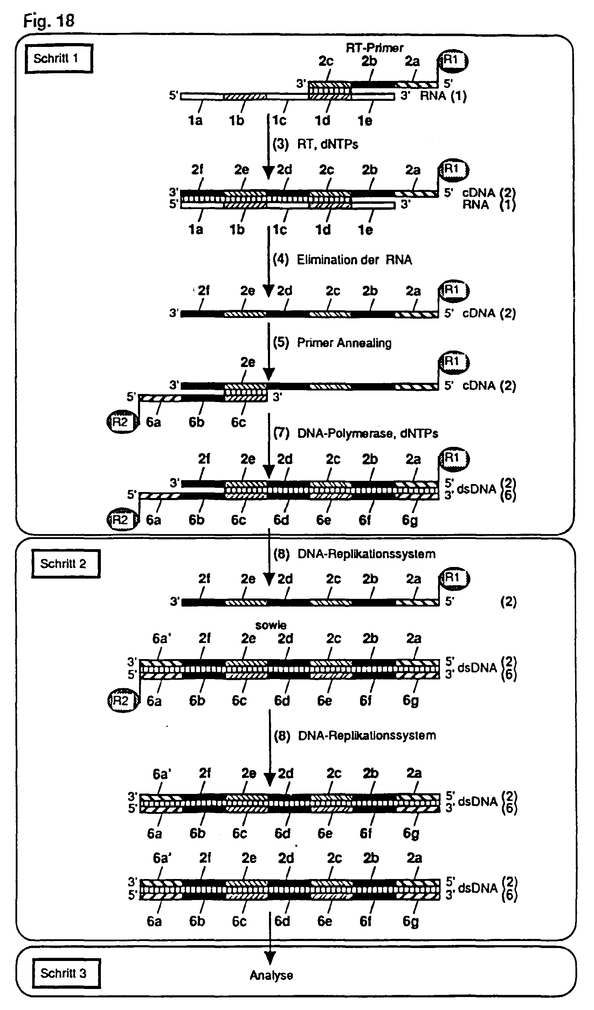

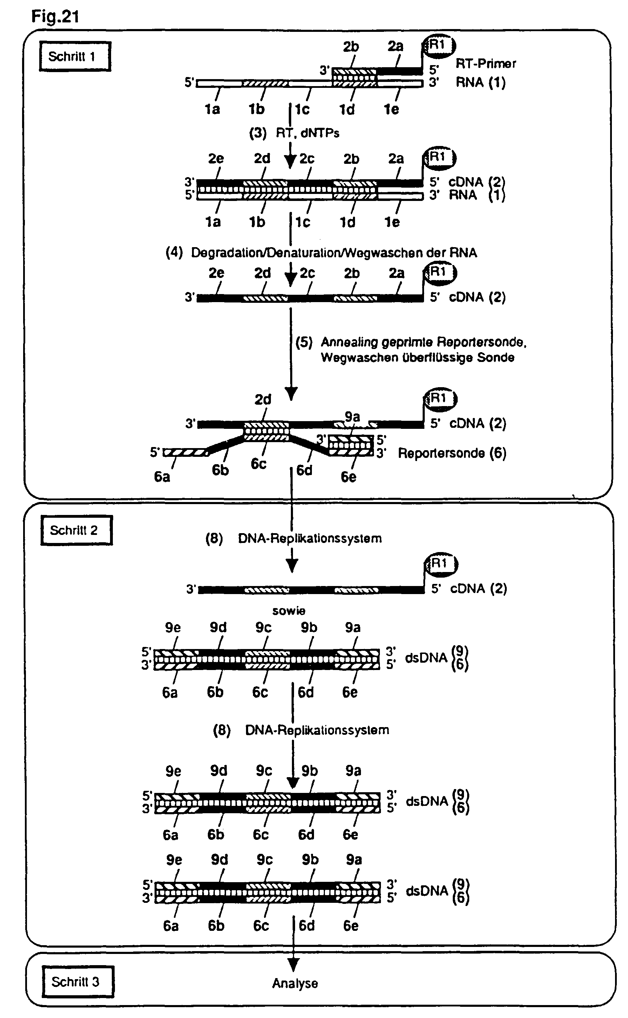

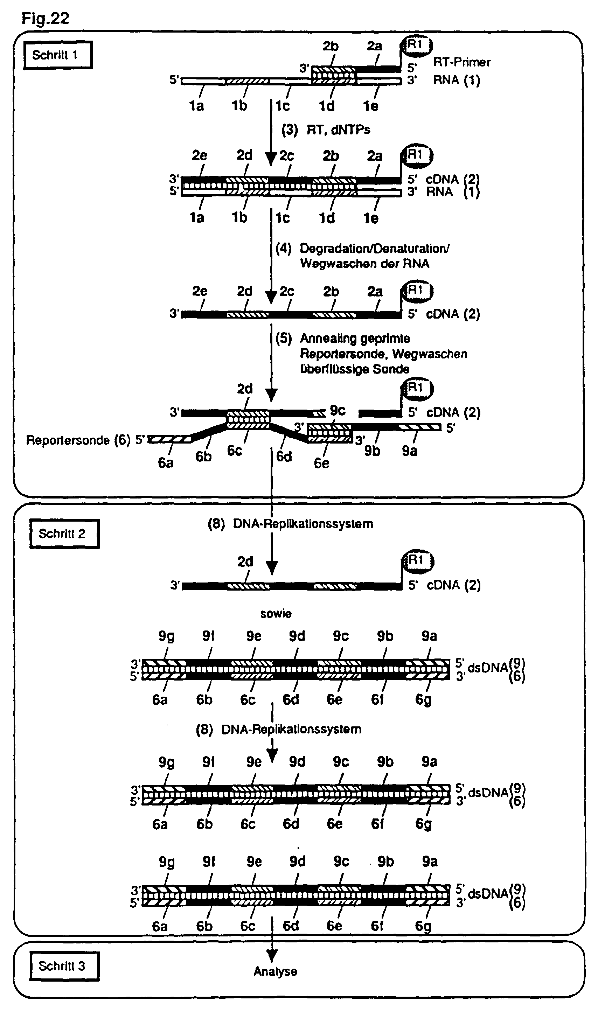

Bei diesen PERT-Varianten wird durch die im Untersuchungsmaterial vorhandene RT-Aktivität aufgrund einer geprimten Template-Nukleinsäure eine cDNA synthetisiert. Falls nicht anders vermerkt, wird als Template-Nukleinsäure bevorzugterweise eine reine RNA eingesetzt. Ausgehend von der cDNA wird sodann eine zumindest partiell doppelsträngige DNA aufgebaut, die mindestens an einem Ende mit einem DNA-ori, und am anderen Ende mit mindestens einer DNA-ori-Sequenz für ein bestimmtes DNA-Replikationssystems versehen ist. Nach Zugabe dieser primären replikativen DNA zum entsprechenden Replikationssystem erfolgt durch vielfach wiederholte Replikation der DNA in Schritt 2 deren Amplifikation. Im Schritt 3 wird die dsDNA nachgewiesen. In Fig. 18 - 20 sind Varianten dargestellt, die nach diesem Prinzip funktionieren. Wie bei den anderen Amplifikationsverfahren besteht auch hier die Möglichkeit, die in der RT-Reaktion (3) synthetisierte cDNA ausschliesslich dazu zu verwenden, um die Amplifikation ausgehend von einer Reporter-DNA zu induzieren. Die Fig. 21 und 22 illustrieren diese Möglichkeit.

**Fig. 18** zeigt einen ersten möglichen Reaktionsablauf, bei welchem die notwendigen funktionellen Sequenzen zur späteren Inititation der DNA-Replikation ausschliesslich durch die Primer in die dsDNA eingebracht werden.

Schritt 1: Die vorgelegte Template-Nukleinsäure (1) besteht aus den folgenden funktionellen Sequenzen: Einer flankierenden Sequenz (1a) am 5'-Ende, einer Hybridisierungssequenz (1b), einer Spacer-Sequenz (1c), einer zweiten Hybridisierungssequenz (1d) für das Annealing des RT-Primers, und einer flankierenden Sequenz (1e) am 3'-Ende. (1a) und (1e) sind fakultativ, alle anderen Sequenzen sind notwendig.

Der RT-Primer (R1)(2a)(2b)(2c) ist eine ssDNA, vorzugsweise ein Oligonukleotid. Er enthält in (2a) eine DNA-ori(-)-Sequenz, daran anschliessend eine Spacer-Sequenz (2b) und am 3'-Ende eine Hybridisierungssequenz (2c) zur Hybridisierung an (1d) der Template-Nukleinsäure (1). (2a) und (2c) sind notwendig, (2b) und (R1) sind fakultativ. Die im aufgearbeiteten Untersuchungsmaterial enthaltene RT-Aktivität synthetisiert in Reaktion (3) eine cDNA (2) der Zusammensetzung (R1)(2a)(2b)(2c)(2d)(2e)(2f). Für das Funktionieren des PERT Assay müssen in der RT-Reaktion zumindest die funktionellen Sequenzen bis (2e) synthetisiert werden.

Das aus Reaktion (3) hervorgehende Gemisch von Nukleinsäuren, bestehend aus unreagierten Template-Primer-Kombinationen (1)//(R1)-(2a)(2b)(2c), freien Primer-Molekülen (R1)(2a)(2b)(2c), nicht geprimten Template-Nukleinsäure-Molekülen (1) und cDNA-Template-Duplexmolekülen (1)//(2), wird in Reaktion (4) derart modifiziert, dass sichergestellt wird, dass keine dieser Komponenten in unerwünscher Weise mit dem optimalen Ablauf der nachfolgenden Reaktionen interferiert. Insbesondere muss verhindert werden, dass die in grosser Menge vorhandene Template-Nukleinsäure (1) und/oder die Template-Primer-Kombinationen (1)//(R1)(2a)(2b)(2c) mit der Hybridisierung (5) des zweiten Oligonukleotidprimers (R2)(6a)(6b) (6c) an die cDNA (2) kompetitiert. Dies führt zu einer verminderten Sensitivität des Nachweises oder gar zu falsch negativen Resultaten.

Es ist daher nötig, in Reaktion (4) die vorhandene Template-Nukleinsäure, die RNA (1), als relevanten Teilnehmer an der Annealingreaktion (5) zu eliminieren. Falls keine maximale Sensitivität erreicht werden muss, kann auch auf die Entfernung der Template-Nukleinsäure aus dem Reaktionsgemisch verzichtet werden.

In Reaktion (5) erfolgt das Annealing eines zweiten ssDNA-Primers, vorzugsweise eines Oligonukleotids. Dieser Primer enthält als notwendige Sequenzen am 5'-Ende eine DNA-ori(-)-Sequenz (6a) und am 3'-Ende eine Hybridisierungssequenz (6c) zur Hybridisierung an (2e) der cDNA (2). Fakultativ sind die Spacer-Sequenz (6b) sowie die funktionelle Gruppe (R2). In Reaktion (7) wird durch Wirkung einer DNA-abhängigen DNA-Polymerase und unter Verwendung von dNTPs eine partiell doppelsträngige DNA, bestehend aus den Strängen (2) und (6) synthetisiert.

Schritt 2: Die partiell doppelsträngige DNA (2)//(6), welche an beiden Enden eine DNA-ori-Sequenz trägt, ist die primäre replikative DNA und wird in das entsprechende Amplifikationssystem eingegeben. Durch die Einwirkung der DNA-Replicase (8) des Replikations-systems entsteht durch Replikation, ausgehend von dem aktiven doppelsträngigen DNA-ori (2a)//(6g), das vollständig doppelsträngige DNA-Produkt (R2)(6)//(2); infolge der Strangtrennungsaktivität der DNA-Replicase wird dabei die (R1) tragende cDNA (2) freigesetzt. Das doppelsträngige DNA-Produkt (R2)(6)//(2) mit den zwei aktiven DNA-ori, (2a)//(6g) und (6a)//(6a'), wird in der Folge durch vielfach wiederholte Replikation (8) amplifiziert.

Schritt 3: Als Produkt der Amplifikationsreaktion entsteht eine dsDNA (6)//(2), die mit geeigneten Methoden nachgewiesen wird.

### Modifikationen:

Die als nicht notwendig bezeichneten funktionellen Sequenzen und funktionellen Gruppen von Template-Nukleinsäure (1) und Oligonukleotidprimern (R1)(2a)(2b)(2c) und (R2)(6a)(6b)(6c) können einzeln oder in den verschiedenen möglichen Kombinationen entfallen.

Abweichend vom Prinzip einer unterschiedlichen Basen-Sequenz in jeder funktionellen Sequenz ist es möglich, die flankierenden Sequenzen (1a) und (1e) sowie die Spacer-Sequenz (1c) der Template-Nukleinsäure (1), und die Spacer-Sequenzen (2b) und (6b) der Primer einzeln oder in Kombination identisch zu gestalten, unter der Voraussetzung, dass dies mit dem Ablauf des Tests in keiner Art und Weise interferiere. Ebenso können die Hybridisierungssequenzen (2c) und (6c) der Oligonukleotid-Primer (und folglich auch die Hybridisierungssequenzen (1b) und (1d) der Template-Nukleinsäure) eine identische, jedoch von den flankierenden und Spacer-Sequenzen unterschiedliche DNA-Sequenz aufweisen.

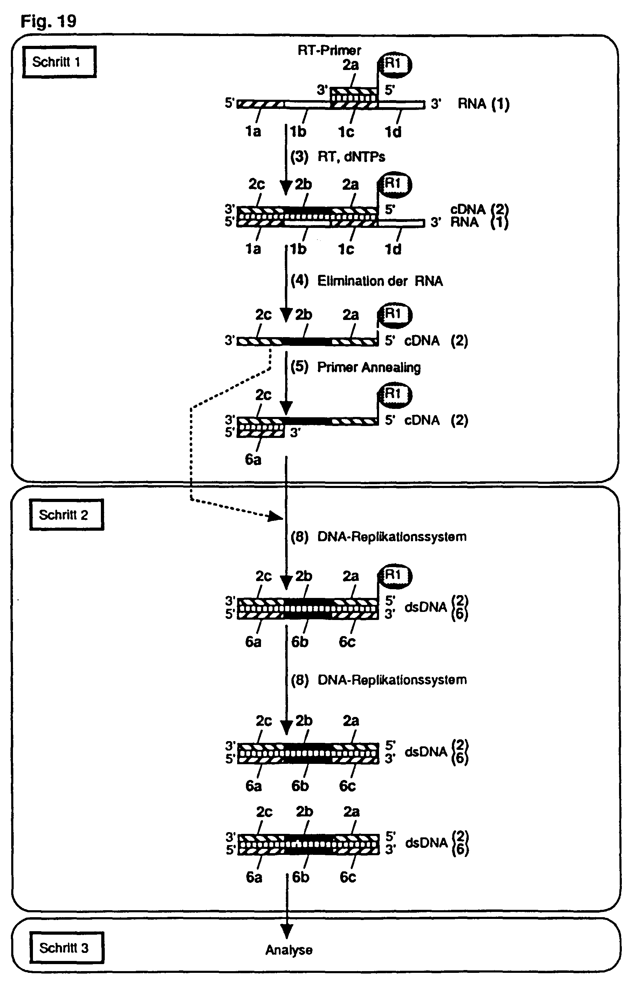

**Fig. 19** zeigt einen Reaktionsablauf, bei dem die zur späteren DNA-Replikation notwendigen DNA-ori-Sequenzen schon vollständig in der Template-Nukleinsäure kodiert vorliegen.

Schritt 1: Die vorgelegte Template-Nukleinsäure (1) besteht aus den folgenden funktionellen Sequenzen: Einer Hybridisierungssequenz (1a) am 5'-Ende, einer Spacer-Sequenz (1b), einer Hybridisierungssequenz (1c) für das Annealing des RT-Primers und einer flankierenden Sequenz (1d) am 3'-Ende. Die beiden Hybridisierungssequenzen (1a) und (1c) kodieren zudem die zwei DNA-ori-Sequenzen für die DNA-Replikation wie folgt: (1a) enthält eine DNA-ori (-)-Sequenz an ihrem 5'-Ende, Hybridisierungssequenz (1d) eine DNA-ori(+)-Sequenz am 3'-Ende. Die flankierende Sequenz (1d) ist fakultativ, alle anderen Sequenzen sind notwendig.

Der RT-Primer (R1)(2a) ist ein DNA-Oligonukleotid. Dieses enthält eine notwendige Hybridisierungssequenz (2a), die gleichzeitig eine DNA-ori(-)-Sequenz ist und zur Hybridisierung an (1d) der Template-Nukleinsäure (1) dient. (R1) ist fakultativ.

Die im aufgearbeiteten Untersuchungsmaterial enthaltene RT-Aktivität synthetisiert in Reaktion (3) eine cDNA (2) der Zusammensetzung (R1)(2a)(2b)(2c). Für das Funktionieren des PERT Assay müssen in der RT-Reaktion (3) zumindest die funktionellen Sequenzen bis (2c) synthetisiert werden.

Aus den gleichen Gründen wie in Fig. 18 ist es nötig, in Reaktion (4) die vorhandene Template-Nukleinsäure, die RNA (1), als relevanten Teilnehmer an der Annealingreaktion (5), wie zuvor beschrieben, zu eliminieren. Falls keine maximale Sensitivität erreicht werden muss, kann unter Umständen auch auf die Entfernung der Template-Nukleinsäure aus dem Reaktionsgemisch verzichtet werden. In Reaktion (5) erfolgt sodann das Annealing eines zweiten DNA-Oligonukleotidprimers. Dieser Primer enthält die notwendige Hybridisierungssequenz (6a) zur Hybridisierung an (2c) der cDNA (2); (6a) ist gleichzeitig eine DNA-ori(-)-Sequenz. (R2) ist fakultativ. Durch das Annealing des Oligonukleotids (6a) entsteht eine primäre replikative DNA mit einem aktiven DNA-ori (2c)//(6a). Diese stellt die zu vermehrende Nukleinsäure dar.

Schritt 2: Die partiell doppelsträngige DNA (R1)(2)//(6a), welche an beiden Enden eine DNA-ori-Sequenz trägt, wird in das entsprechende Amplifikationssystem eingegeben. Durch die Einwirkung der DNA-Replicase (8) des Replikationssytems entsteht zuerst die vollständig doppelsträngige DNA (R1)(2)//(6). Die doppelsträngige, (R1) tragende DNA (2)//(6) mit den zwei aktiven DNA-ori (2a)//(6c) und (6a)//(2c) wird in der Folge durch vielfach wiederholte Replikation (8) amplifiziert.

Schritt 3: Als Produkt entsteht eine dsDNA (6)//(2), die mit geeigneten Methoden nachgewiesen wird.

### Modifikationen:

Die als nicht notwendig bezeichneten flankierende Sequenz (1d) der Template-Nukleinsäure (1) kann auch entfallen.

Abweichend vom Prinzip einer unterschiedlichen Basen-Sequenz in jeder funktionellen Sequenz ist es möglich, die flankierenden Sequenz (1d) und die Spacer-Sequenz (1c) der Template-Nukleinsäure (1) identisch zu gestalten, unter der Voraussetzung, dass dies mit dem Ablauf des Tests in keiner Art und Weise interferiere.

Die zwei Primer (R1)(2a) und (6a), die in der beschriebenen Variante die minimal notwendige DNA-ori (-)-Sequenz umfassen, können derart modifiziert werden, dass sie am 3'-Ende kürzer sind als diese, oder dass sie sich über das 3'-Ende dieser minimalen DNA-ori(-)-Sequenz hinaus erstrecken.

Die cDNA (2) ist in dieser Variante ebenfalls eine replikative DNA, indem unter Bildung einer "Stem-Loop" Struktur die beiden DNA-ori-Sequenzen (2c) und (2a) miteinander hybridisieren und einen funktionellen DNA-ori (2a)//(2c) bilden. Die Zugabe des Oligonukleotids (6a) kann deshalb auch entfallen. Allerdings wird dadurch die Effizienz der initialen DNA-Replikation reduziert.

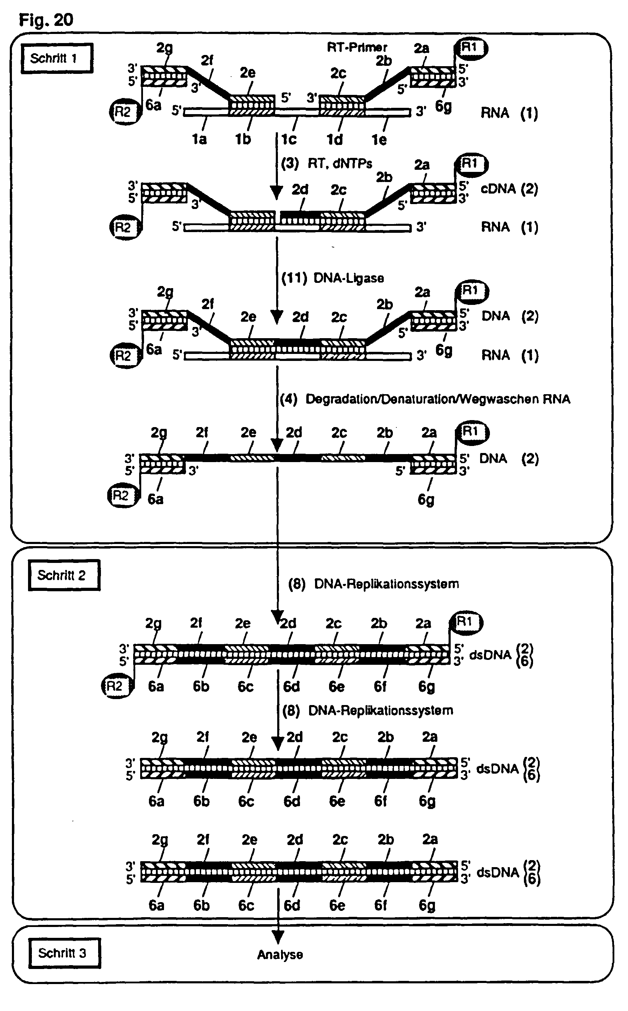

**Fig. 20** illustriert eine Variante, bei der als Primer und zum Einführen der DNA-ori-Sequenzen Adaptoren anstelle von einzelsträngigen Oligonukleotiden eingesetzt werden.

Schritt 1: Die vorgelegte Template-Nukleinsäure (1) besteht aus den folgenden funktionellen Sequenzen: Einer flankierenden Sequenz (1a) am 5'-Ende, einer Hybridisierungssequenz (1b), einer Spacer-Sequenz (1c), einer zweiten Hybridisierungssequenz (1d) für das Annealing des RT-Primers (R1)(2a)(2b)(2c), und einer flankierenden Sequenz (1e) am 3'-Ende. (1a) und (1e) sind fakultativ, alle anderen funktionellen Sequenzen sind notwendig.

Der RT-Primer (R1)(2a)(2b)(2c)//(6g) ist ein mehrteiliger Adaptor und besteht aus einem DNA-Oligonukleotid (R1)(2a)(2b)(2c), das mit einem zweiten Oligonukleotid (6g), welches an (2a) anhybridisiert ist, eine partiell doppelsträngige DNA bildet. Dieser Adaptor enthält an seinem 3'-Ende die Hybridisierungssequenz (2c), die mit (1d) der Template-Nukleinsäure (1) hybridisiert ist. Daran anschliessend befindet sich eine Spacer-Sequenz (2b), gefolgt von einer DNA-ori(-)-Sequenz (2a). Das Oligonukleotid (6g) enthält die zu (2a) komplementäre DNA-ori(+)-Sequenz. Die funktionellen Sequenzen (2a) und (2c) sind notwendig, die restlichen sowie (R1) sind fakultativ.

Der zweite, an die Hybridisierungssequenz (1b) der Template-Nukleinsäure (1) anhybridisierte Adaptor (2e)(2f)(2g)//(R2)(6a) besteht aus den DNA-Oligonukleotiden (2e)(2f)(2g) und (R2)(6a), wobei das Oligonukleotid (2e)(2f)(2g) an seinem 5'-Ende phosphoryliert ist. (2e) enthält die zur Hybridisierung an (1b) verwendete Hybridisierungssequenz; (2f) ist eine Spacer-Sequenz und (2g) eine DNA-ori(+)-Sequenz, welche gleichzeitig eine Hybridisierungssequenz ist. An (2g) ist das DNA-Oligonukleotid (6a) anhybridisiert, welches die zu (2g) komplementäre DNA-ori(-)-Sequenz enthält. Die Spacer-Sequenz (2f) ist fakultativ, alle anderen Sequenzen sind für den Test notwendig. Als fakultatives Element kann das Oligonukleotid (6a) auch eine funktionelle Gruppe (R2) aufweisen.

Die im aufgearbeiteten Untersuchungsmaterial enthaltene RT-Aktivität synthetisiert in Reaktion (3) eine cDNA (2) der Zusammensetzung (R1)(2a)(2b)(2c)(2d)//(6g), welche zusammen mit der Template-Nukleinsäure (1) in Form einer partiellen cDNA-Template-Duplex (2)//(1) vorliegt und vom 5'-Ende des an die Hybridisierungssequenz (1b) der Template-Nukleinsäure (1) hybridisierten 2. Adaptors (2e)(2f)(2g)//(R2)(6a) nur durch ein Nick getrennt ist. In Reaktion (11) werden die zwei durch ein Nick voneinander getrennten DNA-Stränge (2a)(2b)(2c)(2d) der cDNA und (2e)(2f)(2g) des zweiten Adaptors mit Hilfe einer DNA-Ligase, die auch auf DNA-RNA-Heteroduplex-Substrat aktiv ist, z.B. T4-DNA-Ligase, zu einem zusammenhängenden DNA-Molekül (2a)(2b)(2c)(2d)(2e)(2f)(2g) verbunden.

In Reaktion (4) wird die vorhandene Template-Nukleinsäure, die RNA (1), vor der nachfolgenden Amplifikationsreaktion eliminiert. Falls keine maximale Sensitivität erreicht werden muss, kann unter Umständen auch auf die Entfernung der RNA aus dem Reaktionsgemisch verzichtet werden.

Eine Entfernung der RNA ist nötig, da in Schritt 2 infolge der Strangtrennungsaktivität der im Replikationssytem vorliegenden DNA-abhängigen DNA-Polymerase einzelsträngige DNA-Moleküle (2a)(2b) (2c)(2d)(2e)(2f)(2g) entstehen, welche durch intramolekulare Hybridisierung der beiden komplementären DNA-ori-Sequenzen (2a) und (2g) einen funktionellen DNA-ori bilden, der vom Replikationssystem benützt wird, um das einzelsträngige Molekül (2) wieder in eine dsDNA (2)//(6) überzuführen. Da die Template-Nukleinsäure (1) in grosser Menge im Reaktionsgemisch vorliegt, kann sie mit dem teilweise komplementären ssDNA-Molekül (2) hybridisieren und die für die effiziente Amplifikation notwendige intramolekulare Bildung eines funktionellen DNA-ori beeinträchtigen.

Schritt 2: Die DNA (6g)//(R1)(2a)(2b)(2c)(2d)(2e)(2f)(2g)//(R2) (6a), welche an beiden Enden eine doppelsträngige DNA-ori-Sequenz enthält, ansonsten jedoch einzelsträngig ist, stellt die primäre replikative DNA dar und wird in das entsprechende Amplifikationssystem eingegeben. Durch die Einwirkung der DNA-Replicase (8) des Replikationssystems entsteht die vollständig doppelsträngige, (R1) und (R2) tragende DNA (6)//(2). Diese enthält die zwei aktiven DNA-ori (6a)//(2g) und (2a)//(6g) an ihren Enden und wird in der Folge durch vielfach wiederholte Replikation (8) amplifiziert.

Schritt 3: Als Produkt der Amplifikationsreaktion entsteht eine dsDNA (6)//(2), die mit geeigneten Methoden nachgewiesen wird.

### Modifikationen:

Alle als fakultativ bezeichneten funktionellen Sequenzen und funktionellen Gruppen der Template-Nukleinsäure (1) und der Adaptoren (R1)(2a)(2b)(2c)//(6a) und (2e)(2f)(2g)//(R2)(6a) können einzeln oder in den verschiedenen möglichen Kombinationen entfallen.

Abweichend vom Prinzip einer unterschiedlichen Basen-Sequenz in jeder funktionellen Sequenz ist es möglich, die flankierenden Sequenzen (1a) und (1e) sowie die Spacer-Sequenz (1c) der Template-Nukleinsäure (1), und die Spacer-Sequenzen (2b) und (2f) der Adaptoren einzeln oder in Kombination identisch zu gestalten, unter der Voraussetzung, dass sie mit dem Ablauf des Tests in keiner Art und Weise interferieren.

Die Länge der beiden kürzeren Oligonukleotide (6a) und (6g) der zwei Adaptoren, in der illustrierten Variante als diejenige der DNA-ori-Sequenz definiert, kann an ihrem 3'-Ende auch kürzer oder länger sein als die minimale DNA-ori-Sequenz.

Die Unterscheidung in Varianten mit einzelsträngigen Oligonukleotidprimern und mit Adaptoren ist nicht zwingend. Es ist durchaus möglich, sinnvolle Mischformen der in Fig. 18 bis 20 dargestellten Varianten zu entwerfen. Ebenso ist die in den Fig. 18 und 20 beschriebene ausschliessliche Zuordnung einer einzigen Funktion pro funktioneller Sequenz für den PERT Assay nicht zwingend. Eine Hybridisierungssequenz kann durchaus mit einer anderen funktionellen Sequenz überlappen, diese vollständig in sich enthalten oder mit ihr identisch sein, wie in Fig. 19 für die Hybridisierungs- und die DNA-ori(-)-und DNA-ori(+)-Sequenzen beschrieben.

Wie erwähnt, ist die Verwendung einer Template-Nukleinsäure (1), die nebst der Template-RNA auch eine Template-DNA enthält, in gewissen Konstellationen von Vorteil, indem die Synthese einer primären replikativen DNA stark vereinfacht wird. Wenn beispielsweise bei der in Fig. 19 gezeigten Template-Nukleinsäure (1) die DNA-ori(-)-Sequenz (1a) in DNA anstelle von RNA vorgelegt wird, entsteht als Resultat der RT-Reaktion (3) eine primäre replikative DNA mit einem aktiven DNA-ori (2c)//(1a). Diese stellt die zu vermehrende Nukleinsäure dar und kann im entsprechenden Replikationssystem direkt vermehrt werden.

Die in Fig. 21 und 22 beschriebenen Varianten benützen als Template-Nukleinsäure wiederum bevorzugterweise eine reine RNA. Sie verwenden die durch die RT-Aktivität synthetisierte cDNA nur zur spezifischen Anhybridisierung einer teilweise komplementären Reportersonde, welche entweder selbst in Schritt 2 amplifiziert wird (Fig. 21), oder welche als Ziel-DNA, deren Anwesenheit anschliessend nachgewiesen wird, fungiert (Fig. 22).

**Fig. 21** beschreibt einen Reaktionablauf, bei dem als Reportersonde eine primäre replikative DNA verwendet wird, welche in Schritt 2 direkt das Substrat für die Amplifikationsreaktion bildet.

Schritt 1: Sowohl die notwendigen und entbehrlichen funktionellen Sequenzen von Template-Nukleinsäure (1) und RT-Primer (R1)(2a)(2b) als auch die Möglichkeiten und Anforderungen für (R1) sind identisch zu denjenigen in Fig. 3. Im übrigen gilt die Anforderung, dass die zur Hybridisierungsequenz (2d) der cDNA als einzige und ausschliesslich mit der zur spezifischen Hybridisierung bestimmten Sequenz der als Reportersonde eingesetzten DNA reagiert.

Das aus der RT-Reaktion (3) hervorgehende Gemisch von Nukleinsäuren wird in Reaktion (4) derart modifiziert, dass sichergestellt werden kann, dass keine Nukleinsäure in der Hybridisierungsreaktion (5) der Reportersonde (6) mit der cDNA (2) als spezifischer Kompetitor auftreten kann und dadurch zu einer verminderten Sensitivität des Nachweises oder gar zu falsch negativen Resultaten führt. In Reaktion (4) wird die Template-Nukleinsäure (1) deshalb mit einer der genannten Methoden eliminiert. An die cDNA (2) wird zunächst in Reaktion (5a) als Reportersonde (6)//(9a) eine primäre replikative DNA anhybridisiert. Diese Reportersonde (6)//(9a) enthält die folgenden funktionellen Sequenzen: (6a) ist eine DNA-ori(-)-Sequenz; (6b) bezeichnet diejenige Spacer-Sequenz, die (6a) von der zur spezifischen Hybridisierung an (2d) der cDNA eingesetzten Hybridisierungssequenz (6c) trennt; (6d) ist eine weitere Spacer-Sequenz und (6e), am 3'-Ende gelegen, eine DNA-ori(+)-Sequenz. (9a) ist ein mit (6e) hybridisiertes DNA-Oligonukleotid, welches aus einer Hybridisierungssequenz besteht, die gleichzeitig eine DNA-ori(-)-Sequenz ist. Die beiden Spacer-Sequenzen (6b) und (6d) sind fakultativ, alle anderen Sequenzen sind für das Funktionieren des PERT Assay notwendig.

Nach dem Annealing der Reportersonde (6)//(9a) an die cDNA (2) wird nichtgebundene. Reportersonde unter nichtdenaturierenden Bedingungen vollständig aus dem Reaktionsgemisch entfernt, wie in Fig. 3 beschrieben.

Schritt 2: Die Kombination von cDNA (2) mit der anhybridisierten Reportersonde (R1)(2)//(6)//(9a), stellt das Substrat für die Replikation in Schritt 2 dar, wobei die Reportersonde (6)//(9a) die zu vermehrende Nukleinsäure ist. Durch die Einwirkung der DNA-Replicase (8) des Replikationssytems entsteht die vollständig doppelsträngige DNA (6)//(9), und infolge der Strangtrennungsaktivität der DNA-Replicase wird die cDNA (R1)(2) freigesetzt. Die dsDNA (6)//(9) wird in der Folge durch vielfache wiederholte Replikation (8) amplifiziert.

Schritt 3: Das hauptsächliche Produkt der Amplifikation ist die dsDNA (6)//(9), die mit geeigneten Methoden nachgewiesen wird.

### Modifikationen:

Die als fakultativ benannten funktionellen Sequenzen und funktionellen Gruppen der Template-Nukleinsäure (1), des RT-Primers (R1)(2a)(2b) und der Reportersonde (6)//(9a) können, einzeln oder in den verschiedenen möglichen Kombinationen, entfallen.

Die Template-Nukleinsäure kann mehrere Hybridisierungssequenzen (1b) für Reportersonden und/oder mehrere Hybridisierungssequenzen (1d) für RT-Primer enthalten.

Der Primer für die RT-Reaktion kann sowohl eine DNA als auch eine RNA oder eine Kombination mit Anteilen beider sein sein.

Das an die Reportersonde anhybridisierte Oligonukleotid (9a) kann in der Länge variieren. Es muss im Minimum die ersten 6 Nukleotide der DNA-ori-Sequenz umfassen, kann jedoch auch an seinem 3'-Ende länger sein, so dass es zusätzliche, zu (6d) komplementäre Basen enthält. Die Länge des Oligonukleotids (9a) muss so gewählt werden, dass die Stabilität der Bindung an die DNA (6) so gross ist, dass sie auch beim Wegwaschen der ungebundenen Reportersonde (6)//(9a) bestehen bleibt.

Es ist auch möglich, als Reportersonde nur die DNA (6) an die cDNA zu hybridisieren und eine primäre replikative DNA herzustellen, indem das Oligonukleotid (9a) erst nach dem Wegwaschen ungebundener DNA (6) in einer weiteren Annealingreaktion an die DNA (6) angelagert wird.

Abweichend vom Prinzip einer einzigen Funktion in einer funktionellen Sequenz ist es möglich, die DNA-ori-Sequenz (6a) in die zur Hybridisierung der Reportersonde (6)//(9a) an die cDNA (2) verwendete Hybridisierungssequenz teilweise oder auch vollständig einzubeziehen. Die entsprechenden Spacer-Sequenz (6b) entfällt dann. Die Anwesenheit mindestens einer Spacer-Sequenz auf der Reportersonde wird dann allerdings notwendig.

Abweichend vom Prinzip einer unterschiedlichen Basensequenz in jeder funktionellen Sequenz ist es möglich, die flankierenden und die Spacer-Sequenzen von Template-Nukleinsäure (1), des Primers (R1)(2a)(2b) sowie der Reportersonde (6)//(9a), einzeln oder in jeder möglichen Kombination identisch zu gestalten, unter der Voraussetzung, dass dies mit dem Ablauf des Tests in keiner Art und Weise interferiert.

**Fig. 22** zeigt eine Variante, welche zur Hybridisierung an die cDNA eine DNA-Reportersonde verwendet, von welcher ausgehend eine primäre replikative DNA synthesisiert wird.

Schritt 1: Sowohl die notwendigen und entbehrlichen funktionellen Sequenzen von Template-Nukleinsäure (1) und RT-Primer (R1)(2a)(2b) als auch die Möglichkeiten und Anforderungen für (R1) sind identisch zu denjenigen in Fig. 3.

Aus den gleichen Gründen wie in Fig. 18 beschrieben wird zunächst in Reaktion (4) die Template-Nukleinsäure, die RNA (1), mit einer der genannten Methoden eliminiert. An die cDNA (2) wird sodann in Reaktion (5) als Reportersonde eine partiell doppelsträngige DNA (6a)(6b)(6c)(6d)(6e)//(9c)(9b)(9a) anhybridisiert. Die Reportersonde enthält die folgenden funktionellen Sequenzen: (6a) ist eine DNA-ori(-)-Sequenz; (6b) ist eine Spacer-Sequenz; (6c) ist die Hybridisierungssequenz für die spezifische Hybridisierung an die cDNA; (6d) ist eine weitere Spacer-Sequenz; und (6e) ist die Hybridisierungssequenz zur Hybridisierung der DNA (6a)(6b)(6c)(6d) (6e) mit der DNA (9c)(9b)(9a). (9c) ist die Hybridisierungssequenz für die Hybridisierung mit (6e); (9b) ist ein Spacer-Sequenz und (9a) eine DNA-ori(-)-Sequenz. Die Spacer-Sequenzen (6b), (6d) und (9b) sind fakultativ. Alle anderen funktionellen Sequenzen sind für das Funktionieren des PERT Assay notwendig.

Spätestens nach dem Annealing dieser Reportersonde an die cDNA (2) wird diese mittels der funktionellen Gruppe (R1) an einen Carrier gebunden und nichtgebundene Reportersonde unter nichtdenaturierenden Bedingungen vollständig aus dem Reaktionsgemisch entfernt.

Schritt 2: Die Kombination von cDNA mit der anhybridisierten Reportersonde (R1)(2)//(6a)(6b)(6c)(6d)(6e)//(9c)(9b)(9a) stellt das Substrat für die DNA-Replicase in Schritt 2 dar. Durch die Einwirkung der DNA-Replicase (8) des Replikationssytems entsteht zuerst eine vollständig doppelsträngige replikative DNA (6)//(9), und infolge der Strangtrennungsaktivität der DNA-Replicase wird die cDNA (R1)(2) freigesetzt. Die dsDNA (6)//(9) wird in der Folge durch vielfache wiederholte Replikation (8) amplifiziert.

Schritt 3: Das dsDNA-Produkt der Amplifikation wird mit geeigneten Methoden nachgewiesen.

### Modifikationen:

Alle als fakultativ benannten Elemente der Template-Nukleinsäure (1), des RT-Primers (R1)(2a)(2b) und der Reportersonde (6a)(6b) (6c)(6d)(6e)//(9c)(9b)(9a) können, einzeln oder in den verschiedenen möglichen Kombinationen, entfallen. Der für die RT-Reaktion eingesetzte Primer kann sowohl DNA als auch RNA sein.

Abweichend vom Prinzip einer einzigen Funktion in einer funktionellen Sequenz ist es möglich, die zur Hybridisierung der Reportersonde (6a)(6b)(6c)(6d)(6e)//(9c)(9b)(9a) an die cDNA (2) verwendete Hybridisierungssequenz (6c) mit der DNA-ori(-)-Sequenz (6a) teilweise oder vollständig überlappend, oder auch identisch zu gestalten. Die entsprechende Spacer-Sequenz zwischen der Hybridisierungssequenz (6c) und der DNA-ori (-)-Sequenz entfällt dann.

Abweichend vom Prinzip einer unterschiedlichen Sequenz in jeder funktionellen Sequenz ist es möglich, die flankierenden und die Spacer-Sequenzen der Template-Nukleinsäure (1), des Primers (R1)(2a)(2b) sowie der Reportersonde (6a)(6b)(6c)(6d)(6e)//(9c) (9b)(9a), einzeln oder in jeder möglichen Kombination identisch zu gestalten, unter der Voraussetzung, dass dies mit dem Ablauf des Tests in keiner einer Art und Weise interferiere.

Die Herstellung der primären replikativen DNA kann bereits in Schritt 1 durch eine DNA-Polymerase erfolgen, anstatt in Schritt 2 durch die DNA-Replicase.

### Aufbau des PERT bei Verwendung der "Amplification Method for Polynucleotide Assays" (PS-US 4994368) in Schritt 2

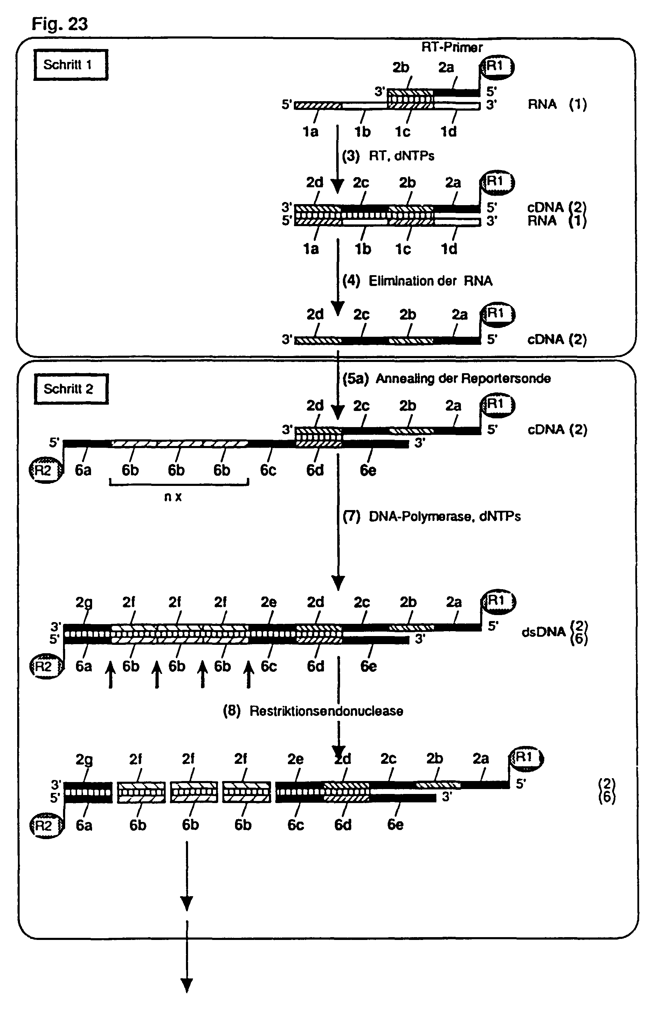

**Fig. 23** zeigt den Reaktionsablauf des PERT Assay, wenn in Schritt 2 zur Amplifikation das in PS-US 4994348 beschriebene Verfahren eingesetzt wird.

Schritt 1: Vorgelegt wird eine Template-Nukleinsäure, welche bevorzugterweise eine reine RNA ist und die funktionellen Sequenzen (1a)(1b)(1c)(1d) enthält. (1a) ist eine notwendige Hybridisierungssequenz die nicht aus DNA bestehen darf. Die davon abgeleitete Sequenz (2d) dient der Bindung der Reportersonde in Reaktion (5a). (1c) ist ebenfalls eine notwendige Hybridisierungssequenz und dient der Bindung des RT-Primers (R1)(2a)(2b). (1b) ist eine Spacer-Sequenz und (1d) ist eine flankierende Sequenz. Diese letzten beiden sind fakultativ.

Der RT-Primer (R1)(2a)(2b) ist eine einzelsträngige Nukleinsäure, vorzugsweise ein DNA- oder RNA-Oligonukleotid, und besteht aus der fakultativen funktionellen Gruppe (R1), der ebenfalls entbehrlichen flankierenden Sequenz (2a) sowie der zur Hybridisierung an (1c) der Template-Nukleinsäure (1) verwendeten notwendigen Hybridisierungssequenz (2b).

Bei Anwesenheit von RT-Aktivität im aufgearbeiteten Untersuchungsmaterial wird in Reaktion (3) unter Einbau der benötigten dNTPs eine cDNA (2) synthetisiert. Für das Funktionieren des PERT Assay müssen alle funktionellen Sequenzen bis (2d) in der cDNA vorhanden sein.

Mit Ausnahme der zu (1a) komplementären Hybridisierungssequenz (2d) der cDNA gilt für alle funktionellen Sequenzen der Template-Nukleinsäure (1) und des RT-Primers (R1)(2a)(2b) sowie alle davon abgeleiteten Sequenzen die Anforderung, dass sie keine spezifische Interaktion mit der Reportersonde eingehen dürfen. Die Hybridisierungssequenz (2d) ist die einzige, die diese Bindung eingehen kann.

Das aus Reaktion (3) hervorgehende Gemisch von Nukleinsäuren, bestehend aus unreagierten Template-Primer-Kombinationen (1)//(R1)-(2a)(2b), freien Primermolekülen (R1)(2a) (2b), nicht geprimten Template-Nukleinsäure-Molekülen (1) und cDNA-Template-Duplexmolekülen (1)//(2), wird in Reaktion (4) derart modifiziert, dass sichergestellt werden kann, dass keine dieser Komponenten, insbesondere die Template-Nukleinsäure (1) oder die Template-Primer-Kombinationen (1)//(R1)(2a)(2b), in den folgenden Reaktionen als spezifischer Kompetitor für die Reportersonde (6) auftreten und dadurch zu falsch-negativen Resultaten führen kann.

In Reaktion (4) wird deshalb die vorhandene Template-Nukleinsäure, die RNA (1), als relevanter Teilnehmer an der Annealingreaktion (5a), wie vorgängig beschrieben, eliminiert. Falls keine maximale Sensitivität erreicht werden muss, kann unter Umständen auf die Entfernung der Template-Nukleinsäure aus dem Reaktionsgemisch verzichtet werden.

Schritt 2: Die in Schritt 2 eingesetzte DNA-Reportersonde (6) sowie die einzelnen Reaktionsschritte zur Amplifikation entsprechen dem in PS-US 4994368 offenbarten Verfahren: An die Hybridisierungssequenz (2d) der cDNA (2) wird zunächst in Reaktion (5a) eine einzelsträngige DNA-Reportersonde (6) anhybridisiert, welche aus den funktionellen Sequenzen (6a)(6b)(6c)(6d)(6e) besteht, wobei (6b) mehrere Male hintereinander in gleicher Orientierung wiederholt ist. (6d) ist die notwendige Hybridisierungssequenz, die spezifisch mit der in der RT-Reaktion (3) synthetisierten Hybridisierungssequenz (2d) der cDNA (2) hybridisiert. Alle anderen funktionellen Sequenzen der Reportersonde (6) dürfen nicht mit der cDNA (2) hybridisieren oder Sequenzidentität mit Teilen von ihr aufweisen. Die mehrfach wiederholte Sequenz (6b) ist ebenfalls notwendig. Sie stellt in ihrer doppelsträngigen Form (6b)//(2f) die zu vermehrende Nukleinsäure sowie das Amplifikationsprodukt dar. Jeweils am Uebergang zur benachbarten funktionellen Sequenz weist sie eine Erkennungssequenz einer bestimmten Restriktionsendonuklease auf (mit Pfeilen markiert). (6c) ist eine fakultative Spacer-Sequenz, und (6a) und (6e) sind fakultative flankierende Sequenzen. Die funktionelle Gruppe (R2) ist fakultativ.

Nach der Hybridisierung (5a) der Reportersonde (6) wird in einer Reaktion (7), ausgehend von dem nun als Primer für diese Reaktion dienenenden 3'-Ende der in Reaktion (3) synthetisierten cDNA (2), mit Hilfe einer DNA-abhängigen DNA-Polymerase (z.B. Klenow-Fragment von E.coli DNA Polymerase I) und unter Einbezug von dNTPs die zu den funktionellen Sequenzen (6c)(6b)(6a) komplementäre DNA (2e)(2f)(2g) synthetisiert. Die dadurch entstandene dsDNA wird anschliessend mit der für die Verbindungsregionen spezifischen Restriktionsendonuklease geschnitten (8), wodurch jeweils mehrere Moleküle des doppelsträngigen Fragments (6b)//(2f) entstehen. In der Folge wird durch vielfache Wiederholung des Reaktionszyklus bestehend aus Hybridisierung (5a), DNA-Synthese (7) und Verdauung mit Restriktionsendonuklease (8) eine grosse Menge des doppelsträngigen Fragments (6b)//(2f) hergestellt.

Schritt 3: Das doppelsträngige Produkt (6b)//(2f) wird mit geeigneten Methoden nachgewiesen.

### Modifikationen:

Die als fakultativ benannten funktionellen Sequenzen und funktionellen Gruppen von Template-Nukleinsäure (1), RT-Primer (R1)(2a) (2b) und Reportersonde (6) können, einzeln oder in den verschiedenen möglichen Kombinationen, entfallen. Die Möglichkeit des Einsatzes einer Reportersonde mit flankierenden Sequenzen erlaubt jedoch die Verwendung sowohl von linearen als auch von zirkulären Molekülen als Reporter-DNA.

Abweichend vom Prinzip einer einzigen Funktion in einer funktionellen Sequenz kann die von (1a) der Template-Nukleinsäure (1) abgeleitete Hybridisierungssequenz (2d) nicht nur die Hybridisierung an die Reportersonde übernehmen, sondern gleichzeitig die zu (6b) komplementäre Sequenz enthalten und somit in Schritt 2 in das spezifische Amplifikationsprodukt eingehen. Die Spacer-Sequenz (6c) der Reportersonde sowie die davon abgeleitete Sequenz (2e) entfallen dann. Ausser einer vollständigen Identität dieser zwei. Funktionen sind auch partielle Ueberlappung dieser beiden Sequenzen oder auch die vollständige Integration der kürzeren der beiden Sequenzen in der längeren anderen möglich.

Abweichend von der Norm können die flankierende Sequenzen sowie die Spacer-Sequenzen der Template-Nukleinsäure (1) und der Reportersonde (6) paarweise oder in den möglichen Kombinationen identisch sein.

Damit diese PERT-Variante funktioniert, muss die zur Hybridisierung und zum Priming verwendete Hybridisierungssequenz (2d) der cDNA am 3'-Ende derselben liegen. In Fig. 23 wird dies dadurch erreicht, dass die zu (2d) komplementäre Hybridisierungssequenz (1a) der Template-Nukleinsäure (1) am 5'-Ende liegt. Es ist jedoch auch möglich, die Template-Nukleinsäure (1) um eine Sequenz am 5'-Ende zu verlängern und die Hybridisierungsequenz innerhalb der cDNA zu plazieren. Dazu muss die Template-Nukleinsäure (1) am Ende der zur Hybridisierung verwendeten funktionellen Sequenz (2d) die Erkennungssequenz einer Restriktionsendonuklease aufweisen. Durch Anhybridisierung eines komplementären DNA-Oligonukleotids und anschliessender Verdauung mit der entsprechenden Restriktionsendonuklease bildet die Hybridierungssequenz das 3'-Ende der cDNA (2). Die das 3'-Ende bildende Sequenz der cDNA kann auch so gewählt werden, dass sie, nach der Entfernung der Template-Nukleinsäure (1), eine Stem-Loop-Struktur einnimmt, in deren Stem die Erkennungssequenz einer Restriktionsendonuklease ist. Durch Verdauung mit dem entsprechenden Enzym entsteht ein 3'-Ende, das nach Hybridisierung zum Priming von Reaktion (7) verwendet werden kann.

### Analyseverfahren zum Nachweis des Reaktionsprodukts (Schritt 3)

Nach Abschluss von Schritt 2 des PERT Assay liegt bei der PCR, der LCR und den auf replikativer DNA basierenden Amplifikationsverfahren eine dsDNA zur weiteren Analyse vor. Bei den auf einem Transcriptionsverfahren sowie bei dem auf replikativer RNA basierenden Amplifikationsverfahren ist es zur Hauptsache einzelsträngige RNA (ssRNA), die im Analyseschritt nachgewiesen wird. Welches Verfahren dabei gewählt wird, ist von verschiedenen Faktoren abhängig, z.B. von der Verfahrensvariante, die gewählt wird, der Ausrüstung, der verfügbaren Arbeitskraft etc. Es ist daher weder möglich, noch notwendig, für jede Variante von PERT Assays alle in Frage kommenden Analyseverfahren aufzulisten. Es bleibt stattdessen dem Fachmann überlassen, das für die jeweiligen Verhältnisse optimale Analyseverfahren auszuwählen.

Im Sinne einer illustrativen, nicht aber vollständigen Darstellung des weiten Feldes von Techniken, die für den Nachweis eines Amplifikationsprodukts aus Schritt 2 zur Verfügung stehen, seien die folgenden Möglichkeiten erwähnt:
a) Nachweis der DNA-, resp. RNA-Synthese in der Amplifikationsreaktion,
b) Nachweis des ssRNA- resp. dsDNA-Amplifikationsprodukts mittels chromatographischer oder elektrophoretischer Analyse,
c) Nachweis des ssRNA- resp. dsDNA-Amplifikationsprodukts mittels Hybridisierung mit spezifischen Sonden,
d) Nachweis mit Hilfe einer Kombination von Elementen aus a)-c).

Für diese Möglichkeiten stehen die folgenden methodischen Elemente zur Verfügung: Electrophorese:
- Gelelectrophorese (nativ, denaturierend): Agarose, Polyacrylamid, andere Gelmaterialien
- Kapillarelectrophorese
   Chromatographie:
- HPLC
   Hybridisierung:
- Hybridisierung in Lösung
- Hybridisierung auf Festphase: Membranen, Kugeln, Platten Reagenzien zur Darstellung von DNA und/oder RNA allgemein:
- Fluoreszenzfarbstoffe: Ethidiumbromid, Acridinorange, Hoechst-Farbstoffe, etc.

Reagenzien zur Markierung von Sonden:
- Nukleotide zur Markierung von Sonden: radioaktiv: ¹²⁵I, ¹³¹I, ³²P, ³⁵S, ³H, ¹⁴C in dNTP und rNTP nicht radioaktiv: Biotin-dUTP, Biotin-UTP, DIG-dUTP, DIG-UTP
- Reagenzien, die nicht Nukleotide sind, zur Markierung von Sonden : Photoreaktives Biotin, photoreaktives DIG, aminoreaktives Biotin, aminoreaktives DIG, sulfhydrylreaktives Biotin, sulfhydrylreaktives DIG, Fluoreszenzfarbstoffe (Rhodamin, FITC, etc.)

Reagenzien zur Markierung der DNA/RNA während der Synthese (in Schritt 2):
- Nukleotide zur Markierung während der Synthese (Schritt 2): radioaktiv: ¹²⁵I, ¹³¹I, ³²P, ³⁵S, ³H, ¹⁴C in dNTP und rNTP nicht radioaktiv: Biotin-dUTP, Biotin-UTP, DIG-dUTP, DIG-UTP.

### Ausführungsbeispiel 1

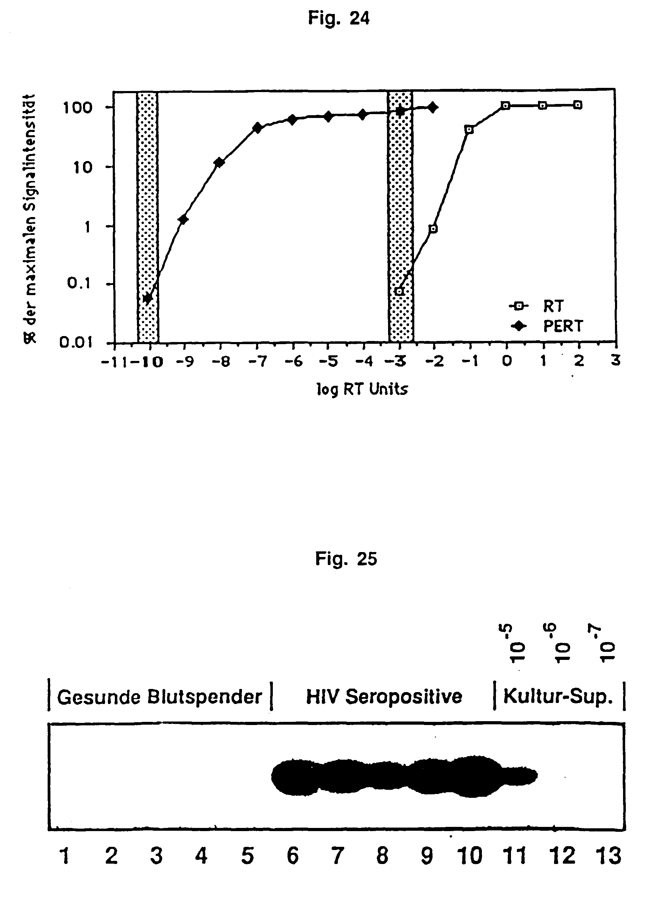

**Fig. 24** illustriert die erhöhte Sensitivität des PERT Assay gegenüber dem konventionellen RT-Test.

PERT Assay gemäss der in Fig. 2 beschriebenen Variante:

Schritt 1: RT-Reaktion: Als Template-Nukleinsäure für die RT-Reaktion im PERT Assay diente die Genom-RNA des Bacteriophagen MS2 (Boehringer Mannheim). Als Primer A wurde ein synthetisch hergestelltes Oligonukleotid mit der Basen-Sequenz 5'-d(CATAGGTCAAACCTCCTAGGAATG)-3' verwendet. Zur Herstellung der Template-Primer-Kombination wurde pro Reaktion 1 µg Template-Nukleinsäure (0.85 pmol) mit 220 ng Primer (27 pmol) in 4 µl Wasser gemischt und anschliessend 5 min bei 95 °C, 30 min bei 37 °C und 5 min bei 4 °C inkubiert. Die frisch hergestellte Template-Primer-Kombination wurde zu einer Lösung mit Reaktionspuffer, dNTPs, Dithiothreitol, RNase-Inhibitor, sowie bovinem Serum-Albumin pipettiert. Als RT diente ein rekombinantes Enzym des Moloney Murinen Leukämie-Virus (New England Biolabs). Zuerst wurde eine zehnfache Verdünnungsreihe, von 102 bis 10-10 Einheiten (U), in einer Lösung von 0.5 M KCl, 17 mM Tris-Cl pH 7.5, 3.3 mM DTT, 0.3 % Triton X-100™ und 33 % Glycerin hergestellt. Jeweils 2.5 µl dieser Lösung, mit der entsprechenden Aktivität, wurden dann zu einer Reaktionslösung mit einem Gesamtvolumen von 25 µl pipettiert. Die Reaktionsbedingungen waren folgendermassen: 50 mM Tris-Cl pH 8.3; 50 mM KCl; 8 mM MgC12; 10 mM Dithiothreitol; 1U/µl RNase-Inhibitor (RNasinTM); 0.12 µg/µl bovines Serum-Albumin; 0.032 % Triton® X-100; und 1mM je dATP, dCTP, dGTP, dTTP. Die Reaktion wurde während 3 h bei 37 °C inkubiert.

Im Anschluss an die RT-Reaktion wurde der RNase-Inhibitor durch Inkubation während 7 min bei 95 °C inaktiviert. Zur Degradierung der Template-Nukleinsäure wurde anschliessend jede Reaktion mit 2.0 ng RNAse A (United States Biochemical) während 30 min bei 37°C behandelt.

Schritt 2: Amplifikation mittels PCR: Die Amplifikation der in Schritt 1 synthetisierten, zu vermehrenden MS-2 cDNA erfolgte mittels PCR mit Primer A (siehe oben) sowie eines Primers B mit der Basen-Sequenz 5'-d(TCCTGCTCAACTTCCTGTCGAG)-3'. Für diese Reaktion wurden jeweils 10 µl (40 %) des mit RNAse A behandelten Produkts der RT-Reaktion in einem Gesamtvolumen von 100 µl eingesetzt und mit 100 µl Mineralöl überschichtet. Die Reaktionsbedingungen waren folgendermassen: 50 mM KCl; 14 mM Tris-Cl pH 8.3; 2.1 mM MgCl₂; 0.01 % Gelatine; 350 nM Primer A; 250 nM Primer B; 300 µM je dATP, dCTP, dGTP, dTTP; und 2.5 U Taq Polymerase. Für die Amplifikation wurden 25 Zyklen mit je einem Denaturierungssegment von 94 °C während 30 sec, einem Hybridisierungssegment von 55 °C während 1 min und 40 sec und einem Synthesesegment von 72 °C während 1 min und 50 sec auf einem programmierbaren Thermoblock (Techne PHC-2) durchgeführt.

Schritt 3: Analyse: Der Nachweis des Reaktionsprodukts erfolgte mittels Southern Blot-Hybridisierung. Dazu wurden 10 % der DNA aus jeder Amplifikationsreaktion auf einem 1.5 % Agarose-Gel aufgetrennt und auf eine Nylonmembran transferiert.. Zur Hybridisierung wurde ein Oligonukleotid C der Basen-Sequenz 5'-d(TTAATGTCTTTAGC-GAGACGC)-3' verwendet, das am 5'-Ende mit einer ³²P-enthaltenden Phosphatgruppe markiert wurde. Die Quantifizierung der radioaktiven Signale erfolgte durch Messung der Cerenkov-Strahlung.

### Konventioneller RT-Test:

Die Pufferbedingungen beim konventionellen RT-Test entsprachen denjenigen des PERT Assay. Die angegebene Menge Enzym war jeweils in 10 µl enthalten, das Reaktionsvolumen betrug 100 µl. Als Template-Primer-Kombination wurden 5 µg poly(rA).oligo(dT) (Pharmacia) eingesetzt, und als Deoxribonukleotidmonomer diente ³H-markiertes Thymidin-Triphosphat. Die Inkubation bei 37 °C erfolgte während 22 h. Die Analyse der in das Reaktionsprodukt inkorporierten Radioaktivität erfolgte im Anschluss an eine Trichloressigsäure-Präzipitation auf Nylonfilter mit einem Szintillationszähler.

Fig. 24 zeigt die Signalintensitäten für beide Verfahren in Abhängigkeit der verwendeten RT-Aktivität, ausgedrückt in Prozent der maximalen Signalintensität. Es ist klar ersichtlich, dass der PERT-Test bis weit unter die Detektionsgrenze (schraffierter Bereich) des konventionellen RT-Tests immer noch maximale Signalintensität zeigt und dass die Detektionsgrenze des PERT Assay mit 10⁻¹⁰ U um einen Faktor 10' tiefer liegt als diejenige des konventionellen RT-Tests.

### Ausführungsbeispiel 2

**Fig. 25** zeigt die Anwesenheit partikelassoziierten RT-Aktivität im Blutplasma von HIV-infizierten Personen sowie im Kulturüberstand von HIV-infizierten Zellen.

Jeweils 1 ml Blutplasma, aus EDTA-antikoaguliertem Blut gewonnen, wurde 30 min bei 14'000 x g zentrifugiert und anschliessend durch 0.2 µM Filter passiert. Anschliessend wurde das Volumen mit Phosphat-gepufferter isotonischer Salzlösung (PBS, GIBCO) auf 2.25 ml eingestellt und partikuläres Material (Viruspartikel) bei 82'000 x g während 2 h in der Ultrazentrifuge sedimentiert. Das Pellet wurde in 40 µl Puffer, bestehend aus 50 mM KCl, 25 mM Tris-Cl pH 7.5, 5 mM Dithiothreitol, 0.25 mM EDTA, 0.025 % Triton® X-100 sowie 50 % Glycerin resuspendiert. Anschliessend wurden mutmasslich vorhandene Viren durch Zugabe von 20 µl einer Lösung von 1.5 M KCl und 0.9 % Triton® X-100 lysiert. 2.5 µl (4 %) dieses Lysats wurden dann in eine RT-Reaktion gegeben.

Die Reaktionsbedingungen und die verwendete Template-Primer-Kombination für die RT-Reaktion entsprachen denjenigen in Beispiel 1. Die Reaktion erfolgte bei 37 °C während 5 h. Anschliessend wurde der RNase-Inhibitor durch Hitzebehandlung bei 95 °C während 7 min inaktiviert. Die Hydrolyse der aus RNA-bestehenden Template-Nukleinnsäure erfolgte durch Inkubation mit 0.8 ng RNase A während 30 min bei 37 °C in der PCR-Reaktionsmischung, unmittelbar vor dem Beginn der Amplifikationsreaktion. Die Bedingungen von PCR-Reaktionslösung, Zykluszahl und Thermoprofil waren identisch mit denen in Ausführungsbeispiel 1. Die Analyse der amplifizierten DNA erfolgte mittels Southern Blot-Hybridisierung mit Oligonukleoitd C, wie unter Ausführungsbeispiel 1 beschrieben. Das Resultat wurde anschliessend autoradiographisch auf Röntgenfilm festgehalten.

Fig. 25 zeigt, dass im Plasma von fünf gesunden Blutspendern (Spur 1 bis 5) keine RT-Aktivität feststellbar ist, während sie in den Plasmen aller HIV-Infizierten (Spur 6 bis 10) eindeutig nachgewiesen werden kann. Desgleichen kann in einem Kulturüberstand von HIV-infizierten Zellen (Spur 11 bis 13) bis zu einer Verdünnung von 1:1 Million eine RT-Aktivität festgestellt werden.

### Anwendung von PERT Assays

Der PERT Assay ist ein ultrasensitiver Assay zum Nachweis von RT-Aktivität. Diese wiederum ist ein charakteristisches Merkmal für Retroelemente. Zu diesen gehören nebst den Retroviren die retrovirusähnlichen Elemente (retrovirus-like elements) mit den Untereinheiten Retrotransposons, Pararetroviren (Caulimoviren und Hepadnaviren) und LINE (long interspersed nuclear elements), andererseits die nicht-retrovirus-ähnlichen Elemente (non-retrovirus-like elements) wie mitochondriale Plasmide oder msDNA (mulitcopy single stranded DNA) [für eine kürzliche Uebersicht über Retroelemente s. Ricchetti M. Bull Inst Pasteur 1991; 89:147-58]. Retroelemente umfassen demnach ein weites Spektrum teilweise infektiöser Agentien, die in der Humanmedizin, der Veterinärmedizin oder in der Pflanzenzucht eine Rolle spielen, indem sie schwere, teilweise tödliche Krankheiten verursachen und, bei Tieren und Pflanzen, zu Mindererträgen führen können.

Die Erfindung beschreibt daher auch Verfahren zum ultrasensitiven Nachweis aller Retroviren sowie aller anderen Retroelemente, die aktive RT enthalten oder exprimieren. Naheliegenderweise werden PERT-Assays bei Mensch, Tier oder Pflanze für den Nachweis von solchen Retroelementen selbst oder für die Diagnostik von Krankheiten, die mit diesen assoziiert sind, eingesetzt.

Beim Menschen gehören zu diesen Retroelementen die gesicherten, humanpathogenen Onko-Retroviren Human T-Cell Leukemia Virus Typ 1 und Typ 2 (HTLV-1 bzw. -2) sowie die mit AIDS und seinen Vorstufen assoziierten Human Immunodeficiency Viruses Typ 1 und Typ 2 (HIV-1 bzw. -2). Für verschiedene weitere Erkrankungen wurde eine Assoziation mit Retroviren postuliert, so bei Multipler Sklerose, dem Kawasaki-Syndrom, dem Chronic Fatigue Syndrom, dem Sjögren Syndrom, Graves Disease, bei gewissen proliferativen Erkrankungen des Bluts wie Polycythämie oder Thrombocythämie, gewissen cutanen T-Zell-Lymphomen oder dem Mammakarzinom. Zur Gruppe der humanpathogenen Pararetroviren gehört das Hepatitis B-Virus, welches mit akuten oder chronischen Hepatitiden sowie Leberkrebs assoziiert ist.

Bei den Tieren gehören zu den Retroelementen zahlreiche Retroviren, darunter alle jene, die im Standardwerk RNA Tumor Viruses, 2. Auflage. R. Weiss, N. Teich, H. Varmus, J. Coffin, Hrsg. Cold Spring Harbor Laboratory 1984 und 1985 aufgeführt sind. Dazu gehören beispielsweise Aviäre Leukose- und Sarkomviren (ALV bzw. ASV), murine Retoriviren (MLV/MSV/MMTV), feline Leukämieviren (FeLV), Equine Infectious Anemia Virus (EIAV), Visna-Virus, Caprine Arthritis/Encephalitis Virus (CAEV), Bovine Leukemia Virus (BLV). Wie die Bezeichnungen verraten, verursachen viele dieser Viren maligne proliferative Erkrankungen, häufig des blutbildenden oder lymphatischen Systems. Andere verursachen chronische Entzündungen und degenerative Erkrankungen des Nervensystems, der Gelenke, oder anderer Organe. Dazu kommen die in den letzten Jahren entdeckten verschiedenen Typen der Siminian Immunodeficiency Viruses (SIV), der Feline Immunodeficiency Viruses (FIV) und der Bovine Immunodeficiency Viruses (BIV). Zu den Pararetroviren gehören die animalen Hepadnaviren mit dem Woodchuck Hepatitits Virus (WHV), dem Ground Squirrel Hepatitis Virus und dem Duck Hepatitis B Virus (DHBV).

In der Gruppe der retrovirusähnlichen Elemente befinden sich neben den bereits erwähnten Pararetroviren, zu denen auch die Pflanzen befallenden Caulimoviren mit dem Vertreter Cauliflower Mosaic Virus gehören, auch die Retrotransposons mit den vorzugsweise transponierbaren Elementen von Drosophila spp., copia, gypsy, 17.6, 297, 412, oder Saccharomyces cerevisiae sowie die LINE. Neben diesen retrovirus-ähnlichen Elementen existiert ferner die Gruppe der nicht-retrovirusähnlichen Elemente, welche die mitochondrialen Plasmide und die msDNA umfasst.

Zu den Retroelementen gehören ferner alle Agentien, die durch natürliche Rekombination aus den bereits existierenden Retroelementen hervorgehen oder durch genetische Manipulationen erzeugt werden. Der Einsatz des PERT Assay zur Entdeckung und Charakterisierung weiterer Retroelemente ist naheliegend und für die Forschung von grösster Bedeutung.

Ferner kann mit dem PERT Assay RT-Aktivität in Assoziation mit prokaryotischen Zellen oder Fragmenten davon, zellulären oder subzellulären Elementen, Viren oder Partikeln jeglicher Art, d.h. RT-Aktivität jeglichen Ursprungs, nachgewiesen werden.

Der diagnostische Einsatz des PERT Assay kann in Form von Screening- oder Typisierungs-Assays, welch letztere auch die Funktion von Bestätigungstests haben, erfolgen. Wichtig ist insbesondere das Screening von Blut- oder Organspendern. Hier ist von Bedeutung, dass mit einem einzigen Screening-Assay sämtliche Retroelemente erfasst werden können, was gegenüber einer Testung mit einer Reihe von typen- oder sequenzspezifischen Tests wesentlich einfacher und kostengünstiger ist.

Wichtig ist auch die quantitative Anwendung des PERT Assay, die Auskunft gibt über das Ausmass einer Infektion, d.h. den "Virus Load". In diesem Zusammenhang ist von Vorteil, dass der PERT Assay nur Elemente mit funktionell aktiver RT-Aktivität erfasst. RT-inaktive Elemente, die für die Uebertragung oder Propagierung einer Infektion irrelevant sind, werden nicht erfasst.

Eine weitere wichtige Einsatzmöglichkeit ist das Screening biologischer Produkte, d.h. Produkten, die mindestens anteilsweise aus biologischen Materialien bestehen oder daraus hergestellt wurden und in der Human- oder Veterinärmedizin oder in der Pflanzenzucht zur Therapie, Prophylaxe oder zu anderen Zwecken eingesetzt werden. Als illustrative Beispiele, die in keiner Weise abschliessend sind, seien Seren, Plasmapräparate oder Plasmafraktionen, Gerinnungsfaktoren, Vakzinen, Zellextrakte, Hormonpräparate, Zytokine sowie durch rekombinante Gentechnologie hergestellte Produkte genannt. Mit dem PERT Assay kann kontrolliert werden, dass über diese Produkte keine infektiösen Retroelemente übertragen werden.

Biologische Produkte werden heute wo immer möglich durch chemische, enzymatische und/oder physikalische Massnahmen inaktiviert. Mit dem PERT Assay kann sehr sensitiv kontrolliert werden, ob die Inaktivierungsmassnahmen den gewünschten Erfolg haben.

Medikamente, welche die RT hemmen, spielen eine wichtige Rolle in der Behandlung von Infektionen mit Retroelementen und den durch sie verursachten Erkrankungen. Wie bei den Antibiotika entwickeln sich jedoch medikamentenresistente Virusvarianten. Bei HIV ist gezeigt worden, dass nach zweijähriger Therapie mit dem RT-Hemmer Azidothymidin (AZT) praktisch alle Patienten AZT-resistente Virusvarianten haben. Mit dem PERT Assay kann direkt ex vivo, d.h. anhand der Untersuchung von Untersuchungsmaterial, das einem Infizierten entnommen wurde, die Wirkung einer die RT-Aktivität hemmenden Substanz an der zu einem bestimmten Zeitpunkt vorherrschenden Population aktiver Retroelemente eruiert werden.

Zusammenfassend ist der PERT Assay bei zahlreichen Fragestellungen aus Forschung, Diagnostik, Klinik und Produktekontrolle sinnvoll anwendbar.

### Kits zur einfachen Durchführung von PERT Assays

Ein wichtiger Aspekt der vorliegenden Erfindung betrifft Kits für den Nachweis von RT-Aktivität in einem Untersuchungsmaterial mittels des PERT Assay. Der Begriff "Kit", wie er hierin verwendet wird, bezeichnet einfach abgepackte und leicht verwendbare Hilfsmittel und Reagentien zum Durchführen von PERT Assays. Der Inhalt eines Kit wird massgeblich durch den Verwendungszweck und die Variante oder Version des PERT Assay, die zur Anwendung gelangt, bestimmt. Der Begriff "Kit" beinhaltet einerseits "Screening-Kits", welche der Abklärung der Frage dienen, ob in einem Untersuchungsmaterial RT-Aktivität vorhanden ist, und die im Falle eines positiven Testresultats keine Aussage bezüglich der Herkunft derselben zulassen. Der Begriff "Kit" beinhaltet andererseits auch "Typisierungs-Kits', welche der Abklärung der Frage nach der Herkunft oder Ursache einer mittels eines "Screening-Kits" nachgewiesenen RT-Aktivität dienen bzw. Aufschluss darüber geben, durch welches Retroelement, oder durch welche Gruppe miteinander verwandter Retroelemente, diese Aktivität bedingt ist. Die Zusammensetzung der Reagentien verschiedener Typisierungskits wird u.a. bestimmt durch die Spezies, von welcher das Untersuchungsmaterial stammt, sowie das Spektrum und die Häufigkeit der in dieser Spezies oder einer bestimmten Untersuchungspopulation bekannterweise vorkommenden Retroelemente. Kits zum Nachweis von RT-Aktivität (Screening-Kits)

In einem solchen Kit kann das Folgende enthalten sein:
für Schritt 1 des PERT Assay:
(1) Geeignete Filter zur Entfernung unerwünschten partikulären Materials aus dem Untersuchungsmaterial;
(2) geeignete Reagentien oder Hilfsmittel zur Anreicherung der RT-Aktivität in einer für die weitere Verarbeitung bestimmten Fraktion oder Phase des Untersuchungsmaterials;
(3) ein geeignetes Reagens zur schonenden Extraktion von RT-Aktivität aus einer für die weitere Verarbeitung bestimmten Fraktion oder Phase;
(4) eine aus einer oder mehreren Lösungen sich zusammensetzende RT-Mastermischung, welche - mit Ausnahme der RT-Aktivität - alle für die RT-Reaktion erforderlichen Reagentien in geeigneter Konzentration in einer geeigneten Pufferlösung enthält; in dieser Mastermischung sind insbesondere enthalten eine entsprechend dem in Schritt 2 verwendeten Amplifikationsverfahren ausgewählte geeignete Template-Primer-Kombination, die benötigten dNTPs, ein geeignetes divalentes Kation (Mn⁺⁺ und/oder Mg⁺⁺) und andere die Reaktion im Sinne optimaler Spezifität und Sensitivität günstig beeinflussende Substanzen;
(5) eine positive RT Kontrolle, bestehend aus einer Lösung, die eine enzymatisch aktive RT enthält;
(6) eine negative RT Kontrolle, bestehend aus einer Lösung, die keine enzymatisch aktive RT enthält;
(7) falls aufgrund der Bedingungen des in Schritt 2 verwendeten Nukleinsäureamplifikationsverfahrens erforderlich oder wünschenswert, ein oder mehrere geeignete Reagentien zur enzymatischen und/oder chemischen Degradation der RNA; zudem eine geeignete Waschlösung zum Wegwaschen derselben, sofern Bindung der cDNA an einen Carrier einen Waschprozess gestattet, oder eine Neutralisationslösung, welche danach bezüglich pH, Salzkonzentrationen, degradierenden Reagentien wieder Reaktionsbedingungen schafft, welche die Durchführung der gemäss der gewählten Variante oder Version des PERT Assay notwendigen nachfolgenden Reaktionen überhaupt zulassen;
(8) eine oder mehrere Lösungen, enthaltend die Reagentien wie Enzyme, Primer bzw. andere Oligonukleotide, Reportersonden, dNTPs, bzw. rNTPs, die gemäss der gewählten Variante und Version des PERT Assay benötigt werden für entweder die Transformation der cDNA in eine Nukleinsäure, die das Edukt von Schritt 2 darstellt, oder für die spezifische Hybridisierung, an die cDNA, einer Reportersonde, welche entweder in Schritt 2 als Edukt verwendet wird oder von welcher ausgehend eine Nukleinsäure gebildet wird, die in Schritt 2 als Edukt verwendet wird, sowie in diesem Fall eine geeignete waschlösung für das Wegwaschen der nicht-hybridisierten überschüssigen Reportersonde;
   für Schritt 2 des PERT Assay:
(9) eine oder mehrere Lösungen enthaltend die je nach der PERT Variante und Version noch zusätzlich benötigten Primer bzw. anderen Oligonukleotide, Enzyme, dNTPs, rNTPs;
   für Schritt 3 des PERT Assay:
(10) der verwendeten PERT Assay-Variante oder -Version angepasste Reagentien und Reaktionsgefässe für die Analyse des aus Schritt 2 resultierenden Amplifikationsprodukts.

### Kits zur Typisierung von RT Aktivität

Diese Kits dienen dazu, die im PERT-Assay nachgewiesene RT-Aktivität einem bestimmten Retroelement oder einer Gruppe miteinander verwandter Retroelemente zuzuordnen. Dies geschieht mittels Reagentien, die mit zumindest einer charakteristischen Determinante eines bestimmten, RT aufweisenden Retroelements, oder einer Gruppe solcher Retroelemente, spezifisch zu reagieren vermögen.

Das Typisierungskit kann enthalten:
(1) Typisierungsreagens: enthaltend mindestens ein geeignetes Reagens mit ausreichender Affinität und Spezifität für mindestens eine Determinante, die für ein bestimmtes Retroelement oder einer Gruppe miteinander verwandter Retroelemente charakteristisch ist.
   Die mit dem Typisierungsreagens reagierenden charakteristischen Determinanten sind Bestandteil eines Partikels oder Fragments, welches mit RT assoziiert ist, oder sie sind auf dem Enzym selbst lokalisiert. Im letzteren Fall kann das Typisierungsreagens auch direkt gegen den "active site" des Enzyms oder dessen Nachbarschaft gerichtet sein und die. Enzymaktivität inhibieren. Die charakteristischen Determinanten bzw. das Typisierungsreagens müssen jedenfalls so gewählt werden, dass die Interaktion des Typisierungsreagens mit den Determinanten auch Interaktion - direkt oder indirekt - mit der RT bedeutet.
   Das Typisierungsreagens kann aus mono- oder polyklonalen Antikörpern mit Spezifität für zumindest eine der charakteristischen Determinanten oder aus anderen Proteinen, pharmakologischen Substanzen oder anderen Molekülen jeglicher Art, die mit zumindest einer der betreffenden Determinanten eine spezifische Bindung eingehen, bestehen.
   Direkt gegen die RT gerichtete Reagentien sind bevorzugterweise nach Aufbereitung des Untersuchungsmaterials, aber vor dem Einbringen desselben in Reaktion (3) von Schritt 1, dem Untersuchungsmaterial beizufügen und die Mischung während geeigneter Zeit zu inkubieren.
   Gegen andere Determinanten gerichtete Reagentien sind während der Materialaufbereitung an dem dafür optimalen Zeitpunkt zuzufügen.
   Das Typisierungsreagens kann in gelöster Form zugegeben werden, besonders wenn es direkt enzyminhibierende (neutralisierende) Aktivität aufweist. Ein nicht-neutralisierendes Typisierungsreagens kann an einen Carrier gebunden vorgelegt werden oder kann, falls in gelöster Form vorliegend, mit reaktiven Gruppen versehen sein, die es mittels irgendeiner der Fachwelt bekannten Fraktionierungsmethode ermöglichen, dass die RT-Aktivität in der für den PERT-Assay bestimmten Fraktion entweder angereichert oder vermindert wird.
(2) Negatives Kontrollreagens: Mindestens ein geeignetes, dem Typisierungsreagens möglichst ähnliches Reagens, welches jedoch keine Bindung mit der vom Typisierungsreagens erkannten Determinante oder einer anderen mit der betreffenden RT direkt oder indirekt assoziierten Determinante eingeht. Das negative Kontrollreagens besteht, je nach Art des Typisierungsreagens, ebenfalls aus monooder polyklonalen Antikörpern, anderen Proteinen, pharmakologischen Substanzen oder anderen Molekülen entsprechender Art und verhält sich bezüglich der Fraktionierung gleich wie das Typisierungsreagens.
(3) Positive RT Kontrolle: Enthält eine enzymatisch aktive Präparation von RT eines bestimmten, durch das Typisierungsreagens definierten Retroelements in geeigneter Konzentration.
(4) Negative RT Kontrolle: Enthält eine geeignete RT, die nicht dem Typisierungsreagens entspricht und mit diesem nicht reagiert.
(5) Fraktionierungsreagens: enthält, falls eine Fraktionierung notwendig ist, alle für diese erforderlichen weiteren Komponenten. Das Fraktionierungsreagens ermöglicht, durch Interaktion mit dem Typisierungsreagens die an dieses gebundene RT in der für den PERT Assay bestimmten Fraktion anzureichern oder zu vermindern. Das Fraktionierungsreagens kann beispielsweise ein Carrier sein, an welchen das Typisierungsreagens gebunden ist oder mittels reaktiver Gruppen gebunden wird, es kann auch ein immunologisches Reagens sein, welches das Typisierungsreagens präzipitiert. Gleicherweise wird auch das negative Kontrollreagens mit dem Fraktionierungsreagens interagieren, nur wird die RT dadurch nicht beeinflusst, da sie mit dem negativen Kontrollreagens keine Bindung eingeht.

Ein Typisierungskit kann die Reagentien (1) bis (4) von jeweils verschiedenen Retroelementen in beliebigen Kombinationen derselben enthalten. Die Reagentien sind bevorzugterweise in geeigneten Kombinationen assortiert, so dass die Erfassung der wichtigsten in einer bestimmten Untersuchungsgruppe vorkommenden Retroelemente mittels eines einzigen Kits möglich ist. Beispielsweise kann ein Typisierungskit für den Nachweis von Retroelementen des Menschen die folgenden Typisierungsreagentien enthalten: solche mit Spezifität für die Gruppe HIV, und innerhalb derselben solche mit Spezifität für entweder HIV-1 oder HIV-2; ferner solche mit Spezifität für die Gruppe HTLV, und innerhalb derselben solche mit Spezifität für entweder HTLV-1 und HTLV-2; ferner ein solches mit Spezifität für Hepatitis B Virus.

Erfindungswesentlich ist, dass der PERT (Product-Enhanced Reverse Transcriptase) Assay ein ultrasensitives 3-schrittiges Verfahren zum Nachweis von Reverse Transcriptase (RT) Aktivität ist.

In einem ersten Schritt wird das Untersuchungsmaterial aufgearbeitet, indem in einem Reaktionsgemisch eine Template-Primer-Kombination vorgelegt wird und die im aufgearbeiteten Untersuchungsmaterial vorhandene RT-Aktivität so zur Bereitstellung einer in einem bestimmten Nukleinsäureamplifikationsverfahren zu vermehrenden Nukleinsäure verwendet wird, dass eine solche ausschliesslich dann bereitgestellt wird, wenn im Untersuchungsmaterial RT-Aktivität vorhanden ist.

Im zweiten Schritt wird die zu vermehrende Nukleinsäure amplifiziert, wobei ein Amplifikationsprodukt erzeugt wird, welches im dritten Schritt analysiert und identifiziert wird.

Gegenüber den herkömmlichen RT-Assays wird mit dem PERT Assay eine Sensitivitätssteigerung in der Grössenordnung von mehreren Millionen erreicht. Die Erfindung ermöglicht den ultrasensitiven Nachweis von RT-Aktivität und damit aller Retroviren sowie aller anderen Retroelemente, die aktive RT enthalten oder exprimieren, und ist von Wichtigkeit für die Diagnose von Infektionen und Krankheiten, die durch diese bei Mensch, Tieren und Pflanzen verursacht werden. PERT Assays werden in Forschung und Diagnostik als Screening- oder als Typisierungs- bzw. Konfirmations-Tests eingesetzt; die Erfindung umfasst entsprechende Kits.

## Patentansprüche

1. Verfahren zum Nachweis von Enzymaktivität des Typs der Reversen Transkriptase (RT) in einem Untersuchungsmaterial, gekennzeichnet dadurch,
(a) dass das Untersuchungsmaterial aufgearbeitet wird, um die darin enthaltene reverse Transkriptase für die RT-Reaktion zugänglich zu machen und um aus diesem Untersuchungsmaterial Faktoren, die mit der RT-Reaktion interferieren, zu eliminieren;
(b) dass das aufgearbeitete Untersuchungsmaterial mit einer Template-Primer-Kombination in einer Reaktionsmischung inkubiert wird, wobei diese Template-Primer-Kombination aus einer Template-Nukleinsäure und mindestens einem RT-Primer besteht,
wobei der RT-Primer eine Nukleinsäure ist, welche mindestens eine funktionelle Sequenz enthält, wovon mindestens eine eine Hybridisierungssequenz ist, welche sich bis zum 3'-Ende erstreckt und durch welche der RT-Primer an die Template-Nukleinsäure hybridisiert ist;
wobei die Template-Nukleinsäure eine heteropolymere Nukleinsäure ist, welche mindestens ein Segment enthält, welches aus einer heteropolymeren RNA besteht und upstream einer Hybridisierungssequenz lokalisiert ist, an welche der RT-Primer hybridisiert ist, und welches Segment in einer RT-Reaktion als Template fungieren kann, und dass diese Template-Nukleinsäure ferner mindestens eine zusätzliche, upstream der Hybridisierungssequenz für den RT-Primer gelegene funktionelle Sequenz enthält;
(c) dass durch reverse Transkription, welche durch die aus dem Untersuchungsmaterial stammende Reverse Transkriptase katalisiert wird, eine cDNA synthetisiert wird;
(d) dass durch ein in vitro Nukleinsäureamplifikationsverfahren mindestens ein Teil der Sequenz einer in Schritt (c) produzierten cDNA oder einer von dieser cDNA abgeleiteten Nukleinsäure amplifiziert wird;
(e) dass das Amplifikationsprodukt aus Schritt (d) analysiert und identifiziert wird; und
(f) dass die Anwesenheit des Amplifikationsprodukts mit der Anwesenheit von Reverser Transkriptaseaktivität im aufgearbeiteten Untersuchungsmaterial korreliert wird.

2. Verfahren nach Anspruch 1, gekennzeichnet dadurch, dass Template-Primer-Kombination aus Schritt (b) alle fünktionellen Sequenzen enthält, welche notwendig sind, um mindestens einen Teil der Sequenz einer in Schritt (c) synthetisierten cDNA zu amplifizieren, und
dass in Schritt (d) derjenige Teil der cDNA-Sequenz, welcher diese funktionellen Sequenzen enthält, amplifiziert wird.

3. Verfahren gemäss Anspruch 2, gekennzeichnet dadurch, dass die Template-Primer-Kombination aus Schritt (b) ein Paar nichtüberlappender Hybridisierungssequenzen enthält, welche in der Amplifikationsreaktion von Schritt (d) verwendet werden, wobei mindestens eine derselben upstream jener Hybridisierungssequenz der Template-Nukleinsäure lokalisiert ist, an welche der RT-Primer hybridisiert ist;
dass eine in Schritt (c) synthetisierte cDNA dieses Paar von Hybridisierungssequenzen enthält;
und dass in Schritt (d) mittels einer Polymerase-Kettenreaktion und unter Verwendung von zwei Amlifikationsprimern, welche an das Paar von Hybridisierungssequenzen anhybridisiert werden, ein Teil der Sequenz jener cDNA selektiv amplifiziert wird.

4. Verfahren gemäss Anspruch 3, gekennzeichnet dadurch, dass die Template-Nukleinsäure die genomische RNA des Bakteriophagen MS2 ist, der RT-Primer ein synthetisches Deoxyoligonukleotid mit der Basensequenz 5'-d(CATAGGTCAAACCTCCTAGGAATG)-3' ist; und dass ein Teil der in Schritt (c) sythetisierten MS2 cDNA in Schritt (d) mittels Polymerase-Kettenreaktion mit synthetischen Deoxyoligonukleotid-Primern der Basensequenz 5'-d(CATAGGTCAAACCTCCTAGGAATG)-3' und 5'-d(TCCTGCTCAACTTCCTGTCGAG)-3' selektiv amplifiziert wird.

5. Verfahren gemäss Anspruch 2, gekennzeichnet dadurch, dass die Template-Primer-Kombination aus Schritt (b) ein Paar unmittelbar aneinandergrenzender Hybridisierungssequenzen enthält, die in der Amplifikationsreaktion von Schritt (d) verwendet werden, wobei mindestens eine dieser Hybridisierungssequenzen vollständig upstream von jener Hybridisierungssequenz der Template-Nukleinsäure gelegen ist, an welche der RT-Primer hybridisiert ist;
dass eine in Schritt (c) synthetisierte cDNA jenes Paar von Hybridisierungssequenzen enthält; und
dass in Schritt (d) ein Teil der Sequenz jener cDNA mittels einer Ligase-Kettenreaktion unter Verwendung jenes Paars von Hybridisierungssequenzen selektiv amplifiziert wird.

6. Verfahren gemäss Anspruch 2, gekennzeichnet dadurch, dass die Template-Primer-Kombination aus Schritt (b) ein Paar von Hybridisierungssequenzen enthält, welche in der Amplifikationsreaktion von Schritt (d) verwendet werden, wobei die Hybridisierungssequenzen dieses Paars voneinander durch eine Spacer-Sequenz getrennt sind und mindestens eine der beiden vollständig upstream von jener Hybridisierungssequenz der Template-Nukleinsäure lokalisiert ist, an welche der RT-Primer hybridisiert ist;
dass eine in Schritt (c) synthetisierte cDNA jenes Paar von Hybridisierungssequenzen enthält; und
dass in Schritt (d) mittels einer Kombination einer Polymerase-Kettenreaktion und einer Ligase-Kettenreaktion unter Verwendung jenes Paars von Hybridisierungssequenzen ein Teil der Sequenz dieser cDNA selektiv amplifiziert wird.

7. Verfahren gemäss Anspruch 2, gekennzeichnet dadurch, dass jene Template-Primer-Kombination aus Schritt (b) ein Paar von nichtüberlappenden Hybridisierungssequenzen enthält, die in der Amplifikationsreaktion von Schritt (d) verwendet werden, wobei mindestens eine derselben vollständig upstream von jener Hybridisierungssequenz der Template-Nukleinsäure lokalisiert ist, an welche der RT-Primer hybridisiert ist;
dass die in Schritt (c) synthetisierte cDNA jenes Paar von Hybridisierungssequenzen enthält;
und dass in Schritt (d) jener Teil der in Schritt (c) synthetisierten cDNA, welcher jenes Paar von Hybridisierungssequenzen enthält, selektiv amplifiziert wird, indem
(i) ein multienzymatisches, auf Transkription basierendes Verfahren verwendet wird, welches jenes Paar von Hybridisierungssequenzen sowie Reaktionsbedingungen benützt, welche die Stabilität der RNA aufrecht erhalten; und
(ii) die Template-RNA von einer Amplifikation ausgeschlossen wird durch vorgängige Behandlung mit mindestens einer Methode ausgewählt aus der Gruppe umfassend enzymatische Degradation, chemische Degradation, physikalische Degradation sowie Dissoziation und Wegwaschen der Template-RNA von einer an eine Festphase gebundenen cDNA.

8. Verfahren gemäss Anspruch 2, gekennzeichnet dadurch, dass die Template-Primer-Kombination aus Schritt (b) ein Paar nichtüberlappender Hybridisierungssequenzen sowie eine Transkriptionspromotersequenz enthält, welche in der Amplifikafionsreaktion von Schritt (d) verwendet werden, wobei mindestens eine Hybridisierungssequenz dieses Paars vollständig upstream jener Hybridisierungssequenz der Template-Nukleinsäure lokalisiert ist, an welche der RT-Primer hybridisiert ist;
dass die in Schritt (c) synthetisierte cDNA die P(-) Sequenz jenes Transkriptionspromoters upstream jenes Paars von Hybridisierungssequenzen enthält;
dass in Schritt (d) jener Teil der in Schritt (c) synthetisierten cDNA, welcher jenes Paar von Hybridisierungssequenzen und jene P(-) Sequenz enthält, selektiv amplifiziert wird, indem
(i) ein multienzymatisches, auf Transkription basierendes Verfahren verwendet wird, welches jenes Paar von Hybridisierungssequenzen und jene P(-) Sequenz sowie Reaktionsbedingungen benützt, welche die Stabilität der RNA aufrecht erhalten; und
(ii) die Template-RNA von einer Amplifikation ausgeschlossen wird durch vorgängige Behandlung mit mindestens einer Methode ausgewählt aus der Gruppe umfassend enzymatische Degradation, chemische Degradation, physikalische Degradation, sowie Dissoziation undWegwaschen der Template-RNA von einer an eine Festphase gebundenen cDNA.

9. Verfahren gemäss Anspruch 2, gekennzeichnet dadurch, dass die Template-Primer-Kombination aus Schritt (b) alle funktionellen Sequenzen einer replikativen DNA enthält;
wobei diese Template-Nukleinsäure in 5' zu 3' Reihenfolge eine DNA-ori(-) Sequenz enthält, die sich bis an das 5'-Ende erstreckt und aus einer Nukleinsäure besteht, welche aus der Gruppe enthaltend RNA und DNA ausgewählt wird, eine Spacer-Sequenz und eine Primer-Hybridisierungssequenz, welche zumindest einen Teil einer DNA-ori(+) Sequenz enthält und sich bis an das 3'-Ende jener Primer-Hybridisierungssequenz erstreckt;
dass jener RT-Primer ein einzelsträngiges DNA-Oligonukleotid ist, welches in seiner gesamten Länge an mindestens den am 3'-terminalen Teil jener DNA-ori(+) Sequenz jener Template-Nukleinsäure hybridisiert ist;
dass jene in Schritt (c) synthetisierte cDNA eine primäre replikative DNA ist, welche die folgenden Elemente enthält: eine bis an das 5'-Ende reichende DNA-ori(-) Sequenz, eine Spacer-Sequenz, und eine bis an das 3'-Ende reichende DNA-ori(+) Sequenz,
dass die Hybridisierung jener 3'-terminalen DNA-ori(+) Sequenz an eine 5'-terminale DNA-ori(-) Sequenz einen aktiven DNA-ori bildet;
dass in Schritt (d) jene cDNA amplifiziert wird, indem
(i) die Template-RNA mindestens teilweise eliminiert wird durch vorgängige Behandlung mit mindestens einer Methode, die ausgewählt wird aus der Gruppe umfassend enzymatische Degradation, chemische Degradation, physikalische Degradation sowie Dissoziation und Wegwaschen der Template-RNA von einer an eine Festphase gebundenen cDNA; und
(ii) in einer Reaktionsmischung, welche dNTPs enthält, ein DNA-Replikationsverfahren eingesetzt wird, in welchem ein Protein-Primer an den aktiven DNA-ori gebunden und die replikative DNA mit Hilfe einer DNA-Replicase, welche an die Protein-geprimte replikative DNA bindet, amplifiziert wird.

10. Verfahren gemäss Anspruch 1, gekennzeichnet dadurch, dass die Template-Nukleinsäure aus Schritt (b) eine RNA ist und mindestens eine weitere Hybridisierungssequenz enthält, welche upstream jener Hybridisierungssequenz liegt, an welche der RT-Primer hybridisiert ist;
dass der RT-Primer eine funktionelle Gruppe enthält;
dass eine in Schritt (c) synthetisierte cDNA jene funktionelle Gruppe und jene mindestens eine weitere Hybridisierungssequenz enthält;
dass eine in Schritt (d) amplifizierte Nukleinsäure von jener cDNA abgeleitet wird, indem jene Template-RNA mindestens teilweise eliminiert wird mittels mindestens einem Verfahren ausgewählt aus der Gruppe umfassend enzymatische Degradation, chemische Degradation, physikalische Degradation sowie Dissoziation und Wegwaschen der Template-RNA von einer cDNA, welche mittels jener funktionellen Gruppe an eine Festphase gebunden wurde;
dass eine Reportersonde, welche alle für eine Amplifikationsreaktion benötigten funktionellen Sequenzen enthält, an mindestens eine jener mindestens einen weiteren Hybridisierungssequenz einer cDNA hybridisiert wird;
dass anhybridisierte Reportersonde mittels jener funktionellen Gruppe der cDNA an eine Festphase gebunden wird;
dass ungebundene Reportersonde entfernt wird;
und dass mindestens ein Teil jener hybridisierten Reportersonde, welche die von der cDNA abgeleitete Nukleinsäure ist, amplifiziert wird.

11. Verfahren gemäss Anspruch 10, gekennzeichnet dadurch, dass jene Reportersonde eine DNA ist, welche eine Hybridisierungssequenz enthält, durch welche sie an die cDNA hybridisiert ist, sowie ein Paar von Hybridisierungssequenzen, welche in der Amplifikationsreaktion von Schritt (d) verwendet werden; und dass jener Teil der Reportersonde, welcher das Paar von Hybridisierungssequenzen enthält, in Schritt (d) amplifiziert wird mittels eines Verfahrens, das ausgewählt wird aus der Gruppe umfassend eine Polymerase-Kettenreaktion (PCR), eine Ligase-Kettenreaktion (LCR), eine Kombination einer PCR und einer LCR, und ein multienzymatisches auf Transkription basierendes Verfahren.

12. Verfahren gemäss Anspruch 10, gekennzeichnet dadurch, dass jene Reportersonde eine RNA ist, welche eine Hybridisierungssequenz enthält, durch welche sie an eine cDNA hybridisiert wird, sowie ein Paar von Hybridisierungssequenzen, welche in der Amplifikationsreaktion von Schritt (d) benützt werden, und dass derjenige Teil dieser Reportersonde, welcher jenes Paar von Hybridisierungssequenzen enthält, in Schritt (d) mittels eines multienzymatischen auf Transkription beruhenden Verfahrens amplifiziert wird.

13. Verfahren gemäss Anspruch 10, gekennzeichnet dadurch, dass jene Reportersonde eine primäre replikative RNA ist, welche eine Hybridisierungssequenz, durch welche sie an eine cDNA hybridisiert wird, eine bis an das 5'-Ende reichende RNA-ori(-) Sequenz, eine bis an das 3'-Ende reichende RNA-ori(+) Sequenz und eine Replicasen-Bindungsdomäne dazwischen enthält; und dass diese Reportersonde in Schritt (d) amplifiziert wird mittels eines RNA-Replikationssystems, welches rNTPs in einem Reaktionsgemisch und eine RNA-Replicase enthält, welche an die Replicasen-Bindungsdomäne der Reportersonde bindet und diese Reportersonde repliziert.

14. Verfahren gemäss Anspruch 10, gekennzeichnet dadurch, dass die Reportersonde mindestens eine Nukleinsäure ist, die ausgewählt wird aus der Gruppe umfassend
(i) eine primäre replikative DNA, welche eine bis an das 5'-Ende reichende DNA-ori(-) Sequenz, eine Hybridisierungssequenz, womit sie an eine cDNA hybridisiert wird, und eine bis an das 3'-Ende reichende DNA-ori(+)Sequenz enthält,
(ii) eine teilweise doppelsträngige primäre replikative DNA, welche eine bis zum 5'-Ende reichende DNA-ori(-) Sequenz, eine Hybridisierungssequenz, mit welcher sie an eine cDNA hybridisiert wird, und eine bis zum 3'-Ende reichende DNA-ori(+) Sequenz enthält, wobei die 3'-terminale Sequenz dieser Reportersonde mit einem einzelsträngigen DNA-Oligonukleotid geprimt ist, welches mindestens die ersten sechs Nukleotide einer DNA-ori(-) Sequenz enthält, und
(iii) eine mindestens teilweise doppelsträngige primäre replikative DNA, deren einer Strang eine DNA-ori(-) Sequenz enthält, welche bis zum 5'-Ende reicht, eine weiter downstream gelegene Hybridisierungssequenz, welche zu jener mindestens einen weiteren Hybridisierungssequenz der cDNA komplementär ist, und eine zweite bis zum 3'-Ende reichende Hybridisierungssequenz, an welche ein zweiter DNA-Strang hybridisiert ist, welcher eine komplementäre, bis zum 3'-Ende reichende Hybridisierungssequenz und eine bis zu seinem 5'-Ende reichende DNA-ori(-) Sequenz enthält; und
dass diese primäre replikative DNA in einer Reaktionsmischung, welche dNTPs enthält, amplifiziert wird mit Hilfe eines DNA-Replikationsverfahrens, das Bindung eines Proteinprimers an den DNA-ori jener primären replikativen DNA sowie Replikation der replikativen DNA mit Hilfe einer DNA-Replicase, welche an jene Protein-geprimte replikative DNA bindet, umfasst.

15. Verfahren gemäss Anspruch 1, gekennzeichnet dadurch, dass eine in Schritt (d) amplifizierte Nukleinsäure von der in Schritt (c) gebildeten cDNA abgeleitet wurde mittels mindestens einer Reaktion ausgewählt aus der Gruppe umfassend
(i) Hybridisierung einer einzelsträngigen DNA, welche mindestens eine sich bis an das 3'-Ende erstreckende Hybridisierungssequenz enthält, an die cDNA
(ii) Synthese einer mindestens teilweise doppelsträngigen DNA mittels enzymatischer Katalyse,
(iii) Synthese einer RNA mittels enzymatischer Katalyse,
(iv) mindestens teilweise Elimination der Template-Nukleinsäure, und
(v) Ligation des 3'-Endes der cDNA mit dem 5'-Ende einer anderen an die Template-Nukleinsäure hybridisierten Nukleinsäure.

16. Verfahren gemäss Anspruch 15, gekennzeichnet dadurch, dass die Template-Nukleinsäure aus Schritt (b) eine RNA ist und ein Paar nicht-überlappender Hybridisierungssequenzen enthält, welches in der Amplifikationsreaktion von Schritt (d) gebraucht wird, wobei mindestens eine der beiden upstream der Hybridisierungssequenz liegt, an welche der RT-Primer hybridisiert ist;
dass diese Template-Nukleinsäure ferner eine P(-) Sequenz eines Transkriptionspromoters enthält, welche upstream jenes Paars von Hybridisierungssequenzen gelegen ist, sowie eine weitere Hybridisierungssequenz enthält, deren 5'-Begrenzung upstream oder an der 5'-Begrenzung jener P(-) Sequenz lokalisiert ist;
dass die in Schritt (c) produzierte cDNA die Hybridisierungssequenz für den RT-Primer, das Paar Hybridisierungssequenzen, weiter downstream eine vollständige P(+) Sequenz und jene weitere Hybridisierungssequenz enthält; und
dass in Schritt (d) von dieser cDNA eine Nukleinsäure abgeleitet wird, indem
(i) die Template-RNA mindestens teilweise eliminiert wird mit mindestens einer Methode, die ausgewählt wird aus der Gruppe umfassend enzymatische Degradation, chemische Degradation, physikalische Degradation sowie Dissoziation und Wegwaschen der Template-RNA von einer an eine Festphase gebundenen cDNA;
(ii) ein DNA Oligonukleotid-Primer an jene weitere Hybridisierungssequenz der cDNA hybridisiert wird;
(iii) durch Katalyse mittels einer DNA-abhängigen DNA-Polymerase und dNTPs eine mindestens teilweise doppelsträngige DNA synthetisiert wird, welche einen funktionellen Transkriptionspromoter enthält; und
(iv) ausgehend von jenem Transkriptionspromoter durch Katalyse mittels einer DNA-abhängigen RNA-Polymerase und mittels rNTPs eine RNA synthetisiert wird, welche jenes Paar nicht-überlappender Hybridisierungssequenzen enthält; und dass
diese RNA, welche die von der cDNA abgeleitete Nukleinsäure ist, mittels eines multienzymatischen auf Transcription beruhenden Verfahrens amplifiziert wird, wobei die Reaktionsbedingungen derart sind, dass die Stabilität der RNA aufrechterhalten wird.

17. Verfahren gemäss Anspruch 15, gekennzeichnet dadurch, dass die Template-Nukleinsäure aus Schritt (b) aus RNA besteht und eine weitere Hybridisierungssequenz enthält, welche upstream von jener Hybridisierungssequenz liegt, an welche der RT-Primer hybridisiert ist, und mit dieser nicht überlappt;
dass der RT-Primer ein einzelsträngiges DNA-Oligonukleotid ist, dessen 5'-terminale Sequenz aus einer DNA-ori(-) Sequenz besteht, die einem DNA-Replikationssystem entspricht;
dass die in Schritt (c) synthesierte cDNA jene DNA-ori(-) Sequenz, welche bis an das 5'-Ende der cDNA reicht, die Hybridisierungssequenz für den RT-Primer und weiter downstream jene weitere Hybridisierungssequenz enthält;
dass in Schritt (d) von dieser cDNA eine Nukleinsäure abgeleitet wird, indem
(i) an jene weitere Hybridisierungssequenz der cDNA ein einzelsträngiger DNA-Primer hybridisiert wird, dessen 5'-terminale Sequenz aus jener DNA-ori(-) Sequenz besteht; und
(ii) mittels Katalyse durch eine DNA-abhängige DNA-Polymerase sowie dNTPs eine mindestens teilweise doppelsträngige DNA synthetisiert wird, welche an mindestens einem Ende einen DNA-ori besitzt und welche eine primäre replikative DNA ist; und dass
diese primäre replikative DNA, welche die von der cDNA abgeleitete Nukleinsäure ist, in einer dNTPs enthaltenden Reaktionsmischung mittels eines DNA-Replikationssystems amplifiziert wird, wobei ein Protein-Primer an den DNA-ori dieser primären replikativen DNA gebunden und die replikative DNA mittels einer DNA-Replicase, welche an diese Protein-geprimte replikative DNA bindet, amplifiziert wird.

18. Verfahren gemäss Anspruch 15, gekennzeichnet dadurch, dass die Template-Nukleinsäure von Schritt (b) aus RNA besteht und eine weitere Hybridisierungssequenz enthält, die upstream der Hybridisierungssequenz liegt, an welche der RT-Primer hybridisiert ist, und von dieser durch eine Spacer-Sequenz getrennt ist;
dass der RT-Primer ein partiell doppelsträngiger DNA-Adaptor ist, dessen einer, an die Template-Nukleinsäure hybridisierter Strang in seiner 5'-terminalen Sequenz die DNA-ori(-) Sequenz einer replikativen DNA enthält, an welche ein zweites DNA-Oligonukleotid hybridisiert ist, dessen 3'-terminale Sequenz aus einer DNA-ori(+) Sequenz besteht;
dass an jene weitere Hybridisierungssequenz der Template-Nukleinsäure ein zweiter teilweise doppelsträngiger DNA-Adaptor hybridisiert ist mittels einer Hybridisierungssequenz, welche bis zum 5'-Ende des längeren DNA-Strangs des Adaptors reicht;
dass der längere Strang dieses zweiten Adaptors am 5'-Ende phosphoryliert ist und eine DNA-ori(+) Sequenz enthält, welche sich bis an sein 3'-Ende erstreckt und an welche ein DNA-Oligonukleotid hybridisiert ist, dessen 5'-terminale Sequenz aus einer vollständigen DNA-ori(-) Sequenz besteht;
dass die in Schritt (c) synthetisierte cDNA die Lücke zwischen diesen zwei Adaptoren derart auffüllt, dass die cDNA vom zweiten Adaptor nur gerade durch ein Nick getrennt ist;
dass in Schritt (d) von dieser cDNA eine Nukleinsäure abgeleitet wird, indem das Nick mittels einer enzymatischen Ligation des 3'-Endes der cDNA mit dem 5'-Ende des zweiten Adaptors geschlossen wird, wodurch eine primäre replikative DNA gebildet wird; und
dass diese primäre replikative DNA, welche die von der cDNA abgeleitete Nukleinsäure ist, mittels eines DNA-Replikationssystems in einer dNTPs enthaltenden Reaktionsmischung amplifiziert wird, wobei ein Protein-Primer an den DNA-ori der besagten primären replikativen DNA gebunden und diese replikative DNA mittels einer DNA-Replicase, welche an jene Protein-geprimte replikative DNA bindet, amplifiziert wird.

19. Verfahren gemäss Anspruch 1, gekennzeichnet dadurch, dass die Template-Nukleinsäure von Schritt (b) eine RNA ist und mindestens eine weitere Hybridisierungssequenz enthält, welche upstream von jener Hybridisierungssequenz, an welche der RT-Primer hybridisiert ist, lokalisiert ist;
dass die in Schritt (c) synthetisierte cDNA jene mindestens eine weitere Hybridisierungssequenz enthält;
dass eine in Schritt (d) amplifizierte Nukleinsäure von dieser cDNA abgeleitet wird durch Hybridisierung einer Reportersonde an mindestens eine jener mindestens einen weiteren Hybridisierungssequenz jener cDNA und dass die Reaktionsmischung mindestens einer weiteren Reaktion unterzogen wird, die ausgewählt wird aus der Gruppe umfassend:
(i) Synthese einer RNA durch enzymatische Katalyse,
(ii) mindestens teilweise Elimination der Template-RNA,
(iii) Synthese einer mindestens teilweise doppelsträngigen DNA durch enzymatische Katalyse, sowie
(iv) Spaltung einer doppelsträngigen DNA durch enzymatische Katalyse.

20. Verfahren gemäss Anspruch 19, gekennzeichnet dadurch, dass die Template-Nukleinsäure eine sich bis an ihr 5'-Ende erstreckende weitere Hybridisierungssequenz enthält; wobei diese weitere Hybridisierungssequenz an ihrem 3-Ende eine P(-) Sequenz eines Transkriptionspromoters besitzt, welche ustream direkt gefolgt wird von mindestens den ersten drei Nukleotiden einer 3'-RNA-ori(+) Sequenz;
dass die in Schritt (c) synthetisierte cDNA jene weitere Hybridisierungssequenz enthält, welche sich bis zum 3'-Ende erstreckt;
dass eine in Schritt (d) amplifizierte Nukleinsäure von der cDNA abgeleitet wird, indem
(i) eine Reportersonde an jene weitere Hybridisierungssequenz der cDNA hybridisiert wird, wobei diese Reportersonde eine einzelsträngige DNA ist, welche eine sich bis an ihr 5'-Ende erstreckende 5'-RNA ori(-) Sequenz einer replikativen RNA, weiter downstream eine Replicasen-Bindungsdomäne und noch weiter downstream eine 3'-RNA-ori(+) Sequenz enthält, welche direkt gefolgt wird von der P(-) Sequenz jenes Transkriptionspromoters; wobei die Hybridisierungssequenz dieser Reportersonde aus jenen mindestens drei letzten Nukleotiden der besagten 3'-RNA-ori(+) Sequenz und der gesamten P(-) Sequenz jenes Transkriptionspromoters besteht; wobei die Hybridisierung dieser Reportersonde an die weitere Hybridisierungssequenz der cDNA zur Ausbildung eines funktionellen Transkriptionspromoters führt, und
(ii) ausgehend von diesem Transkriptionspromoter mittels Katalyse durch die entsprechende DNA-abhängige RNA-Polymerase und rNTPs eine primäre replikative RNA synthetisiert wird; und
dass diese primäre replikative RNA, welche die von der cDNA abgeleitete Nukleinsäure ist, mit Hilfe eines RNA-Replikationssystems amplifiziert wird, welches rNTPs in einer Reaktionsmischung sowie eine RNA-Replicase enthält, welche an die Replicasen-Bindungsdomäne dieser primären replikativen RNA bindet und diese primäre replikative RNA repliziert.

21. Verfahren gemäss Anspruch 19, gekennzeichnet dadurch, dass eine in Schritt (d) amplifizierte Nukleinsäure von der cDNA abgeleitet wird, indem
(i) die Template-RNA mindestens teilweise eliminiert wird mittels mindestens einer Methode, die ausgewählt wird aus der Gruppe umfassend enzymatische Degradation, chemische Degradaton, physikalische Degradation sowie Dissoziation der Template-RNA von einer cDNA durch Denaturierung und Wegwaschen;
(ii) eine Smart Probe an jene mindestens eine weitere Hybridsierungssequenz der cDNA hybridisiert wird, wobei diese Smart Probe eine einzelsträngige DNA ist, welche in 5' zu 3' Reihenfolge die P(+) Sequenz eines Transkriptionspromoters, eine Spacer-Sequenz, eine zu jener mindestens einen zusätzlichen Hybridisierungssequenz der cDNA komplementäre Hybridisierungssequenz, eine weitere Spacer-Sequenz, welche nicht komplementär ist zu der ersten, sowie die P(-) Sequenz jenes Transkriptionspromoters enthält, welche direkt gefolgt wird von mindestens den ersten drei Nukleotiden einer 5'-RNA-ori(-) Sequenz, wobei die P(-) Sequenz und die 5'-RNA-ori(-) Nukleotide eine weitere Hybridisierungssequenz bilden, welche bis zum 3'-Ende der Smart Probe reicht und welche bestimmt ist für die Anhybridisierung einer DNA-Reportersonde;
(iii) an diese weitere Hybridisierungssequenz der Smart Probe eine einzelsträngige DNA-Reportersonde hybridisiert wird, welche in 5' zu 3' Reihenfolge eine 5'-RNA-ori (-) Sequenz, eine Replicasen-Bindungsdomäne und eine 3'-RNA-ori(+) Sequenz enthält, die direkt gefolgt wird von der P(+) Sequenz jenes Transkriptionspromoters, wodurch ein funktioneller Transkriptionspromoter generiert wird; und indem
(iv) ausgehend von diesem Transkriptionspromoter mittels Katalyse durch die entsprechende DNA-abhängige RNA-Polymerase sowie rNTPs eine primäre replikative RNA synthetisiert wird; und
dass diese primäre replikative RNA, welche die von der cDNA abgeleitete Nukleinsäure ist, amplifiziert wird mittels eines RNA-Replikationssystems, welches rNTPs in einer Rektionsmischung sowie eine RNA-Replicase enthält, welche an die Replicasen-Bindungsdomäne dieser primären replikativen RNA bindet und diese primäre replikative RNA repliziert.

22. Verfahren gemäss Anspruch 19, gekennzeichnet dadurch, dass die Template-Nukleinsäure aus Schritt (b) eine weitere Hybridisierungssequenz enthält, welche bis an ihr 5'-Ende reicht; dass die in Schritt (c) synthetisierte cDNA jene weitere Hybridisierungssequenz enthält, die bis an das 3'-Ende reicht; dass eine in Schritt (d) amplifizierte Nukleinsäure von dieser cDNA abgeleitet wird, indem
(i) eine einzelsträngige DNA-Reportersonde an jene weitere Hybridisierungssequenz der cDNA hybridsiert wird, wobei diese Reportersonde upstream einer Hybridisierungssequenz, mittels welcher sie an jene weitere Hybridisierungssequenz der cDNA hybridisiert ist, multiple repetitive Kopien einer Sequenz enthält, welche eine Erkennungsstelle für eine Restriktionsendonuklease enthält;
(ii) mittels Katalyse durch eine DNA-abhängige DNA-Polymerase und dNTPs eine doppelsträngige DNA synthetisiert wird;
(iii) diese doppelsträngige DNA mittels der besagten Restriktionsendonuklease gespalten wird, wodurch mehrere Moleküle jenes doppelsträngigen Fragments erzeugt werden; wobei jenes Fragment die von der cDNA abgeleitete Nukleinsäure ist, die in wiederholten Zyklen von Denaturierung, Hybridisierung an die Reportersonde, DNA-Synthese und Restriktionsendonukleasespaltung amplifiziert wird.

23. Verfahren gemäss Anspruch 1, gekennzeichnet dadurch, dass mindestens eine Nukleinsäure aus der Gruppe umfassend den RT-Primer, eine vom RT-Primer verschiedene an die Template-Nukleinsäure hybridisierte Nukleinsäure, eine cDNA, eine an die cDNA hybridisierte Nukleinsäure, eine an eine Reportersonde hybridisierte Nukleinsäure und eine in einer Amplifikationsreaktion amplifizierte Nukleinsäure eine funktionelle Gruppe trägt, welche mindestens eine Eigenschaft aufweist, die ausgewählt wird aus der Gruppe der folgenden Eigenschaften: eine Festphase seiend, die Bindung an eine Festphase vermittelnd, ein Ligand seiend, und mindestens eine antigene Determinante besitzend.

24. Verfahren gemäss Anspruch 1, gekennzeichnet dadurch, dass nach der Synthese der cDNA von Schritt (c) die Template-RNA mindestens teilweise eliminiert wird durch Behandlung mit mindestens einer Methode, die ausgewählt wird aus der Gruppe umfassend enzymatische Degradation, chemische Degradation, physikalische Degradation sowie Dissoziation und Wegwaschen der Template-RNA von einer an eine Festphase gebundenen cDNA.

25. Ein Screening Kit für den Nachweis einer Reversen Transkriptase gemäss Anspruch 1 mindestens enthaltend:
(i) Reagenzien und Hilfsmittel für die Probenaufbereitung umfassend einen isotonischen Probenverdünnungspuffer, 0,2 µm Filter, einen Salze, Proteinstabilisatoren und ein mildes Detergens enthaltenden Extraktionspuffer für die Reverse Transkriptase;
(ii) eine RT-Mastermischung umfassend mindestens ein Puffersystem, Salze, eine heteropolymere Template-RNA, einen heteropolymeren RT-Primer, dATP, dCTP, dGTP und dTTP, ein divalentes Kation ausgewählt aus der Gruppe umfassend Mg²+ und Mn²+, mindestens einen Proteinstabilisator, einen RNase-Inhibitor,
(iii) eine RNase für die Verdauung der Template-RNA;
(iv) mindestens ein weiteres Oligonukleotid, welches auf die retrotranskribierte Template-RNA abgestimmt ist, sowie Reagenzien für eine in vitro Nukleinsäureamplifikationsreaktion umfassend mindestens die Enzyme, Nukleotidtriphosphate, Salze, divalente Kationen und Puffer;
(v) mindestens eine positive RT-Kontrolle umfassend eine Lösung, welche eine enzymatisch aktive Reverse Transkriptase enthält; und mindestens eine negative RT-Kontrolle, welche besteht aus einer Lösung, die keine enzymatisch aktive Reverse Transkriptase enthält.

26. Ein Screening Kit für den Nachweis einer reversen Transkriptase gemäss dem Kit aus Anspruch 25, gekennzeichnet dadurch, dass die Reagenzien für eine in vitro Nukleinsäureamplifikationsreaktion solche sind, die in einer Polymerase-Kettenreaktion verwendet werden, und mindestens eine Taq Polymerase, dATP, dCTP, dGTP, dTTP, Salze, ein divalentes Kation und Puffer enthalten.

## Claims

1. A method for detection of reverse transcriptase (RT) in a sample comprising:
(a) pretreating the sample to make the total amount of reverse transcriptase contained therein available for a RT reaction; and eliminating from said sample factors which interfere with the RT reaction;
(b) incubating the pretreated sample wit a template-primer combination in a reaction mixture, said template-primer combination comprising a template nucleic acid and at least a RT primer;
said RT primer being a nucleic acid which contains at least one functional sequence at least one of which is a hybridization sequence which extends to the 3'-end and by which the RT primer is hybridized to the template nucleic acid;
said template nucleic acid being a heteropolymeric nucleic acid which contains at least one segment consisting of a heteropolymeric RNA which is located upstream of a hybridization sequence to which said RT primer is hybridized and which is capable of functioning as a template in a RT reaction, said template nucleic acid further containing at least one additional functional sequence located upstream of said hybridization sequence for the RT primer
(c) synthesizing a cDNA by reverse transcription catalyzed by said reverse transcriptase from the sample;
(d) amplifying by an in vitro nucleic acid amplification reaction at least part of the sequence of a cDNA produced in step (c) or of a nucleic acid which has been derived from said cDNA,
(e) analyzing and identifying the amplification product of step (d); and
(f) correlating the presence of amplification product to the presence of reverse transcriptase activity in the pretreated sample.

2. A method according to claim 1, wherein said template-primer combination incubated in step (b) contains all functional sequences which will be required to amplify at least part of the sequence of a cDNA synthesized in step (c); and in step (d) that part of the cDNA sequence which contains these functional sequences is amplified.

3. A method according to claim 2, wherein said template-primer combination of step (b) contains a pair of non-overlapping hybridization sequences which will be used in the amplification reaction of step (d), at least one of these being located upstream of that hybridization sequence of the template nucleic acid to which the RT primer is hybridized; a cDNA synthesized in step (c) contains said pair of hybridization sequences; and in step (d) part of the sequence of said cDNA is selectively amplified by a polymerase chain reaction using two amplification primers which anneal to said pair of hybridization sequences.

4. A method according to claim 3, wherein the template nucleic acid is the genomic RNA of the bacteriophage MS2, the RT primer is a synthetic deoxyoligonucleotide of the base sequence 5'-d(CATAGGTCAAACCTCCTAGGAATG)-3'; and part of the MS2 cDNA synthesized in step (c) is selectively amplified in step (d) by polymerase chain reaction with synthetic deoxyoligonucleotide primers of the base sequence 5'-d(CATAGGTCAAACCTCCTAGGAATG)-3' and 5'-d(TCCTGCTCAACTTCCTGTCGAG)-3'.

5. A method according to claim 2, wherein said template-primer combination of step (b) contains a pair of hybridization sequences immediately adjacent to each other which will be used in the amplification reaction of step (d), at least one of these being located entirely upstream of that hybridization sequence of the template nucleic acid to which the RT primer is hybridized; a cDNA synthesized in step (c) contains said pair of hybridization sequences; and in step (d) part of the sequence of said cDNA is selectively amplified by ligase chain reaction using said pair of hybridization sequences.

6. A method according to claim 2, wherein said template-primer combination of step (b) contains a pair of hybridization sequences which will be used in the amplification reaction of step (d), the hybridization sequences of said pair being separated from each other by a spacer sequence and at least one of them being located entirely upstream of that hybridization sequence of the template nucleic acid to which the RT primer is hybridized; a cDNA synthesized in step (c) contains said pair of hybridization sequences; and in step (d) part of the sequence of said cDNA is selectively amplified by a combination of a polymerase chain reaction and a ligase chain reaction using said pair of hybridization sequences.

7. A method according to claim 2, wherein
said template-primer combination of step (b) contains a pair of non-overlapping hybridization sequences which will be used in the amplification reaction of step (d), at least one of these being located entirely upstream of that hybridization sequence of the template nucleic acid to which the RT primer is hybridized;
said cDNA synthesized in step (c) contains said pair of hybridization sequences;
in step (d) that part of said cDNA synthesized in step (c) which contains said pair of hybridization sequences is selectively amplified by
(i) employing a multienzymatic transcription-based procedure which uses said pair of hybridization sequences and reaction conditions that maintain the stability of the RNA; and
(ii) excluding the template RNA from amplification by prior treatment with at least one method selected from the group consisting of enzymatic degradation, chemical degradation, physical degradation, and dissociating the template RNA from a solid-phase-bound cDNA and washing it away.

8. A method according to claim 2, wherein
said template-primer combination of step (b) contains a pair of non-overlapping hybridization sequences and a transcription promoter sequence which will be used in the amplification reaction of step (d), at least one hybridization sequence of said pair being located entirely upstream of that hybridization sequence of the template nucleic acid to which the RT primer is hybridized;
said cDNA synthesized in step (c) contains the P(-) sequence of said transcription promoter located upstream of said pair of hybridization sequences;
in step (d) that part of said cDNA synthesized in step (c) which contains said pair of hybridization sequences and said P(-) sequence is selectively amplified by
(i) employing a multienzymatic transcription-based procedure using said pair of hybridization sequences and said P(-) sequence under reaction conditions that maintain the stability of the RNA, and by
(ii) excluding the template RNA from amplification by prior treatment with at least one method selected from the group consisting of enzymatic degradation, chemical degradation, physical degradation, and dissociating the template RNA from a solid-phase-bound cDNA and washing it away.

9. A method according to claim 2, wherein the template-primer combination of step (b) contains all sequence elements of a replicative DNA;
said template nucleic acid comprises, in 5' to 3' order, a DNA-ori(-) sequence which extends to the 5'-end and consists of a nucleic acid selected from the group comprising RNA and DNA, a spacer sequence, and a primer hybridization sequence which contains at least part of a DNA-ori(+) sequence extending to the 3'-end of said primer hybridization sequence;
said RT primer is a single-stranded DNA oligonucleotide which is in its entire length hybridized to at least the 3' terminal part of said DNA-ori(+) sequence of said template nucleic acid;
said cDNA synthesized in step (c) is a primary replicative DNA which contains a DNA-ori(-) sequence which extends to the 5-end, a spacer sequence, and a DNA-ori(+) sequence which extends to the 3'-end, the hybridization of said 3' terminal DNA-ori(+) sequence to a 5' terminal DNA-ori(-) sequence constituting an active DNA-ori;
in step (d) said cDNA is amplified by
(i) eliminating the template RNA at least partially by at least one method selected from the group comprising enzymatic degradation, chemical degradation, physical degradation, and dissociating the template RNA from a solid-phase-bound cDNA and washing it away, and
(ii) performing in a reaction mixture which contains dNTPs, a DNA replication procedure which involves binding of a protein primer to said active DNA-ori and replicating said replicative DNA by means of a DNA replicase that binds to said protein-primed replicative DNA.

10. A method according to claim 1, wherein said template nucleic acid of step (b) is RNA and contains at least one further hybridization sequence located upstream of that to which said RT primer is hybridized; said RT primer carries a functional group; a cDNA synthesized in step (c) contains said functional group and said at least one further hybridization sequence; a nucleic acid amplified in step (d) is derived from said cDNA by eliminating the template RNA at least partially with at least one method selected from the group consisting of enzymatic degradation, chemical degradation, physical degradation, and dissociating the template RNA and washing it away from a cDNA which has been bound to a solid phase by means of said functional group; hybridizing a reporter probe which contains all functional sequences used in an amplification reaction to at least one of said at least one further hybridization sequence of a cDNA; hybridized reporter probe is bound to a solid phase by means of said functional group of the cDNA; unbound reporter probe is removed; and at least part of said hybridized reporter probe which is the nucleic acid derived from said cDNA is amplified.

11. A method according to claim 10, wherein said reporter probe is a DNA which contains a hybridization sequence by which it is hybridized to said cDNA and a pair of hybridization sequences which will be used in the amplification reaction of step (d); and that part of said reporter probe which contains said pair of hybridization sequences is amplified in step (d) by a method selected from the group consisting of a polymerase chain reaction (PCR), a ligase chain reaction (LCR), a combination of PCR and LCR, and a multienzymatic transcription-based procedure.

12. A method according to claim 10, wherein said reporter probe is a RNA which contains a hybridization sequence by which it is hybridized to a cDNA and a pair of hybridization sequences which will be used in the amplification reaction of step (d) and a part of this reporter probe which contains said pair of hybridization sequences is amplified in step (d) by a multienzymatic transcription-based procedure.

13. A method according to claim 10, wherein said reporter probe is a primary replicative RNA which comprises a hybridization sequence by which it is hybridized to a cDNA, a RNA-ori (-) sequence extending to its 5'-end, a RNA-ori (+) sequence extending to its 3'-end and a replicase-binding domain in between; and said reporter probe is amplified in step (d) by means of a RNA replication system which comprises rNTPs in a reaction mixture and a RNA replicase that binds to the replicase binding domain of said reporter probe and replicates said reporter probe.

14. A method according to claim 10, wherein said reporter probe is at least one nucleic acid selected from the group comprising
(i) a primary replicative DNA which comprises a DNA-ori(-) sequence extending to its 5'-end, a hybridization sequence by which it is hybridized to a cDNA, and a DNA-ori(+) sequence extending to its 3'-end,
(ii) a partially double-stranded primary replicative DNA which comprises a DNA-ori(-) sequence extending to its 5'-end, a hybridization sequence by which it is hybridized to a cDNA, and a DNA-ori(+) sequence extending to its 3'-end, said 3'-terminal sequence of said reporter probe being primed with a single-stranded DNA oligonucleotide which comprises at least the first 6 nucleotides of a DNA-ori(-) sequence, and
(iii) a partially double-stranded primary replicative DNA which consists of one strand comprising a DNA-ori(-) sequence extending to the 5'-end, a hybridization sequence located further downstream which is complementary to said at least one further hybridization sequence of said cDNA, and a second hybridization sequence extending to the 3'-end to which is hybridized a second strand of DNA which contains a corresponding hybridization sequence extending to the 3'-end and a DNA-ori(-) sequence extending to the 5'-end;
and said primary replicative DNA is amplified in a reaction mixture which contains dNTPs by means of a DNA replication system which involves binding of a protein primer to the DNA-ori of said primary replicative DNA and replicating said replicative DNA by means of a DNA replicase that binds to said protein-primed replicateive DNA.

15. A method according to claim 1, wherein a nucleic acid amplified in step (d) is one which has been derived from said cDNA produced in step (c) by at least one further reaction selected from the group conisting of:
(i) hybridizing to said cDNA a single-stranded DNA which comprises at least a hybridization sequence which extends to the 3'-end,
(ii) synthesizing by enzymatic catalysis an at least partially double-stranded DNA,
(iii) synthesizing a RNA by enzymatic catalysis,
(iv) eliminating the template nucleic acid at least partially, and
(v) ligating the 3'-end of the cDNA with the 5'-end of another nucleic acid which is hybridized to the template nucleic acid.

16. A method according to claim 15, wherein said template nucleic acid of step (b) is RNA and contains a pair of non-overlapping hybridization sequences which will be used in the amplification reaction of step (d), at least one of these being located upstream of that hybridization sequence to which said RT primer is hybridized; said template nucleic acid further contains a P(-) sequence of a transcription promoter which is located upstream of said pair of hybridization sequences and a further hybridization sequence whose 5' boundary is located upstream of or at the 5' boundary of said P(-) sequence; said cDNA produced in step (c) contains the hybridization sequence of said RT primer, said pair of hybridization sequences, further downstream a complete P(+) sequence and said further hybridization sequence; in step (d) a nucleic acid is derived from said cDNA by
(i) eliminating the template RNA at least partially by treatment with at least one method selected from the group comprising enzymatic degradation, chemical degradation, physical degradation, and dissociating the template RNA from a solid-phase-bound cDNA and washing it away;
(ii) hybridizing a DNA oligonucleotide primer to said further hybridization sequence of the cDNA;
(iii) synthesizing by means of catalysis by a DNA-dependent DNA polymerase and dNTPs an at least partially double-stranded DNA which contains a functional transcription promoter; and
(iv) synthesizing by means of catalysis by the corresponding DNA-dependent RNA polymerase and rNTPs from said transcription promoter a RNA which contains said pair of non-overlapping hybridization sequences;
said synthesized RNA, which is the nucleic acid derived from the cDNA, is amplified by means of a multienzymatic transcription-based procedure; and the reaction conditions are such that the stability of the RNA is maintained.

17. A method according to claim 15, wherein said template nucleic acid of step (b) consists of RNA and contains a further hybridization sequence located upstream of and non-overlapping with that hybridization sequence to which said RT primer is hybridized;
said RT primer is a single-stranded DNA oligonucleotide, whose 5' terminal sequence consists of a DNA-ori(-) sequence corresponding to a DNA replication system;
said cDNA synthesized in step (c) contains said DNA-ori(-) sequence which extends to the cDNA's 5'-end, said hybridization sequence of the RT primer and further downstream said further hybridization sequence;
in step (d) a nucleic acid is derived from said cDNA by
(i) hybridizing to said further hybridization sequence of the cDNA a single-stranded DNA primer whose 5' terminal sequence consists of said DNA-ori(-) sequence; and
(ii) synthesizing by means of catalysis by a DNA-dependent DNA polymerase and dNTPs an at least partially double-stranded DNA which has a DNA-ori at least at one end and is a primary replicative DNA;
and said primary replicative DNA, which is the nucleic acid derived from said cDNA, is amplified in a reaction mixture which contains dNTPs by means of a DNA replication system which involves binding of a protein primer to the DNA-ori of said primary replicative DNA and replicating said replicative DNA by means of a DNA replicase that binds to said protein-primed replicative DNA.

18. A method according to claim 15 wherein
said template nucleic acid of step (b) consists of RNA and contains a further hybridization sequence located upstream of and separated by a spacer sequence from that hybridization sequence to which said RT primer is hybridized;
said RT primer is a partially double-stranded DNA adaptor of which the strand hybridized to said template nucleic acid comprises at its 5' terminal sequence the DNA-ori(-) sequence of a replicative DNA, to which a second DNA oligonucleotide is hybridized whose 3' terminal sequence consists of a DNA-ori(+) sequence;
to said further hybridization sequence of the template nucleic acid a second partially double-stranded DNA adaptor is hybridized by a hybridization sequence which extends to the 5'-end of the adaptor's longer DNA strand; said second adaptor's longer strand is phosphorylated at its 5'-end and comprises a DNA-ori(+) sequence which extends to its 3'-end and to which a DNA oligonucleotide whose 5' terminal sequence consists of a complete DNA-ori(-) sequence is hybridized;
said cDNA synthesized in step (c) fills the gap between these two adaptors such that said cDNA is separated from said second adaptor by just one nick;
in step (d) a nucleic acid is derived from said cDNA by closing said nick by means of enzymatic ligation of the 3'-end of the cDNA with the 5'-end of said second adaptor, whence a primary replicative DNA is formed;
and said primary replicative DNA, which is the nucleic acid derived from the cDNA, is amplified in a reaction mixture which contains dNTPs by means of a DNA replication system which involves binding of a protein primer to the DNA-on of said primary replicative DNA and replicating said replicative DNA by means of a DNA replicase that binds to said protiein-primed replicative DNA.

19. A method according to claim 1, wherein
said template nucleic acid of step (b) is RNA and contains at least one further hybridization sequence located upstream of that to which said RT primer is hybridized; said cDNA synthesized in step (c) contains said at least one further hybridization sequence; a nucleic acid amplified in step (d) is derived from said cDNA by hybridizing a reporter probe to at least one of said at least one further hybridization sequence of said cDNA and by subjecting the reaction mixture to at least one further reaction selected from
(i) synthesizing a RNA by enzymatic catalysis,
(ii) eliminating the template RNA at least partially,
(iii) synthesizing an at least partially double-stranded DNA by enzymatic catalysis, and
(iv) cleaving a double-stranded DNA by enzymatic catalysis.

20. A method according to claim 19, wherein
said template nucleic acid contains one further hybridization sequence that extends to its 5'-end; said further hybridization sequence consists in its 3' terminal sequence of a P(-) sequence of a transcription promoter which is upstream directly followed by at least the first three nucleotides of a 3'-RNA-ori(+) sequence; said cDNA synthesized in step (c) contains said further hybridization sequence which extends to the 3'-end;
a nucleic acid amplified in step (d) is derived from said cDNA by
(i) hybridizing a reporter probe to said further hybridization sequence of the cDNA, said reporter probe being a single-stranded DNA which contains, extending to its 5'-end, the 5'-RNA-ori(-) sequence of a replicative RNA, further downstream a replicase binding domain, still further downstream a 3'-RNA-ori(+) sequence which is directly followed by the P(-) sequence of said transcription promoter; the hybridization sequence of said reporter probe consisting of said at least three last nucleotides of said 3'-RNA-ori(+) sequence and the entire P(-) sequence of said transcription promoter; the hybridization of said reporter probe to said further hybridization sequence of said cDNA constituting a functional transcription promoter, and
(ii) synthesizing from said transcription promoter a primary replicative RNA by means of catalysis by the corresponding DNA-dependent RNA polymerase and rNTPs;
and said primary replicative RNA, which is the nucleic acid derived from the cDNA, is amplified by means of an RNA replication system which comprises rNTPs in a reaction mixture and an RNA replicase that binds to the replicase binding domain of said primary replicative RNA and replicates said primary replicative RNA.

21. A method according to claim 19, wherein a nucleic acid amplified in step (d) is derived from said cDNA by
(i) eliminating the template RNA at least partially with at least one method selected from the group comprising enzymatic degradation, chemical degradation, physical degradation, and dissociating the template RNA from a cDNA by denaturation and washing it away;
(ii) hybridizing a smart probe to said at least one further hybridization sequence of said cDNA, said smart probe being a single-stranded DNA which contains, in 5' to 3' order, the P(+) sequence of a transcription promoter, a spacer sequence, a hybridization sequence complementary to said at least one further hybridization sequence of said cDNA, a further spacer sequence not complementary to the first, and the P(-) sequence of said transcription promoter directly followed by at least the first three nucleotides of a 5'-RNA-ori(-) sequence, whereby said P(-) sequence and said 5'-RNA-ori(-) nucleotides form a further hybridization sequence which extends to the 3'-end of said smart probe and is designated for the hybridization of a DNA reporter probe;
(iii) hybridizing to said further hybridization sequence of said smart probe a single-stranded DNA reporter probe which contains, in 5' to 3' order, a 5'-RNA-ori(-) sequence, a replicase binding domain, and a 3'-RNA-ori(+) sequence directly followed by the P(+) sequence of said transcription promoter, whence a functional transcription promoter is generated; and
(iv) synthesizing from said transcription promoter a primary replicative RNA by means of catalysis by the corresponding DNA-dependent RNA polymerase and rNTPs;
and said primary replicative RNA, which is the nucleic acid derived from the cDNA, is amplified by means of an RNA replication system which comprises rNTPs in a reaction mixture and an RNA replicase that binds to the replicase binding domain of said primary replicative RNA and replicates said primary replicative RNA.

22. A method according to claim 19, wherein
said template nucleic acid of step (b) contains one further hybridization sequence which extends to its 5'-end;
said cDNA synthesized in step (c) contains the corresponding further hybridization sequence extending to its 3'-end;
a nucleic acid amplified in step (d) is derived from said cDNA by
(i) hybridizing a single-stranded DNA reporter probe to said further hybridization sequence of the cDNA, said reporter probe containing upstream of a hybridization sequence by which it is hybridized to said further hybridization sequence of the cDNA multiple repetitive copies of a sequence which contains a recognition site for a restriction endonuclease;
(ii) synthesizing a double-stranded DNA by means of catalysis by a DNA-dependent DNA polymerase and dNTPs;
(iii) cleaving said double-stranded DNA by means of said restriction endonuclease, thereby generating several molecules of said double-stranded fragment;
said fragment, which is the nucleic acid derived from said cDNA, is amplified in repeated cycles of denaturation, hybridization to said reporter probe, DNA synthesis, and restriction endonuclease cleavage.

23. A method according to claim 1, wherein at least one nucleic acid of the group consisting of the RT primer, a nucleic acid other than the RT primer which is hybridized to the template nucleic acid, a cDNA, a nucleic acid that is hybridized to the cDNA, a nucleic acid that is hybridized to a reporter probe, and a nucleic acid amplified in a amplification reaction carries a functional group which has at least one property selected from the group comprising being a solid phase, mediating the binding to a solid phase, being a ligand, and possessing at least one antigenic determinant.

24. A method according to claim 1 wherein, after having synthesized said cDNA in step (c), the template RNA is eliminated at least partially by treatment with at least one method selected from the group consisting of enzymatic degradation, chemical degradation, physical degradation, and dissociating the template RNA from a solid-phase-bound cDNA and washing it away.

25. A screening kit for the detection of a reverse transcriptase in accordance with the method of claim 1 comprising at least:
(i) reagents and auxiliary devices for the pretreatment of a sample including a isotonic sample dilution buffer, 0.2µm filters, a reverse transcriptase extraction buffer containing salts, protein stabilizers and a mild detergent;
(ii) a RT master mixture comprising at least a buffer system, salts, a heteropolymeric template RNA, a heteropolymeric RT primer, dATP, dCTP, dGTP, and dTTP, a divalent cation selected from the group comprising Mg²⁺ and Mn²⁺, at least one protein stabilizer, a RNase inhibitor,
(iii) a RNase for the digestion of the template RNA;
(iv) at least one further oligonucleotide matched to the reverse-transcribed template RNA and reagents for a in vitro nucleic acid amplification reaction comprising at least the enzymes, nucleotide triphosphates, salts, divalent cations, and buffers;
(v) at least one positive RT control consisting of a solution which contains an enzymatically active reverse transcriptase; and at least one negative RT control consisting of a solution which does not contain any enzymatically active reverse transcriptase.

26. A screening kit for the detection of a reverse transcriptase in accordance with the kit of claim 25, wherein said reagents for a in vitro nucleic acid amplification reaction are those used in a polymerase chain reaction and comprise at least a Taq polymerase, dATP, dCTP, dGTP, dTTP, salts, a divalent cation, and buffers.

## Revendications

1. Procédé en vue de prouver l'activité enzymatique du type de la transcriptase inverse (TI) dans un matériel de recherche, caractérisé par le fait
(a) que le matériel de recherche est élaboré en vue de rendre la transcriptase inverse qu'il contient accessible pour la réaction TI et d'éliminer de ce matériel les facteurs qui interfèrent avec la réaction TI;
(b) que le matériel de recherche élaboré est incubé avec une combinaison matrice-amorce dans un mélange de réaction, cette combinaison matrice-amorce se composant d'une matrice acide nucléique et au moins d'une amorce TI,
l'amorce TI étant un acide nucléique, lequel contient au moins une séquence fonctionnelle, dont au moins une est une séquence d'hybridation, laquelle s'étend jusqu'à l'extrémité 3' et par laquelle l'amorce TI est hybridée à la matrice acide nucléique;
la matrice acide nucléique étant un acide nucléique hétéropolymère, lequel contient au moins un segment, lequel se compose d'un ARN hétéropolymère et est localisé en amont d'une séquence d'hybridation, à laquelle l'amorce TI est hybridée, et lequel segment peut fonctionner dans une réaction TI comme matrice, et que cette matrice acide nucléique contient en outre au moins une séquence fonctionnelle supplémentaire en amont de la séquence d'hybridation pour l'amorce TI;
(c) que par la transcription inverse, laquelle est catalysée par la transcriptase inverse provenant du matériel de recherche, un ADNc est synthétisé;
(d) que par un processus d'amplification de l'acide nucléique in vitro, au moins une partie de la séquence d'un ADNc produit à l'étape ( c) ou d'un acide nucléique dérivé de ce ADNc est amplifiée;
(e) que le produit d'amplification de l'étape (d) est analysé et identifié; et
(f) que la présence du produit d'amplification est mise en corrélation avec la présence d'une activité de transcriptase inverse dans le matériel de recherche élaboré.

2. Procédé selon l'exigence 1, caractérisé par le fait que la combinaison matrice-amorce de l'étape (b) comprend toutes les séquences fonctionnelles qui sont nécessaires en vue d'amplifier au moins une partie de la séquence d'un ADNc synthétisé à l'étape (c) et
que, à l'étape (d), cette partie de la séquence ADNc, qui contient ces séquences fonctionnelles, est amplifiée.

3. Procédé selon l'exigence 2, caractérisé par le fait que la combinaison matrice-amorce de l'étape (b) contient une paire de séquences d'hybridation non chevauchantes, lesquelles sont utilisées dans la réaction d'amplification de l'étape (d), au moins l'une d'entre elles se situant en amont de cette séquence d'hybridation de la matrice acide nucléique, à laquelle l'amorce TI est hybridée;
qu'un ADNc synthétisé à l'étape (c) contient cette paire de séquences d'hybridation;
et que à l'étape (d), au moyen d'une réaction en chaîne polymérase et avec utilisation de deux amorces d'amplification, lesquelles sont hybridées à la paire de séquences d'hybridation, une partie de la séquence de ce ADNc est sélectivement amplifiée.

4. Procédé selon l'exigence 3, caractérisé par le fait que la matrice acide nucléique est le ARN génomique du bactériophage MS2, que l'amorce TI est un oligonucléotide d'ADN synthétique avec la séquence basique 5'-d(CATAGGTCAAACCTCCTAGGAATG)-3'; et qu'une partie du MS2 ADNc synthétisé à l'étape (c) est amplifiée sélectivement à l'étape (d), au moyen d'une réaction en chaîne polymérase avec des amorces oligonucléotides d'ADN synthétiques de la séquence basique 5'-d(CATAGGTCAAACCTCCTAGGAATG)-3' et 5'-d(TCCTGCTCAACTTCCTGTCGAG)-3'.

5. Procédé selon l'exigence 2, caractérisé par le fait que la combinaison matrice-amorce de l'étape (b) contient une paire de séquences d'hybridation directement contiguës, qui sont utilisées dans la réaction d'amplification de l'étape (d), au moins une de ces séquences d'hybridation étant située entièrement en amont de la séquence d'hybridation de la matrice acide nucléique, à laquelle l'amorce TI est hybridée;
qu'un ADNc synthétisé à l'étape (c) contient cette paire de séquences d'hybridation; et
que, à l'étape (d), une partie de la séquence du ADNc est sélectivement amplifiée au moyen d'une réaction en chaîne ligase, avec utilisation de cette paire de séquences d'hybridation.

6. Procédé selon l'exigence 2, caractérisé par le fait que la combinaison matrice-amorce de l'étape (b) contient une paire de séquences d'hybridation, lesquelles sont utilisées dans la réaction d'amplification de l'étape (d), les séquences d'hybridation de cette paire étant séparées par une séquence d'espacement et au moins l'une des deux étant localisée entièrement en amont de la séquence d'hybridation de la matrice acide nucléique, à laquelle l'amorce TI est hybridée;
qu'un ADNc synthétisé à l'étape (c) contient cette paire de séquences d'hybridation; et
que, à l'étape (d), une partie de la séquence de ce ADNc est sélectivement amplifiée, au moyen d'une combinaison d'une réaction en chaîne polymérase et d'une réaction en chaîne ligase, avec utilisation de la paire de séquences d'hybridation.

7. Procédé selon l'exigence 2, caractérisé par le fait que la combinaison matrice-amorce de l'étape (b) contient une paire de séquences d'hybridation non chevauchantes, qui sont utilisées dans la réaction d'amplification de l'étape (d), au moins l'une d'entre elles étant localisée entièrement en amont de la séquence d'hybridation de la matrice acide nucléique, à laquelle l'amorce TI est hybridée;
que le ADNc synthétisé à l'étape (c) contient cette paire de séquences d'hybridation;
et que, à l'étape (d), une partie du ADNc synthétisé à l'étape (c), laquelle contient une paire de séquences d'hybridation, est sélectivement amplifiée au moyen de
(i) un procédé multienzymatique basé sur la transcription étant alors utilisé, lequel utilise la paire de séquences d'hybridation, ainsi que des conditions de réaction, qui maintiennent la stabilité du ARN; et
(ii) la matrice ARN étant exclue d'une amplification par un traitement préalable avec au moins une méthode choisie dans le groupe comprenant la dégradation enzymatique, la dégradation chimique, la dégradation physique, ainsi que la dissociation et le lavage de la matrice ARN d'un ADNc lié à une phase solide.

8. Procédé selon l'exigence 2, caractérisé par le fait que la combinaison matrice-amorce de l'étape (b) contient une paire de séquences d'hybridation non chevauchantes, ainsi qu'une séquence de promoteur de la transcription, lesquelles sont utilisées dans la réaction d'amplification de l'étape (d), au moins une séquence d'hybridation de cette paire étant localisée entièrement en amont de la séquence d'hybridation de la matrice acide nucléique, à laquelle l'amorce TI est hybridée;
que le ADNc synthétisé à l'étape (c) contient la séquence P(-) du promoteur de transcription en amont de la paire de séquences d'hybridation;
que, à l'étape (d), la partie du ADNc synthétisé à l'étape (c), laquelle contient la paire de séquences d'hybridation et la séquence P(-), est sélectivement amplifiée au moyen de
(i) un procédé multienzymatique, basé sur la transcription, étant utilisé, lequel utilise la paire de séquences d'hybridation et la séquence P(-), ainsi que des conditions de réaction, qui maintiennent la stabilité de l'ARN; et
(ii) la matrice ARN étant exclue d'une amplification par un traitement préalable avec au moins une méthode choisie parmi le groupe comprenant la dégradation enzymatique, la dégradation chimique, la dégradation physique, ainsi que la dissociation et le lavage de la matrice ARN d'un ADNc lié à une phase solide.

9. Procédé selon l'exigence 2, caractérisé par le fait que la combinaison matrice-amorce de l'étape (b) comprend toutes les séquences fonctionnelles d'un ADN réplicatif;
cette matrice acide nucléique contenant dans l'ordre 5' à 3' une séquence ADN-ori(-), qui s'étend jusqu'à l'extrémité 5' et se compose d'un acide nucléique, lequel est choisi dans le groupe contenant ARN et ADN, une séquence d'espacement et une séquence d'hybridation d'amorce, laquelle comprend au moins une partie d'une séquence ADN-ori(+) et s'étend jusqu'à l'extrémité 3' de la séquence d'hybridation d'amorce;
que l'amorce TI est un oligonucléotide d'ADN monocaténaire, lequel est hybridé sur toute sa longueur au moins à la partie à l'extrémité 3' de la séquence ADN-ori(+) de la matrice acide nucléique;
que le ADNc synthétisé à l'étape (c) est un ADN réplicatif primaire, lequel confient les éléments suivants: une séquence ADN-ori(-) s'étendant jusqu'à la 5' extrémité, une séquence d'espacement, et une séquence ADN-ori(+) atteignant l'extrémité 3',
que l'hybridation de cette extrémité 3' de la séquence ADN-ori(+) à une séquence ADN-ori(-) localisée à l'extrémité 5' forme un ADN-ori actif;
que, à l'étape (d), le ADNc est amplifié au moyen de
(i) la matrice ARN étant au moins partiellement éliminée par un traitement préalable avec au moins une méthode choisie parmi le groupe comprenant la dégradation enzymatique, la dégradation chimique, la dégradation physique, ainsi que la dissociation et le lavage de la matrice ARN d'un ADNc lié à une phase solide; et
(ii) dans un mélange de réaction contenant des dNTPs un procédé de réplication d'ADN étant entamé, dans lequel une amorce protéique est liée à l'ADN-ori actif et l'ADN réplicatif étant amplifié à l'aide d'une ADN réplicase, laquelle se lie à l'ADN réplicatif à amorce protéique.

10. Procédé selon l'exigence 1, caractérisé par le fait que la matrice acide nucléique de l'étape (b) est un ARN et contient au moins une autre séquence d'hybridation, laquelle se situe en amont de la séquence d'hybridation à laquelle l'amorce TI est hybridée;
que l'amorce TI contient un groupe fonctionnel;
qu'un ADNc synthétisé à l'étape (c) contient ce groupe fonctionnel et au moins une autre séquence d'hybridation;
qu'un acide nucléique amplifié à l'étape (d) est dérivé du ADNc, la matrice ARN étant au moins partiellement éliminée au moyen d'au moins un procédé choisi parmi le groupe comprenant la dégradation enzymatique, la dégradation chimique, la dégradation physique, ainsi que la dissociation et le lavage de la matrice ARN d'un ADNc, lequel a été lié à une phase solide au moyen du groupe fonctionnel;
qu'une sonde Reporter, qui contient toutes les séquences fonctionnelles nécessaires pour une réaction d'amplification, est hybridée à au moins une autre séquence d'hybridation d'un ADNc;
que la sonde Reporter hybridée est liée à une phase solide au moyen du groupe fonctionnel du ADNc;
que la sonde Reporter non liée est éloignée;
et que au moins une partie de la sonde Reporter hybridée, qui est l'acide nucléique dérivé du ADNc, est amplifiée.

11. Procédé selon l'exigence 10, caractérisé par le fait que la sonde Reporter est un ADN, qui contient une séquence d'hybridation, par laquelle elle est hybridée au ADNc, ainsi qu'une paire de séquences d'hybridation, lesquelles sont utilisées dans la réaction d'amplification de l'étape (d); et que la partie de la sonde Reporter qui contient la paire de séquences d'hybridation, est amplifiée à l'étape (d) au moyen d'un procédé qui est choisi parmi le groupe comprenant une réaction en chaîne polymérase (PCR), une réaction en chaîne ligase (LCR), une combinaison d'une PCR et d'une LCR, et un procédé multienzymatique basé sur la transcription.

12. Procédé selon l'exigence 10, caractérisé par le fait que la sonde Reporter est un ARN qui contient une séquence d'hybridation, par laquelle elle est hybridée à un ADNc, ainsi qu'une paire de séquences d'hybridation qui sont utilisées dans la réaction d'amplification de l'étape (d), et que cette partie de cette sonde Reporter, qui contient la paire de séquences d'hybridation, est amplifiée à l'étape (d) au moyen d'un procédé multienzymatique basé sur la transcription.

13. Procédé selon l'exigence 10, caractérisé par le fait que la sonde Reporter est un ARN réplicatif primaire, qui contient une séquence d'hybridation par laquelle elle est hybridée à un ADNc, une séquence ARN-ori(-) atteignant l'extrémité 5', une séquence ARN-ori(+) atteignant l'extrémité 3' et une domaine de liaison pour une réplicase entre les deux; et que cette sonde Reporter est amplifiée à l'étape (d) au moyen d'un système de réplication ARN, lequel contient des rNTPs dans un mélange de réaction et une réplicase d'ARN, laquelle se lie au domaine de liaison pour une réplicase de la sonde Reporter et réplique cette sonde Reporter.

14. Procédé selon l'exigence 10, caractérisé par le fait que la sonde Reporter est au moins un acide nucléique, choisi parmi le groupe comprenant
(i) un ADN réplicatif primaire, qui contient une séquence ADN-ori(-) atteignant la 5' extrémité, une séquence d'hybridation avec laquelle il est hybridé à un ADNc et une séquence ADN-ori(+) atteignant la 3' extrémité,
(ii) un ADN réplicatif primaire partiellement bicaténaire, lequel contient une séquence ADN-ori(-) atteignant la 5' extrémité, une séquence d'hybridation, à laquelle il est hybridé à un ADNc, et une séquence ADN-ori(+) atteignant la 3' extrémité, la séquence extrémité 3' de cette sonde Reporter étant amorcée avec un oligonucléotide d'ADN monocaténaire, lequel contient au moins les six premiers nucléotides d'une séquence ADN-ori(-), et
(iii) un ADN réplicatif primaire au moins partiellement bicaténaire, dont l'un des brins contient une séquence ADN-ori(-), laquelle atteint la 5' extrémité, une séquence d'hybridation située en aval, qui est complémentaire à au moins cette autre séquence d'hybridation du ADNc, et une deuxième séquence d'hybridation atteignant la 3' extrémité, à laquelle un deuxième brin d'ADN est hybridé, lequel contient une séquence d'hybridation complémentaire atteignant l'extrémité 3' et une séquence ADN-ori(-) atteignant la 5' extrémité; et
que cet ADN réplicatif primaire est amplifié dans un mélange de réaction contenant des dNTPs, à l'aide d'un procédé de réplication d'ADN, qui comprend la liaison d'une amorce protéique au ADN-ori de l'ADN réplicatif primaire, ainsi que la réplication de l'ADN réplicatif à l'aide d'une réplicase d'ADN, laquelle se lie à l'ADN réplicatif à amorce protéique.

15. Procédé selon l'exigence 1, caractérisé par le fait qu'un acide nucléique amplifié à l'étape (d) a été dérivé d'un ADNc formé à l'étape (c), au moyen d'au moins une réaction choisie parmi le groupe comprenant
(i) l'hybridation d'un ADN monocaténaire, qui contient au moins une séquence d'hybridation atteignant l'extrémité 3', au ADNc,
(ii) la synthèse d'un ADN au moins partiellement bicaténaire au moyen d'une catalyse enzymatique,
(iii) la synthèse d'un ARN au moyen d'une catalyse enzymatique
(iv) l'élimination au moins partielle de la matrice acide nucléique, et
(v) la ligature de l'extrémité 3' du ADNc avec l'extrémité 5' d'un autre acide nucléique hybridé à la matrice acide nucléique.

16. Procédé selon l'exigence 15, caractérisé par le fait que la matrice acide nucléique de l'étape (b) est un ARN et contient une paire de séquences d'hybridation non chevauchantes, laquelle est utilisée dans la réaction d'amplification de l'étape (d), au moins l'une des deux se situant en amont de la séquence d'hybridation à laquelle l'amorce TI est hybridée;
que cette matrice acide nucléique contient en outre une séquence P(-) d'un promoteur de transcription, laquelle est située en amont de la paire de séquences d'hybridation, ainsi qu'une autre séquence d'hybridation, dont la limitation 5' est localisée en amont ou à la limitation 5' de la séquence P(-);
que le ADNc produit à l'étape (c) contient la séquence d'hybridation pour l'amorce TI, la paire de séquences d'hybridation et, en aval, une séquence P(+) complète et une autre séquence d'hybridation; et
que, à l'étape (d) un acide nucléique est dérivé de ce ADNc au moyen de
(i) la matrice ARN étant au moins partiellement éliminée avec au moins une méthode choisie parmi le groupe comprenant la dégradation enzymatique, la dégradation chimique, la dégradation physique, ainsi que la dissociation et le lavage de la matrice ARN d'un ADNc lié à une phase solide;
(ii) une amorce oligonucléotide d'ADN étant hybridée à cette autre séquence d'hybridation du ADNc;
(iii) par catalyse, au moyen d'une polymérase d'ADN dépendante d'ADN et de dNTPs, un ADN au moins partiellement bicaténaires étant synthétisé, lequel contient un promoteur de transcription fonctionnel; et
(iv) partant de ce promoteur de transcription par catalyse au moyen d'une polymérase d'ARN dépendante d'ARN et au moyen de rNTPs, un ARN étant synthétisé, lequel contient la paire de séquences d'hybridation non chevauchantes; et que
cet ARN, qui est l'acide nucléique dérivé du ADNc, est amplifié au moyen d'un procédé multienzymatique basé sur la transcription, les conditions de réaction étant alors telles que la stabilité de l'ARN est maintenue.

17. Procédé selon l'exigence 15, caractérisé par le fait que la matrice acide nucléique de l'étape (b) se compose d'ARN et contient une autre séquence d'hybridation, laquelle se trouve en amont de la séquence d'hybridation à laquelle l'amorce TI est hybridée, et ne se chevauche pas avec celle-ci;
que l'amorce TI est un oligonucléotide d'ADN monocaténaire, dont la séquence à l'extrémité 5' se compose d'une séquence ADN-ori(-), qui correspond à un système de réplication ADN;
que le ADNc synthétisé à l'étape (c) contient la séquence ADN-ori(-) qui atteind l'extrémité 5' du ADNc, la séquence d'hybridation pour l'amorce TI et, plus en aval, cette autre séquence d'hybridation;
que à l'étape (d) un acide nucléique est dérivé de ce ADNc au moyen de
(i) une amorce ADN monocaténaire étant hybridée à cette autre séquence d'hybridation du ADNc, et dont la séquence localisée à l'extrémité 5' se compose de la séquence ADN-ori(-); et
(ii) une catalyse par une polymérase d'ADN dépendante d'ADN, ainsi que des dNTPs, un ADN au moins partiellement bicaténaire étant synthétisé, lequel possède au moins à une extrémité une ADN-ori et lequel est un ADN réplicatif primaire; et que
cet ADN réplicatif primaire, lequel est l'acide nucléique dérivé du ADNc, est amplifié dans un mélange de réaction contenant des dNTPs, au moyen d'un système de réplication ADN, une amorce protéique étant liée à la ADN-ori de cet ADN réplicatif primaire et l'ADN réplicatif étant amplifié au moyen d'une réplicase d'ADN, laquelle se lie à l'ADN réplicatif à amorce protéique.

18. Procédé selon l'exigence 15, caractérisé par le fait que la matrice acide nucléique de l'étape (b) se compose d'ARN et contient une autre séquence d'hybridation, qui se situe en amont de la séquence d'hybridation à laquelle l'amorce TI est hybridée et est séparée de celle-ci par une séquence d'espacement;
que l'amorce TI est un adaptateur ADN partiellement bicaténaire, dont un brin, hybridé à la matrice acide nucléique contient, dans la séquence localisée à son extrémité 5', la séquence ADN-ori(-) d'un ADN réplicatif, à laquelle un deuxième oligonucléotide d'ADN est hybridé, dont la séquence à l'extrémité 3' se compose d'une séquence ADN-ori(+);
que à l'autre séquence d'hybridation de la matrice acide nucléique, un deuxième adaptateur ADN partiellement bicaténaire est hybridé au moyen d'une séquence d'hybridation, laquelle atteint l'extrémité 5' du brin d'ADN plus long de l'adaptateur;
que le brin plus long de ce deuxième adaptateur est phosphorylé à l'extrémité 5' et contient une séquence ADN-ori(+), laquelle s'étend jusqu'à son extrémité 3' et à laquelle un oligonucléotide d'ADN est hybridé, dont la séquence à l'extrémité 5' se compose d'une séquence ADN-ori(-) complète;
que le ADNc synthétisé à l'étape (c) comble les lacunes entre ces deux adaptateurs, de telle sorte que le ADNc est maintenant séparé du deuxième adaptateur justement par une coupure;
que à l'étape (d) un acide nucléique est dérivé de ce ADNc, la coupure étant bouclée au moyen d'une ligature enzymatique de l'extrémité 3' du ADNc avec l'extrémité 5' du deuxième adaptateur, un ADN réplicatif primaire étant de ce fait constitué; et
que cet ADN réplicatif primaire, qui est l'acide nucléique dérivé du ADNc, est amplifié au moyen d'un système de réplication d'ADN dans un mélange de réaction contenant des dNTPs, une amorce protéique étant liée à la ADN-ori de l'ADN réplicatif primaire susdit, et que cet ADN réplicatif est amplifié au moyen d'une réplicase d'ADN, laquelle se lie à l'ADN réplicatif à amorce protéique.

19. Procédé selon l'exigence 1, caractérisé par le fait que la matrice acide nucléique de l'étape (b) est un ARN et contient au moins une autre séquence d'hybridation, laquelle est localisée en amont de la séquence d'hybridation à laquelle l'amorce TI est hybridée;
que le ADNc synthétisé à l'étape (c) contient au moins une autre séquence d'hybridation;
qu'un acide nucléique amplifié à l'étape (d) est dérivé de ce ADNc par l'hybridation d'une sonde Reporter à au moins une de cette au moins une autre séquence d'hybridation du ADNc et que le mélange de réaction de cet ADNc est soumis au moins à une autre réaction, laquelle est choisie parmi le groupe comprenant:
(i) la synthèse d'un ARN par catalyse enzymatique,
(ii) l'élimination au moins partielle de la matrice ARN,
(iii) la synthèse d'un ADN au moins partiellement bicaténaire par catalyse enzymatique, ainsi que
(iv) la coupure de l'ADN bicaténaire par catalyse enzymatique.

20. Procédé selon l'exigence 19, caractérisé par le fait que la matrice acide nucléique contient une autre séquence d'hybridation s'étendant jusqu'à son extrémité 5'; cette autre séquence d'hybridation possédant à son extrémité 3' une séquence P(-) d'un promoteur de transcription, laquelle est directement suivie, en amont, par au moins les trois premiers nucléotides d'une séquence 3'-ARN-ori(+);
que le ADNc synthétisé à l'étape (c) contient cette autre séquence d'hybridation, qui s'étend jusqu'à son extrémité 3';
qu'un acide nucléique amplifié à l'étape (d) est dérivé du ADNc au moyen de
(i) une sonde Reporter étant hybridée à cette autre séquence d'hybridation du ADNc, cette sonde Reporter étant un ADN monocaténaire, lequel contient une séquence 5'-ARN ori(-) d'un ARN réplicatif s'étendant jusqu'à son extrémité 5', plus loin en aval une domaine de liaison pour une réplicase et encore plus loin en aval une séquence 3'-ARN-ori(+), laquelle est directement suivie par la séquence P(-) du promoteur de transcription; la séquence d'hybridation de cette sonde Reporter se composant au moins des trois derniers nucléotides de la séquence 3'-ARN-ori(+) susdite et de la séquence P(-) complète du promoteur de transcription; l'hybridation de cette sonde Reporter à l'autre séquence d'hybridation du ADNc conduisant à la formation d'un promoteur de transcription fonctionnel, et
(ii) partant de ce promoteur de transcription au moyen d'une catalyse par la polymérase d'ARN dépendante d'ADN correspondante et de rNTPs, un ARN réplicatif primaire étant synthétisé; et
que cet ARN réplicatif primaire, lequel est l'acide nucléique dérivé du ADNc, est amplifié au moyen d'un système de réplication ARN, lequel contient des rNTPs dans un mélange de réaction, ainsi qu'une réplicase d'ARN, laquelle se lie à la domaine de liaison pour une réplicase de Cet ARN réplicatif primaire et réplique cet ARN réplicatif primaire.

21. Procédé selon l'exigence 19, caractérisé par le fait qu'un acide nucléique amplifié à l'étape (d) est dérivé du ADNc au moyen de
(i) la matrice ARN étant au moins partiellement éliminée au moyen d'au moins une méthode choisie parmi le groupe comprenant la dégradation enzymatique, la dégradation chimique, la dégradation physique, ainsi que la dissociation de la matrice ARN d'un ADNc par dénaturation et lavage;
(ii) une sonde Smart étant hybridé à cette au moins une autre séquence d'hybridation du ADNc, cette sonde Smart étant un ADN monocaténaire, lequel contient, dans l'ordre 5' à 3', la séquence P(+)d'un promoteur de transcription, une séquence d'espacement, une séquence d'hybridation complémentaire de cette au moins une séquence d'hybridation additionelle du ADNc, une autre séquence d'espacement n'étant pas complémentaire à la première, ainsi que la séquence P(-) du promoteur de transcription, laquelle étant directement suivie par au moins les trois premiers nucléotides d'une séquence 5'-ARN-ori(-), la séquence P(-) et les nucléotides 5'-ARN-ori(-) formant une autre séquence d'hybridation, laquelle s'étend jusqu'à l'extrémité 3' de la sonde Smart et est destinée à l'hybridation d'une sonde Reporter ADN;
(iii) à cette autre séquence d'hybridation de la sonde Smart, une sonde Reporter monocaténaire étant hybridée, laquelle contient, dans l'ordre 5' à 3', une séquence 5'-ARN-ori(-), une domaine de liaison pour une réplicase et une séquence 3'-ARN-ori(+), qui est directement suivie par la séquence P(+) du promoteur de transcription, un promoteur de transcription fonctionnel étant ainsi généré; et
(iv) partant de ce promoteur de transcription au moyen d'une catalyse par la polymérase d'ARN dépendante d'ADN correspondante, ainsi que de rNTPs, une ARN réplicatif primaire étant synthétisé; et
que cet ARN réplicatif primaire, qui est l'acide nucléique dérivé du ADNc, est amplifié au moyen d'un système de réplication d'ARN, lequel contient des rNTPs dans un mélange de réaction, ainsi qu'une réplicase d'ARN, laquelle se lie à la domaine de liaison pour une réplicase de cet ARN réplicatif primaire et réplique cet ARN réplicatif primaire.

22. Procédé selon l'exigence 19, caractérisé par le fait que la matrice acide nucléique de l'étape (b) contient une autre séquence d'hybridation qui s'étend jusqu'à son extrémité 5'; que le ADNc synthétisé à l'étape (c) contient cette autre séquence d'hybridation qui s'étend jusqu'à l'extrémité 3'; qu'un acide nucléique amplifié à l'étape (d) est dérivé de ce ADNc au moyen de
(i) une sonde Reporter ADN monocaténaire étant hybridée à l'autre séquence d'hybridation du ADNc, cette sonde Reporter contenant, en amont d'une séquence d'hybridation, au moyen de laquelle elle est hybridée à l'autre séquence d'hybridation du ADNc, des copies répétitives multiples d'une séquence, laquelle contient un site de reconnaissance pour une endonucléase de restriction;
(ii) au moyen d'une catalyse par une polymérase d'ADN dépendante d'ADN et de dNTPs, un ADN bicaténaire étant synthétisé;
(iii) cet ADN bicaténaire étant coupé par l'endonucléase de restriction susdite, plusieurs molécules de ce fragment bicaténaire étant produites; ce fragment étant l'acide nucléique dérivé du ADNc, qui est amplifié en cycles répétés de dénaturation, d'hybridation à la sonde Reporter, de synthèse ADN et de coupure d'endonucléase de restriction.

23. Procédé selon l'exigence 1, caractérisé par le fait qu'au moins un acide nucléique du groupe comprenant l'amorce TI, un acide nucléique différent de l'amorce TI hybridé à la matrice acide nucléique, un ADNc, un acide nucléique hybridé au ADNc, un acide nucléique hybridé à une sonde Reporter et un acide nucléique amplifié dans une réaction d'amplification, porte un groupe fonctionnel, lequel atteste au moins d'une propriété qui est choisie parmi le groupe de propriétés suivant: étant une phase solide, produisant la liaison à une phase solide, étant un ligaturant et possédant au moins un déterminant antigènique.

24. Procédé selon l'exigence 1, caractérisé par le fait qu'après la synthèse du ADNc de l'étape (c), la matrice ARN est au moins partiellement éliminée par traitement avec au moins une méthode qui est choisie parmi le groupe comprenant la dégradation enzymatique, la dégradation chimique, la dégradation physique, ainsi que la dissociation et le lavage de la matrice ARN d'un ADNc lié à une phase solide.

25. Un Screening Kit pour la preuve d'une transcriptase inverse selon l'exigence 1, contenant au moins:
(i) des réactifs et accessoires pour la préparation du matériel de recherche comprenant un tampon de dilution isotonique pour le matériel de recherche, un filtre 0,2 µm, un tampon d'extraction pour la transcriptase inverse contenant des sels, des stabilisateurs protéiques et un détergent doux;
(ii) un mélange-mère TI comprenant au moins un système de tampon, des sels, une matrice ARN hétéropolymère, une amorce TI hétéropolymère, des dATP, des dCTP, des dGTP et des dTTP, un cation divalent choisi parmi le groupe contenant Mg²⁺ et Mn²⁺, au moins un stabilisateur protéique, un inhibiteur RNase,
(iii) une RNase pour la digestion de la matrice ARN;
(iv) au moins un autre oligonucléotide, lequel est adapté à la matrice ARN rétrotranscrite, ainsi que des réactifs pour une réaction d'amplification d'acides nucléiques in vitro contenant au moins les enzymes, les triphosphates de nucléotide, les sels, les cations divalents et les tampons;
(v) au moins un contrôle TI positif comprenant une solution contenant une transcriptase inverse enzymatiquement active; et au moins un contrôle TI négatif, qui se compose d'une solution contenant aucune transcriptase inverse enzymatiquement active.

26. Un Screening Kit pour la preuve d'une transcriptase inverse selon le kit de l'exigence 25, caractérisé par le fait que les réactifs pour une réaction d'amplification d'acides nucléiques in vitro sont ceux qui sont utilisés dans une réaction en chaîne polymérase et contiennent au moins une polymérase Taq, des dATP, des dCTP, des dGTP, des dTTP, des sels, un cation divalent et des tampons.
